# EUROPEAN PATENT SPECIFICATION

(11) **EP 2 527 456 B1**
(45) Date of publication and mention of the grant of the patent: **16.05.2018**
(21) Application number: 12161321.0
(22) Date of filing: 24.10.2005
(51) Int. Cl.: C12N 15/85, C07K 16/00, A01K 67/027, C12N 15/00, C12N 5/10, C12N 15/873

(54) **Transgenic porcines lacking endogenous immunoglobulin light chain**
Transgenschweine ohne endogene leichte Kette von Immunglobulin
Porcs transgéniques déficients en chaîne légère d'immunoglobuline endogène

(30) Priority: 22.10.2004 US 621433 P
(43) Date of publication of application: 28.11.2012
(62) Divisional of application: 05818708.9
(73) Proprietor: Revivicor Inc., Blacksburg, VA 24060 (US)
(72) Inventor: Wells, Kevin, Christiansburg, VA 24073 (US); Ayares, David, Blacksburgh, VA 24060 (US)
(74) Representative: Carpmaels & Ransford LLP

(56) References cited:
- WO-A-01/35735
- WO-A-02/070648
- WO-A-03/047336
- WO-A-2004/028243
- WO-A1-2010/051288
- MENDICINO M ET AL: "Generation of antibody- and B cell-deficient pigs by targeted disruption of the J-region gene segment of the heavy chain locus", TRANSGENIC RESEARCH, KLUWER ACADEMIC PUBLISHERS-PLENUM PUBLISHERS, NE, vol. 20, no. 3, 26 September 2010 (2010-09-26), pages 625-641, XP019900628, ISSN: 1573-9368, DOI: 10.1007/S11248-010-9444-Z
- RAMSOONDAR J ET AL: "Targeted disruption of the porcine immunoglobulin kappa light chain locus", TRANSGENIC RESEARCH, KLUWER ACADEMIC PUBLISHERS-PLENUM PUBLISHERS, NE, vol. 20, no. 3, 26 September 2010 (2010-09-26), pages 643-653, XP019900627, ISSN: 1573-9368, DOI: 10.1007/S11248-010-9445-Y

## Description

### FIELD OF THE INVENTION

The present invention provides porcine animals, tissue and organs as well as cells and cell lines derived from such animals, tissue and organs, which lack expression of functional endogenous immunoglobulin. The present invention also provides porcine animals, tissue and organs as well as cells and cell lines derived from such animals, tissue and organs, which express xenogenous, such as human, immunoglobulin. Also disclosed are ungulate, such as porcine genomic DNA sequences of porcine heavy and light chain immunoglobulins. Such animals, tissues, organs and cells can be used in research and medical therapy. In addition, methods are disclosed to prepare such animals, organs, tissues, and cells.

### BACKGROUND OF THE INVENTION

An antigen is an agent or substance that can be recognized by the body as 'foreign'. Often it is only one relatively small chemical group of a larger foreign substance which acts as the antigen, for example a component of the cell wall of a bacterium. Most antigens are proteins, though carbohydrates can act as weak antigens. Bacteria, viruses and other microorganisms commonly contain many antigens, as do pollens, dust mites, molds, foods, and other substances. The body reacts to antigens by making antibodies. Antibodies (also called immunoglobulins (Igs)) are proteins that are manufactured by cells of the immune system that bind to an antigen or foreign protein. Antibodies circulate in the serum of blood to detect foreign antigens and constitute the gamma globulin part of the blood proteins. These antibodies interact chemically with the antigen in a highly specific manner, like two pieces of a jigsaw puzzle, forming an antigen/antibody complex, or immune complex. This binding neutralises or brings about the destruction of the antigen.

When a vertebrate first encounters an antigen, it exhibits a primary humoral immune response. If the animal encounters the same antigen after a few days the immune response is more
rapid and has a greater magnitude. The initial encounter causes specific immune cell (B-cell) clones to proliferate and differentiate. The progeny lymphocytes include not only effector cells (antibody producing cells) but also clones of memory cells, which retain the capacity to produce both effector and memory cells upon subsequent stimulation by the original antigen. The effector cells live for only a few days. The memory cells live for a lifetime and can be reactivated by a second stimuation with the same antigen. Thus, when an antigen is encountered a second time, its memory cells quickly produce effector cells which rapidly produce massive quantities of antibodies.

By exploiting the unique ability of antibodies to interact with antigens in a highly specific manner, antibodies have been developed as molecules that can be manufactured and used for both diagnostic and therapeutic applications. Because of their unique ability to bind to antigenic epitopes, polyclonal and monoclonal antibodies can be used to identify molecules carrying that epitope or can be directed, by themselves or in conjunction with another moiety, to a specific site for diagnosis or therapy. Polyclonal and monoclonal antibodies can be generated against practically any pathogen or biological target. The term polyclonal antibody refers to immune sera that usually contain pathogen-specific antibodies of various isotypes and specificities. In contrast, monoclonal antibodies consist of a single immunoglobulin type, representing one isotype with one specificity.

In 1890, Shibasaburo Kitazato and Emil Behring conducted the fundamental experiment that demonstrated immunity can be transmitted from one animal to another by transferring the serum from an immune animal to a non-immune animal. This landmark experiment laid the foundation for the introduction of passive immunization into clinical practice. However, wide scale serum therapy was largely abandoned in the 1940s because of the toxicity associated with the administration of heterologous sera and the introduction of effective antimicrobial chemotherapy. Currently, such polyclonal antibody therapy is indicated to treat infectious diseases in relatively few situations, such as replacement therapy in immunoglobulin-deficient patients, post-exposure prophylaxis against several viruses (e.g., rabies, measles, hepatitis A and B, varicella), and toxin neutralization (diphtheria, tetanus, and botulism). Despite the limited use of serum therapy, in the United States, more than 16 metric tons of human antibody are required each year for intravenous antibody therapy. Comparable levels of use exist in the economies of most highly industrialized countries, and the demand can be expected to grow rapidly in developing countries. Currently, human antibody for passive immunization is obtained from the pooled serum of donors. Thus, there is an inherent limitation in the amount of human antibody available for therapeutic and prophylactic therapies.

The use of antibodies for passive immunization against biological warfare agents represents a very promising defense strategy. The final line of defense against such agents is the immune system of the exposed individual. Current defense strategies against biological weapons include such measures as enhanced epidemiologic surveillance, vaccination, and use of antimicrobial agents. Since the potential threat of biological warfare and bioterrorism is inversely proportional to the number of immune persons in the targeted population, biological agents are potential weapons only against populations with a substantial proportion of susceptible persons.

Vaccination can reduce the susceptibility of a population against specific threats, provided that a safe vaccine exists that can induce a protective response. Unfortunately, inducing a protective response by vaccination may take longer than the time between exposure and onset of disease. Moreover, many vaccines require multiple doses to achieve a protective immune response, which would limit their usefulness in an emergency to provide rapid prophylaxis after an attack. In addition, not all vaccine recipients mount a protective response, even after receiving the recommended immunization schedule.

Drugs can provide protection when administered after exposure to certain agents, but none are available against many potential agents of biological warfare. Currently, no small-molecule drugs are available that prevent disease following exposure to preformed toxins. The only currently available intervention that could provide a state of immediate immunity is passive immunization with protective antibody (Arturo Casadevall "Passive Antibody Administration (Immediate Immunity) as a Specific Defense Against Biological Weapons" from Emerging Infectious Diseases, Posted 09/12/2002).

In addition to providing protective immunity, modem antibody-based therapies constitute a potentially useful option against newly emergent pathogenic bacteria, fungi, virus and parasites (A. Casadevall and M. D. Scharff, Clinical Infectious Diseases 1995; 150). Therapies of patients with malignancies and cancer (C. Botti et al, Leukemia 1997; Suppl 2:S55-59; B. Bodey, S.E. Siegel, and H.E. Kaiser, Anticancer Res 1996; 16(2):661), therapy of steroid resistant rejection of transplanted organs as well as autoimmune diseases can also be achieved through the use of monoclonal or polyclonal antibody preparations (N. Bonnefoy-Berard and J.P. Revillard, J Heart Lung Transplant 1996; 15(5):435-442; C. Colby, et al Ann Pharmacother 1996; 30(10):1164-1174; M.J. Dugan, et al, Ann Hematol 1997; 75(1-2):41 2; W. Cendrowski, Boll Ist Sieroter Milan 1997; 58(4):339-343; L.K. Kastrukoff, et al Can J Neurol Sci 1978; 5(2):175178; J.E. Walker et al J Neurol Sci 1976; 29(2-4):303309).

Recent advances in the technology of antibody production provide the means to generate human antibody reagents, while avoiding the toxicities associated with human serum therapy. The advantages of antibody-based therapies include versatility, low toxicity, pathogen specificity, enhancement of immune function, and favorable pharmacokinetics.

The clinical use of monoclonal antibody therapeutics has just recently emerged. Monoclonal antibodies have now been approved as therapies in transplantation, cancer, infectious disease, cardiovascular disease and inflammation. In many more monoclonal antibodies are in late stage clinical trials to treat a broad range of disease indications. As a result, monoclonal antibodies represent one of the largest classes of drugs currently in development.

Despite the recent popularity of monoclonal antibodies as therapeutics, there are some obstacles for their use. For example, many therapeutic applications for monoclonal antibodies require repeated administrations, especially for chronic diseases such as autoimmunity or cancer. Because mice are convenient for immunization and recognize most human antigens as foreign, monoclonal antibodies against human targets with therapeutic potential have typically been of murine origin. However, murine monoclonal antibodies have inherent disadvantages as human therapeutics. For example, they require more frequent dosing to maintain a therapeutic level of monoclonal antibodies because of a shorter circulating half-life in humans than human antibodies. More critically, repeated administration of murine immunoglobulin creates the likelihood that the human immune system will recognize the mouse protein as foreign, generating a human anti-mouse antibody response, which can cause a severe allergic reaction. This possibility of reduced efficacy and safety has lead to the development of a number of technologies for reducing the immunogenicity of murine monoclonal antibodies.

Polyclonal antibodies are highly potent against multiple antigenic targets. They have the unique ability to target and kill a plurality of "evolving targets" linked with complex diseases. Also, of all drug classes, polyclonals have the highest probability of retaining activity in the event of antigen mutation. In addition, while monoclonals have limited therapeutic activity against infectious agents, polyclonals can both neutralize toxins and direct immune responses to eliminate pathogens, as well as biological warfare agents.

The development of polyclonal and monoclonal antibody production platforms to meet future demand for production capacity represents a promising area that is currently the subject of much research. One especially promising strategy is the introduction of human immunoglobulin genes into mice or large domestic animals. An extension of this technology would include inactivation of their endogenous immunoglobulin genes. Large animals, such as sheep, pigs and cattle, are all currently used in the production of plasma derived products, such as hyperimmune serum and clotting factors, for human use. This would support the use of human polyclonal antibodies from such species on the grounds of safety and ethics. Each of these species naturally produces considerable quantities of antibody in both serum and milk.

### Arrangement of Genes Encoding Immunoglobulins

Antibody molecules are assembled from combinations of variable gene elements, and the possibilities resulting from combining the many variable gene elements in the germline enable the host to synthesize antibodies to an extraordinarily large number of antigens. Each antibody molecule consists of two classes of polypeptide chains, light (L) chains (that can be either kappa (κ) L-chain or lambda (λ) L-chain) and heavy (H) chains. The heavy and light chains join together to define a binding region for the epitope. A single antibody molecule has two identical copies of the L chain and two of the H chain. Each of the chains is comprised of a variable region (V) and a constant region (C). The variable region constitutes the antigen-binding site of the molecule. To achieve diverse antigen recognition, the DNA that encodes the variable region undergoes gene rearrangement. The constant region amino acid sequence is specific for a particular isotype of the antibody, as well as the host which produces the antibody, and thus does not undergo rearrangement.

The mechanism of DNA rearrangement is similar for the variable region of both the heavy- and light-chain loci, although only one joining event is needed to generate a light-chain gene whereas two are needed to generate a complete heavy-chain gene. The most common mode of rearrangement involves the looping-out and deletion of the DNA between two gene segments. This occurs when the coding sequences of the two gene segments are in the same orientation in the DNA. A second mode of recombination can occur between two gene segments that have opposite transcriptional orientations. This mode of recombination is less common, although such rearrangements can account for up to half of all V_{κ} to J_{κ} joins; the transcriptional orientation of half of the human V_{κ} gene segments is opposite to that of the J_{κ} gene segments.

The DNA sequence encoding a complete V region is generated by the somatic recombination of separate gene segments. The V region, or V domain, of an immunoglobulin heavy or light chain is encoded by more than one gene segment. For the light chain, the V domain is encoded by two separate DNA segments. The first segment encodes the first 95-101 amino acids of the light chain and is termed a V gene segment because it encodes most of the V domain. The second segment encodes the remainder of the V domain (up to 13 amino acids) and is termed a joining or J gene segment. The joining of a V and a J gene segment creates a continuous exon that encodes the whole of the light-chain V region. To make a complete immunoglobulin light-chain messenger RNA, the V-region exon is joined to the C-region sequence by RNA splicing after transcription.

- A heavy-chain- V region is encoded in three-gene segments. In addition to the V and J gene segments (denoted V_{H} and J_{H} to distinguish them from the light-chain V_{L} and J_{L}), there is a third gene segment called the diversity or D_{H} gene segment, which lies between the V_{H} and J_{H} gene segments. The process of recombination that generates a complete heavy-chain V region occurs in two separate stages. In the first, a D_{H} gene segment is joined to a J_{H} gene segment; then a V_{H} gene segment rearranges to DJ_{H} to make a complete V_{H}-region exon. As with the light-chain genes, RNA splicing joins the assembled V-region sequence to the neighboring C-region gene.

Diversification of the antibody repertoire occurs in two stages: primarily by rearrangement ("V(D)J recombination") of Ig V, D and J gene segments in precursor B cells resident in the bone marrow, and then by somatic mutation and class switch recombination of these rearranged Ig genes when mature B cells are activated. Immunoglobulin somatic mutation and class switching are central to the maturation of the immune response and the generation of a "memory" response.

The genomic loci of antibodies are very large and they are located on different chromosomes. The immunoglobulin gene segments are organized into three clusters or genetic loci: the κ, λ, and heavy-chain loci. Each is organized slightly differently. For example, in humans, immunoglobulin genes are organized as follows. The λ light-chain locus is located on chromosome 22 and a cluster of V_{λ} gene segments is followed by four sets of J_{λ} gene segments each linked to a single C_{λ} gene. The κ light-chain locus is on chromosome 2 and the cluster of V_{κ} gene segments is followed by a cluster of Jκ gene segments, and then by a single Cκ gene. The organization of the heavy-chain locus, on chromosome 14, resembles that of the κ locus, with separate clusters of V_{H}, D_{H}, and J_{H} gene segments and of C_{H} genes. The heavy-chain locus differs in one important way: instead of a single C-region, it contains a series of C regions arrayed one after the other, each of which corresponds to a different isotype. There are five immunoglobulin heavy chain isotypes: IgM, IgG, IgA, IgE and IgD. Generally, a cell expresses only one at a time, beginning with IgM. The expression of other isotypes, such as IgG, can occur through isotype switching.

The joining of various V, D and J genes is an entirely random event that results in approximately 50,000 different possible combinations for VDJ(H) and approximately 1,000 for VJ(L). Subsequent random pairing of H and L chains brings the total number of antibody specificities to about 10⁷ possibilities. Diversity is further increased by the imprecise joining of different genetic segments. Rearrangements occur on both DNA strands, but only one strand is transcribed (due to allelic exclusion). Only one rearrangement occurs in the life of a B cell because of irreversible deletions in DNA. Consequently, each mature B cell maintains one immunologic specificity and is maintained in the progeny or clone. This constitutes the molecular basis of the clonal selection; i.e., each antigenic determinant triggers the response of the pre-existing clone of B lymphocytes bearing the specific receptor molecule. The primary repertoire of B cells, which is established by V(D)J recombination, is primarily controlled by two closely linked genes, recombination activating gene (RAG)-1 and RAG-2.

Over the last decade, considerable diversity among vertebrates in both Ig gene diversity and antibody repertoire development has been revealed. Rodents and humans have five heavy chain classes, IgM, IgD, IgG, IgE and IgA, and each have four subclasses of IgG and one or two subclasses of IgA, while rabbits have a single IgG heavy chain gene but 13 genes for different IgA subclasses (Burnett, R.C et al. EMBO J 8:4047; Honjo, In Honjo, T, Alt. F.W. T.H. eds, Immunoglobulin Genes p. 123 Academic Press, New York). Swine have at least six IgG subclasses (Kacskovics, I et al. 1994 J Immunol 153:3565), but no IgD (Butler et al. 1996 Inter. Immunol 8:1897-1904). A gene encoding IgD has only been described in rodents and primates. Diversity in the mechanism of repertoire development is exemplified by contrasting the pattern seen in rodents and primates with that reported for chickens, rabbits, swine and the domesticated Bovidae. Whereas the former group have a large number of V_{H} genes belonging to seven to 10 families (Rathbun, G. In Hongo, T. Alt. F.W. and Rabbitts, T.H., eds, Immunoglobulin Genes, p. 63, Academic press New York), the V_{H} genes of each member of the latter group belong to a single V_{H} gene family (Sun, J. et al. 1994 J. Immunol. 1553:56118; Dufour, V et al.1996, J Immunol. 156:2163). With the exception of the rabbit, this family is composed of less than 25 genes. Whereas rodents and primates can utilize four to six J_{H} segments, only a single J_{H} is available for repertoire development in the chicken (Reynaud et al. 1989 Adv. Immunol. 57:353). Similarly, Butler et al. (1996 Inter. Immunol 8:1897-1904) hypothesized that swine may resemble the chicken in having only a single J_{H} gene. These species generally have fewer V, D and J genes; in the pig and cow a single VH gene family exists, consisting of less than 20 gene segments (Butler et al, Advances in Swine in Biomedical Research, eds: Tumbleson and Schook, 1996; Sinclair et al, J. Immunol. 159: 3883, 1997). Together with lower numbers of J and D gene segments, this results in significantly less diversity being generated by gene rearrangement. However, there does appear to be greater numbers of light chain genes in these species. Similar to humans and mice, these species express a single κ light chain but multiple λ light chain genes. However, these do not seem to affect the restricted diversity that is achieved by rearrangement.

Since combinatorial joining of more than 100 V_{H}, 20-30 D_{H} and four to six J_{H} gene segments is a major mechanism of generating the antibody repertoire in humans, species with fewer V_{H}, D_{H} or J_{H} segments must either generate a smaller repertoire or use alternative mechanisms for repertoire development. Ruminants, pigs, rabbits and chickens, utilize several mechanisms to generate antibody diversity. In these species there appears to be an important secondary repertoire development, which occurs in highly specialized lymphoid tissue such as ileal Peyer's patches (Binns and Licence, Adv. Exp. Med. Biol. 186: 661, 1985). Secondary repertoire development occurs in these species by a process of somatic mutation which is a random and not fully understood process. The mechanism for this repertoire diversification appears to be templated mutation, or gene conversion (Sun et al, J. Immunol. 153: 5618, 1994) and somatic hypermutation.

Gene conversion is important for antibody diversification in some higher vertebrates, such as chickens, rabbits and cows. In mice, however, conversion events appear to be infrequent among endogenous antibody genes. Gene conversion is a distinct diversifying mechanism characterized by transfers of homologous sequences from a donor antibody V gene segment to an acceptor V gene segment. If donor and acceptor segments have numerous sequence differences then gene conversion can introduce a set of sequence changes into a V region by a single event. Depending on the species, gene conversion events can occur before and/or after antigen exposure during B cell differentiation (Tsai et al. International Immunology, Vol. 14, No. 1, 55-64, January 2002).

Somatic hypermutation achieves diversification of antibody genes in all higher vertebrate. species. It is typified by the introduction of single point mutations into antibody V(D)J segments. Generally, hypermutation appears to be activated in B cells by antigenic stimulation.

### Production of Animals with Humanized Immune Systems

In order to reduce the immunogenicity of antibodies generated in mice for human therapeutics, various attempts have been made to replace murine protein sequences with human protein sequences in a process now known as humanization. Transgenic mice have been constructed which have had their own immunoglobulin genes functionally replaced with human immunoglobulin genes so that they produce human antibodies upon immunization. Elimination of mouse antibody production was achieved by inactivation of mouse Ig genes in embryonic stem (ES) cells by using gene-targeting technology to delete crucial *cis*-acting sequences involved in the process of mouse Ig gene rearrangement and expression. B cell development in these mutant mice could be restored by the introduction of megabase-sized YACs containing a human germline-configuration H- and κ L-chain minilocus transgene. The expression of fully human antibody in these transgenic mice was predominant, at a level of several 100 µg/l of blood. This level of expression is several hundred-fold higher than that detected in wild-type mice expressing the human Ig gene, indicating the importance of inactivating the endogenous mouse Ig genes in order to enhance human antibody production by mice.

The first humanization attempts utilized molecular biology techniques to construct recombinant antibodies. For example, the complementarity determining regions (CDR) from a mouse antibody specific for a hapten were grafted onto a human antibody framework, effecting a CDR replacement. The new antibody retained the binding specificity conveyed by the CDR sequences (P. T. Jones et al. Nature 321: 522-525 (1986)). The next level of humanization involved combining an entire mouse VH region with a human constant region such as gamma₁ (S. L. Morrison et al., Proc. Natl. Acad. Sci., 81, pp. 6851-6855 (1984)). However, these chimeric antibodies, which still contain greater than 30% xenogeneic sequences, are sometimes only marginally less immunogenic than totally xenogeneic antibodies (M. Bruggemann et al., J. Exp. Med., 170, pp. 2153-2157 (1989)).

Subsequently, attempts were carried out to introduce human immunoglobulin genes into the mouse, thus creating transgenic mice capable of responding to antigens with antibodies having human sequences (Bruggemann et al. Proc. Nat'l. Acad. Sci. USA 86:6709-6713 (1989)). Due to the large size of human immunoglobulin genomic loci, these attempts were thought to be limited by the amount of DNA, which could be stably maintained by available cloning vehicles. As a result, many investigators concentrated on producing mini-loci containing limited numbers of V region genes and having altered spatial distances between genes as compared to the natural or germline configuration (See, for example, U.S. Pat. No. 5,569,825). These studies indicated that producing human sequence antibodies in mice was possible, but serious obstacles remained regarding obtaining sufficient diversity of binding specificities and effector functions (isotypes) from these transgenic animals to meet the growing demand for antibody therapeutics.

In order to provide additional diversity, work has been conducted to add large germline fragments of the human Ig locus into transgenic mammals. For example, a majority of the human V, D, and J region genes arranged with the same spacing found in the unrearranged germline of the human genome and the human Cµ and Cδ constant regions was introduced into mice using yeast artificial chromosome (YAC) cloning vectors (See, for example, WO 94/02602). A 22 kb DNA fragment comprising sequences encoding a human gamma-2 constant region and the upstream sequences required for class-switch recombination was latter appended to the foregoing transgene. In addition, a portion of a human kappa locus comprising Vκ, Jκ and Cκ region genes, also arranged with substantially the same spacing found in the unrearranged germline of the human genome, was introduced into mice using YACS. Gene targeting was used to inactivate the murine IgH & kappa light chain immunoglobulin gene loci and such knockout strains were bred with the above transgenic strains to generate a line of mice having the human V, D, J, Cµ, Cδ. and Cγ₂ constant regions as well as the human Vκ, Jκ and Cκ region genes all on an inactivated murine immunoglobulin background (See, for example, PCT patent application WO 94/02602 to Kucherlapati et al.; see also Mendez et al., Nature Genetics 15:146-156 (1997)).

Yeast artificial chromosomes as cloning vectors in combination with gene targeting of endogenous loci and breeding of transgenic mouse strains provided one solution to the problem of antibody diversity. Several advantages were obtained by this approach. One advantage was that YACs can be used to transfer hundreds of kilobases of DNA into a host cell. Therefore, use of YAC cloning vehicles allows inclusion of substantial portions of the entire human Ig heavy and light chain regions into a transgenic mouse thus approaching the level of potential diversity available in the human. Another advantage of this approach is that the large number of V genes has been shown to restore full B cell development in mice deficient in murine immunoglobulin production. This ensures that these reconstituted mice are provided with the requisite cells for mounting a robust human antibody response to any given immunogen. (See, for example, WO 94/02602.; L. Green and A. Jakobovits, J. Exp. Med. 188:483-495 (1998)). A further advantage is that sequences can be deleted or inserted onto the YAC by utilizing high frequency homologous recombination in yeast. This provides for facile engineering of the YAC transgenes.

In addition, Green et al. Nature Genetics 7:13-21 (1994) describe the generation of YACs containing 245 kb and 190 kb-sized germline configuration fragments of the human heavy chain locus and kappa light chain locus, respectively, which contained core variable and constant region sequences. The work of Green et al. was recently extended to the introduction of greater than approximately 80% of the human antibody repertoire through introduction of megabase sized, germline configuration YAC fragments of the human heavy chain loci and kappa light chain loci, respectively, to produce XenoMouse™ mice. See, for example, Mendez et al. Nature Genetics 15:146-156 (1997), Green and Jakobovits J. Exp. Med. 188:483-495 (1998), European Patent No. EP 0 463 151 B1, PCT Publication Nos. WO 94/02602, WO 96/34096 and WO 98/24893.

Several strategies exist for the generation of mammals that produce human antibodies. In particular, there is the "minilocus" approach that is typified by work of GenPharm International, Inc. and the Medical Research Council, YAC introduction of large and substantially germline fragments of the Ig loci that is typified by work of Abgenix, Inc. (formerly Cell Genesys). The introduction of entire or substantially entire loci through the use microcell fusion as typified by work of Kirin Beer Kabushiki Kaisha.

In the minilocus approach, an exogenous Ig locus is mimicked through the inclusion of pieces (individual genes) from the Ig locus. Thus, one or more V_{H} genes, one or more D_{H} genes, one or more J_{H} genes, a mu constant region, and a second constant region (such as a gamma constant region) are formed into a construct for insertion into an animal. See, for example, U.S. Patent Nos. 5,545,807, 5,545,806, 5,625,825, 5,625,126, 5,633,425, 5,661,016, 5,770,429, 5,789,650, 5,814,318, 5,591,669, 5,612,205, 5,721,367, 5,789,215, 5,643,763; European Patent No. 0 546 073; PCT Publication Nos. WO 92/03918, WO 92/22645, WO 92/22647, WO 92/22670, WO 93/12227, WO 94/00569, WO 94/25585, WO 96/14436, WO 97/13852, and WO 98/24884; Taylor et al. Nucleic Acids Research 20:6287-6295 (1992), Chen et al. International Immunology 5:647-656 (1993), Tuaillon et al. J. Immunol. 154:6453-6465 (1995), Choi et al. Nature Genetics 4:117-123 (1993), Lonberg et al. Nature 368:856-859 (1994), Taylor et al. International Immunology 6:579-591 (1994), Tuaillon et al. J. Immunol. 154:6453-6465 (1995), and Fishwild et al. Nature Biotech. 14:845-851 (1996).

In the microcell fusion approach, portions or whole human chromosomes can be introduced into mice (see, for example, European Patent Application No. EP 0 843 961 A1). Mice generated using this approach and containing the human Ig heavy chain locus will generally possess more than one, and potentially all, of the human constant region genes. Such mice will produce, therefore, antibodies that bind to particular antigens having a number of different constant regions.

While mice remain the most developed animal for the expression of human immunoglobulins in humans, recent technological advances have allowed for progress to begin in applying these techniques to other animals, such as cows. The general approach in mice has been to genetically modify embryonic stem cells of mice to knock-out murine immunoglobulins and then insert YACs containing human immunoglobulins into the ES cells. However, ES cells are not available for cows or other large animals such as sheep and pigs. Thus, several fundamental developments had to occur before even the possibility existed to generate large animals with immunoglobulin genes knocked-out and that express human antibody. The alternative to ES cell manipulation to create genetically modified animals is cloning using somatic cells that have been genetically modified. Cloning using genetically modified somatic cells for nuclear transfer has only recently been accomplished.

Since the announcement of Dolly's (a cloned sheep) birth from an adult somatic cell in 1997 (Wilmut, I., et al (1997) Nature 385: 810-813), ungulates, including cattle (Cibelli, J et al 1998 Science 280: 1266-1258; Kubota, C. et al.2000 Proc. Nat'l. Acad. Sci 97: 990-995), goats (Baguisi, A. et al., (1999) Nat. Biotechnology 17: 456-461), and pigs (Polejaeva, I.A., et al. 2000 Nature 407: 86-90; Betthauser, J. et al. 2000 Nat. Biotechnology 18: 1055-1059) have been cloned.

The next technological advance was the development of the technique to genetically modify the cells prior to nuclear transfer to produce genetically modified animals. PCT publication No. WO 00/51424 to PPL Therapeutics describes the targetted genetic modification of somatic cells for nuclear transfer.

Subsequent to these fundamental developments, single and double allele knockouts of genes and the birth of live animals with these modifications have been reported. Between 2002 and 2004, three independent groups, Immerge Biotherapeutics, Inc. in collaboration with the University of Missouri (Lai et al. (Science (2002) 295: 1089-1092) & Kolber-Simonds et al. (PNAS. (2004) 101(19):7335-40)), Alexion Pharmaceuticals (Ramsoondar et al. (Biol Reprod (2003)69: 437-445) and Revivicor, Inc. (Dai et al. (Nature Biotechnology (2002) 20: 251-255) & Phelps et al. (Science (2003) Jan 17;299(5605):411-4)) produced pigs that lacked one allele or both alleles of the alpha-1,3-GT gene via nuclear transfer from somatic cells with targeted genetic deletions. In 2003, Sedai et al. (Transplantation (2003) 76:900-902) reported the targeted disruption of one allele of the alpha-1,3-GT gene in cattle, followed by the successful nuclear transfer of the nucleus of the genetically modified cell and production of transgenic fetuses.

Thus, the feasibility of knocking-out immunoglobulin genes in large animals and inserting human immunoglobulin loci into their cells is just now beginning to be explored. However, due to the complexity and species differences of immunoglobulin genes, the genomic sequences and arrangement of Ig kappa, lambda and heavy chains remain poorly understood in most species. For example, in pigs, partial genomic sequence and organization has only been described for heavy chain constant alpha, heavy chain constant mu and heavy chain constant delta (Brown and Butler Mol Immunol. 1994 Jun;31(8):633-42, Butler et al Vet Immunol. Immunopathol. 1994 Oct;43(1-3):5-12, and Zhao et al J Immunol. 2003 Aug 1;171(3):1312-8).

In cows, the immunoglobulin heavy chain locus has been mapped (Zhao et al. 2003 J. Biol. Chem. 278:35024-32) and the cDNA sequence for the bovine kappa gene is known (See, for example, U.S. Patent Publication No. 2003/0037347). Further, approximately 4.6kb of the bovine mu heavy chain locus has been sequenced and transgenic calves with decreased expression of heavy chain immunoglobulins have been created by disrupting one or both alleles of the bovine mu heavy chain. In addition, a mammalian artificial chromosome (MAC) vector containing the entire unarranged sequences of the human Ig H-chain and K L-chain has been introduced into cows (TC cows) with the technology of microcell-mediated chromosome transfer and nuclear transfer of bovine fetal fibroblast cells (see, for example, Kuroiwa et al. 2002 Nature Biotechnology 20:889, Kuroiwa et al. 2004 Nat Genet. Jun 6 Epub, U.S. Patent Publication Nos. 2003/0037347, 2003/0056237, 2004/0068760 and PCT Publication No. WO 02/07648).

While significant progress has been made in the production of bovine that express human immunoglobulin, little has been accomplished in other large animals, such as sheep, goats and pigs. Although cDNA sequence information for immunoglobulin genes of sheeps, goats and pigs is readily available in Genbank, the unique nature of immunoglobulin loci, which undergo massive rearrangements, creates the need to characterize beyond sequences known to be present in mRNAs (or cDNAs). Since immunoglobulin loci are modular and the coding regions are redundant, deletion of a known coding region does not ensure altered function of the locus. For example, if one were to delete the coding region of a heavy-chain variable region, the function of the locus would not be significantly altered because hundreds of other function variable genes remain in the locus. Therefore, one must first characterize the locus to identify a potential "Achilles heel".

WO 03/047336 discloses transgenic mice which are homozygous for an inactivated endogenous immunoglobulin kappa light chain locus.

Despite some advancements in expressing human antibodies in cattle, greater challenges remain for inactivation of the endogenous bovine Ig genes, increasing expression levels of the human antibodies and creating human antibody expression in other large animals, such as porcine, for which the sequence and arrangement of immunoglobulin genes are largely unknown.

It is therefore an object of the present disclosure to provide the arrangement of ungulate immunoglobulin germline gene sequence.

It is another object of the present disclosure to provide novel ungulate immunoglobulin genomic sequences.

It is a further object of the present disclosure to provide cells, tissues and animals lacking expression of at least one allele of a heavy and/or light chain immunoglobulin gene.

It is another object of the present disclosure to provide ungulates that express human immunoglobulins.

It is a still further object of the present disclosure to provide methods to generate cells, tissues and animals lacking at least one allele of novel ungulate immunoglobulin gene sequences and/ or express human immunoglobulins.

### SUMMARY OF THE INVENTION

The invention in its broadest sense is as defined in the independent claims. The invention provides a transgenic porcine animal lacking expression of at least one allele of porcine endogenous kappa light chain immunoglobulin gene, in which the at least one allele of porcine kappa light chain gene is inactivated via a genetic targeting event, wherein the genetic targeting event comprises disruption of the kappa constant region.

The invention also provides a method for producing the transgenic porcine of the invention, comprising (i) a step of gene disruption through (a) substitution, deletion or insertion techniques and/or (b) homologous recombination; and/or (ii) a step of cloning using a donor nucleus from a cell in which at least one allele of the immunoglobulin gene has been inactivated.

The invention also provides a cell, tissue or organ derived from a porcine animal of the invention, wherein the cell tissue or organ lacks expression of at least one allele of porcine endogenous kappa light chain immunoglobulin gene, in which the at least one allele of porcine kappa light chain gene is inactivated via a genetic targeting event, wherein the genetic targeting event comprises disruption of the kappa constant region.

### SUMMARY OF THE DISCLOSURE

Disclosed herein for the first time are ungulate immunoglobulin germline gene sequence arrangement as well as novel genomic sequences thereof. In addition, novel ungulate cells, tissues and animals that lack expression of at least one allele of a heavy or light chain immunoglobulin gene are disclosed. Based on this discovery, ungulates can be produced that completely lack expression of at least one allele of a heavy and/or light chain immunoglobulin gene. In addition, these ungulates can be further modified to express xenogenous, such as human, immunoglobulin loci or fragments thereof.

Disclosed herein is a transgenic ungulate that lacks any expression of functional endogenous immunoglobulins. The ungulate can lack any expression of endogenous heavy and/ or light chain immunoglobulins.

The light chain immunoglobulin can be a kappa and/ or lambda immunoglobulin. Transgenic ungulates are disclosed that lack expression of at least one allele of an endogenous immunoglobulin wherein the immunoglobulin is selected from the group consisting of heavy chain, kappa light chain and lambda light chain or any combination thereof. The expression of functional endogenous immunoglobulins can be accomplished by genetic targeting of the endogenous immunoglobulin loci to prevent expression of the endogenous immunoglobulin. The genetic targeting can be accomplished via homologous recombination. The transgenic ungulate can be produced via nuclear transfer.

The transgenic ungulate that lacks any expression of functional endogenous immunoglobulins can be further genetically modified to express xenogenous immunoglobulin loci. Porcine animals are disclosed that contain an xenogenous immunoglobulin locus. The xenogenous immunoglobulin locus can be a heavy and/ or light chain immunoglobulin or fragment thereof. The xenogenous immunoglobulin locus can be a kappa chain locus or fragment thereof and/ or a
lambda chain locus or fragment thereof. An artificial chromosome (AC) can contain the xenogenous immunoglobulin locus. Then AC can be a yeast AC or a mammalian AC. The
xenogenous locus can be a human immunoglobulin locus or fragment thereof. The human immunoglobulin locus can be human chromosome 14, human chromosome 2, and human chromosome 22 or fragments thereof. The human immunoglobulin locus can include any fragment of a human immunoglobulin locus that can undergo rearrangement. The human immunoglobulin locus can include any fragment of a human immunoglobulin heavy chain locus and a human immunoglobulin light chain locus that can undergo rearrangement. The human immunoglobulin locus can include any human immunoglobulin locus or fragment thereof that can produce an antibody upon exposure to an antigen. In particular the exogenous human immunoglobulin can be expressed in B cells to produce xenogenous immunoglobulin in response to exposure to one or more antigens.

In an aspect of the present invention, transgenic porcine animals are provided that express a xenogenous immunoglobulin or fragment thereof, wherein the immunoglobulin can be expressed from an immunoglobulin locus that is integrated within an endogenous ungulate chromosome. In one embodiment, cells derived from the transgenic porcine animals are provided. In one embodiment, the xenogenous immunoglobulin locus can be inherited by offspring. In another embodiment, the xenogenous immunoglobulin locus can be inherited through the male germ line by offspring. In still further embodiments, an artificial chromosome (AC) can contain the xenogenous immunoglobulin locus. In one embodiment, the AC can be a mammalian AC. In a further embodiment, the xenogenous locus can be a human immunoglobulin locus or fragment thereof that can undergo rearrangement. In one embodiment, the human immunoglobulin locus can be human chromosome 14, human chromosome 2, and human chromosome 22 or fragments thereof. The human immunoglobulin locus can include any fragment of a human immunoglobulin that can undergo rearrangement. In an embodiment, the human immunoglobulin locus can include any fragment of a human immunoglobulin heavy chain locus and a human immunoglobulin light chain locus that can undergo rearrangement. The human immunoglobulin locus can include any human immunoglobulin locus or fragment thereof that can produce an antibody upon exposure to an antigen. In a particular embodiment, the exogenous human immunoglobulin can be expressed in B cells to produce xenogenous immunoglobulin in response to exposure to one or more antigens.

Novel genomic sequences encoding the heavy chain locus of ungulate immunoglobulin are disclosed herein. An isolated nucleotide sequence encoding porcine heavy chain is disclosed that includes at least one variable region, two diversity regions, at least four joining regions and at least one constant region, such as the mu constant region, for example, as represented in Seq ID No. 29. An isolated nucleotide sequence is disclosed that includes at least four joining regions and at least one constant region, such as the mu constant region, of the porcine heavy chain genomic sequence, for example, as represented in Seq ID No. 4. Nucleotide sequence is disclosed that includes 5' flanking sequence to the first joining region of the porcine heavy chain genomic sequence, for example, as represented in Seq ID No 1. Still further, nucleotide sequence is disclosed that includes 3' flanking sequence to the first joining region of the porcine heavy chain genomic sequence, for example, as represented in the 3' region of Seq ID No 4. Isolated nucleotide sequences as depicted in Seq ID Nos 1, 4 or 29 are disclosed herein. Nucleic acid sequences at least 80, 85, 90, 95, 98 or 99% homologous to Seq ID Nos 1, 4 or 29 are also disclosed. In addition, nucleotide sequences that contain at least 10, 15, 17, 20, 25 or 30 contiguous nucleotides of Seq ID Nos 1, 4 or 29 are disclosed. The nucleotide sequence can contain at least 17, 20, 25 or 30 contiguous nucleotides of Seq ID No 4 or residues 1- 9,070 of Seq ID No 29.
The nucleotide sequence can contain residues 9,070-11039 of Seq ID No 29. Also disclosed are nucleotide sequences that hybridize, optionally under stringent conditions, to Seq ID Nos 1, 4 or 29, as well as, nucleotides homologous thereto.

Novel genomic sequences encoding the kappa light chain locus of ungulate immunoglobulin are also disclosed. Disclosed herein is the first reported genomic sequence of ungulate kappa light chain regions. Nucleic acid sequence is disclosed that encodes the porcine kappa light chain locus. The nucleic acid sequence can contain at least one joining region, one constant region and/or one enhancer region of kappa light chain locus. The nucleotide sequence can include at least five joining regions, one constant region and one enhancer region, for example, as represented in Seq ID No. 30. An isolated nucleotide sequence is disclosed herein that contains at least one, at least two, at least three, at least four or five joining regions and 3' flanking sequence to the joining region of porcine genomic kappa light chain, for example, as represented in Seq ID No 12.An isolated nucleotide sequence of porcine genomic kappa light chain is disclosed herein that contains 5' flanking sequence to the first joining region, for example, as represented in Seq ID No 25. An isolated nucleotide sequence is disclosed herein that contains 3' flanking sequence to the constant region and, optionally, the 5' portion of the enhancer region, of porcine genomic kappa light chain, for example, as represented in Seq ID Nos. 15, 16 and/or 19.

Isolated nucleotide sequences as depicted in Seq ID Nos 30, 12, 25, 15, 16 or 19 are also disclosed herein. Nucleic acid sequences at least 80, 85, 90, 95, 98 or 99% homologous to Seq ID Nos 30, 12, 25, 15, 16 or 19 are also disclosed herein. In addition, nucleotide sequences that contain at least 10, 15, 17, 20, 25 or 30 contiguous nucleotides of Seq ID Nos 30, 12, 25, 15, 16 or 19 are disclosed herein. Further disclosed herein are nucleotide sequences that hybridizes, optionally under stringent conditions, to Seq ID Nos 30, A2, 25, 15, 16 or 19, as well as, nucleotides homologous thereto.

Novel genomic sequences encoding the lambda light chain locus of ungulate immunoglobulin are disclosed herein. Disclosed herein is the first reported genomic sequence of ungulate lambda light chain regions. The porcine lambda light chain nucleotides include a concatamer of J to C units. An isolated porcine lambda nucleotide sequence is disclosed herein, such as that depicted in Seq BD No. 28. Nucleotide sequence is disclosed herein that includes 5' flanking sequence to the first lambda J/C region of the porcine lambda light chain genomic sequence, for example, as represented by Seq ID No 32. Nucleotide sequence is disclosed herein that includes 3' flanking sequence to the J/C cluster region of the porcine lambda light chain genomic sequence, for example, approximately 200 base pairs downstream of lambda J/C, such as that represented by Seq ID No 33. Alternatively, nucleotide sequence is disclosed herein that includes 3' flanking sequence to the J/C cluster region of the porcine lambda light chain genomic sequence, for example, as represented by Seq ID No 34, 35, 36, 37, 38, and/or 39.Nucleic acid sequences are disclosed herein that encode bovine lambda light chain locus, which can include at least one joining region-constant region pair and/or at least one variable region, for example, as represented by Seq ID No. 31.Isolated nucleotide
sequences as depicted in Seq ID Nos 28, 31, 32, 33, 34, 35, 36, 37, 38, or 39 are disclosed herein. Nucleic acid sequences at least 80, 85, 90, 95, 98 or 99% homologous to Seq ID Nos 28, 31, 32, 33, 34, 35, 36, 37, 38, or 39 are also disclosed herein. In addition, nucleotide sequences that contain at least 10, 15, 17, 20, 25 or 30 contiguous nucleotides of Seq ED Nos 28, 31, 32, 33, 34, 35, 36, 37, 38, or 39 are disclosed herein. Further disclosed herein are nucleotide sequences that hybridizes, optionally under stringent conditions, to Seq ED Nos 28, 31, 32, 33, 34, 35, 36, 37, 38, or 39, as well as, nucleotides homologous thereto.

Nucleic acid targeting vector constructs are also disclosed herein. The targeting vectors can be designed to accomplish homologous recombination in cells. These targeting vectors can be transformed into mammalian cells to target the ungulate heavy chain, kappa light chain or lambda light chain genes via homologous recombination. The targeting vectors can contain a 3' recombination arm and a 5' recombination arm (i.e. flanking sequence) that is homologous to the genomic sequence of ungulate heavy chain, kappa light chain or lambda light chain genomic sequence, for example, sequence represented by Seq ID Nos. 1, 4, 29, 30, 12, 25, 15, 16, 19, 28 or 31, as described above. The homologous DNA sequence can include at least 15 bp, 20 bp, 25 bp, 50 bp, 100 bp, 500 bp, lkbp, 2 kbp, 4 kbp, 5 kbp, 10 kbp, 15 kbp, 20 kbp, or 50 kbp of sequence homologous to the genomic sequence. The 3' and 5' recombination arms can be designed such that they flank the 3' and 5' ends of at least one functional variable, joining, diversity, and/or constant region of the genomic sequence. The targeting of a functional region can render it inactive, which results in the inability of the cell to produce functional immunoglobulin molecules. The homologous DNA sequence can include one or more intron and/or exon sequences. In addition to the nucleic acid sequences, the expression vector can contain selectable marker sequences, such as, for example, enhanced Green Fluorescent Protein (eGFP) gene sequences, initiation and/or enhancer sequences, poly A-tail sequences, and/or nucleic acid sequences that provide for the expression of the construct in prokaryotic and/or eukaryotic host cells. The selectable marker can be located between the 5' and 3' recombination arm sequence.

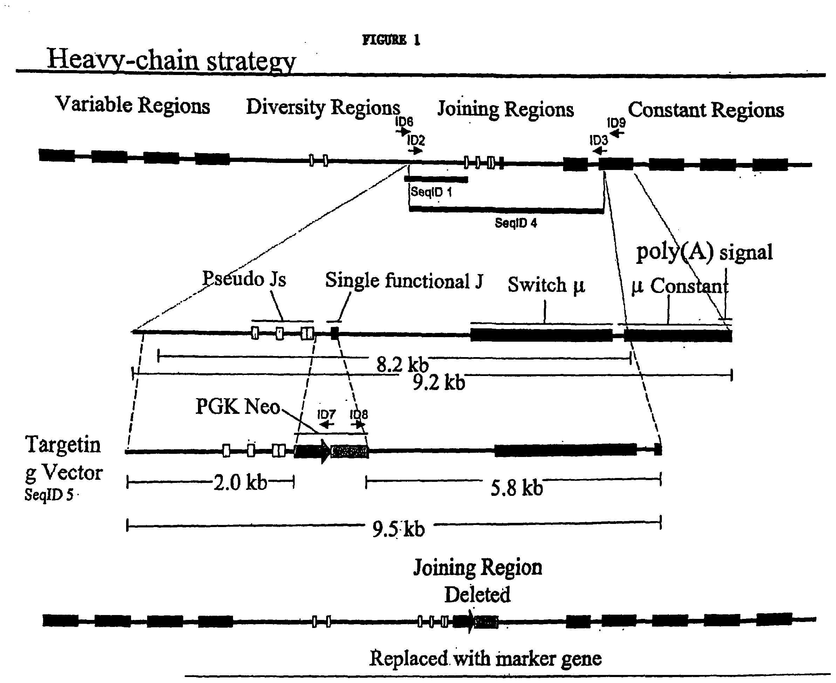

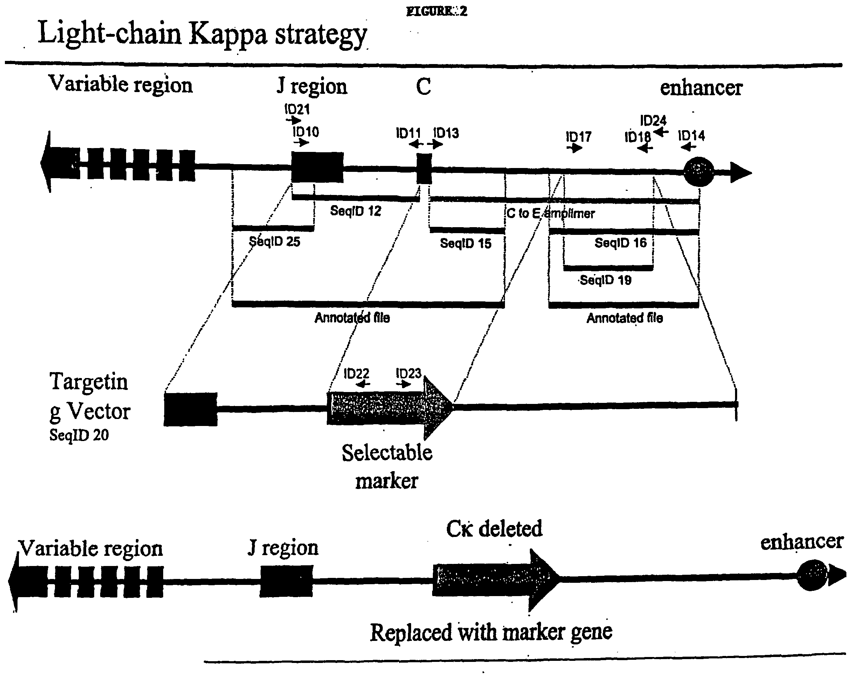

The targeting vector can contain 5' and 3' recombination arms that contain homologous sequence to the 3' and 5' flanking sequence of the J6 region of the porcine immunoglobulin heavy chain locus. Since the J6 region is the only functional joining region of the porcine immunoglobulin heavy chain locus, this will prevent the expression of a
functional porcine heavy chain immunoglobulin. The targeting vector can contain a 5' recombination arm that contains sequence homologous to genomic sequence 5' of the J6 region, including J1-4, and a 3' recombination arm that contains sequence homologous to genomic sequence 3' of the J6 region, including the mu constant region (a "J6 targeting construct"), see for example, Figure 1. Further, this J6 targeting construct can also contain a selectable marker gene that is located between the 5' and 3' recombination arms, see for example, Seq ID No 5 and Figure 1.The targeting vector can contain a 5' recombination arm that contains sequence homologous to genomic sequence 5' of the diversity region, and a 3' recombination arm that contains sequence homologous to genomic sequence 3' of the diversity region of the porcine heavy chain locus. The targeting vector can contain a 5' recombination arm that contains sequence homologous to genomic sequence 5' of the mu constant region and a 3' recombination arm that contains sequence homologous to genomic sequence 3' of the mu constant region of the porcine heavy chain locus. The targeting vector can contain 5' and 3' recombination arms that contain homologous sequence to the 3' and 5' flanking sequence of the constant region of the porcine immunoglobulin heavy chain locus. Since the present inventors discovered that there is only one constant region of the porcine immunoglobulin kappa light chain locus, this will prevent the expression of a functional porcine kappa light chain immunoglobulin. The targeting vector can contain a 5' recombination arm that contains sequence homologous to genomic sequence 5' of the constant region, optionally including the joining region, and a 3' recombination arm that contains sequence homologous to genomic sequence 3' of the constant region, optionally including at least part of the enhancer region (a "Kappa constant targeting construct"), see for example, Figure 2. Further, this kappa constant targeting construct can also contain a selectable marker gene that is located between the 5' and 3' recombination arms, see for example, Seq ID No 20 and Figure 2. The targeting vector can contain a 5' recombination arm that contains sequence homologous to genomic sequence 5' of the joining region, and a 3' recombination arm that contains sequence homologous to genomic sequence 3' of the joining region of the porcine kappa light chain locus.

Primers are disclosed herein to generate 3' and 5' sequences of a targeting vector. The oligonucleotide primers can be capable of hybridizing to porcine
immunoglobulin genomic sequence, such as Seq ID Nos. 1, 4, 29, 30, 12, 25, 15, 16, 19, 28 or 31, as described above. The primers may hybridize under stringent conditions to Seq ID Nos. 1, 4, 29, 30, 12, 25, 15, 16, 19, 28 or 31, as described above. Disclosed herein are oligonucleotide probes capable of hybridizing to porcine heavy chain, kappa light chain or lambda light chain nucleic acid sequences, such as Seq ID Nos. 1, 4, 29, 30, 12, 25, 15, 16, 19, 28 or 31, as described above. The polynucleotide primers or probes can have at least 14 bases, 20 bases, 30 bases, or 50 bases which hybridize to a polynucleotide disclosed herein. The probe or primer can be at least 14 nucleotides in length, and may be at least 15,20,25,28, or 30 nucleotides in length.

Primers are disclosed herein to amplify a fragment of porcine Ig heavy-chain that includes the functional joining region (the J6 region). The amplified fragment of heavy chain can be represented by Seq ID No 4 and the primers used to amplify this fragment can be complementary to a portion of the J-region, such as, but not limited to Seq ID No 2, to produce the 5' recombination arm and complementary to a portion of Ig heavy-chain mu constant region, such as, but not limited to Seq ID No 3, to produce the 3' recombination arm. Regions of the porcine Ig heavy chain (such as, but not limited to Seq ID No 4) can be subcloned and assembled into a targeting vector.

Primers are disclosed herein to amplify a fragment of porcine Ig kappa light-chain that includes the constant region. Primers are disclosed herein to amplify a fragment of porcine Ig kappa light-chain that includes the J region. The primers used to amplify this fragment can be complementary to a portion of the J-region, such as, but not limited to Seq ID No 21 or 10, to produce the 5' recombination arm and complementary to genomic sequence 3' of the constant region, such as, but not limited to Seq ID No 14, 24 or 18, to produce the 3' recombination arm. Regions of the porcine Ig heavy chain (such as, but not limited to Seq ID No 20) can be subcloned and assembled into a targeting vector.

Ungulate cells lacking expression of at least one allele of a functional region of an ungulate heavy chain, kappa light chain and/or lambda light chain locus produced according to the process, sequences and/or constructs described herein are disclosed. These cells can be obtained as a result of homologous recombination. Particularly, by inactivating at least one allele of an ungulate heavy chain, kappa light chain or lambda light
chain gene, cells can be produced which have reduced capability for expression of ungulate antibodies. Mammalian cells lacking expression of both alleles of an ungulate heavy chain, kappa light chain and/or lambda light chain gene can be produced according to the process, sequences and/or constructs described herein. Porcine animals are disclosed herein in which at least one allele of an ungulate heavy chain, kappa light chain and/or lambda light chain gene is inactivated via a genetic targeting event produced according to the process, sequences and/or constructs described herein. In another aspect of the present disclosure, porcine animals are provided in which both alleles of an ungulate heavy chain, kappa light chain and/or lambda light chain gene are inactivated via a genetic targeting event. The gene can be targeted via homologous recombination. The gene can be disrupted, i.e. a portion of the genetic code can be altered, thereby affecting transcription and/or translation of that segment of the gene. For example, disruption of a gene can occur through substitution, deletion ("knock-out") or insertion ("knock-in") techniques. Additional genes for a desired protein or regulatory sequence that modulate transcription of an existing sequence can be inserted. To achieve multiple genetic modifications of ungulate immunoglobulin genes, cells can be modified sequentially to contain multiple genetic modifications. Animals can be bred together to produce animals that contain multiple genetic modifications of immunoglobulin genes. As an illustrative example, animals that lack expression of at least one allele of an ungulate heavy chain gene can be further genetically modified or bred with animals lacking expression of at least one allele of a kappa light chain gene.

Alleles of ungulate heavy chain, kappa light chain or lambda light chain gene may be rendered inactive according to the process, sequences and/or constructs described herein, such that functional ungulate immunoglobulins can no longer be produced. The targeted immunoglobulin gene can be transcribed into RNA, but not translated into protein. The targeted immunoglobulin gene can be transcribed in an inactive truncated form. Such a truncated RNA may either not be translated or can be translated into a nonfunctional protein. The targeted immunoglobulin gene can be inactivated in such a way that no transcription of the gene occurs. The targeted immunoglobulin gene can be transcribed and then translated into a nonfunctional protein.

Ungulate, such as porcine or bovine, cells lacking expression of one allele, optionally both alleles of an ungulate heavy chain, kappa light chain and/or lambda light chain gene can be used as donor cells for nuclear transfer into recipient cells to produce cloned, transgenic animals. Alternatively, ungulate heavy chain, kappa light chain and/or lambda light chain gene knockouts can be created in embryonic stem cells, which are then used to produce offspring. Offspring lacking expression of a single allele of a functional ungulate heavy chain, kappa light chain and/or lambda light chain gene produced according to the process, sequences and/or constructs described herein can be breed to further produce offspring lacking functionality in both alleles through mendelian type inheritance.

A method is disclosed herein to disrupt the expression of an ungulate immunoglobulin gene by (i) analyzing the germline configuration of the ungulate heavy chain, kappa light chain or lambda light chain genomic locus; (ii) determining the location of nucleotide sequences that flank the 5' end and the 3' end of at least one functional region of the locus; and (iii) transfecting a targeting construct containing the flanking sequence into a cell wherein, upon successful homologous recombination, at least one functional region of the immunoglobulin locus is disrupted thereby reducing or preventing the expression of the immunoglobulin gene. The germline configuration of the porcine heavy chain locus is disclosed herein. The porcine heavy chain locus contains at least four variable regions, two diversity regions, six joining regions and five constant regions, for example, as illustrated in Figure 1. Only one of the six joining regions, J6, may be functional. The germline configuration of the porcine kappa light chain locus is disclosed herein. The porcine kappa light chain locus contains at least six variable regions, six joining regions, one constant region and one enhancer region, for example, as illustrated in Figure 2.The germline configuration of the porcine lambda light chain locus is disclosed herein.

Disclosed herein are ungulates and ungulate cells that lack at least one allele of a functional region of an ungulate heavy chain, kappa light chain and/or lambda light chain locus produced according to the processes, sequences and/or constructs described herein, which are further modified to express at least part of a human antibody (i.e. immunoglobulin (Ig)) locus. Porcine animals are disclosed herein that express xenogenous immunoglobulin. This human locus can undergo rearrangement and
express a diverse population of human antibody molecules in the ungulate. These cloned, transgenic ungulates provide a replenishable, theoretically infinite supply of human antibodies (such as polyclonal antibodies), which can be used for therapeutic, diagnostic, purification, and other clinically relevant purposes. Artificial chromosomes (ACs), such as yeast or mammalian artificial chromosomes (YACS or MACS) can be used to allow expression of human immunoglobulin genes into ungulate cells and animals. All or part of human immunoglobulin genes, such as the Ig heavy chain gene (human chromosome 414), Ig kappa chain gene (human chromosome #2) and/or the Ig lambda chain gene (chromosome #22) can be inserted into the artificial chromosomes, which can then be inserted into ungulate cells. Ungulates and ungulate cells are disclosed herein that contain either part or all of at least one human antibody gene locus, which undergoes rearrangement and expresses a diverse population of human antibody molecules.

Methods of producing xenogenous antibodies are disclosed herein, wherein the method can include: (a) administering one or more antigens of interest to an ungulate whose cells comprise one or more artificial chromosomes and lack any expression of functional endogenous immunoglobulin, each artificial chromosome comprising one or more xenogenous immunoglobulin locus that undergoes rearrangement, resulting in production of xenogenous antibodies against the one or more antigens; and/ or (b) recovering the xenogenous antibodies from the ungulate. The immunoglobulin locus can undergo rearrangement in a B cell.

An ungulate, such as a pig or a cow, can be prepared by a method disclosed herein. These cloned, transgenic ungulates (e.g., porcine and bovine -animals) provide a replenishable, theoretically infinite supply of human polyclonal antibodies, which can be used as therapeutics, diagnostics and for purification purposes. For example, transgenic animals produced according to the process, sequences and/or constructs described herein that produce polyclonal human antibodies in the bloodstream can be used to produce an array of different antibodies which are specific to a desired antigen. The availability of large quantities of polyclonal antibodies can also be used for treatment and prophylaxis of infectious disease, vaccination against biological warfare agents, modulation of the immune system, removal of undesired human cells such as cancer cells, and modulation of specific human molecules.

Animals or cells lacking expression of functional immunoglobulin, produced according to the process, sequences and/or constructs described herein, can contain additional genetic modifications to eliminate the expression of xenoantigens. Such animals can be modified to elimate the expression of at least one allele of the alpha-1,3-galactosyltransferase gene, the CMP-Neu5Ac hydroxylase gene (see, for example, USSN 10/863,116), the iGb3 synthase gene (see, for example, U.S. Patent Application 60/517,524), and/or the Forssman synthase gene (see, for example, U.S. Patent Application 60/568,922). The animals disclosed herein can also contain genetic modifications to express fucosyltransferase and/ or sialyltransferase. To achieve these additional genetic modifications cells can be modified to contain multiple genetic modifications. Animals can be bred together to achieve multiple genetic modifications. Animals, such as pigs, lacking expression of functional immunoglobulin, produced according to the process, sequences and/or constructs described herein, can be bred with animals, such as pigs, lacking expression of alpha-1,3-galactosyl transferase (for example, as described in WO 04/028243).

### BRIEF DESCRIPTION OF THE DRAWINGS

**Figure 1** illustrates the design of a targeting vector that disrupts the expression of the joining region of the porcine heavy chain immunoglobulin gene.
**Figure 2** illustrates the design of a targeting vector that disrupts the expression of the constant region of the porcine kappa light chain immunoglobulin gene.
**Figure 3** illustrates the genomic organization of the porcine lambda immunoglobulin locus, including a concatamer of J-C sequences as well as flanking regions that include the variable region 5' to the JC region. Bacterial artificial chromosomes (BAC1 and BAC2) represent fragments of the porcine immunoglobulin genome that can be obtained from BAC libraries.
**Figure 4** represents the design of a targeting vector that disrupts the expression of the JC cluster region of the porcine lambda light chain immunoglobulin gene. "SM" stands for a selectable marker gene, which can be used in the targeting vector.
**Figure 5** illustrates a targeting strategy to insert a site specific recombinase target or recognition site into the region 5' of the JC cluster region of the porcine lambda immunoglobulin locus. "SM" stands for a selectable marker gene, which can be used in the targeting vector. "SSRRS" stands for a specific recombinase target or recognition site.
**Figure 6** illustrates a targeting strategy to insert a site specific recombinase target or recognition site into the region 3' of the JC cluster region of the porcine lambda immunoglobulin locus. "SM" stands for a selectable marker gene, which can be used in the targeting vector. "SSRRS" stands for a specific recombinase target or recognition site.
**Figure 7** illustrates the site specific recombinase mediated transfer of a YAC into a host genome. "SSRRS" stands for a specific recombinase target or recognition site.

### DETAILED DESCRIPTION

Disclosed herein for the first time are ungulate immunoglobulin germline gene sequence arrangement as well as novel genomic sequences thereof. In addition, novel ungulate cells, tissues and animals that lack expression of at least one allele of a heavy or light chain immunoglobulin gene are disclosed herein. Based on this discovery, ungulates can be produced that completely lack expression of at least one allele of a heavy and/or light chain immunoglobulin gene. In addition, these ungulates can be further modified to express xenogenous, such as human, immunoglobulin loci or fragments thereof.

A transgenic ungulate that lacks any expression of functional endogenous immunoglobulins is disclosed herein. The ungulate can lack any expression of endogenous heavy and/ or light chain immunoglobulins. The light chain immunoglobulin can be a kappa and/ or lambda immunoglobulin. Transgenic ungulates are disclosed herein that lack expression of at least one allele of an endogenous immunoglobulin wherein the immunoglobulin is selected from the group consisting of heavy chain, kappa light chain and lambda light chain or any combination thereof. The expression of functional endogenous immunoglobulins can be accomplished by genetic targeting of the endogenous immunoglobulin loci to prevent expression of the endogenous immunoglobulin. The genetic targeting can be accomplished via
homologous recombination. The transgenic ungulate can be produced via nuclear transfer.

The transgenic ungulate that lacks any expression of functional endogenous immunoglobulins can be further genetically modified to express a xenogenous immunoglobulin. Porcine animals are disclosed herein that contain an xenogenous immunoglobulin locus. In one embodiment, the xenogenous immunoglobulin locus can be a heavy and/ or light chain immunoglobulin or fragment thereof. In another embodiment, the xenogenous immunoglobulin locus can be a kappa chain locus or fragment thereof and/ or a lambda chain locus or fragment thereof. In still further embodiments, an artificial chromosome (AC) can contain the xenogenous immunoglobulin locus. In one embodiment, the AC can be a yeast AC or a mammalian AC. In a further embodiment, the xenogenous locus can be a human immunoglobulin locus or fragment thereof. In one embodiment, the human immunoglobulin locus can be human chromosome 14, human chromosome 2, and human chromosome 22 or fragments thereof. In another embodiment, the human immunoglobulin locus can include any fragment of a human immunoglobulin locus that can undergo rearrangement. In a further embodiment, the human immunoglobulin locus can include any fragment of a human immunoglobulin heavy chain locus and a human immunoglobulin light chain locus that can undergo rearrangement. In still further embodiment, the human immunoglobulin locus can include any human immunoglobulin locus or fragment thereof that can produce an antibody upon exposure to an antigen. In a particular embodiment, the exogenous human immunoglobulin can be expressed in B cells to produce xenogenous immunoglobulin in response to exposure to one or more antigens.

In an aspect of the present invention, transgenic porcine animals are provided that express a xenogenous immunoglobulin or fragment thereof, wherein the immunoglobulin can be expressed from an immunoglobulin locus that is integrated within an endogenous ungulate chromosome. In one embodiment, cells derived from the transgenic animals are provided. In one embodiment, the xenogenous immunoglobulin locus can be inherited by offspring. In another embodiment, the xenogenous immunoglobulin locus can be inherited through the male germ line by offspring. In still further embodiments, an artificial chromosome (AC) can contain the xenogenous immunoglobulin locus. In one embodiment, the AC can be a yeast AC or a mammalian AC. In a further embodiment, the xenogenous locus can be a human immunoglobulin locus or fragment thereof that can undergo rearrangement. In one embodiment, the human immunoglobulin locus can be human chromosome 14, human chromosome 2, and human chromosome 22 or fragments thereof. The human immunoglobulin locus can include any fragment of a human immunoglobulin locus that can undergo rearrangement. In a further embodiment, the human immunoglobulin locus can include any fragment of a human immunoglobulin heavy chain locus and a human immunoglobulin light chain locus that can undergo rearrangement. The human immunoglobulin locus can include any human immunoglobulin locus or fragment thereof that can produce an antibody upon exposure to an antigen. In a particular embodiment, the exogenous human immunoglobulin can be expressed in B cells to produce xenogenous immunoglobulin in response to exposure to one or more antigens.

### Definitions

The terms "recombinant DNA technology," "DNA cloning," "molecular cloning," or "gene cloning" refer to the process of transferring a DNA sequence into a cell or organism. The transfer of a DNA fragment can be from one organism to a self-replicating genetic element (e.g., bacterial plasmid) that permits a copy of any specific part of a DNA (or RNA) sequence to be selected among many others and produced in an unlimited amount. Plasmids and other types of cloning vectors such as artificial chromosomes can be used to copy genes and other pieces of chromosomes to generate enough identical material for further study. In addition to bacterial plasmids, which can carry up to 20 kb of foreign DNA, other cloning vectors include viruses, cosmids, and artificial chromosomes (e.g., bacteria artificial chromosomes (BACs) or yeast artificial chromosomes (YACs)). When the fragment of chromosomal DNA is ultimately joined with its cloning vector in the lab, it is called a "recombinant DNA molecule." Shortly after the recombinant plasmid is introduced into suitable -host cells, the newly inserted segment will be reproduced along with the host cell DNA.

"Cosmids" are artificially constructed cloning vectors that carry up to 45 kb of foreign DNA. They can be packaged in lambda phage particles for infection into *E. coli* cells.

As used herein, the term "mammal" (as in "genetically modified (or altered) mammal") is meant to include any non-human mammal, including but not limited to pigs, sheep, goats, cattle (bovine), deer, mules, horses, monkeys, dogs, cats, rats, mice, birds, chickens, reptiles, fish, and insects. In one embodiment of the invention, genetically altered pigs and methods of production thereof are provided.

The term "ungulate" refers to hoofed mammals. Artiodactyls are even-toed (cloven-hooved) ungulates, including antelopes, camels, cows, deer, goats, pigs, and sheep. Perissodactyls are odd toes ungulates, which include horses, zebras, rhinoceroses, and tapirs. The term ungulate as used herein refers to an adult, embryonic or fetal ungulate animal.

As used herein, the terms "porcine", "porcine animal", "pig" and "swine" are generic terms referring to the same type of animal without regard to gender, size, or breed.

A "homologous DNA sequence or homologous DNA" is a DNA sequence that is at least about 80%, 85%, 90%, 95%, 98% or 99% identical with a reference DNA sequence. A homologous sequence hybridizes under stringent conditions to the target sequence, stringent hybridization conditions include those that will allow hybridization occur if there is at least 85, at least 95% or 98% identity between the sequences.

An "isogenic or substantially isogenic DNA sequence" is a DNA sequence that is identical to or nearly identical to a reference DNA sequence. The term "substantially isogenic" refers to DNA that is at least about 97-99% identical with the reference DNA sequence, or at least about 99.5-99.9% identical with the reference DNA sequence, and in certain uses 100% identical with the reference DNA sequence.

"Homologous recombination" refers to the process of DNA recombination based on sequence homology.

"Gene targeting" refers to homologous recombination between two DNA sequences, one of which is located on a chromosome and the other of which is not.

"Non-homologous or random integration" refers to any process by which DNA is integrated into the genome that does not involve homologous recombination.

A "selectable marker gene" is a gene, the expression of which allows cells containing the gene to be identified. A selectable marker can be one that allows a cell to proliferate on a medium that prevents or slows the growth of cells without the gene. Examples include antibiotic resistance genes and genes which allow an organism to grow on a selected metabolite. Alternatively, the gene can facilitate visual screening of transformants by conferring on cells a phenotype that is easily identified. Such an identifiable phenotype can be, for example, the production of luminescence or the production of a colored compound, or the production of a detectable change in the medium surrounding the cell.

The term "contiguous" is used herein in its standard meaning, i.e., without interruption, or uninterrupted.

"Stringent conditions" refers to conditions that (1) employ low ionic strength and high temperature for washing, for example, 0.015 M NaC1/0.0015 M sodium citrate/0.1% SDS at 50°C, or (2) employ during hybridization a denaturing agent such as, for example, formamide. One skilled in the art can determine and vary the stringency conditions appropriately to obtain a clear and detectable hybridization signal. For example, stringency can generally be reduced by increasing the salt content present during hybridization and washing, reducing the temperature, or a combination thereof. See, for example, Sambrook et aL, Molecular Cloning: A Laboratory Manual, Cold Spring Harbour Laboratory Press, Cold Spring Harbour, New York, (1989).

### I. Immunoglobulin Genes

A transgenic ungulate that lacks any expression of functional endogenous immunoglobulins is disclosed herein. The ungulate can lack any expression of endogenous heavy and/ or light chain immunoglobulins. The light chain immunoglobulin can be a kappa and/ or lambda immunoglobulin. Transgenic ungulates are disclosed herein that lack expression of at least one allele of an endogenous immunoglobulin wherein the immunoglobulin is selected from the group consisting of heavy chain, kappa light chain and lambda light chain or any combination thereof. The expression of functional endogenous immunoglobulins can be accomplished by genetic targeting of the endogenous immunoglobulin locus to prevent expression of the endogenous immunoglobulin. The genetic targeting can be accomplished via homologous recombination. The transgenic ungulate can be produced via nuclear transfer.

Disclosed herein is a method to disrupt the expression of an ungulate immunoglobulin gene by (i) analyzing the germline configuration of the ungulate heavy chain, kappa light chain or lambda light chain genomic locus; (ii) determining the location of nucleotide sequences that flank the 5' end and the 3' end of at least one functional region of the locus; and (iii) transfecting a targeting construct containing the flanking sequence into a cell wherein, upon successful homologous recombination, at least one functional region of the
immunoglobulin locus is disrupted thereby reducing or preventing the expression of the immunoglobulin gene.

The germline configuration of the porcine heavy chain locus may be as disclosed herein. The porcine heavy chain locus contains at least four variable regions, two diversity regions, six joining regions and five constant regions, for example, as illustrated in Figure 1. Only one of the six joining regions, J6, may be functional.

The germline configuration of the porcine kappa light chain locus may be as disclosed herein. The porcine kappa light chain locus contains at least six variable regions, six joining regions, one constant region and one enhancer region, for example, as illustrated in Figure 2.

The germline configuration of the porcine lambda light chain locus may be as disclosed herein.

Isolated nucleotide sequences as depicted in Seq ID Nos 1-39 are disclosed herein. Nucleic acid sequences at least 80, 85, 90, 95, 98 or 99% homologous to any one of Seq ID Nos 1-39 are also disclosed herein. In addition, nucleotide sequences that contain at least 10, 15, 17, 20, 25 or 30 contiguous nucleotides of any one of Seq ID Nos 1- 39 are disclosed herein. Further disclosed are nucleotide sequences that hybridizes, optionally under stringent conditions, to Seq ID Nos 1-39, as well as, nucleotides homologous thereto.

Homology or identity at the nucleotide or amino acid sequence level can be determined by BLAST (Basic Local Alignment Search Tool) analysis using the algorithm employed by the programs blastp, blastn, blastx, tblastn and tblastx (see, for example, Altschul, S.F. et al (1 997) Nucleic Acids Res 25:3389-3402 and Karlin et al, (1 900) Proc. Natl. Acad. Sci. USA 87, 2264-2268) which are tailored for sequence similarity searching. The approach used by the BLAST program is to first consider similar-segments, with and without gaps, between a-query sequence and a database sequence, then to evaluate the statistical significance of all matches that are identified and finally to summarize only those matches which satisfy a preselected threshold of significance. See, for example, Altschul et aL, (1994) (Nature Genetics 6, 119-129). The search parameters for histogram, descriptions, alignments, expect (ie. , the statistical significance threshold for reporting matches against database sequences), cutoff, matrix and filter (low co M'plexity) are at the default settings. The default scoring matrix used by blastp, blastx, tblastn, and tblastx is the BLOSUM62 matrix (Henikoff et aL, (1 992) Proc. Natl. Acad. Sci. USA 89, 10915-10919), which is recommended for query sequences over 85 in length (nucleotide bases or amino acids).

### Porcine Heavy Chain

Novel genomic sequences encoding the heavy chain locus of ungulate immunoglobulin are disclosed herein. An isolated nucleotide sequence encoding porcine heavy chain is disclosed herein that includes at least one variable region, two diversity regions, at least four joining regions and at least one constant region, such as the mu constant region, for example, as represented in Seq ID No. 29. An isolated nucleotide sequence is also disclosed herein that includes at least four joining regions and at least one constant region, such as the mu constant region, of the porcine heavy chain genomic sequence, for example, as represented in Seq ID No. 4.Nucleotide sequence is disclosed herein that includes 5' flanking sequence to the first joining region of the porcine heavy chain genomic sequence, for example, as represented in Seq ID No 1. Still further, nucleotide sequence is disclosed herein that includes 3' flanking sequence to the first joining region of the porcine heavy chain genomic sequence, for example, as represented in the 3' region of Seq **ID** No 4. Isolated nucleotide sequences as depicted in Seq ID Nos 1, 4 or 29 are disclosed herein. Nucleic acid sequences at least 80, 85, 90, 95, 98 or 99% homologous to Seq ID Nos 1, 4 or 29 are also disclosed herein. Further disclosed herein are nucleotide sequences that hybridizes, optionally under stringent conditions, to Seq **ID** Nos 1, 4 or 29, as well as, nucleotides homologous thereto.

In addition, nucleotide sequences that contain at least 10, 15, 17, 20, 25 or 30 contiguous nucleotides of Seq ID Nos **1,4** or 29 are disclosed herein. The nucleotide sequence may contain at least 17, 20, 25 or 30 contiguous nucleotides of Seq **ID** No 4 or residues 1- 9,070 of Seq **ID** No 29.Nucleotide sequences that contain at least 50, 100, 1,000, 2,500, 4,000, 4,500, 5,000, 7,000, 8,000, 8,500, 9,000, 10,000 or 15,000 contiguous nucleotides of Seq ID No. 29 are disclosed herein. The nucleotide sequence may contain residues 9,070-11039 of Seq ID No 29.

Isolated nucleotide sequences as depicted in Seq ID Nos **1, 4** or 29 are disclosed herein. Nucleic acid sequences at least 80, 85, 90, 95, 98 or 99% homologous to Seq ID Nos **1, 4** or 29 are also disclosed herein. In addition, nucleotide sequences that contain at least 10,

15, 17, 20, 25 or 30 contiguous nucleotides of Seq ID Nos 1, 4 or 29 are disclosed herein. Further disclosed are nucleotide sequences that hybridizes, optionally under stringent conditions, to Seq ID Nos 1,4 or 29, as well as, nucleotides homologous thereto.

An isolated nucleotide sequence encoding porcine heavy chain is disclosed herein that includes at least one variable region, two diversity regions, at least four joining regions and at least one constant region, such as the mu constant region, for example, as represented in Seq ID No. 29. In Seq ID No. 29, the Diversity region of heavy chain is represented, for example, by residues 1089-1099 (D(pseudo)), the Joining region of heavy chain is represented, for example, by residues 1887-3352 (for example: J(psuedo): 1887-1931, J(psuedo): 2364-2411, J(psuedo): 2756-2804, J (functional J): 3296-3352), the recombination signals are represented, for example, by residues 3001-3261 (Nonamer), 3292-3298 (Heptamer), the Constant Region is represented by the following residues: 3353-9070 (J to C mu intron),
5522-8700 (Switch region), 9071-9388 (Mu Exon 1), 9389-9469 (Mu Intron A), 9470-9802 (Mu Exon 2), 9830- 10069 (Mu Intron B), 10070-10387 (Mu Exon 3), 10388-10517 (Mu Intron C), 10815-11052 (Mu Exon 4), 11034-11039 (Poly(A) signal).

| | |
|---|---|
| Seq ID No. 29 | |
| | |
| | |
| | |
| | |
| Seq ID No.1 | |
| | |
| Seq ID No.4 | |
| | |
| | |
| | |
| | |

### Porcine Kappa Light Chain

Novel genomic sequences encoding the kappa light chain locus of ungulate immunoglobulin are disclosed herein. Disclosed herein is the first reported genomic sequence of ungulate kappa light chain regions. Nucleic acid sequence is disclosed herein that encodes the porcine kappa light chain locus. The nucleic acid sequence can contain at least one joining region, one constant region and/or one enhancer region of kappa light chain locus. The nucleotide sequence can
include at least five joining regions, one constant region and one enhancer region, for example, as represented in Seq ID No. 30. An isolated nucleotide sequence is disclosed herein that contains at least one, at least two, at least three, at least four or five joining regions and 3' flanking sequence to the joining region of porcine genomic kappa light chain, for example, as represented in Seq ID No 12.An isolated nucleotide sequence of porcine genomic kappa light chain is disclosed herein that contains 5' flanking sequence to the first joining region, for example, as represented in Seq ID No. 25. An isolated nucleotide sequence is disclosed herein that contains 3' flanking sequence to the constant region and, optionally, the 5' portion of the enhancer region, of porcine genomic kappa light chain, for example, as represented in Seq 1D Nos. 15, 16 and/or 19.

Isolated nucleotide sequences as depicted in Seq ID Nos 30, 12, 25, 15, 16 or 19 are disclosed herein. Nucleic acid sequences at least 80, 85, 90, 95, 98 or 99% homologous to Seq ED Nos 30, 12, 25, 15, 16 or 19 are also disclosed herein. In addition, nucleotide sequences that contain at least 10, 15, 17, 20, 25 or 30 contiguous nucleotides of Seq ID Nos 30, 12, 25, 15, 16 or 19 are disclosed herein. In addition, nucleotide sequences that contain at least 10, 15, 17, 20, 25 or 30 contiguous nucleotides of Seq ID Nos 1, 4 or 29 are disclosed herein. Nucleotide sequences that contain at least 50, 100, 1,000, 2,500, 5,000, 7,000, 8,000, 8,500, 9,000, 10,000 or 15,000 contiguous nucleotides of Seq ID No. 30 are disclosed herein. Also disclosed herein are nucleotide sequences that hybridizes, optionally under stringent conditions, to Seq ID Nos 30, 12, 25, 15, 16 or 19, as well as, nucleotides homologous thereto.

An isolated nucleotide sequence encoding kappa light chain is disclosed herein that includes at least five joining regions, one constant region and one enhancer region, for example, as represented in Seq 1D No. 30. In Seq ID No. 30, the coding region of kappa light chain is represented, for example by residues 1-549 and 10026-10549, whereas the intronic sequence is represented, for example, by residues 550-10025, the Joining region of kappa light chain is represented, for example, by residues 5822- 7207 (for example, J1:5822-5859, J2:6180- 6218, J3:6486-6523, J4:6826-6863, J5:7170-7207), the Constant Region is represented by the following residues: 10026- 10549 (C exon) and 10026-10354 (C coding), 10524-10529 (Poly(A) signal) and 11160-11264 (SINE element).

| | |
|---|---|
| Seq ID No 30 | |
| | |
| | |
| | |
| | |

| | |
|---|---|
| | |
| Seq ID No.12 | |
| | |
| | |
| Seq ID No.15 | |
| | |
| | |
| Seq ID No.16 | |
| | |
| | |
| | |
| Seq ID No.19 | |
| | |
| Seq ID No.25 | |
| | |
| | |

### Porcine Lambda Light Chain

Novel genomic sequences encoding the lambda light chain locus of ungulate immunoglobulin are disclosed herein. Disclosed herein is the first reported genomic sequence of ungulate lambda light chain regions. The porcine lambda light chain nucleotides may include a concatamer of J to C units. An isolated porcine lambda nucleotide sequence is disclosed herein, such as that depicted in Seq ID No. 28.

Nucleotide sequence is disclosed herein that includes 5' flanking sequence to the first lambda J/C region of the porcine lambda light chain genomic sequence, for example, as represented by Seq ID No 32. Nucleotide sequence is disclosed herein that includes 3' flanking sequence to the J/C cluster region of the porcine lambda light chain genomic sequence, for example, approximately 200 base pairs downstream of lambda J/C, such as that represented by Seq ID No 33. Alternatively, nucleotide sequence is disclosed herein that includes 3' flanking sequence to the J/C cluster region of the porcine lambda light chain genomic sequence, for example, approximately 11.8 kb downstream of the J/C cluster, near the enhancer (such as that represented by Seq ID No. 34), approximately 12 Kb downstream of lambda, including the enhancer region (such as that represented by Seq ID No. 35), approximately 17.6 Kb downstream of lambda (such as that represented by Seq ID No. 36, approximately 19.1 Kb downstream of lambda (such as that represented by Seq ID No. 37), approximately 21.3 Kb downstream of lambda (such as that represented by Seq ID No. 38), and/or approximately 27 Kb downstream of lambda (such as that represented by Seq ID No. 39).

Isolated nucleotide sequences as depicted in Seq ID Nos 28, 31, 32, 33, 34, 35, 36, 37, 38, or 39 are disclosed herein. Nucleic acid sequences at least 80, 85, 90, 95, 98 or 99% homologous to Seq ID Nos 28, 31, 32, 33, 34, 35, 36, 37, 38, or 39 are also disclosed herein. In addition, nucleotide sequences that contain at least 10, 15, 17, 20, 25, 30, 40, 50, 75, 100, 150, 200, 250, 500 or 1,000 contiguous nucleotides of Seq ID Nos 28, 31, 32, 33, 34, 35, 36, 37, 38, or 39 are disclosed herein. Further disclosed are nucleotide sequences that hybridizes, optionally under stringent conditions, to Seq ID Nos 28, 31, 32, 33, 34, 35, 36, 37, 38, or 39, as well as, nucleotides homologous thereto.

| | |
|---|---|
| | |
| Seq ID No.28 | |
| | |
| | |

| | |
|---|---|
| Seq ID No.32 | |
| | |
| | |
| | |
| | |

| | |
|---|---|
| | |
| Seq ID No.33 | |
| | |
| | |

| | |
|---|---|
| | |
| Seq ID No.34 | |
| | |

| | |
|---|---|
| | |
| Seq ID No.35 | |

| | |
|---|---|
| | |
| Seq II7 No. 36 : | |

| | |
|---|---|
| | |
| Seq ID No. 37 : | |
| | |

| | |
|---|---|
| | |
| Seq ID No.38 | |
| | |
| | |

| | |
|---|---|
| Seq ID No.39 | |
| | |

### Bovine Lambda Light Chain

Nucleic acid sequences are disclosed herein that encode bovine lambda light chain locus, which can include at least one joining region-constant region pair and/or at least one variable region, for example, as represented by Seq ID No. 31. In Seq ID No 31, bovine lambda C can be found at residues 993-1333, a J to C pair can be found at the complement of residues 33848-35628 where C is the complement of 33848-34328 and J is the complement of 35599-35628, V regions can be found at (or in the complement of) residues 10676-10728, 11092-11446, 15088-15381. 25239-25528, 29784-30228, and 51718-52357. Seq ID No. 31 can be found in Genbank ACCESSION No. AC1 17274. Also disclosed are vectors and/or targetting constructs that contain all or part of Seq ID No. 31, for example at least 100, 250,500, 1000, 2000, 5000, 10000, 20000, 500000, 75000 or 100000 contiguouos nucleotides of Seq ID No. 31, as well as ceels and animals that contain a disrupted bovine lambda gene.

| | |
|---|---|
| Seq ID No 31 | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |

### Primers

Primers are disclosed herein to generate 3' and 5' sequences of a targeting vector. The oligonucleotide primers can be capable of hybridizing to porcine immunoglobulin genomic sequence, such as Seq ID Nos. 1, 4, 29, 30, 12, 25, 15, 16, 19, 28 or 31, as described above. The primers may hybridize under stringent conditions to Seq ID Nos. 1,4, 29, 30, 12, 25, 15, 16, 19, 28 or 31, as described above. Also disclosed herein are oligonucleotide probes capable of hybridizing to porcine heavy chain, kappa light chain or lambda light chain nucleic acid sequences, such as Seq ID Nos. 1, 4, 29, 30, 12, 25, 15, 16, 19, 28 or 31, as described above. The polynucleotide primers or probes can have at least 14 bases, 20 bases, 30 bases, or 50 bases which hybridize to a polynucleotide disclosed herein. The probe or primer can be at least 14 nucleotides in length, and maybe at least 15, 20, 25, 28, or 30 nucleotides in length.

Primers are disclosed herein to amplify a fragment of porcine Ig heavy-chain that includes the functional joining region (the J6 region). The amplified fragment of heavy chain can be represented by Seq ID No 4 and the primers used to amplify this fragment can be complementary to a portion of the J-region, such as, but not limited to Seq ID No 2, to produce the 5' recombination arm and complementary to a portion of Ig heavy-chain mu constant region, such as, but not limited to Seq ID No 3, to produce the 3' recombination arm. Regions of the porcine Ig heavy chain (such as, but not limited to Seq ID No 4) can be subcloned and assembled into a targeting vector.

Primers are disclosed herein to amplify a fragment of porcine Ig kappa light-chain that includes the constant region. Primers are disclosed herein to amplify a fragment of porcine Ig kappa light-chain that includes the J region. The primers used to amplify this fragment can be complementary to a portion of the J-region, such as, but not limited to Seq ID No 21 or 10, to produce the 5' recombination arm and complementary to genomic sequence 3' of the constant region, such as, but not limited to Seq ID No 14, 24 or 18, to produce the 3' recombination arm. Regions of the porcine Ig heavy chain (such as, but not limited to Seq ID No 20) can be subcloned and assembled into a targeting vector.

### II. Genetic Targeting of the Immunoglobulin Genes

The present disclosure provides porcine cells that have been genetically modified to inactivate immunoglobulin genes, for example, immunoglobulin genes described above. Animal cells that can be genetically modified can be obtained from a variety of different organs and tissues such as, but not limited to, skin, mesenchyme, lung, pancreas, heart, intestine, stomach, bladder, blood vessels, kidney, urethra, reproductive organs, and a disaggregated preparation of a whole or part of an embryo, fetus, or adult animal. The cells can be selected from the group consisting of, but not limited to, epithelial cells, fibroblast cells, neural cells, keratinocytes, hematopoietic cells, melanocytes, chondrocytes, lymphocytes (B and T), macrophages, monocytes, mononuclear cells, cardiac muscle cells, other muscle cells, granulosa cells, cumulus cells, epidermal cells, endothelial cells, Islets of Langerhans cells, blood cells, blood precursor cells, bone cells, bone precursor cells, neuronal stem cells, primordial stem cells, hepatocytes, keratinocytes, umbilical vein endothelial cells, aortic endothelial cells, microvascular endothelial cells, fibroblasts, liver stellate cells, aortic smooth muscle cells, cardiac myocytes, neurons, Kupffer cells, smooth muscle cells, Schwann cells, and epithelial cells, erythrocytes, platelets, neutrophils, lymphocytes, monocytes, eosinophils, basophils, adipocytes, chondrocytes, pancreatic islet cells, thyroid cells, parathyroid cells, parotid cells, tumor cells, glial cells, astrocytes, red blood cells, white blood cells, macrophages, epithelial cells, somatic cells, pituitary cells, adrenal cells, hair cells, bladder cells, kidney cells, retinal cells, rod cells, cone cells, heart cells, pacemaker cells, spleen cells, antigen presenting cells, memory cells, T cells, B cells, plasma cells, muscle cells, ovarian cells, uterine cells, prostate cells, vaginal epithelial cells, sperm cells, testicular cells, germ cells, egg cells, leydig cells, peritubular cells, sertoli cells, lutein cells, cervical cells, endometrial cells, mammary cells, follicle cells, mucous cells, ciliated cells, nonkeratinized epithelial cells, keratinized epithelial cells, lung cells, goblet cells, columnar epithelial cells, squamous epithelial cells, osteocytes, osteoblasts, and osteoclasts. Alternatively, embryonic stem cells can be used. An embryonic stem cell line can be employed or embryonic stem cells can be obtained freshly from a host, such as a porcine animal. The cells can be grown on an appropriate fibroblast-feeder layer or grown in the presence of leukemia inhibiting factor (LIF).

In particular the cells can be fibroblasts; specifically the cells can be fetal fibroblasts. Fibroblast cells are a suitable somatic cell type because they can be obtained from developing fetuses and adult animals in large quantities. These cells can be easily propagated in vitro with a rapid doubling time and can be clonally propagated for use in gene targeting procedures.

### Targeting constructs

### Homologous Recombination

The immunoglobulin genes can be genetically targeted in cells through homologous recombination. Homologous recombination permits site-specific modifications in endogenous genes and thus novel alterations can be engineered into the genome. In homologous recombination, the incoming DNA interacts with and integrates into a site in the genome that contains a substantially homologous DNA sequence. In non-homologous ("random" or "illicit") integration, the incoming DNA is not found at a homologous sequence in the genome but integrates elsewhere, at one of a large number of potential locations. In general, studies with higher eukaryotic cells have revealed that the frequency of homologous recombination is far less than the frequency of random integration. The ratio of these frequencies has direct implications for "gene targeting" which depends on integration via homologous recombination (i.e. recombination between the exogenous "targeting DNA" and the corresponding "target DNA" in the genome).

A number of papers describe the use of homologous recombination in mammalian cells. Illustrative of these papers are Kucherlapati et al., Proc. Natl. Acad. Sci. USA 81:3153-3157, 1984; Kucherlapati et al., Mol. Cell. Bio. 5:714-720, 1985; Smithies et al, Nature 317:230-234, 1985; Wake et al., Mol. Cell. Bio. 8:2080-2089, 1985; Ayares et al., Genetics 111:375-388, 1985; Ayares et al., Mol. Cell. Bio. 7:1656-1662, 1986; Song et al., Proc. Natl. Acad. Sci. USA 84:6820-6824, 1987; Thomas et al. Cell 44:419-428, 1986; Thomas and Capecchi, Cell 51: 503-512, 1987; Nandi et al., Proc. Natl. Acad. Sci. USA 85:3845-3849, 1988; and Mansour et al., Nature 336:348-352, 1988. Evans and Kaufman, Nature 294:146-154, 1981; Doetschman et al., Nature 330:576-578, 1987; Thoma and Capecchi, Cell 51:503-512,4987; Thompson et al., Cell 56:316-321, 1989. Homologous recombination can be used to inactivate an immunoglobulin gene in cells, such as the cells described above. The DNA can comprise at least a portion of the gene(s) at the particular locus with introduction of an alteration into at least one, optionally both copies, of the native gene(s), so as to prevent expression of functional immunoglobulin. The alteration can be an insertion, deletion, replacement or combination thereof. When the alteration is introduce into only one copy of the gene being inactivated, the cells having a single unmutated copy of the target gene are amplified and can be subjected to a second targeting step, where the alteration can be the same or different from the first alteration, usually different, and where a deletion, or replacement is involved, can be overlapping at least a portion of the alteration originally introduced. In this second targeting step, a targeting vector with the same arms of homology, but containing a different mammalian selectable markers can be used. The resulting transforrnants are screened for the absence of a functional target antigen and the DNA of the cell can be further screened to ensure the absence of a wild-type target gene. Alternatively, homozygosity as to a phenotype can be achieved by breeding hosts heterozygous for the mutation.

### Targeting Vectors

Disclosed herein are nucleic acid targeting vector constructs. The targeting vectors can be designed to accomplish homologous recombination in cells. These targeting vectors can be transformed into mammalian cells to target the ungulate heavy chain, kappa light chain or lambda light chain genes via homologous recombination. The targeting vectors can contain a 3' recombination arm and a 5' recombination arm (i.e. flanking sequence) that is homologous to the genomic sequence of ungulate heavy chain, kappa light chain or lambda light chain genomic sequence, for example, sequence represented by Seq BD Nos. 1, 4, 29, 30, 12, 25, 15, 16, 19, 28 or 31, as described above. The homologous DNA sequence can include at least 15 bp, 20 bp, 25 bp, 50 bp, 100 bp, 500 bp, lkbp, 2 kbp, 4 kbp, 5 kbp, 10 kbp, 15 kbp, 20 kbp, or 50 kbp of sequence, particularly contiguous sequence, homologous to the genomic sequence. The 3' and 5' recombination arms
can be designed such that they flank the 3' and 5' ends of at least one functional variable, joining, diversity, and/or constant region of the genomic sequence. The targeting of a functional region can render it inactive, which results in the inability of the cell to produce functional immunoglobulin molecules. The homologous DNA sequence can include one or more intron and/or exon sequences. In addition to the nucleic acid sequences, the expression vector can contain selectable marker sequences, such as, for example, enhanced Green Fluorescent Protein (eGFP) gene sequences, initiation and/or enhancer sequences, poly A-tail sequences, and/or nucleic acid sequences that provide for the expression of the construct in prokaryotic and/or eukaryotic host cells. The selectable marker can be located between the 5' and 3' recombination arm sequence.

Modification of a targeted locus of a cell can be produced by introducing DNA into the cells, where the DNA has homology to the target locus and includes a marker gene, allowing for selection of cells comprising the integrated construct. The homologous DNA in the target vector will recombine with the chromosomal DNA at the target locus. The marker gene can be flanked on both sides by homologous DNA sequences, a 3' recombination arm and a 5' recombination arm. Methods for the construction of targeting vectors have been described in the art, see, for example, Dai et al., Nature Biotechnology 20: 251-255, 2002; WO 00/51424.

Various constructs can be prepared for homologous recombination at a target locus. The construct can include at least 50 bp, 100 bp, 500 bp, 1 kbp, 2 kbp, 4 kbp, 5 kbp, 10 kbp, 15 kbp, 20 kbp, or 50 kbp of sequence homologous with the target locus. The sequence can include any contiguous sequence of an immunoglobulin gene.

Various considerations can be involved in determining the extent of homology of target DNA sequences, such as, for example, the size of the target locus, availability of sequences, relative efficiency of double cross-over events at the target locus and the similarity of the target sequence with other sequences.

The targeting DNA can include a sequence in which DNA substantially isogenic flanks the desired sequence modifications with a corresponding target sequence in the genome to be modified. The substantially isogenic sequence can be at least about 95%, 97-98%, 99.0-99.5%, 99.6-99.9%, or 100% identical to the corresponding target sequence (except for the desired sequence modifications). The targeting DNA and the target DNA can share stretches of DNA at least about 75, 150 or 500 base pairs that are 100% identical.
Accordingly, targeting DNA can be derived from cells closely related to the cell line being targeted; or the targeting DNA can be derived from cells of the same cell line or animal as the cells being targeted.

### Porcine Heavy Chain Targeting

Targeting vectors are disclosed herein to target the porcine heavy chain locus. The targeting vector can contain 5' and 3' recombination arms that contain homologous sequence to the 3' and 5' flanking sequence of the J6 region of the porcine immunoglobulin heavy chain locus. Since the J6 region is the only functional joining region of the porcine immunoglobulin heavy chain locus, this will prevent the expression of a functional porcine heavy chain immunoglobulin. The targeting vector can contain a 5' recombination arm that contains sequence homologous to genomic sequence 5' of the J6 region, optionally including J1-4 and a 3' recombination arm that contains sequence homologous to genomic sequence 3' of the J6 region, including the mu constant region (a "J6 targeting construct"), see for example, Figure 1. Further, this J6 targeting construct can also contain a selectable marker gene that is located between the 5' and 3' recombination arms, see for example, Seq ID No 5 and Figure 1.The 5' targeting arm can contain sequence 5' of J1, such as depicted in Seq ID No. 1 and/ or Seq ID No 4. The 5' targeting arm can contain sequence 5' of J1, J2 and/ or J3, for example, as depicted in approximately residues 1-300, 1-500, 1-750, 1-1000 and/ or 1-1500 Seq ID No 4.The 5' targeting arm can contain sequence 5' of the constant region, for example, as depicted in approximately residues 1-300, 1-500, 1- 750, 1-1000, 1-1500 and/ or 1-2000 or any fragment thereof of Seq ID No 4 and/ or any contiguous sequence of Seq ID No. 4 or fragment thereof. The 3' targeting arm can contain sequence 3' of the constant region and/ or including the constant region, for example, such as resides 7000-8000 and/ or 8000-9000 or fragment thereof of Seq ID No 4. Targeting vector can contain any contiguous sequence or fragment thereof of Seq ID No 4. Sequence. The targeting vector can contain a 5' recombination arm that contains sequence homologous to genomic sequence 5' of the diversity region, and a 3' recombination arm that contains sequence homologous to genomic sequence 3' of the diversity region of the porcine heavy chain locus. The targeting
vector can contain a 5' recombination arm that contains sequence homologous to genomic sequence 5' of the mu constant region and a 3' recombination arm that contains sequence homologous to genomic sequence 3' of the mu constant region of the porcine heavy chain locus.

The targeting vector can include, but is not limited to any of the following sequences: the Diversity region of heavy chain is represented, for example, by residues 1089-1099 of Seq ID No 29 (D(pseudo)), the Joining region of heavy chain is represented, for example, by residues 1887-3352 of Seq ID No 29 (for example: J(psuedo): 1887-1931 of Seq ID No 29, J(psuedo): 2364-2411 of Seq ID No 29, J(pseudo) 2756-2804 of Seq ID No 29, J (functional J): 3296-3352 of Seq ID No 29), the recombination signals are represented, for example, by residues 3001-3261 of Seq ID No 29 (Nonamer), 3292-3298 of Seq ID No 29 (Heptarner), the Constant Region is represented by the following residues: 3353-9070 of Seq ID No 29 (J to C mu intron), 5522-8700 of Seq ID No 29 (Switch region), 9071-9388 of Seq ID No 29 (Mu Exon 1), 9389-9469 of Seq ID No 29 (Mu Intron A), 9470-9802 of Seq ID No 29 (Mu Exon 2), 9830- 10069 of Seq ID No 29 (Mu Intron B), 10070-10387 of Seq ID No 29 (Mu Exon 3), 10388-10517 of Seq ID No 29 (Mu Intron C), 10815-11052 of Seq ID No 29 (Mu Exon 4), 11034-11039 of Seq ID No 29 (Poly(A) signal) or any fragment or combination thereof. Still further, any contiguous sequence at least about 17, 20, 30, 40, 50, 100, 150, 200 or 300 nucleotides of Seq ID No 29 or fragment and/ or combination thereof can be used as targeting sequence for the heavy chain targeting vector. It is understood that in general when designing a targeting construct one targeting arm will be 5' of the other targeting arm.

Targeting vectors designed to disrupt the expression of porcine heavy chain genes can contain recombination arms, for example, the 3' or 5' recombination arm, that target the constant region of heavy chain. The recombination arm can target the mu constant region, for example, the C mu sequences described above or as disclosed in Sun & Butler Immunogenetics (1997) 46: 452-460. The recombination arm can target the delta constant region, such as the sequence disclosed in Zhao et al. (2003) J imunol 171: 1312-1318, or the alpha constant region, such as the sequence disclosed in Brown & Butler (1994) Molec Immunol 31: 633-642.

| | |
|---|---|
| Seq ID No.5 | |
| | |
| | |
| | |
| | |

### Porcine Kappa Chain Targeting

Targeting vectors are disclosed herein to target the porcine kappa chain locus. The targeting vector can contain 5' and 3' recombination arms that contain homologous sequence to the 3' and 5' flanking sequence of the constant region of the porcine immunoglobulin kappa chain locus. Since the inventors discovered that there is only one constant region of the porcine immunoglobulin kappa light chain locus, this will prevent the expression of a functional porcine kappa light chain immunoglobulin. The targeting vector can contain a 5' recombination arm that contains sequence homologous to genomic sequence 5' of the constant region, optionally including the joining region, and a 3' recombination arm that contains sequence homologous to genomic sequence 3' of the constant region, optionally including at least part of the enhancer region (a "Kappa constant targeting construct"), see for example, Figure 2. Further, this kappa constant targeting construct can also contain a selectable marker gene that is located between the 5' and 3' recombination arms, see for example, Seq ID No 20 and Figure 2. The targeting vector can contain a 5' recombination arm that contains sequence homologous to genomic sequence 5' of the joining region, and a 3' recombination arm that contains sequence homologous to genomic sequence 3' of the joining region of the porcine kappa light chain locus. The 5' arm of the targeting vector can include Seq ID No 12 and/ or Seq ID No 25 or any contiguous sequence or fragment thereof. The 3' arm of the targeting vector can include Seq ID No 15, 16 and/ or 19 or any contiguous sequence or fragment thereof.

The targeting vector can include, but is not limited to any of the following sequences: the coding region of kappa light chain is represented, for example by residues 1-549 of Seq ID No 30 and 10026-10549 of Seq ID No 30, whereas the intronic sequence is represented, for example, by residues 550-10025 of Seq ID No 30, the Joining region of kappa light chain is represented, for example, by residues 5822- 7207 of Seq ID No 30 (for example, J1:5822-5859 of Seq ID No 30, J2:6180-6218 of Seq ID No 30, J3:6486-6523 of Seq ID No 30, J4:6826-6863 of Seq ID No 30, J5:7170-7207 of Seq ID No 30), the Constant Region is represented by the following residues: 10026- 10549 of Seq ID No 30 (C exon) and 10026-10354 of Seq ID No 30 (C coding), 10524-10529 of Seq ID No 30 (Poly(A) signal) and 11160-11264 of Seq ID No 30 (SINE element) or any fragment or combination thereof. Still further, any contiguous sequence at least about 17, 20, 30, 40, 50, 100, 150, 200 or 300 nucleotides of Seq ID No 30 or fragment and/ or combination thereof can be used as targeting sequence for the heavy chain targeting vector. It is understood that in general when designing a targeting construct one targeting arm will be 5' of the other targeting arm.

| | |
|---|---|
| Seq ID No.20 | |
| | |
| | |
| | |

### Porcine Lambda Chain Targeting

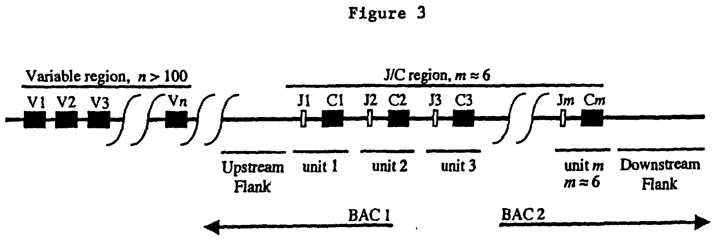

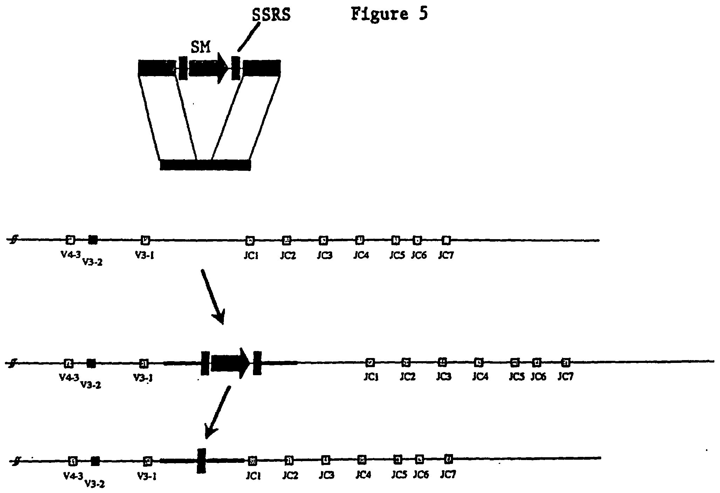

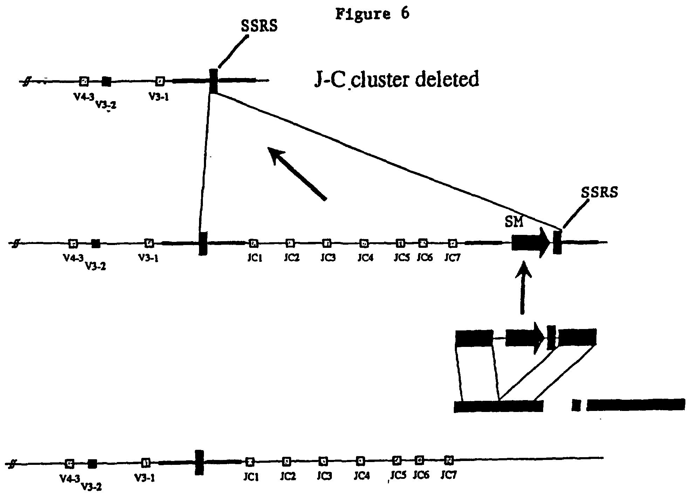

Targeting vectors are disclosed herein to target the porcine heavy chain locus. Lambda can be targeted by designing a targeting construct that contains a 5' arm containing sequence located 5' to the first JC cluster and a 3' arm containing sequence 3' to the last JC cluster, thus preventing functional expression of the lambda locus (see, Figures 3-4). The targeting vector can contain any contiguous sequence (such as about 17, 20, 30, 40, 50, 75, 100, 200, 300 or 5000 nucleotides of contiguous sequence) or fragment thereof Seq ID No 28. The 5' targeting arm can contain Seq ID No. 32, which includes 5' flanking sequence to the first lambda J/C region of the porcine lambda light chain genomic sequence or any contiguous sequence (such as about 17, 20, 30, 40, 50, 75, 100, 200, 300 or 5000 nucleotides of contiguous sequence) or fragment thereof (see also, for example Figure 5). The 3' targeting arm can contain, but is not limited to one or more of the following: Seq ID No. 33, which includes 3' flanking sequence to the J/C cluster region of the porcine lambda light chain genomic sequence, from approximately 200 base pairs downstream of lambda J/C; Seq ID No. 34, which includes 3' flanking sequence to the J/C cluster region of the porcine lambda light chain genomic sequence, approximately 11.8 Kb downstream of the J/C cluster, near the enhancer; Seq II) No. 35, which includes approximately 12 Kb downstream of lambda, including the enhancer region; Seq ID No 36, which includes approximately 17.6 Kb downstream of lambda; Seq ID No. 37, which includes approximately 19.1 Kb downstream of lambda; Seq ID No. 38, which includes approximately 21.3 Kb downstream of lambda; and Seq ID No. 39, which includes approximately 27 Kb downstream of lambda, or any contiguous sequence (such as about 17, 20, 30, 40, 50, 75, 100, 200, 300 or 5000 nucleotides of contiguous sequence) or fragment thereof of Seq ID Nos 32-39 (see also, for example Figure 6). It is understood that in general when designing a targeting construct one targeting arm will be 5' of the other targeting arm.

The targeting constructs for the lambda locus can contain site specific recombinase sites, such as, for example, lox. The targeting arms can insert the site specific recombinase site into the targeted region. Then, the site specific recombinase can be activated and/ or applied to the cell such that the intervening nucleotide sequence between the two site specific recombinase sites is excised (see, for example, Figure 6).

### Selectable Marker Genes

The DNA constructs can be designed to modify the endogenous, target immunoglobulin gene. The homologous sequence for targeting the construct can have one or more deletions, insertions, substitutions or combinations thereof. The alteration can be the insertion of a selectable marker gene fused in reading frame with the upstream sequence of the target gene.

Suitable selectable marker genes include, but are not limited to: genes conferring the ability to grow on certain media substrates, such as the tk gene (thymidine kinase)
or the hprt gene (hypoxanthine phosphoribosyltransferase) which confer the ability to grow on HAT medium (hypoxanthine, aminopterin and thymidine); the bacterial gpt gene (guanine/xanthine phosphoribosyltransferase) which allows growth on MAX medium (mycophenolic acid, adenine, and xanthine). See, for example, Song, K-Y., et al. Proc. Nat'l Acad. Sci. U.S.A. 84:6820-6824 (1987); Sambrook, J., et al., Molecular Cloning--A Laboratory Manual, Cold Spring Harbor Laboratory, Cold Spring Harbor, N.Y. (1989), Chapter 16. Other examples of selectable markers include: genes conferring resistance to compounds such as antibiotics, genes conferring the ability to grow on selected substrates, genes encoding proteins that produce detectable signals such as luminescence, such as green fluorescent protein, enhanced green fluorescent protein (eGFP). A wide variety of such markers are known and available, including, for example, antibiotic resistance genes such as the neomycin resistance gene (neo) (Southern, P., and P. Berg, J. Mol. Appl. Genet. 1:327-341 (1982)); and the hygromycin resistance gene (hyg) (Nucleic Acids Research 11:6895-6911 (1983), and Te Riele, H., et al., Nature 348:649-651 (1990)). Other selectable marker genes include: acetohydroxyacid synthase (AHAS), alkaline phosphatase (AP), beta galactosidase (LacZ), beta glucoronidase (GUS), chloramphenicol acetyltransferase (CAT), green fluorescent protein (GFP), red fluorescent protein (RFP), yellow fluorescent protein (YFP), cyan fluorescent protein (CFP), horseradish peroxidase (HRP), luciferase (Luc), nopaline synthase (NOS), octopine synthase (OCS), and derivatives thereof. Multiple selectable markers are available that confer resistance to ampicillin, bleomycin, chloramphenicol, gentamycin, hygromycin, kanamycin, lincomycin, methotrexate, phosphinothricin, puromycin, and tetracycline.

Methods for the incorporation of antibiotic resistance genes and negative selection factors will be familiar to those of ordinary skill in the art (see, e.g., WO 99/15650; U.S. Patent No. 6,080,576; U.S. Patent No. 6,136,566; Niwa et al., J. Biochem. 113:343-349 (1993); and Yoshida et al., Transgenic Research 4:277-287 (1995)).

Combinations of selectable markers can also be used. For example, to target an immunoglobulin gene, a neo gene (with or without its own promoter, as discussed above) can be cloned into a DNA sequence which is homologous to the immunoglobulin gene. To use a combination of markers, the HSV-tk gene can be cloned such that it is outside of the targeting DNA (another selectable marker could be placed on the opposite flank, if desired). After introducing the DNA construct into the cells to be targeted, the cells can be selected on the appropriate antibiotics. In this particular example, those cells which are resistant to G418 and gancyclovir are most likely to have arisen by homologous recombination in which the neo gene has been recombined into the immunoglobulin gene but the tk gene has been lost because it was located outside the region of the double crossover.

Deletions can be at least about 50 bp, more usually at least about 100 bp, and generally not more than about 20 kbp, where the deletion can normally include at least a portion of the coding region including a portion of or one or more exons, a portion of or one or more introns, and can or can not include a portion of the flanking non-coding regions, particularly the 5'-non-coding region (transcriptional regulatory region). Thus, the homologous region can extend beyond the coding region into the 5'-non-coding region or alternatively into the 3'-non-coding region. Insertions can generally not exceed 10 kbp, usually not exceed 5 kbp, generally being at least 50 bp, more usually at least 200 bp.

The region(s) of homology can include mutations, where mutations can further inactivate the target gene, in providing for a frame shift, or changing a key amino acid, or the mutation can correct a dysfunctional allele, etc. The mutation can be a subtle change, not exceeding about 5% of the homologous flanking sequences. Where mutation of a gene is desired, the marker gene can be inserted into an intron or an exon.

The construct can be prepared in accordance with methods known in the art, various fragments can be brought together, introduced into appropriate vectors, cloned, analyzed and then manipulated further until the desired construct has been achieved. Various modifications can be made to the sequence, to allow for restriction analysis, excision, identification of probes, etc. Silent mutations can be introduced, as desired. At various stages, restriction analysis, sequencing, amplification with the polymerase chain reaction, primer repair, in vitro mutagenesis, etc. can be employed.

The construct can be prepared using a bacterial vector, including a prokaryotic replication system, e.g. an origin recognizable by E. coli, at each stage the construct can be cloned and analyzed. A marker, the same as or different from the marker to be used for insertion, can be employed, which can be removed prior to introduction into the target cell. Once the vector containing the construct has been completed, it can be further manipulated, such as by deletion of the bacterial sequences, linearization, introducing a short deletion in the homologous sequence. After final manipulation, the construct can be introduced into the cell.

Also disclosed herein are recombinant constructs containing sequences of immunoglobulin genes. The constructs comprise a vector, such as a plasmid or viral vector, into which a sequence disclosed herein has been inserted, in a forward or reverse orientation. The construct can also include regulatory sequences, including, for example, a promoter, operably linked to the sequence. Large numbers of suitable vectors and promoters are known to those of skill in the art, and are commercially available. The following vectors are provided by way of example. Bacterial: pBs, pQE-9 (Qiagen), phagescript, PsiX174, pBluescript SK, pBsKS, pNH8a, pNH16a, pNH18a, pNH46a (Stratagene); pTrc99A, pKIC223-3, pKK233-3, pDR540, pRIT5 (Pharmacia). Eukaryotic: pWLneo, pSv2cat, p0G44, pXT1, pSG (Stratagene) pSVK3, pBPv, pMSG, pSVL (Pharmiacia), viral origin vectors (M13 vectors, bacterial phage 1 vectors, adenovirus vectors, and retrovirus vectors), high, low and adjustable copy number vectors, vectors which have compatible replicons for use in combination in a single host (pACYC184 and pBR322) and eukaryotic episomal replication vectors (pCDM8). Other vectors include prokaryotic expression vectors such as pcDNA H, pSL301, pSE280, pSE380, pSE420, pTrcHisA, B, and C, pRSET A, B, and C (Invitrogen, Corp.), pGEMEX-1, and pGEMEX-2 (Promega, Inc.), the pET vectors (Novagen, Inc.), pTrc99A, pKK223-3, the pGEX vectors, pEZZ18, pRIT2T, and pMC1871 (Pharmacia, Inc.), pKK233-2 and pKK388-1 (Clontech, Inc.), and pProEx-HT (Invitrogen, Corp.) and variants and derivatives thereof. Other vectors include eucaryotic expression vectors such as pFastBac, pFastBacHT, pFastBacDUAL, pSFV, and pTet-Splice (Invitrogen), pEUK-C1, pPUR, pMAM, pMAMneo, . pBI101, pBI121, pDR2, pCMVEBNA, and pYACneo (Clontech), pSVK3, pSVL, pMSG, pCH110, and pKI(232-8 (Pharmacia, Inc.), p3'SS, pXT1, pSG5, pPbac, pMbac, pMClneo, and p0G44 (Stratagene, Inc.), and pYES2, pAC360, pBlueBacHis A, B, and C, pVL1392, pBlueBacIII, pCDM8, pcDNA1, pZeoSV, pcDNA3 pREP4, pCEP4, and pEBVHis (Invitrogen, Corp.) and variants or derivatives thereof. Additional vectors that can be used include: pUC18, pUC19, pBlueScript, pSPORT, cosmids, phagemids, YAC's (yeast artificial chromosomes), BAC's (bacterial artificial chromosomes), P1 (Escherichia coli phage), pQE70, pQE60, pQE9 (quagan), pBS vectors, PhageScript vectors, BlueScript vectors, pNH8A, pNH16A, pNH18A, pNH46A (Stratagene), pcDNA3 (Invitrogen), pGEX, pTrsfus, pTrc99A, pET-5, pET-9, pKIC223-3, pKK233-3, pDR540, pRIT5 (Pharmacia), pSPORT1, pSPORT2, pCMVSPORT2.0 and pSV-SPORT1 (Invitrogen), pTrxFus, pThioHis, pLEX, pTrcHis, pTrcHis2, pRSET, pBlueBacHis2, pcDNA3.1/His, pcDNA3.1(-)/Myc-His, pSecTag, pEBVHis, pPIC9K, pPIC3.5K, pAO815, pPICZ, pPICZ□, pGAPZ, pGAPZ□, pBlueBac4.5, pBlueBacHis2, pMelBac, pSinRep5, pSinHis, pIND, pIND(SP1), pVgRXR, pcDNA2.1, pYES2, pZErO1.1, pZErO-2.1, pCR-Blunt, pSE280, pSE380, pSE420, pVL1392, pVL1393, pCDM8, pcDNA1.1, pcDNA1.1/Amp, pcDNA3.1, pcDNA3.1/Zeo, pSe, SV2, pRc/CMV2, pRc/RSV, pREP4, pREP7, pREP8, pREP9, pREP 10, pCEP4, pEBVHis, pCR3.1, pCR2.1, pCR3.1-Uni, and pCRBac from Invitrogen; 0 ExCell, □ gt11, pTrc99A, pKK223-3, pGEX-1□T, pGEX-2T, pGEX-2TK, pGEX-4T-1, pGEX-4T-2, pGEX-4T-3, pGEX-3X, pGEX-5X-1, pGEX-5X-2, pGEX-5X-3, pEZZ18, pRIT2T, pMC1871, pSVK3, pSVL, pMSG, pCH110, pKK232-8, pSL1180, pNEO, and pUC4K from Pharmacia; pSCREEN-1b(+), pT7Blue(R), pT7Blue-2, pCITE-4abc(+), pOCUS-2, pTAg, pET-32LIC, pET-30LIC, pBAC-2cp LIC, pBACgus-2cp LIC, pT7Blue-2 LIC, pT7Blue-2, □SCREEN-1, □BlueSTAR, pET-3abcd, pET-7abc, pET9abcd, pET11abcd, pET12abc, pET-14b, pET-15b, pET-16b, pET-17b-pET-17xb, pET-19b, pET-20b(+), pET-21abcd(+), pET-22b(+), pET-23abcd(+), pET-24abcd(+), pET-25b(+), pET-26b(+), pET-27b(+), pET-28abc(+), pET-29abc(+), pET-30abc(+), pET-31b(+), pET-32abc(+), pET-33b(+), pBAC-1, pBACgus-1, pBAC4x-1, pBACgus4x-1, pBAC-3cp, pBACgus-2cp, pBACsurf-1, plg, Signal plg, pYX, Selecta Vecta-Neo, Selecta Vecta-Hyg, and Selecta Vecta-Gpt from Novagen; pLexA, pB42AD, pGBT9, pAS2-1, pGAD424, pACT2, pGAD GL, pGAD GH, pGAD10, pGilda, pEZM3, pEGFP, pEGFP-1, pEGFP-N, pEGFP-C, pEBFP, pGFPuv, pGFP, p6xHis-GFP, pSEAP2-Basic, pSEAP2-Contral, pSEAP2-Promoter, pSEAP2-Enhancer, p□gal-Basic, p□gal-Control, p□gal-Promoter, p□gal-Enhancer, pCMV□, pTet-Off, pTet-On, pTK-Hyg, pRetro-Off, pRetro-On, pIRES1neo, pIRES1hyg, pLXSN, pLNCX, pLAPSN, pMAMneo, pMAMneo-CAT, pMAMneo-LUC, pPUR, pSV2neo, pYEX4T-1/2/3, pYEX-S1, pBacPAK-His, pBacPAK8/9, pAcUW31, BacPAK6, pTriplEx, □gt10, □gt11, pWE15, and □TriplEx from Clontech; Lambda ZAP II, pBK-CMV, pBK-RSV, pBluescript II KS +/-, pBluescript II SK +/-, pAD-GAL4, pBD-GAL4 Cam, pSurfscript, Lambda FIX II, Lambda DASH, Lambda EMBL3, Lambda EMBL4, SuperCos, pCR-Scrigt Amp, pCR-Script Cam, pCR-Script Direct, pBS +/-, pBC KS +/-, pBC SK +/-, Phagescript, pCAL-n-EK, pCAL-n, pCAL-c, pCAL-kc, pET-3abcd, pET-11abcd, pSPUTK, pESP-1, pCMVLacI, pOPRSVI/MCS, pOPI3 CAT,pXT1, pSG5, pPbac, pMbac, pMC1neo, pMC1neo Poly A, pOG44, pOG45, pFRT□GAL, pNEO□GAL, pRS403, pRS404, pRS405, pRS406, pRS413, pRS414, pRS415, and pRS416 from Stratagene and variants or derivatives thereof. Two-hybrid and reverse two-hybrid vectors can also be used, for example, pPC86, pDBLeu, pDBTrp, pPC97, p2.5, pGAD1-3, pGAD10, pACt, pACT2, pGADGL, pGADGH, pAS2-1, pGAD424, pGBT8, pGBT9, pGAD-GAL4, pLexA, pBD-GAL4, pHISi, pHISi-1, placZi, pB42AD, pDG202, pJK202, pJG4-5, pNLexA, pYESTrp and variants or derivatives thereof. Any other plasmids and vectors may be used as long as they are replicable and viable in the host.

Techniques which can be used to allow the DNA construct entry into the host cell include, for example, calcium phosphate/DNA co precipitation, microinjection of DNA into the nucleus, electroporation, bacterial protoplast fusion with intact cells, transfection, or any other technique known by one skilled in the art. The DNA can be single or double stranded, linear or circular, relaxed or supercoiled DNA. For various techniques for transfecting mammalian cells, see, for example, Keown et al., Methods in Enzymology Vol. 185, pp. 527-537 (1990).

Heterozygous or homozygous knockout cells can be produced by transfection of primary fetal fibroblasts with a knockout vector containing immunoglobulin gene sequence isolated from isogenic DNA. The vector can incorporate a promoter trap strategy, using, for example, IRES (internal ribosome entry site) to initiate translation of the Neor gene.

### Site Specific Recombinases

The targeting constructs can contain site specific recombinase sites, such as, for example, lox. The targeting arms can insert the site specific recombinase target sites into the targeted region such that one site specific recombinase target site is located 5' to the second site specific recombinase target site. Then, the site specific recombinase can be activated and/ or applied to the cell such that the intervening nucleotide sequence between the two site specific recombinase sites is excised.

Site-specific recombinases include enzymes or recombinases that recognize and bind to a short nucleic acid site or sequence-specific recombinase target site, i.e., a recombinase recognition site, and catalyze the recombination of nucleic acid in relation to these sites. These enzymes include recombinases, transposases and integrases. Examples of sequence-specific recombinase target sites include, but are not limited to, lox sites, aft sites, dif sites and frt sites. Non-limiting examples of site-specific recombinases include, but are not limited to, bacteriophage P1 Cre recombinase, yeast FLP recombinase, Inti integrase, bacteriophage X, phi 80, P22, P2, 186, and P4 recombinase, Tn3 resolvase, the Hin recombinase, and the Cin recombinase, *E. coli* xerC and xerD recombinases, *Bacillus thuringiensis* recombinase, TpnI and the 13-lactamase transposons, and the immunoglobulin recombinases.

The recombination site can be a lox site that is recognized by the Cre recombinase of bacteriophage P1. Lox sites refer to a nucleotide sequence at which the product of the cre gene of bacteriophage P 1, the Cre recombinase, can catalyze a site-specific recombination event. A variety of lox sites are known in the art, including the naturally occurring loxP, loxB, loxL and loxR, as well as a number of mutant, or variant, lox sites, such as loxP511, loxP514, lox.DELTA.86, lox.DELTA.117, loxC2, loxP2, loxP3 and lox P23. Additional example of lox sites include, but are not limited to, loxB, loxL, loxR, loxP, loxP3, loxP23, loxA86, loxA117, loxP511, and loxC2.

The recombination site may be a recombination site that is recognized by a recombinases other than Cre. The recombinase site can be the FRT sites recognized by FLP recombinase of the 2 pi plasmid of *Saccharomyces cerevisiae.* FRT sites refer to a nucleotide sequence at which the product of the FLP gene of the yeast 2 micron plasmid, FLP recombinase, can catalyze site-specific recombination. Additional examples of the non-Cre recombinases include, but are not limited to, site-specific recombinases include: att sites recognized by the Int recombinase of bacteriophage X (e.g. att1, att2, att3, attP, attB, attL, and attR), the recombination sites recognized by the resolvase family, and the recombination site recognized by transposase of *Bacillus thuringiensis.*

The targeting constructs can contain: sequence homologous to a porcine immunoglobulin gene as described herein, a selectable marker gene and/ or a site specific recombinase target site.

### Selection of Homologously Recombined Cells

The cells can then be grown in appropriately-selected medium to identify cells providing the appropriate integration. The presence of the selectable marker gene inserted into the immunoglobulin gene establishes the integration of the target construct into the host genome. Those cells which show the desired phenotype can then be further analyzed by restriction
analysis, electrophoresis, Southern analysis, polymerase chain reaction, etc to analyze the DNA in order to establish whether homologous or non-homologous recombination occurred. This can be determined by employing probes for the insert and then sequencing the 5' and 3' regions flanking the insert for the presence of the immunoglobulin gene extending beyond the flanking regions of the construct or identifying the presence of a deletion, when such deletion is introduced. Primers can also be used which are complementary to a sequence within the construct and complementary to a sequence outside the construct and at the target locus. In this way, one can only obtain DNA duplexes having both of the primers present in the complementary chains if homologous recombination has occurred. By demonstrating the presence of the primer sequences or the expected size sequence, the occurrence of homologous recombination is supported.

The polymerase chain reaction used for screening homologous recombination events is known in the art, see, for example, Kim and Smithies, Nucleic Acids Res. 16:8887-8903, 1988; and Joyner et al., Nature 338:153-156, 1989. The specific combination of a mutant polyoma enhancer and a thymidine kinase promoter to drive the neomycin gene has been shown to be active in both embryonic stem cells and EC cells by Thomas and Capecchi, supra, 1987; Nicholas and Berg (1983) in Teratocarcinoma Stem Cell, eds. Siver, Martin and Strikland (Cold Spring Harbor Lab., Cold Spring Harbor, N.Y. (pp. 469-497); and Linney and Donerly, Cell 35:693-699, 1983.

The cell lines obtained from the first round of targeting are likely to be heterozygous for the targeted allele. Homozygosity, in which both alleles are modified, can be achieved in a number of ways. One approach is to grow up a number of cells in which one copy has been modified and then to subject these cells to another round of targeting using a different selectable marker. Alternatively, homozygotes can be obtained by breeding animals heterozygous for the modified allele, according to traditional Mendelian genetics. In some situations, it can be desirable to have two different modified alleles. This can be achieved by successive rounds of gene targeting or by breeding heterozygotes, each of which carries one of the desired modified alleles.

### Identification Of Cells That Have Undergone Homologous Recombination

The selection method can detect the depletion of the immunoglobulin gene directly, whether due to targeted knockout of the immunoglobulin gene by homologous recombination, or a mutation in the gene that results in a nonfunctioning or nonexpressed immunoglobulin. Selection via antibiotic resistance has been used most commonly for screening (see above). This method can detect the presence of the resistance gene on the targeting vector, but does not directly indicate whether integration was a targeted recombination event or a random integration. Certain technology, such as Poly A and promoter trap technology, increase the probability of targeted events, but again, do not give direct evidence that the desired phenotype, a cell deficient in immunoglobulin gene expression, has been achieved. In addition, negative forms of selection can be used to select for targeted integration; in these cases, the gene for a factor lethal to the cells is inserted in such a way that only targeted events allow the cell to avoid death. Cells selected by these methods can then be assayed for gene disruption, vector integration and, finally, immunoglobulin gene depletion. In these cases, since the selection is based on detection of targeting vector integration and not at the altered phenotype, only targeted knockouts, not point mutations, gene rearrangements or truncations or other such modifications can be detected.

Animal cells believed to lacking expression of functional immunoglobulin genes can be further characterized. Such characterization can be accomplished by the following techniques, including, but not limited to: PCR analysis, Southern blot analysis, Northern blot analysis, specific lectin binding assays, and/or sequencing analysis.

PCR analysis as described in the art can be used to determine the integration of targeting vectors. In one embodiment, amplimers can originate in the antibiotic resistance gene and extend into a region outside the vector sequence. Southern analysis can also be used to characterize gross modifications in the locus, such as the integration of a targeting vector into the immunoglobulin locus. Whereas, Northern analysis can be used to characterize the transcript produced from each of the alleles.

Further, sequencing analysis of the cDNA produced from the RNA transcript can also be used to determine the precise location of any mutations in the immunoglobulin allele.

Ungulate cells lacking expression of at least one allele of a functional region of an ungulate heavy chain, kappa light chain and/or lambda light chain locus produced according to the process, sequences and/or constructs described herein are disclosed herein. These cells can be obtained as a result of homologous recombination. Particularly, by inactivating at least one allele of an ungulate heavy chain, kappa light chain or lambda light chain gene, cells can be produced which have reduced capability for expression of porcine antibodies. Mammalian cells lacking expression of both alleles of an ungulate heavy chain, kappa light chain and/or lambda light chain gene can be produced according to the process, sequences and/or constructs described herein. In a further embodiment, porcine animals are provided in which at least one allele of an ungulate heavy chain, kappa light chain and/or lambda light chain gene is inactivated via a genetic targeting event produced according to the process, sequences and/or constructs described herein. In another aspect of the present invention, porcine animals are provided in which both alleles of an ungulate heavy chain, kappa light chain and/or lambda light chain gene are inactivated via a genetic targeting event. The gene can be targeted via homologous recombination. In other embodiments, the gene can be disrupted, i.e. a portion of the genetic code can be altered, thereby affecting transcription and/or translation of that segment of the gene. For example, disruption of a gene can occur through substitution, deletion ("knock-out") or insertion ("knock-in") techniques. Additional genes for a desired protein or regulatory sequence that modulate transcription of an existing sequence can be inserted.

Alleles of ungulate heavy chain, kappa light chain or lambda light chain gene can be rendered inactive according to the process, sequences and/or constructs described herein, such that functional ungulate immunoglobulins can no longer be produced. In one embodiment, the targeted immunoglobulin gene can be transcribed into RNA, but not translated into protein. In another embodiment, the targeted immunoglobulin gene can be transcribed in an inactive truncated form. Such a truncated RNA may either not be translated or can be translated into a nonfunctional protein. In an alternative embodiment, the targeted immunoglobulin gene can be inactivated in such a way that no transcription of the gene occurs. In a further embodiment, the targeted immunoglobulin gene can be transcribed and then translated into a nonfunctional protein.

### Insertion of Artificial Chromosomes Containing Human Immunoglobulin Genes

### Artificial Chromosomes

Disclosed herein are ungulates and ungulate cells that lack expression of at least one allele of a functional region of an ungulate heavy chain, kappa light chain and/or lambda light chain produced according to the processes, sequences and/or constructs described herein, which are further modified to express at least part of a human antibody (i.e. immunoglobulin (Ig)). The human immunoglobulin locus can undergo rearrangement and express a diverse population of human antibody molecules in the ungulate. These cloned, transgenic ungulates provide a replenishable, theoretically infinite supply of human antibodies (such as polyclonal antibodies), which can be used for therapeutic, diagnostic, purification, and other clinically relevant purposes.

In one particular embodiment, artificial chromosome (ACs) can be used to accomplish the transfer of human immunoglobulin genes into ungulate cells and animals. ACs permit targeted integration of megabase size DNA fragments that contain single or multiple genes. The ACs, therefore, can introduce heterologous DNA into selected cells for production of the gene product encoded by the heterologous DNA. In a one embodiment, one or more ACs with integrated human immunoglobulin DNA can be used as a vector for introduction of human Ig genes into ungulates (such as pigs).

First constructed in yeast in 1983, ACs are man-made linear DNA molecules constructed from essential cis-acting DNA sequence elements that are responsible for the proper replication and partitioning of natural chromosomes (Murray et al. (1983), Nature 301:189-193). A chromosome requires at least three elements to function. Specifically, the elements of an artificial chromosome include at least: (1) autonomous replication sequences (ARS) (having properties of replication origins - which are the sites for initiation of DNA replication), (2) centromeres (site of kinetochore assembly that is responsible for proper distribution of replicated chromosomes at mitosis and meiosis), and (3) telomeres (specialized structures at the ends of linear chromosomes that function to both stabilize the ends and facilitate the complete replication of the extreme termini of the DNA molecule).

In one embodiment, the human Ig can be maintained as an independent unit (an episome) apart from the ungulate chromosomal DNA. For example, episomal vectors contain the necessary DNA sequence elements required for DNA replication and maintenance of the vector within the cell. Episomal vectors are available commercially (see, for example, Maniatis, T. et al., Molecular Cloning, A Laboratory Manual (1982) pp. 368-369). The AC can stably replicate and segregate along side endogenous chromosomes. In an alternative embodiment, the human IgG DNA sequences can be integrated into the ungulate cell's chromosomes thereby permitting the new information to be replicated and partitioned to the cell's progeny as a part of the natural chromosomes (see, for example, Wigler et al. (1977), Cell 11:223). The AC can be translocated to, or inserted into, the endogenous chromosome of the ungulate cell. Two or more ACs can be introduced to the host cell simultaneously or sequentially.

ACs, furthermore, can provide an extra-genomic locus for targeted integration of megabase size DNA fragments that contain single or multiple genes, including multiple copies of a single gene operatively linked to one promoter or each copy or several copies linked to separate promoters. ACs can permit the targeted integration of megabase size DNA fragments that contain single or multiple human immunoglobulin genes. The ACs can be generated by culturing the cells with dicentric chromosomes (i.e., chromosomes with two centromeres) under such conditions known to one skilled in the art whereby the chromosome breaks to form a minichromosome and formerly dicentric chromosome.

ACs can be constructed from humans (human artificial chromosomes: "HACs"), yeast (yeast artificial chromosomes: "YACs"), bacteria (bacterial artificial chromosomes: "BACs"), bacteriophage P1-derived artificial chromosomes: "PACs") and other mammals (mammalian artificial chromosomes: "MACs"). The ACs derive their name (e.g., YAC, BAC, PAC, MAC, HAC) based on the origin of the centromere. A YAC, for example, can derive its centromere from *S. cerevisiae.* MACs, on the other hand, include an active mammalian centromere while HACs refer to chromosomes that include human centromeres. Furthermore, plant artificial chromosomes ("PLACs") and insect artificial chromosomes can also be constructed. The ACs can include elements derived from chromosomes that are responsible for both replication and maintenance. ACs, therefore, are capable of stably maintaining large genomic DNA fragments such as human Ig DNA..

Ungulates containing YACs are disclosed herein. YACs are genetically engineered circular chromosomes that contain elements from yeast chromosomes, such as *S. cerevisiae*, and segments of foreign DNAs that can be much larger than those accepted by conventional cloning vectors (e.g., plasmids, cosmids). YACs allow the propagation of very large segments of exogenous DNA (Schlessinger, D. (1990), Trends in Genetics 6:248-253) into mammalian cells and animals (Choi et al. (1993), Nature Gen 4:117-123). YAC transgenic approaches are very powerful and are greatly enhanced by the ability to efficiently manipulate the cloned DNA. A major technical advantage of yeast is the ease with which specific genome modifications can be made via DNA-mediated transformation and homologous recombination (Ramsay, M. (1994), Mol Biotech 1:181-201). In one embodiment, one or more YACs with integrated human Ig DNA can be used as a vector for introduction of human Ig genes into ungulates (such as pigs).

The YAC vectors contain specific structural components for replication in yeast, including: a centromere, telomeres, autonomous replication sequence (ARS), yeast selectable markers (e.g., *TRP1*, *URA3*, and *SUP4),* and a cloning site for insertion of large segments of greater than 50 kb of exogenous DNA. The marker genes can allow selection of the cells carrying the YAC and serve as sites for the synthesis of specific restriction endonucleases. For example, the *TRP1* and *URA3* genes can be used as dual selectable markers to ensure that only complete artificial chromosomes are maintained. Yeast selectable markers can be carried on both sides of the centromere, and two sequences that seed telomere formation *in vivo* are separated. Only a fraction of one percent of a yeast cell's total DNA is necessary for replication, however, including the center of the chromosome (the centromere, which serves as the site of attachment between sister chromatids and the sites of spindle fiber attachment during mitosis), the ends of the chromosome (telomeres, which serve as necessary sequences to maintain the ends of eukaryotic chromosomes), and another short stretch of DNA called the ARS which serves as DNA segments where the double helix can unwind and begin to copy itself.

In one embodiment, YACs can be used to clone up to about 1, 2, or 3 Mb of immunoglobulin DNA. In another embodiment, at least 25, 30, 40, 50, 60, 70, 75, 80, 85, 90, or 95 kilobases.

Yeast integrating plasmids, replicating vectors (which are fragments of YACs),can also be used to express human Ig. The yeast integrating plasmid can contain bacterial plasmid sequences that provide a replication origin and a drug-resistance gene for growth in bacteria (e.g., *E. coli*), a yeast marker gene for selection of transformants in yeast, and restriction sites for inserting Ig sequences. Host cells can stably acquire this plasmid by integrating it directly into a chromosome. Yeast replicating vectors can also be used to express human Ig as free plasmid circles in yeast. Yeast or ARS-containing vectors can be stabilized by the addition of a centromere sequence. YACs have both centromeric and telomeric regions, and can be used for cloning very large pieces of DNA because the recombinant is maintained essentially as a yeast chromosome.

YACs are disclosed, for example, as disclosed in U.S. Pat. Nos. 6,692,954, 6,495,318, 6,391,642, 6,287,853, 6,221,588, 6,166,288, 6,096,878, 6,015,708, 5,981,175, 5,939,255, 5,843,671, 5,783,385, 5,776,745, 5,578,461, and 4,889,806; European Patent Nos. 1 356 062 and 0 648 265; PCT Publication Nos. WO 03/025222, WO 02/057437, WO 02/101044, WO 02/057437, WO 98/36082, WO 98/12335, WO 98/01573, WO 96/01276, WO 95/14769, WO 95/05847, WO 94/23049, and WO 94/00569.

Ungulates containing BACs are disclosed herein. BACs are F-based plasmids found in bacteria, such as *E. Coli*, that can transfer approximately 300 kb of foreign DNA into a host cell. Once the Ig DNA has been cloned into the host cell, the newly inserted segment can be replicated along with the rest of the plasmid. As a result, billions of copies of the foreign DNA can be made in a very short time. In a particular embodiment, one or more BACs with integrated human Ig DNA are used as a vector for introduction of human Ig genes into ungulates (such as pigs).

The BAC cloning system is based on the *E. coli* F-factor, whose replication is strictly controlled and thus ensures stable maintenance of large constructs (Willets, N., and R. Skurray (1987), Structure and function of the F-factor and mechanism of conjugation. In Escherichia coli and Salmonella Typhimurium: Cellular and Molecular Biology (F. C. Neidhardt, Ed) Vol.2 pp 1110-1133, Am. Soc. Microbiol., Washington, D.C.). BACs have been widely used for cloning of DNA from various eukaryotic species (Cai et al. (1995), Genomics 29:413-425; Kim et al. (1996), Genomics 34:213-218; Misumi et al. (1997), Genomics 40:147-150; Woo et al. (1994), Nucleic Acids Res 22:4922-4931; Zimmer, R. and Gibbins, A.M.V. (1997), Genomics 42:217-226). The low occurance of the F-plasmid can reduce the potential for recombination between DNA fragments and can avoid the lethal overexpression of cloned bacterial genes. BACs can stably maintain the human immunoglobulin genes in a single copy vector in the host cells, even after 100 or more generations of serial growth.

BAC (or pBAC) vectors can accommodate inserts in the range of approximately 30 to 300 kb pairs. One specific type of BAC vector, pBeloBac11, uses a complementation of the lacZ gene to distinguish insert-containing recombinant molecules from colonies carrying the BAC vector, by color. When a DNA fragment is cloned into the lacZ gene of pBeloBac11, insertional activation results in a white colony on X-Gal/IPTG plates after transformation (Kim et al. (1996), Genomics 34:213-218) to easily identify positive clones.

For example, BACs can be provided such as disclosed in U.S. Pat. Nos. 6,713,281, 6,703,198, 6,649,347, 6,638,722, 6,586,184, 6,573,090, 6,548,256, 6,534,262, 6,492,577, 6,492,506, 6,485,912, 6,472,177, 6,455,254, 6,383,756, 6,277,621, 6,183,957, 6,156,574, 6,127,171, 5,874,259, 5,707,811, and 5,597,694; European Patent Nos. 0 805 851; PCT Publication Nos. WO 03/087330, WO 02/00916, WO 01/39797, WO 01/04302, WO 00/79001, WO 99/54487, WO 99/27118, and WO 96/21725.

Ungulates containing bacteriophage PACs are disclosed herein. One or more bacteriophage PACs with integrated human Ig DNA may be used as a vector for introduction of human Ig genes into ungulates (such as pigs). For example,
PACs can be provided such as disclosed in U.S. Pat. Nos. 6,743,906, 6,730,500, 6,689,606, 6,673,909, 6,642,207, 6,632,934, 6,573,090, 6,544,768, 6,489,458, 6,485,912, 6,469,144, 6,462,176,6,413,776, 6,399,312, 6,340,595, 6,287,854, 6,284,882, 6,277,621, 6,271,008, 6,187,533, 6,156,574, 6,153,740, 6,143,949, 6,017,755, and 5,973,133; European Patent Nos. 0 814 156; PCT Publication Nos. WO 03/091426, WO 03/076573, WO 03/020898, WO 02/101022, WO 02/070696, WO 02/061073, WO 02/31202, WO 01/44486, WO 01/07478, WO 01/05962, and WO 99/63103,.

Ungulates containing MACs are disclosed herein. MACs possess high mitotic stability, consistent and regulated gene expression, high cloning capacity, and nonimmunogenicity. Mammalian chromosomes can be comprised of a continuous linear strand of DNA ranging in size from approximately 50 to 250 Mb. The DNA construct can further contain one or more sequences necessary for the DNA construct to multiply in yeast cells. The DNA construct can also contain a sequence encoding a selectable marker gene. The DNA construct **can** be capable of being maintained as a chromosome in a transformed cell with the DNA construct. MACs provide extra-genomic specific integration sites for introduction of genes encoding proteins of interest and permit megabase size DNA integration so that, for example, genes encoding an entire metabolic pathway, a very large gene [e.g., such as the cystic fibrosis (CF) gene (∼ 600 kb)], or several genes [e.g., a series of antigens for preparation of a multivalent vaccine] can be stably introduced into a cell.

Mammalian artificial chromosomes [MACs] are disclosed herein. Also disclosed are artificial chromosomes for other higher eukaryotic species, such as insects and fish, produced using the MACS are disclosed herein. Methods for generating and isolating such chromosomes. Methods using the MACs to construct artificial chromosomes from other species, such as insect and fish species are also disclosed herein. The artificial chromosomes are fully functional stable chromosomes. Two types of artificial chromosomes are disclosed herein. One type, herein referred to as SATACs [satellite artificial chromosomes] are stable heterochromatic chromosomes, and the another type are minichromosomes based on amplification of euchromatin. As used herein, a formerly dicentric chromosome is a chromosome that is produced when a dicentric chromosome fragments and acquires new telomeres so that two chromosomes, each having one of the centromeres, are produced. Each of the fragments can be replicable chromosomes.

Also disclosed herein are artificial chromosomes for other higher eukaryotic species, such as insects and fish, produced using the MACS are disclosed herein. SATACs [satellite artificial chromosomes] are disclosed herein. SATACs are stable heterochromatic chromosomes. Minichromosomes are disclosed herein wherein the minichromosomes are based on amplification of euchromatin.

Artificial chromosomes can be generated by culturing the cells with the dicentric chromosomes under conditions whereby the chromosome breaks to form a minichromosome and formerly dicentric chromosome. The SATACs can be generated from the minichromosome fragment, see, for example, in U.S. Pat. No. 5,288,625. The SATACs can be generated from the fragment of the formerly dicentric chromosome. The SATACs can be made up of repeating units of short satellite DNA and can be fully heterochromatic. Absent insertion of heterologous or foreign DNA, the SATACs may not contain genetic information. SATACs of various sizes are disclosed herein that are formed by repeated culturing under selective conditions and subcloning of cells that contain chromosomes produced from the formerly dicentric chromosomes. These chromosomes can be based on repeating units 7.5 to 10 Mb in size, or megareplicons. These megareplicons can be tandem blocks of satellite DNA flanked by heterologous non-satellite
DNA. Amplification can produce a tandem array of identical chromosome segments [each called an amplicon] that contain two inverted megareplicons bordered by heterologous ["foreign"] DNA. Repeated cell fusion, growth on selective medium and/or. BrdU [5- bromodeoxyuridine] treatment or other genome destabilizing reagent or agent, such as ionizing radiation, including X-rays, and subcloning can result in cell lines that carry stable heterochromatic or partially heterochromatic chromosomes, including a 150-200 Mb "sausage" chromosome, a 500-1000 Mb gigachromosome, a stable 250-400 Mb megachromosome and various smaller stable chromosomes derived therefrom. These chromosomes are based on these repeating units and can include human immunoglobulin DNA that is expressed. (See also US Patent No. 6,743,967

In other embodiments, MACs can be provided, for example, as disclosed in U.S. Pat. Nos. 6,743,967, 6,682,729, 6,569,643, 6,558,902, 6,548,287, 6,410,722, 6,348,353, 6,297,029, 6,265,211, 6,207,648, 6,150,170, 6,150,160, 6,133,503, 6,077,697, 6,025,155, 5,997,881, 5,985,846, 5,981,225, 5,877,159, _ 5,851,760, and 5,721,118; PCT Publication Nos. WO 04/066945, WO 04/044129, WO 04/035729, WO 04/033668, WO 04/027075, WO 04/016791, WO 04/009788, WO 04/007750, WO 03/083054, WO 03/068910, WO 03/068909, WO 03/064613, WO 03/052050, WO 03/027315, WO 03/023029, WO 03/012126, WO 03/006610, WO 03/000921, WO 02/103032, WO 02/097059, WO 02/096923, WO 02/095003, WO 02/092615, WO 02/081710, WO 02/059330, WO 02/059296, WO 00/18941, WO 97/16533, and WO 96/40965.

Ungulates and ungulate cells containing HACs are disclosed herein. One or more HACs with integrated human Ig DNA are used as a vector for introduction of human Ig genes into ungulates (such as pigs). One or more HACs with integrated human Ig DNA are used to generate ungulates (for example, pigs) by nuclear transfer which express human Igs in response to immunization and which undergo affinity maturation.

Various approaches may be used to produce ungulates that express human antibodies ("human Ig"). These approaches include, for example, the insertion of a HAC containing both heavy and light chain Ig genes into an ungulate or the insertion of human B-cells or B-cell precursors into an ungulate during its fetal stage or after it is born (e.g., an immune deficient or immune suppressed ungulate) (see, for example, WO 01/35735, filed November 17, 2000, US 02/08645, filed March 20, 2002). In either case, both human antibody producing cells and ungulate antibody-producing B-cells may be present in the ungulate. In an ungulate containing a HAC, a single B-cell may produce an antibody that contains a combination of ungulate and human heavy and light chain proteins. In still other embodiments, the total size of the HAC is at least to approximately 1, 2, 3, 4, 5, 6, 7, 8, 9 or 10 Mb.

For example, HACs can be provided such as disclosed in U.S. Pat. Nos. 6,642,207, 6,590,089, 6,566,066, 6,524,799, 6,500,642, 6,485,910, 6,475,752, 6,458,561, 6,455,026, 6,448,041, 6,410,722, 6,358,523, 6,277,621, 6,265,211, 6,146,827, 6,143,566, 6,077,697, 6,025,155, 6,020,142, and 5,972,649; U.S. Pat. Application No. 2003/0037347; PCT Publication Nos. WO 04/050704, WO 04/044156, WO 04/031385, WO 04/016791, WO 03/101396, WO 03/097812, WO 03/093469, WO 03/091426, WO 03/057923, WO 03/057849, WO 03/027638, WO 03/020898, WO 02/092812, and WO 98/27200.

Additional examples of ACs into which human immunoglobulin sequences can be inserted for use in the invention include, for example, BACs (e.g., pBeloBAC11 or pBAC108L; see, e.g., Shizuya et al. (1992), Proc Natl Acad Sci USA 89(18):8794-8797; Wang et al. (1997), Biotechniques 23(6):992-994), bacteriophage PACs, YACs (see, e.g., Burke (1990), Genet Anal Tech Appl 7(5):94-99), and MACs (see, e.g., Vos (1997), Nat. Biotechnol. 15(12):1257-1259; Ascenzioni et al. (1997), Cancer Lett 118(2):135-142), such as HACs, see also, U.S. Pat. Nos. 6,743,967, 6,716,608, 6,692,954, 6,670,154, 6,642,207, 6,638,722, 6,573,090, 6,492,506, 6,348,353, 6,287,853, 6,277,621, 6,183,957, 6,156,953, 6,133,503, 6,090,584, 6,077,697, 6,025,155, 6,015,708, 5,981,175, 5,874,259, 5,721,118, and 5,270,201; European Patent Nos. 1 437 400, 1 234 024, 1 356 062, 0 959 134, 1 056 878, 0 986 648, 0 648 265, and 0 338 266; PCT Publication Nos. WO 04/013299, WO 01/07478, WO 00/06715, WO 99/43842, WO 99/27118, WO 98/55637, WO 94/00569, and WO 89/09219. Additional examples incluse those AC provided in, for example, PCT Publication No. WO 02/076508, WO 03/093469, WO 02/097059; WO 02/096923; US Publication Nos US 2003/0113917 and US 2003/003435; and US Patent No 6,025,155.

In other embodiments of the present invention, ACs transmitted through male gametogenesis in each generation. The AC can be integrating or non-integrating. In one ambodiment, the AC can be transmitted through mitosis in substantially all dividing cells. In another embodiment, the AC can provide for position independent expression of a human immunoglobulin nucleic acid sequence. In a particular embodiment, the AC can have a transmittal efficiency of at least 10% through each male and female gametogenesis. In one particular embodiment, the AC can be circular. In another particular embodiment, the non-integrating AC can be that deposited with the Belgian Coordinated Collections of Microorganisms--BCCM on Mar. 27, 2000 under accession number LMBP 5473 CB. Methods for producing an AC are disclosed herein wherein a mitotically stable unit containing an exogenous nucleic acid transmitted through male gametogenesis is identified; and an entry site in the mitotically stable unit allows for the integration of human immunoglobulin genes into the unit.

ACs are disclosed that include: a functional centromere, a selectable marker and/or a unique cloning site. The AC can exhibit one or more of the following properties: it can segregate stably as an independent chromosome, immunoglobulin sequences can be inserted in a controlled way and can expressed from the AC, it can be efficiently transmitted through the male and female germline and/ or the transgenic animals can bear the chromosome in greater than about 30, 40, 50, 60, 70, 80 or 90% of its cells.

The AC can be isolated from fibroblasts (such as any mammalian or human fibroblast) in which it was mitotically stable. After transfer of the AC into hamster cells, a lox (such as loxP) site and a selectable marker site can be inserted. The AC can maintain mitotic stability, for example, showing a loss of less than about 5, 2, 1, 0.5 or 0.25 percent per mitosis in the absence of selection. See also, US 2003/0064509 and WO 01/77357.

### Xenogenous Immunoglobulin Genes

In another aspect of the present invention, transgenic porcine animals are provided that expresses a xenogenous immunoglobulin or fragment thereof, wherein the immunoglobulin can be expressed from an immunoglobulin locus that is integrated within an endogenous ungulate chromosome. In one embodiment, ungulate cells derived from the transgenic animals are provided. In one embodiment, the xenogenous immunoglobulin locus can be inherited by offspring. In another embodiment, the xenogenous immunoglobulin locus can be inherited through the male germ line by offspring. In still further embodiments, an artificial chromosome (AC) can contain the xenogenous immunoglobulin locus. In one embodiment, the AC can be a yeast AC or a mammalian AC. In a further embodiment, the xenogenous locus can be a human immunoglobulin locus or fragment thereof. In one embodiment, the human immunoglobulin locus can be human chromosome 14, human chromosome 2, and human chromosome 22 or fragments thereof. In another embodiment, the human immunoglobulin locus can include any fragment of a human immunoglobulin locus that can undergo rearrangement In a further embodiment, the human immunoglobulin locus can include any fragment of a human immunoglobulin heavy chain locus and a human immunoglobulin light chain locus that can undergo rearrangement In still further embodiment, the human immunoglobulin locus can include any human immunoglobulin locus or fragment thereof that can produce an antibody upon exposure to an antigen. In a particular embodiment, the exogenous human immunoglobulin can be expressed in **B** cells to produce xenogenous immunoglobulin in response to exposure to one or more antigens.
In other embodiments, the transgenic porcine that lacks any expression of functional endogenous kappa immunoglobulins can be further genetically modified to express a xenogenous immunoglobulin. In an alternative embodiment, porcine animals are provided that contain an xenogenous immunoglobulin locus. In one embodiment, the xenogenous immunoglobulin locus can be a heavy and/ or light chain immunoglobulin locus or fragment thereof In another embodiment, the xenogenous immunoglobulin locus can be a kappa chain locus or fragment thereof and/ or a lambda chain locus or fragment thereof. In still further embodiments, an artificial chromosome (AC) can contain the xenogenous immunoglobulin locus. In one embodiment, the AC can be a yeast AC or a mammalian AC. In a further embodiment, the xenogenous locus can be a human immunoglobulin locus or fragment thereof. In one embodiment, the human immunoglobulin locus can be human chromosome 14, human chromosome 2, and human chromosome 22 or fragments thereof. In another embodiment, the human immunoglobulin locus can include any fragment of a human immunoglobulin that can undergo rearrangement. In a further embodiment, the human immunoglobulin locus can include any fragment of a human immunoglobulin heavy chain locus and a human immunoglobulin light chain locus that can undergo rearrangement. In still further embodiment, the human immunoglobulin locus can include any human immunoglobulin locus or fragment thereof that can produce an antibody upon exposure to an antigen. In a particular embodiment, the exogenous human immunoglobulin can be expressed in **B** cells to produce xenogenous immunoglobulin in response to exposure to one or more antigens.

In other embodiments, the transgenic porcine that lacks any expression of functional endogenous kappa immunoglobulins can be further genetically modified to express a xenogenous immunoglobulin. In an alternative embodiment, porcine animals are provided that contain an xenogenous immunoglobulin locus. In one embodiment, the xenogenous immunoglobulin locus can be a heavy and/ or light chain immunoglobulin locus or fragment thereof In another embodiment, the xenogenous immunoglobulin locus can be a kappa chain locus or fragment thereof and/ or a lambda chain locus or fragment thereof In still further embodiments, an artificial chromosome (AC) can contain the xenogenous immunoglobulin locus. In one embodiment, the AC can be a yeast AC or a mammalian AC. In a further embodiment, the xenogenous locus can be a human immunoglobulin locus or fragment thereof. In one embodiment, the human immunoglobulin locus can be human chromosome 14, human chromosome 2, and human chromosome 22 or fragments thereof In another embodiment, the human immunoglobulin locus can include any fragment of a human immunoglobulin locus that can undergo rearrangement. In a further embodiment, the human immunoglobulin locus can include any fragment of a human immunoglobulin heavy chain locus and a human immunoglobulin light chain locus that can undergo rearrangement. In still further embodiment, the human immunoglobulin locus can include any human immunoglobulin locus or fragment thereof that can produce an antibody upon exposure to an antigen. In a particular embodiment, the exogenous human immunoglobulin can be expressed in B cells to produce xenogenous immunoglobulin in response to exposure to one or more antigens.

In another embodiment, porcine animals are provided that contain an xenogenous immunoglobulin locus. In one embodiment, the xenogenous immunoglobulin locus can be a heavy and/ or light chain immunoglobulin locus or fragment thereof. In another embodiment, the xenogenous immunoglobulin locus can be a kappa chain locus or fragment thereof and/ or a lambda chain locus or fragment thereof. In still further embodiments, an artificial chromosome (AC) can contain the xenogenous immunoglobulin locus. In one embodiment, the AC can be a yeast AC or a mammalian AC. In a further embodiment, the xenogenous locus can be a human immunoglobulin locus or fragment thereof In one embodiment, the human immunoglobulin locus can be human chromosome 14, human chromosome 2, and human chromosome 22 or fragments thereof In another embodiment, the human immunoglobulin locus can include any fragment of a human immunoglobulin locus that can undergo rearrangement. In a further embodiment, the human immunoglobulin locus can include any fragment of a human immunoglobulin heavy
chain locus and a human immunoglobulin light chain locus that can undergo rearrangement In still further embodiment, the human immunoglobulin locus can include any human immunoglobulin locus or fragment thereof that can produce an antibody upon exposure to an antigen. In a particular embodiment, the exogenous human immunoglobulin can be expressed in B cells to produce xenogenous immunoglobulin in response to exposure to one or more antigens.

Human immunoglobulin genes, such as the Ig heavy chain gene (human chromosome 414), Ig kappa chain gene (human chromosome #2) and/or the Ig lambda chain gene (chromosome #22) can be inserted into Acs, as described above. In a particular embodiment, any portion of the human heavy, kappa and/or lambda Ig genes can be inserted into ACs. In one embodiment, the nucleic acid can be at least 70, 80, 90, 95, or 99% identical to the corresponding region of a naturally-occurring nucleic acid from a human. In other embodiments, more than one class of human antibody is produced by the ungulate. In various embodiments, more than one different human Ig or antibody is produced by the ungulate. In one embodiment, an AC containing both a human Ig heavy chain gene and Ig light chain gene, such as an automatic human artificial chromosome ("AHAC," a circular recombinant nucleic acid molecule that is converted to a linear human chromosome *in vivo* by an endogenously expressed restriction endonuclease) can be introduced. In one embodiment, the human heavy chain locus and the light chain locus are on different chromosome arms (i.e., on different side of the centromere). In one embodiments, the heavy chain can include the mu heavy chain, and the light chain can be a lambda or kappa light chain. The Ig genes can be introduced simultaneously or sequentially in one or more than one ACs.

In particular embodiments, the ungulate or ungulate cell expresses one or more nucleic acids encoding all or part of a human Ig gene which undergoes rearrangement and expresses more than one human Ig molecule, such as a human antibody protein. Thus, the nucleic acid encoding the human Ig chain or antibody is in its unrearranged form (that is, the nucleic acid has not undergone V(D)J recombination). In particular embodiments, all of the nucleic acid segments encoding a V gene segment of an antibody light chain can be separated from all of the nucleic acid segments encoding a J gene segment by one or more nucleotides. In a particular embodiment, all of the nucleic acid segments encoding a V gene segment of an antibody heavy chain can be separated from all of the nucleic acid segments encoding a D gene segment by one or more nucleotides, and/or all of the nucleic acid segments encoding a D gene segment of an antibody heavy chain are separated from all of the nucleic acid segments encoding a J gene segment by one or more nucleotides. Administration of an antigen to a transgenic ungulate containing an unrearranged human Ig gene is followed by the rearrangement of the nucleic acid segments in the human Ig gene locus and the production of human antibodies reactive with the antigen.

In one embodiment, the AC can express a portion or fragment of a human chromosome that contains an immunoglobulin gene. In one embodiment, the AC can express at least 300 or 1300 kb of the human light chain locus, such as described in Davies et al. 1993 Biotechnology 11: 911-914.

In another embodiment, the AC can express a portion of human chromosome 22 that contains at least the X light-chain locus, including V), gene segments, **JA**, gene segments, and the single **CA**, gene. In another embodiment, the AC can express at least one VA, gene segment, at least one **JA**, gene segment, and the Cx gene. In other embodiment, ACs can contain portions of the lambda locus, such as described in Popov et al. J Exp Med. 1999 May 17;189(10):1611-20.

In another embodiment, the AC can express a portion of human chromosome 2 that contains at least the lc light-chain locus, including V" gene segments, J" gene segments and the single C" gene. In another embodiment, the AC can express at least one V), gene segment, at least one J" gene segment and the C" gene. In other embodiments, AC containing portions of the kappa light chain locus can be those describe, for example, in Li et al. 2000 J Immunol 164: 812-824 and Li S Proc Natl Acad Sci U S A. 1987 Jun;84(12):4229-33. In another embodiment, AC containing approximatelty 1.3 Mb of human kappa locus are disclosed herein, such as described in Zou et al FASEB J. 1996 Aug;10(10):1227-32.

In further embodiments, the AC can express a portion of human chromosome 14 that contains at least the human heavy-chain locus, including **VH**, **DH**, **JH** and **CH** gene segments. In another embodiment, the AC can express at least one **VH** gene segment, at least one **DH** gene segment, at least one **JH** gene segment and at least one at least one **CH** gene segment. In other embodiments, the AC can express at least 85 kb of the human heavy chain locus, such as described in Choi et al. 1993 Nat Gen 4:117-123 and/or Zou et al. 1996 PNAS 96:14100-14105.

In other embodiments, the AC can express portions of both heavy and light chain, such as, at least 220, 170, 800 or 1020 kb, for example, as disclosed in Green et al. 1994 Nat Gen 7:13-22; Mendez et al 1995 Genomics 26: 294-307; Mendez et al. 1997 Nat Gen 15: 146-156; Green et al. 1998 J Exp Med 188: 483-495 and/or Fishwild et al. 1996 Nat Biotech 14: 845-851. In another embodiment, the AC can express megabase amounts of human immunoglobulin, such as described in Nicholson J Immunol. 1999 Dec 15;163(12):6898-906 and Popov Gene. 1996 Oct 24;177(1-2):195-201. In addition, in one particular embodiment, MACs derived from human chromosome #14 (comprising the Ig heavy chain gene), human chromosome #2 comprising the Ig kappa chain gene) and human chromosome #22 (comprising the Ig lambda chain gene) can be introduced simultaneously or successively, such as described in US Patent Publication No. 2004/0068760 to Robl et al.. In another embodiments, the total size of the MAC is less than or equal to approximately 10, 9, 8, or 7 megabases.

In a particular embodiment, human Vh, human Dh, human Jh segments and human mu segments of human immunoglobulins in germline configuration can be inserted into an AC, such as a YAC, such that the Vh, Dh, Jh and mu DNA segments form a repertoire of immunoglobulins containing portions which correspond to the human DNA segments, for example, as described in U.S. Patent No. 5,545,807 to the Babraham Insttitute. Such ACs, after insertion into ungulate cells and generation of ungulates can produce heavy chain immunoglobulins. In one embodiment, these immunoglobulins can form functional heavy chain-light chain immunoglobulins. In another embodiment, these immunoglobulins can be expressed in an amount allowing for recovery from suitable cells or body fluids of the ungulate. Such immunglobulins can be inserted into yeast artifical chromosome vectors, such as decribed by Burke, D T, Carle, G F and Olson, M V (1987) "Cloning of large segments of exogenous DNA into yeast by means of artifical chromosome vectors" Science, 236, 806-812, or by introduction of chromosome fragments (such as described by Richer, J and Lo, C W (1989) "Introduction of human DNA into mouse eggs by injection of dissected human chromosome fragments" Science 245, 175-177).

Additional information on specific ACs containing human immunoglobulin genes can be found in, for example, recent reviews by Giraldo & Montoliu (2001) Transgenic Research 10: 83-103 and Peterson (2003) Expert Reviews in Molecular Medicine 5: 1-25.

### AC Transfer Methods

The human immunoglobulin genes can be first inserted into ACs and then the human-immunoglobulin-containing ACs can be inserted into the ungulate cells. Alternatively, the ACs can be transferred to an intermediary mammalian cell, such as a CHO cell, prior to insertion into the ungulate call. In one embodiment, the intermediary mammalian cell can also contain and AC and the first AC can be inserted into the AC of the mammalian cell. In particular, a YAC containing human immunoglobulin genes or fragments thereof in a yeast cell can be transferred to a mammalian cell that harbors an MAC. The YAC can be inserted into the MAC. The MAC can then be transferred to an ungulate cell. The human Ig genes can be inserted into ACs by homologous recombination. The resulting AC containing human Ig genes, can then be introduced into ungulate cells. One or more ungulate cells can be selected by techniques described herein or those known in the art, which contain an AC containing a human Ig.

Suitable hosts for introduction of the ACs are provided herein, which include but are not limited to any animal or plant, cell or tissue thereof, including, but not limited to: mammals, birds, reptiles, amphibians, insects, fish, arachnids, tobacco, tomato, wheat, monocots, dicots and algae. In one embodiment, the ACscan be condensed (Marschall et al Gene Ther. 1999 Sep;6(9):1634-7) by any reagent known in the art, including, but not limited to, spermine, spermidine, polyethylenimine, and/ or polylysine prior to introduction into cells.The ACs can be introduced by cell fusion or microcell fusion or subsequent to isolation by any method known to those of skill in this art, including but not limited to: direct DNA transfer, electroporation, nuclear transfer, microcell fusion, cell fusion, spheroplast fusion, lipid-mediated transfer, lipofection, liposomes, microprojectile bombardment, microinjection, calcium phosphate precipitation and/or any other suitable method. Other methods for introducing DNA into cells, include nuclear microinjection, electroporation, bacterial protoplast fusion with intact cells. Polycations, such as polybrene and polyornithine, may also be used. For various techniques for transforming mammalian cells, see e.g., Keown et al. Methods in Enzymology (1990) Vol.185, pp. 527-537; and Mansour et al. (1988) Nature 336:348-352.

The ACs can be introduced by direct DNA transformation; microinjection in cells or embryos, protoplast regeneration for plants, electroporation, microprojectile gun and other such methods known to one skilled in the art (see, e.g., Weissbach et al. (1988) Methods for Plant Molecular Biology, Academic Press, N.Y., Section VIII, pp. 421-463; Grierson et al. (1988) Plant Molecular Biology, 2d Ed., Blackie, London, Ch. 7-9; see, also U.S. Pat. Nos. 5,491,075; 5,482,928; and 5,424,409; see, also, e.g., U.S. Pat. No. 5,470,708,).

In particular embodiments, one or more isolated YACs can be used that harbor human Ig genes. The isolated YACs can be condensed (Marschall et al Gene Ther. 1999 Sep;6(9):1634-7) by any reagent known in the art, including, but not limited to spermine, spermidine, polyethylenimine, and/ or polylysine. The condensed YACs can then be transferred to porcine cells by any method known in the art (for example, microinjection, electroporation, lipid mediated transfection, etc). Alternatively, the condensed YAC can be transferred to oocytes via sperm-mediated gene transfer or intracytoplasmic sperm injection (ICSI) mediated gene transfer. In one embodiment, spheroplast fusion can be used to transfer YACs that harbor human Ig genes to porcine cells.

In other embodiments of the invention, the AC containing the human Ig can be inserted into an adult, fetal, or embryonic ungulate cell. Additional examples of ungulate cells include undifferentiated cells, such as embryonic cells (e.g., embryonic stem cells), differentiated or somatic cells, such as epithelial cells, neural cells epidermal cells, keratinocytes, hematopoietic cells, melanocytes, chondrocytes, B-lymphocytes, T-lymphocytes, erythrocytes, macrophages, monocytes, fibroblasts, muscle cells, cells from the female reproductive system, such as a mammary gland, ovarian cumulus, granulosa, or oviductal cell, germ cells, placental cell, or cells derived from any organ, such as the bladder, brain, esophagus, fallopian tube, heart, intestines, gallbladder, kidney, liver, lung, ovaries, pancreas, prostate, spinal cord, spleen, stomach, testes, thymus, thyroid, trachea, ureter, urethra, and uterus or any other cell type described herein.

### Site Specific Recombinase Mediated Transfer

In particular embodiments of the present invention, the transfer of ACs containing human immunoglobulin genes to porcine cells, such as those described herein or known in the art, can be accomplished via site specific recombinase mediated transfer. In one particular embodiment, the ACs can be transferred into porcine fibroblast cells. In another particular embodiment, the ACs can be YACs.

In other embodiments of the present invention, the circularized DNA, such as an AC, that contain the site specific recombinase target site can be transferred into a cell line that has a site specific resombinase target site within its genome. In one embodiment, the cell's site specific recombinase target site can be located within an exogenous chromosome. The exogenous chromosome can be an artificial chromosome that does not integrate into the host's endogenous genome. In one embodiment, the AC can be transferred via germ line transmission to offspring. In one particular embodiment, a YAC containing a human immunoglobulin gene or fragment thereof can be circularized via a site specific recombinase and then transferred into a host cell that contains a MAC, wherein the MAC contains a site specific recombinase site. This MAC that now contains human immunoglobulin loci or fragments thereof can then be fused with a porcine cell, such as, but not limited to, a fibroblast. The porcine cell can then be used for nuclear transfer.

In certain embodiments of the present invention, the ACs that contain human immunoglobulin genes or fragments thereof can be transferred to a mammalian cell, such as a CHO cell, prior to insertion into the ungulate call. In one embodiment, the intermediary mammalian cell can also contain and AC and the first AC can be inserted into the AC of the mammalian cell. In particular, a YAC containing human immunoglobulin genes or fragments thereof in a yeast cell can be transferred to a mammalian cell that harbors a MAC. The YAC can be inserted in the MAC. The MAC can then be transferred to an ungulate cell. In particular embodiments, the YAC harboring the human Ig genes or fragments thereof can contain site specific recombinase trarget sites. The YAC can first be circularized via application of the appropriate site specific recombinase and then inserted into a mammalian cell that contains its own site specific recombinase target site. Then, the site specific recombinase can be applied to inegrate the YAC into the MAC in the intermediary mammalian cell. The site specific recoombinase can be applied in cis or trans. In particular, the site specific recombinase can be applied in trans. In one embodiment, the site specific recombinase can be expressed via transfection of a site specific recombainse expression plasmid, such as a Cre expression plasmid. In addition, one telomere region of the YAC can also be retrofitted with a selectable marker, such as a selectable marker described herein or known in the art. The human Ig genes or fragments thereof within the MAC of the intermediary mammalian cell can then be transferred to an ungulate cell, such as a fibroblast.

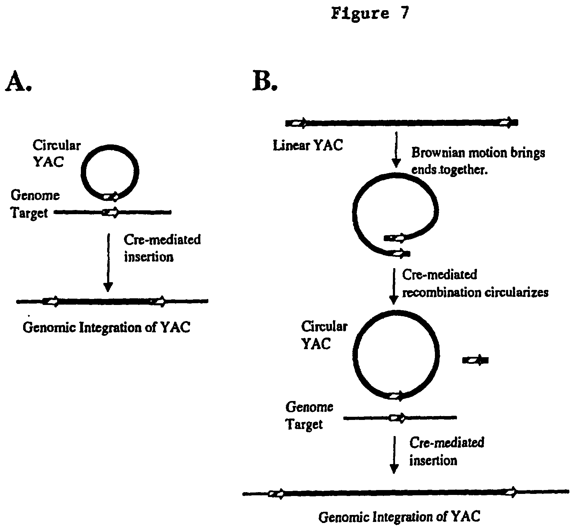

Alternatively, the AC, such as a YAC, harboring the human Ig genes or fragments thereof can contain site specific recombinase target sites optionally located near each telomere. The YAC can first be circularized via application of the appropriate site specific recombinase and then inserted into an ungulate cell directly that contains its own site specific recombinase target site within it genome. Alternatively, the ungulate cell can harbor its own MAC, which contains a site specific recombinase target site. In this embodiment, the YAC can be inserted directly into the endogenous genome of the ungulate cell. In particular embodiments, the ungulate cell can be a fibroblast cell or any other suitable cell that can be used for nuclear transfer. See, for example, Figure 7; Call et al., Hum Mol Genet. 2000 Jul 22;9(12):1745-51.

Methods to circularize at least 100 kb of DNA are disclosed herein wherein the DNA can then be integrated into a host genome via a site specific recombinase. In one embodiment, at least 100, 200, 300, 400, 500, 1000, 2000, 5000, 10,000 kb of DNA can be circularized. In another embodiment, at least 1000, 2000, 5000, 10,000, or 20,000 megabases of DNA can be circularized. In one embodiment, the circularization of the DNA can be accomplished by attaching site specific recombinase target sites at each end of the DNA sequence and then applying the site specific recombinase to result in circularization of the DNA. In one embodiment, the site specific recombinase target site can be lox. In another embodiment, the site specific recombinase target site can be Flt. In certain embodiments, the DNA can be an artificial chromosome, such as a YAC or any AC described herein or known in the art. In another embodiment, the AC can contain human immunoglobulin loci or fragments thereof.

In another preferred embodiment, the YAC can be converted to, or integrated within, an artificial mammalian chromosome. The mammalian artificial chromosome is either transferred to or harbored within a porcine cell. The artificial chromosome can be introduced within the porcine genome through any method known in the art including but not limited to direct injection of metaphase chromosomes, lipid mediated gene transfer, or microcell fusion.

Site-specific recombinases include enzymes or recombinases that recognize and bind to a short nucleic acid site or sequence-specific recombinase target site, i.e., a recombinase recognition site, and catalyze the recombination of nucleic acid in relation to these sites. These enzymes include recombinases, transposases and integrases. Examples of sequence-specific recombinase target sites include, but are not limited to, lox sites, aft sites, dif sites and frt sites. Non-limiting examples of site-specific recombinases include, but are not limited to, bacteriophage P1 Cre recombinase, yeast FLP recombinase, Inti integrase, bacteriophage A., phi 80, P22, P2, 186, and P4 recombinase, Tn3 resolvase, the Hin recombinase, and the Cin recombinase, *E. coil* xerC and xerD recombinases, *Bacillus thuringiensis* recombinase, TpnI and the 13-lactamase transposons, and the immunoglobulin recombinases.

In one embodiment, the recombination site can be a lox site that is recognized by the Cre recombinase of bacteriophage PI. Lox sites refer to a nucleotide sequence at which the product of the cre gene of bacteriophage P1, the Cre recombinase, can catalyze a site-specific recombination event. A variety of lox sites are known in the art, including the naturally occurring loxP, loxB, loxL and loxR, as well as a number of mutant, or variant, lox sites, such as loxP511, loxP514, lox.DELTA.86, lox.DELTA.117, loxC2, loxP2, loxP3 and lox P23. Additional example of lox sites include, but are not limited to, loxB, loxL, loxR, loxP, loxP3, loxP23, lox6,86, lox6.117, loxP511, and loxC2.

In another embodiment, the recombination site is a recombination site that is recognized by a recombinases other than Cre. In one embodiment, the recombinase site can be the FRT sites recognized by FLP recombinase of the 2 pi plasmid of *Saccharomyces cerevisiae.* FRT sites refer to a nucleotide sequence at which the product of the FLP gene of the yeast 2 micron plasmid, FLP recombinase, can catalyze site-specific recombination. Additional examples of the non-Cre recombinases include, but are not limited to, site-specific recombinases include: an sites recognized by the hit recombinase of bacteriophage (e.g. att1, att2, att3, attP, attB, attL, and attR), the recombination sites recognized by the resolvase family, and the recombination site recognized by transposase of *Bacillus thuringiensis.*

### IV. Production of Genetically Modified Animals

In additional aspects of the present invention, porcine animals that contain the genetic modifications described herein can be produced by any method known to one skilled in the art. Such methods include, but are not limited to: nuclear transfer, intracytoplasmic sperm injection, modification of zygotes directly and sperm mediated gene transfer.

In another embodiment, a method to clone such animals, for example, pigs, includes: enucleating an oocyte, fusing the oocyte with a donor nucleus from a cell in which at least one allele of at least one immunoglobulin gene has been inactivated, and implanting the nuclear transfer-derived embryo into a surrogate mother.

Alternatively, a method is disclosed herein for producing viable animals that lack any expression of functional immunoglobulin by inactivating both alleles of the immunoglobulin gene in embryonic stem cells, which can then be used to produce offspring.

Disclosed herein is a method for producing viable animals, such as pigs, in which both alleles of the immunoglobulin gene have been rendered inactive. The animals may be produced by cloning using a donor nucleus from a cell in which both alleles of the immunoglobulin gene have been inactivated. Both alleles of the immunoglobulin gene may be inactivated via a genetic targeting event.

Genetically altered animals that can be created by modifying zygotes directly. For mammals, the modified zygotes can be then introduced into the uterus of a pseudopregnant female capable of carrying the animal to term. For example, if whole animals lacking an immunoglobulin gene are desired, then embryonic stem cells derived from that animal can be targeted and later introduced into blastocysts for growing the modified cells into chimeric animals. For embryonic stem cells, either an embryonic stem cell line or freshly obtained stem cells can be used.

The totipotent cells may be embryonic stem (ES) cells. The isolation of ES cells from blastocysts, the establishing of ES cell lines and their subsequent cultivation are carried out by conventional methods as described, for example, by Doetclunann et al., J. Embryol. Exp. Morph. 87:27-45 (1985); Li et al., Cell 69:915-926 (1992); Robertson, E. J. "Tetracarcinomas and Embryonic Stem Cells: A Practical Approach," ed. E. J. Robertson, IRL Press, Oxford, England (1987); Wurst and Joyner, "Gene Targeting: A Practical Approach," ed. A. L. Joyner, IRL Press, Oxford, England (1993); Hogen et al., "Manipulating the Mouse Embryo: A Laboratory Manual," eds. Hogan, Beddington, Costantini and Lacy, Cold Spring Harbor Laboratory Press, New York (1994); and Wang et al., Nature 336:741-744 (1992). The totipotent cells may be embryonic germ (EG) cells. Embryonic Germ cells are undifferentiated cells functionally equivalent to ES cells, that is they can be cultured and transfected in vitro, then contribute to somatic and germ cell lineages of a chimera (Stewart et al., Dev. Biol. 161:626-628 (1994)). EG cells are derived by culture of primordial germ cells, the progenitors of the gametes, with a combination of growth factors: leukemia inhibitory factor, steel factor and basic fibroblast growth factor (Matsui et al., Cell 70:841-847 (1992); Resnick et al., Nature 359:550-551 (1992)). The cultivation of EG cells can be carried out using methods described in the article by Donovan et al., "Transgenic Animals, Generation and Use," Ed. L. M. Houdebine, Harwood Academic Publishers (1997), and in the original literature cited therein.

Tetraploid blastocysts may be obtained by natural zygote production and development, or by known methods by electrofusion of two-cell embryos and subsequently cultured as described, for example, by James et al., Genet. Res. Camb. 60:185-194 (1992); Nagy and Rossant, "Gene Targeting: A Practical Approach," ed. A. L. Joyner, IRL Press, Oxford, England (1993); or by Kubiak and Tarkowslci, Exp. Cell Res. 157:561-566 (1985).

The introduction of the ES cells or EG cells into the blastocysts can be carried out by any method known in the art. A suitable method is the microinjection method as described by Wang et al., EMBO J. 10:2437-2450 (1991).

Alternatively, by modified embryonic stem cells transgenic animals can be produced. The genetically modified embryonic stem cells can be injected into a blastocyst and then brought to term in a female host mammal in accordance with conventional techniques. Heterozygous progeny can then be screened for the presence of the alteration at the site of the target locus, using techniques such as PCR or Southern blotting. After mating with a wild-type host of the same species, the resulting chimeric progeny can then be cross-mated to achieve homozygous hosts.

After transforming embryonic stem cells with the targeting vector to alter the immunoglobulin gene, the cells can be plated onto a feeder layer in an appropriate medium, e.g., fetal bovine serum enhanced DMEM. Cells containing the construct can be detected by employing a selective medium, and after sufficient time for colonies to grow, colonies can be picked and analyzed for the occurrence of homologous recombination. Polymerase chain reaction can be used, with primers within and without the construct sequence but at the target locus. Those colonies which show homologous recombination can then be used for embryo manipulating and blastocyst injection. Blastocysts can be obtained from superovulated females. The embryonic stem cells can then be trypsinized and the modified cells added to a droplet containing the blastocysts. At least one of the modified embryonic stem cells can be injected into the blastocoel of the blastocyst. After injection, at least one of the blastocysts can be returned to each uterine horn of pseudopregnant females. Females are then allowed to go to term and the resulting litters screened for mutant cells having the construct. The blastocysts are selected for different parentage from the transformed ES cells. By providing for a different phenotype of the blastocyst and the ES cells, chimeric progeny can be readily detected, and then genotyping can be conducted to probe for the presence of the modified immunoglobulin gene.

In other embodiments, sperm mediated gene transfer can be used to produce the genetically modified ungulates described herein. The methods and compositions described herein to either eliminate expression of endogenous immunoglobulin genes or insert xenogenous immunoglobulin genes can be used to genetically modify the sperm cells via any technique described herein or known in the art. The genetically modified sperm can then be used to impregnate a female recipient via artificial insemination, intracytoplasmic sperm injection or any other known technique. In one embodiment, the sperm and/ or sperm head can be incubated with the exogenous nucleic acid for a sufficient time period. Sufficient time periods include, for example, about 30 seconds to about 5 minutes, typically about 45 seconds to about 3 minutes, more typically about 1 minute to about 2 minutes. In particular embodiments, the expression of xenogenous, such as human, immunoglobulin genes in ungulates as descrbed herein, can be accomplished via intracytoplasmic sperm injection.

The potential use of sperm cells as vectors for gene transfer was first suggested by Brackett et al., Proc., Natl. Acad. Sci. USA 68:353-357 (1971). This was followed by reports of the production of transgenic mice and pigs after in vitro fertilization of oocytes with sperm that had been incubated by naked DNA (see, for example, Lavitrano et al., Cell 57:717-723 (1989) and Gandolfi et al. Journal of Reproduction and Fertility Abstract Series 4, 10 (1989)), although other laboratories were not able to repeat these experiments (see, for example, Brinster et al. Cell 59:239-241 (1989) and Gavora et al., Canadian Journal of Animal Science 71:287-291 (1991)). Since then, there have been several reports of successful sperm mediated gene transfer in chicken (see, for example, Nakanishi and Iritani, Mol. Reprod. Dev. 36:258-261 (1993)); mice (see, for example, Maione, Mol. Reprod. Dev. 59:406 (1998)); and pigs (see, for example, Lavitrano et al. Transplant. Proc. 29:3508-3509 (1997); Lavitrano et al., Proc. Natl. Acad. Sci. USA 99:14230-5 (2002); Lavitrano et al., Mol. Reprod. Dev. 64-284-91 (2003)). Similar techniques are also described in U.S. Pat. No. 6,376,743; issued Apr. 23, 2002; U.S. Patent Publication Nos. 20010044937, published Nov. 22, 2001, and 20020108132, published Aug. 8,2002.

In other embodiments, intracytoplasmic sperm injection can be used to produce the genetically modified ungulates described herein. This can be accomplished by coinserting an exogenous nucleic acid and a sperm into the cytoplasm of an unfertilized oocyte to form a transgenic fertilized oocyte, and allowing the transgenic fertilized oocyte to develop into a transgenic embryo and, if desired, into a live offspring. The sperm can be a membrane-disrupted sperm head or a demembranated sperm head. The coinsertion step can include the substep of preincubating the sperm with the exogenous nucleic acid for a sufficient time period, for example, about 30 seconds to about 5 minutes, typically about 45 seconds to about 3 minutes, more typically about 1 minute to about 2 minutes. The coinsertion of the sperm and exogenous nucleic acid into the oocyte can be via microinjection. The exogenous nucleic acid mixed with the sperm can contain more than one transgene, to produce an embryo that is transgenic for more than one transgene as described herein. The intracytoplasmic sperm injection can be accomplished by any technique known in the art, see, for example, US Patent No. 6,376,743. In particular embodiments, the expression of xenogenous, such as human, immunoglobulin genes in ungulates as described herein, can be accomplished via intracytoplasmic sperm injection.

Any additional technique known in the art may be used to introduce the transgene into animals. Such techniques include, but are not limited to pronuclear microinjection (see, for example, Hoppe, P. C. and Wagner, T. E., 1989, U.S. Pat. No. 4,873,191); retrovirus mediated gene transfer into germ lines (see, for example, Van der Putten et al., 1985, Proc. Natl. Acad. Sci., USA 82:6148-6152); gene targeting in embryonic stem cells (see, for example, Thompson et al., 1989, Cell 56:313-321; Wheeler, M. B., 1994, WO 94/26884); electroporation of embryos (see, for example, Lo, 1983, Mol Cell. Biol. 3:1803-1814); cell gun; transfection; transduction; retroviral infection; adenoviral infection; adenoviral-associated infection; liposome-mediated gene transfer; naked DNA transfer; and sperm-mediated gene transfer (see, for example, Lavitrano et al., 1989, Cell 57:717-723); etc. For a review of such techniques, see, for example, Gordon, 1989, Transgenic Animals, Intl. Rev. Cytol. 115:171-229. In particular embodiments, the expression of xenogenous, such as human, immunoglobulin genes in ungulates as described herein, can be accomplished via these techniques.

### Somatic Cell Nuclear Transfer to Produce Cloned, Transgenic Offspring

Ungulate, such as porcine or bovine, cells lacking expression of one allele, optionally both alleles of an ungulate heavy chain, kappa light chain and/or lambda light chain gene can be used as donor cells for nuclear transfer into recipient cells to produce cloned, transgenic animals. Alternatively, ungulate heavy chain, kappa light chain and/or lambda light chain gene knockouts can be created in embryonic stem cells, which are then used to produce offspring. Offspring lacking expression of a single allele of a functional ungulate heavy chain, kappa light chain and/or lambda light chain gene produced according to the process, sequences and/or constructs described herein can be breed to further produce offspring lacking functionality in both alleles through mendelian type inheritance.

Disclosed herein is a method for producing viable pigs that lack any expression of functional alpha-1,3-GT by breeding a male pig heterozygous for the alpha-1,3-GT gene with a female pig heterozygous for the alpha-1,3-GT gene. In one embodiment, the pigs are heterozygous due to the genetic modification of one allele of the alpha1,3-GT gene to prevent expression of that allele. In another embodiment, the pigs are heterozygous due to the presence of a point mutation in one allele of the alpha-1,3-GT gene. In another embodiment, the point mutation can be a T-to-G point mutation at the second base of exon 9 of the alpha-1,3-GT gene. In one specific embodiment, a method to produce a porcine animal that lacks any expression of functional alpha-1,3-GT is disclosed herein wherein a male pig that contains a T-toG point mutation at the second base of exon 9 of the alpha-1,3-GT gene is bred with a female pig that contains a T-to-G point mutation at the second base of exon 9 of the alpha1,3-GT gene, or vise versa.

Disclosed herein is a method for cloning an animal, such as a pig, lacking a functional immunoglobulin gene via somatic cell nuclear transfer. In general, the animal can be produced by a nuclear transfer process comprising the following steps: obtaining desired differentiated cells to be used as a source of donor nuclei; obtaining oocytes from the animal; enucleating said oocytes; transferring the desired differentiated cell or cell nucleus into the enucleated oocyte, e.g., by fusion or injection, to form NT units; activating the resultant NT unit; and transferring said cultured NT unit to a host animal such that the NT unit develops into a fetus.

Nuclear transfer techniques or nuclear transplantation techniques are known in the art(Dai et al. Nature Biotechnology 20:251-255; Polejaeva et al Nature 407:86-90 (2000); Campbell et al, Theriogenology, 43:181 (1995); Collas et al, Mol. Report Dev., 38:264-267 (1994); Keefer et al, Biol. Reprod., 50:935-939 (1994); Sims et al, Proc. Natl. Acad. Sci., USA, 90:6143-6147 (1993); WO 94/26884; WO 94/24274, and WO 90/03432, U.S. Pat. Nos. 4,944,384 and 5,057,420).

A donor cell nucleus, which has been modified to alter the immunoglobulin gene, is transferred to a recipient oocyte. The use of this method is not restricted to a particular donor cell type. The donor cell can be as described herein, see also, for example, Wilmut et al Nature 385 810 (1997); Campbell et al Nature 380 64-66 (1996); Dai et al., Nature Biotechnology 20:251-255, 2002 or Cibelli et al Science 280 1256-1258 (1998). All cells of normal karyotype, including embryonic, fetal and adult somatic cells which can be used successfully in nuclear transfer can be employed. Fetal fibroblasts are a particularly useful class of donor cells. Generally suitable methods of nuclear transfer are described in Campbell et al Theriogenology 43 181 (1995), Dai et al. Nature Biotechnology 20:251-255, Polejaeva et al Nature 407:86-90 (2000), Collas et al Mol. Reprod. Dev. 38 264-267 (1994), Keefer et al Biol. Reprod. 50 935-939 (1994), Sims et al Proc. Nat'1. Acad. Sci. USA 90 6143-6147 (1993), WO-A-9426884, WO-A-9424274, WO-A-9807841, WO-A-9003432, U.S. Pat. No. 4,994,384 and U.S. Pat. No. 5,057,420. Differentiated or at least partially differentiated donor cells can also be used. Donor cells can also be, but do not have to be, in culture and can be quiescent. Nuclear donor cells which are quiescent are cells which can be induced to enter quiescence or exist in a quiescent state in vivo._ Prior art methods have also used embryonic cell types in cloning procedures (Campbell et al (Nature, 380:64-68, 1996) and Stice et al (Biol. Reprod., 20 54:100-110, 1996).

Somatic nuclear donor cells may be obtained from a variety of different organs and tissues such as, but not limited to, skin, mesenchyme, lung, pancreas, heart, intestine, stomach, bladder, blood vessels, kidney, urethra, reproductive organs, and a disaggregated preparation of a whole or part of an embryo, fetus, or adult animal. Nuclear donor cells may be selected from the group consisting of epithelial cells, fibroblast cells, neural cells, keratinocytes, hematopoietic cells, melanocytes, chondrocytes, lymphocytes (B and T), macrophages, monocytes, mononuclear cells, cardiac muscle cells, other muscle cells, xtended cells, cumulus cells, epidermal cells or endothelial cells. In another embodiment, the nuclear donor cell is an embryonic stem cell. In a particular embodiment, fibroblast cells can be used as donor cells.

The nuclear donor cells may be germ cells of an animal. Any germ cell of an animal species in the embryonic, fetal, or adult stage may be used as a nuclear donor cell. In a suitable embodiment, the nuclear donor cell is an embryonic germ cell.

Nuclear donor cells may be arrested in any phase of the cell cycle (GO, G1, G2, S, M) so as to ensure coordination with the acceptor cell. Any method known in the art may be used to manipulate the cell cycle phase. Methods to control the cell cycle phase include, but are not limited to, G0 quiescence induced by contact inhibition of cultured cells, G0 quiescence induced by removal of serum or other essential nutrient, G0 quiescence induced by senescence, G0 quiescence induced by addition of a specific growth factor; G0 or G1 quiescence induced by physical or chemical means such as heat shock, hyperbaric pressure or other treatment with a chemical, hormone, growth factor or other substance; S-phase control via treatment with a chemical agent which interferes with any point of the replication procedure; M-phase control via selection using fluorescence activated cell sorting, mitotic shake off, treatment with microtubule disrupting agents or any chemical which disrupts progression in mitosis (see also Freshney, R. I,. "Culture of Animal Cells: A Manual of Basic Technique," Alan R. Liss, Inc, New York (1983).

Methods for isolation of oocytes are well known in the art. Essentially, this can comprise isolating oocytes from the ovaries or reproductive tract of an animal. A readily available source of oocytes is slaughterhouse materials. For the combination of techniques such as genetic engineering, nuclear transfer and cloning, oocytes must generally be matured in vitro before these cells can be used as recipient cells for nuclear transfer, and before they can be fertilized by the sperm cell to develop into an embryo. This process generally requires collecting immature (prophase I) oocytes from mammalian ovaries, e.g., bovine ovaries obtained at a slaughterhouse, and maturing the oocytes in a maturation medium prior to fertilization or enucleation until the oocyte attains the metaphase II stage, which in the case of bovine oocytes generally occurs about 18-24 hours post-aspiration. This period of time is known as the "maturation period". In certain embodiments, the oocyte is obtained from a gilt. A "gilt" is a female pig that has never had offspring. In other embodiments, the oocyte is obtained from a sow. A "sow" is a female pig that has previously produced offspring.

A metaphase II stage oocyte can be the recipient oocyte, at this stage it is believed that the oocyte can be or is sufficiently "activated" to treat the introduced nucleus as it does a fertilizing sperm. Metaphase II stage oocytes, which have been matured in vivo have been successfully used in nuclear transfer techniques. Essentially, mature metaphase II oocytes can be collected surgically from either non-superovulated or superovulated animal 35 to 48, or 39-41, hours past the onset of estrus or past the injection of human chorionic gonadotropin (hCG) or similar hormone. The oocyte can be placed in an appropriate medium, such as a hyalurodase solution.

After a fixed time maturation period, which ranges from about 10 to 40 hours, about 16-18 hours, about 40-42 hours or about 39-41 hours, the oocytes can be enucleated. Prior to enucleation the oocytes can be removed and placed in appropriate medium, such as HECM containing 1 milligram per milliliter of hyaluronidase prior to removal of cumulus cells. The stripped oocytes can then be screened for polar bodies, and the selected metaphase II oocytes, as determined by the presence of polar bodies, are then used for nuclear transfer. Enucleation follows.

Enucleation can be performed by known methods, such as described in U.S. Pat. No. 4,994,384. For example, metaphase II oocytes can be placed in either HECM, optionally containing 7.5 micrograms per milliliter cytochalasin B, for immediate enucleation, or can be placed in a suitable medium, for example an embryo culture medium such as CR1aa, plus 10% estrus cow serum, and then enucleated later, such as not more than 24 hours later,or not more than 16-18 hours later.

Enucleation can be accomplished microsurgically using a micropipette to remove the polar body and the adjacent cytoplasm. The oocytes can then be screened to identify those of which have been successfully enucleated. One way to screen the oocytes is to stain the oocytes with 1 microgram per milliliter 33342 Hoechst dye in HECM, and then view the oocytes under ultraviolet irradiation for less than 10 seconds. The oocytes that have been successfully enucleated can then be placed in a suitable culture medium, for example, CR1aa plus 10% serum.

A single mammalian cell of the same species as the enucleated oocyte can then be transferred into the perivitelline space of the enucleated oocyte used to produce the NT unit. The mammalian cell and the enucleated oocyte can be used to produce NT units according to methods known in the art. For example, the cells can be fused by electrofusion. Electrofusion is accomplished by providing a pulse of electricity that is sufficient to cause a transient breakdown of the plasma membrane. This breakdown of the plasma membrane is very short because the membrane reforms rapidly. Thus, if two adjacent membranes are induced to breakdown and upon reformation the lipid bilayers intermingle, small channels can open between the two cells. Due to the thermodynamic instability of such a small opening, it enlarges until the two cells become one. See, for example, U.S. Pat. No. 4,997,384 by Prather et al. A variety of electrofusion media can be used including, for example, sucrose, mannitol, sorbitol and phosphate buffered solution. Fusion can also be accomplished using Sendai virus as a fusogenic agent (Graham, Wister Inot. Symp. Monogr., 9, 19, 1969). Also, the nucleus can be injected directly into the oocyte rather than using electroporation fusion. See, for example, Collas and Barnes, Mol. Reprod. Dev., 38:264-267 (1994). After fusion, the resultant fused NT units are then placed in a suitable medium until activation, for example, CR1aa medium. Typically activation can be effected shortly thereafter, for example less than 24 hours later, or about 4-9 hours later, or optimally 1-2 hours after fusion. In a particular embodiment, activation occurs at least one hour post fusion and at 40-41 hours post maturation.

The NT unit can be activated by known methods. Such methods include, for example, culturing the NT unit at sub-physiological temperature, in essence by applying a cold, or actually cool temperature shock to the NT unit. This can be most conveniently done by culturing the NT unit at room temperature, which is cold relative to the physiological temperature conditions to which embryos are normally exposed. Alternatively, activation can be achieved by application of known activation agents. For example, penetration of oocytes by sperm during fertilization has been shown to activate prefusion oocytes to yield greater numbers of viable pregnancies and multiple genetically identical calves after nuclear transfer. Also, treatments such as electrical and chemical shock can be used to activate NT embryos after fusion. See, for example, U.S. Pat. No. 5,496,720, to Susko-Parrish et al. Fusion and activation can be induced by application of an AC pulse of 5 V for 5 s followed by two DC pulses of 1.5 kV/cm for 60 µs each using an ECM2001 Electrocell Manipulator (BTX Inc., San Diego, CA). Additionally, activation can be effected by simultaneously or sequentially by increasing levels of divalent cations in the oocyte, and reducing phosphorylation of cellular proteins in the oocyte. This can generally be effected by introducing divalent cations into the oocyte cytoplasm, e.g., magnesium, strontium, barium or calcium, e.g., in the form of an ionophore. Other methods of increasing divalent cation levels include the use of electric shock, treatment with ethanol and treatment with caged chelators. Phosphorylation can be reduced by known methods, for example, by the addition of kinase inhibitors, e.g., serine-threonine kinase inhibitors, such as 6-dimethyl-aminopurine, staurosporine, 2-aminopurine, and sphingosine. Alternatively, phosphorylation of cellular proteins can be inhibited by introduction of a phosphatase into the oocyte, e.g., phosphatase 2A and phosphatase 2B.

The activated NT units, or "fused embyos", can then be cultured in a suitable in vitro culture medium until the generation of cell colonies. Culture media suitable for culturing and maturation of embryos are well known in the art. Examples of known media, which can be used for embryo culture and maintenance, include Ham's F-10+10% fetal calf serum (FCS), Tissue Culture Medium-199 (TCM-199)+10% fetal calf serum, Tyrodes-Albumin-Lactate-Pyruvate (TALP), Dulbecco's Phosphate Buffered Saline (PBS), Eagle's and Whitten's media, and, in one specific example, the activated NT units can be cultured in NCSU-23 medium for about 1-4 h at approximately 38.6°C in a humidified atmosphere of 5% CO2.

Afterward, the cultured NT unit or units can be washed and then placed in a suitable media contained in well plates which can contain a suitable confluent feeder layer. Suitable feeder layers include, by way of example, fibroblasts and epithelial cells. The NT units are cultured on the feeder layer until the NT units reach a size suitable for transferring to a recipient female, or for obtaining cells which can be used to produce cell colonies. These NT units can be cultured until at least about 2 to 400 cells, about 4 to 128 cells, or at least about 50 cells.

Activated NT units can then be transferred (embryo transfers) to the oviduct of an female pigs. In one embodiment, the female pigs can be an estrus-synchronized recipient gilt. Crossbred gilts (large white/Duroc/Landrace) (280-400 lbs) can be used. The gilts can be synchronized as recipient animals by oral administration of 18-20 mg Regu-Mate (Altrenogest, Hoechst, Warren, NJ) mixed into the feed. Regu-Mate can be fed for 14 consecutive days. One thousand units of Human Chorionic Gonadotropin (hCG, Intervet America, Millsboro, DE) can then be administered i.m. about 105 h after the last Regu-Mate treatment. Embryo transfers can then be performed about 22-26 h after the hCG injection. In one embodiment, the pregnancy can be brought to term and result in the birth of live offspring. In another embodiment, the pregnancy can be terminated early and embryonic cells can be harvested.

### Breeding for Desired Homozygous Knockout Animals

Disclosed herein is a method for producing viable animals that lack any expression of a functional inununoglobulin gene by breeding a male heterozygous for the immunoglobulin gene with a female heterozygous for the immunoglobulin gene. The animals may be heterozygous due to the genetic modification of one allele of the immunoglobulin gene to prevent expression of that allele.
The animals may be heterozygous due to the presence of a point mutation in one allele of the alpha-immunoglobulin gene. Such heterozygous knockouts can be bred with an ungulate that expresses xenogenous immunoglobulin, such as human. An animal can be obtained by breeding a transgenic ungulate that lacks expression of at least one allele of an endogenous immunoglobulin wherein the immunoglobulin is selected from the group consisting of heavy chain, kappa light chain and lambda light chain or any combination thereof with an ungulate that expresses an xenogenous immunoglobulin. An animal can be obtained by breeding a transgenic ungulate that lacks expression of one allele of heavy chain, kappa light chain and lambda light chain with an ungulate that expresses an xenogenous, such as human, immunoglobulin. An animal can be obtained by breeding a transgenic ungulate that lacks expression of one allele of heavy chain, kappa light chain and lambda light chain and expresses an xenogenous, such as human, immunoglobulin with another transgenic ungulate that lacks expression of one allele of heavy chain, kappa light chain and lambda light chain with an ungulate and expresses an xenogenous, such as human, immunoglobulin to produce a homozygous transgenic ungulate that lacks expression of both alleles of heavy chain, kappa light chain and lambda light chain and expresses an xenogenous, such as human, immunoglobulin. Methods to produce such animals are also disclosed herein.

In one embodiment, sexually mature animals produced from nuclear transfer from donor cells that carrying a double knockout in the immunoglobulin gene, can be bred and their offspring tested for the homozygous knockout. These homozygous knockout animals can then be bred to produce more animals.

In another embodiment, oocytes from a sexually mature double knockout animal can be in vitro fertilized using wild type sperm from two genetically diverse pig lines and the embryos implanted into suitable surrogates. Offspring from these matings can be tested for the presence of the knockout, for example, they can be tested by cDNA sequencing, and/ or PCR. Then, at sexual maturity, animals from each of these litters can be mated. In certain methods disclosed herein, pregnancies can be terminated early so that fetal fibroblasts can be isolated and further characterized phenotypically and/or genotypically. Fibroblasts that lack expression of the immunoglobulin gene can then be used for nuclear transfer according to the
methods described herein to produce multiple pregnancies and offspring carrying the desired double knockout.

### Additional Genetic Modifications

In other embodiments, animals or cells lacking expression of functional immunoglobulin, produced according to the process, sequences and/or constructs described herein, can contain additional genetic modifications to eliminate the expression of xenoantigens. The additional genetic modifications can be made by further genetically modifying cells obtained from the transgenic cells and animals described herein or by breeding the animals described herein with animals that have been further genetically modified. Such animals can be modified to climate the expression of at least one allele of the alpha-1,3-galactosyltransferase gene, the CM1)-Neu5Ac hydroxylase gene (see, for example, USSN 10/863,116), the iGb3 synthase gene (see, for example, U.S. Patent Application 60/517,524), and/or the Forssman synthase gene (see, for example,.U,S. Patent Application 60/568,922). In additional embodiments, the animals discloses herein can also contain genetic modifications to express fucosyltransferase, sialyltransferase and/ or any member of the family of glucosyltransferases. To achieve these additional genetic modifications, in one embodiment, cells can be modified to contain multiple genetic modifications. In other embodiments, animals can be bred together to achieve multiple genetic modifications. In one specific embodiment, animals, such as pigs, lacking expression of functional immunoglobulin, produced according to the process, sequences and/or constructs described herein, can be bred with animals, such as pigs, lacking expression of alpha-1,3-galactosyl transferase (for example, as described in WO 04/028243).

In another embodiment, the expression of additional genes responsible for xenograft rejection can be eliminated or reduced. Such genes include, but are not limited to the CMP-NEUAc Hydroxylase Gene, the isoGloboside 3 Synthase gene, and the Forssman synthase gene. In addition, genes or cDNA encoding complement related proteins, which are responsible for the suppression of complement mediated lysis can also be expressed in the animals and tissues of the present invention. Such genes include,, but are not limited to CD59, DAF, MCP and CD46 (see, for example, WO 99/53042; Chen et al. Xenotransplantation, Volume 6 Issue 3 Page 194 - August 1999, which describes pigs that express CD59/DAF transgenes; Costa C et al, Xenotransplantation. 2002 Jan;9(1):45-57, which describes transgenic pigs that express human CD59 and H-transferase; Zhao L et al.; Diamond LE et al. Transplantation. 2001 Jan 15;71(1):132-42, which describes a human CD46 transgenic pigs.

Additional modifications can include expression of tissue factor pathway inhibitor (TFPI). heparin, antithrombin, hirudin, TFPI, tick anticoagulant peptide, or a snake venom factor, such as described in WO 98/42850 and US Patent No. 6,423,316, entitled "Anticoagulant fusion protein anchored to cell membrane"; or compounds, such as antibodies, which down-regulate the expression of a cell adhesion molecule by the cells, such as described in WO 00/31126, entitled "Suppression of xenograft rejection by down regulation of a cell adhesion molecules" and compounds in which co-stimulation by signal 2 is prevented, such as by administration to the organ recipient of a soluble form of CTLA-4 from the xenogeneic donor organism, for eample as described in WO 99/57266, entitled "Immunosuppression by blocking T cell co-stimulation signal 2 (B7/CD28 interaction)".

Certain aspects of the invention are described in greater detail in the non-limiting Examples that follow.

### EXAMPLES

### EXAMPLE 1:Porcine Heavy Chain Targeting and Generation of Porcine Animals that Lack Expression of Heavy Chain

A portion of the porcine Ig heavy-chain locus was isolated from a 3X redundant porcine BAC library. In general, BAC libraries can be generated by fragmenting pig total genomic DNA, which can then be used to derive a BAC library representing at least three times the genome of the whole animal. BACs that contain porcine heavy chain immunoglobulin can then be selected through hybridization of probes selective for porcine heavy chain immunoglobulin as described herein.

Sequence from a clone (Seq ID 1) was used to generate a primer complementary to a portion of the J-region (the primer is represented by Seq ID No. 2). Separately, a primer was designed that was complementary to a portion of Ig heavy-chain mu constant region (the promer is represented by Seq ID No. 3). These primers were used to amplify a fragment of porcine Ig heavy-chain (represented by Seq ID No. 4) that led the functional joining region (J-region) and sufficient flanking region to design and build a targeting vector. To maintain this fragment and sublcones of this fragment in a native state, the *E. coli* (Stable 2, Invitrogen cat #1026-019) that harbored these fragments was maintained at 30°C. Regions of Seq. ID No. 4 were subcloned and used to assemble a targeting vector as shown in Seq. ID No. 5. This vector was transfected into porcine fetal fibroblasts that were subsequently subjected to selection with G418. Resulting colonies were screened by PCR to detect potential targeting events (Seq ID No. 6 and Seq ID No. 7, 5' screen prmers; and Seq ID No. 8 and Seq ID No. 9, 3' screen primers). See Figure 1. for a schematic illustrating the targeting. Targeting was confirmed by southern blotting. Piglets were generated by nuclear transfer using the targeted fetal fibroblasts as nuclear donors.

### Nuclear Transfer.

The targeted fetal fibroblasts were used as nuclear donor cells. Nuclear transfer was performed by methods that are well known in the art (see, e.g., Dai et al., Nature Biotechnology 20: 251-255, 2002; and Polejaeva et al., Nature 407:86-90, 2000).

Oocytres were collected 46-54 h after the hCG injection by reverse flush of the oviducts using pre-warmed Dulbecco's phosphate buffered saline (PBS) containing bovine serum albumin (BSA; 4 gl⁻¹) (as described in Polejaeva, I.A., et al. (Nature 407, 86-90 (2000)). Enucleation of in *vitro*-matured oocytes (BioMed, Madison, WI) was begun between 40 and 42 hours post-maturation as described in Polejaeva, I.A., et al. (Nature 407, 86-90 (2000)). Recovered oocytes were washed in PBS containing 4 gl⁻¹ BSA at 38°C, and transferred to calcium-free phosphate-buffered NCSU-23 medium at 38°C for transport to the laboratory. For enucleation, we incubated the oocytes in calcium-free phosphate-buffered NCSU-23 medium containing 5 µg ml⁻¹ cytochalasin B (Sigma) and 7.5 µg ml⁻¹ Hoechst 33342 (Sigma) at 38°C for 20 min. A small amount of cytoplasm from directly beneath the first polar body was then aspirated using an 18 µM glass pipette (Humagen, Charlottesville, Virginia). We exposed the aspirated karyoplast to ultraviolet light to confirm the presence of a metaphase plate.

For nuclear transfer, a single fibroblast cell was placed under the zona pellucida in contact with each enucleated oocyte. Fusion and activation were induced by application of an AC pulse of 5 V for 5 s followed by two DC pulses of 1.5 kV/cm for 60 µs each using an ECM2001 Electrocell Manipulator (BTX Inc., San Diego, CA). Fused embryos were cultured in NCSU-23 medium for 1-4 h at 38.6°C in a humidified atmosphere of 5% CO₂, and then transferred to the oviduct of an estrus-synchronized recipient gilt. Crossbred gilts (large white/Duroc/landrace) (280-400 lbs) were synchronized as recipients by oral administration of 18-20 mg Regu-Mate (Altrenogest, Hoechst, Warren, NJ) mixed into their feed. Regu-Mate was fed for 14 consecutive days. Human chorionic gonadotropin (hCG, 1,000 units; Intervet America, Millsboro, DE) was administered intra-muscularly 105 h after the last Regu-Mate treatment. Embryo transfers were done 22-26 h after the hCG injection.

Nuclear transfer produced 18 healthy piglets from four litters. These animals have one functional wild-type Ig heavy-chain locus and one disrupted Ig heavy chain locus.

| | |
|---|---|
| Seq ID 2: primer from Butler subclone to amplify J to C heavychain (637Xba5') | ggccagacttcctcggaacagctca |
| | |
| Seq ID 3: primer for C to amplify J to C heavychain (JM1L) | ttccaggagaaggtgacggagct |
| | |
| Seq ID 6: heavychain 5' primer for 5' screen (HCKOXba5'2) | tctagaagacgctggagagaggccag |
| | |
| Seq ID 7: heavychain 3' primer for 5' screen (5'arm5') | taaagcgcatgctccagactgcctt |
| | |
| Seq ID 8: heavychain 5' primer for 3' screen (NEO4425) | catcgccttctatcgccttctt |
| | |
| Seq ID 9: heavychain 3' primer for 3' screen (650+CA) | Aagtacttgccgcctctcagga |

*Southern blot analysis of cell and pig tissue samples.* Cells or tissue samples were lysed overnight at 60°C in lysis buffer (10mM Tris, pH 7.5, 10mM EDTA, 10mM NaCl, 0.5% (w/v) Sarcosyl, 1 mg/ml proteinase K) and the DNA precipitated with ethanol. The DNA was then digested with NcoI or XbaI, depending on the probe to be used, and separated on a 1% agarose gel. After electrophoresis, the DNA was transferred to a nylon membrane and probed with digoxigenin-labeled probe (SEQ ID No 41 for NcoI digest, SEQ ID No 40 for XbaI digest). Bands were detected using a chemiluminescent substrate system (Roche Molecular Biochemicals).

### Probes for Heavy Chain Southern:

HC J Probe (used with Xba I digest)
HC Mu Probe (used with NcoI digest)

### EXAMPLE 2: Porcine Kappa Light Chain Targeting and Generation of Porcine Lacking Expression of Kappa Light Chain

A portion of the porcine Ig kappa-chain locus was isolated from a 3X redundant porcine BAC library. In general, BAC libraries can be generated by fragmenting pig total genomic DNA, which can then be used to derive a BAC library representing at least three times the genome of the whole animal. BACs that contain porcine kappa chain immunoglobulin can then be selected through hybridization of probes selective for porcine kappa chain immunoglobulin as described herein.

A fragment of porcine Ig light-chain kappa was amplified using a primer complementary to a portion of the J-region (the primer is represented by Seq ID No. 10) and a primer complementary to a region of kappa C-region (represented by Seq ID No. 11). The resulting amplimer was cloned into a plasmid vector and maintained in Stable2 cells at 30°C (Seq ID No. 12). See Figure 2 for a schematic illustration.

Separately, a fragment of porcine Ig light-chain kappa was amplified using a primer complementary to a portion of the C-region (Seq ID No. 13) and a primer complementary to a region of the kappa enhancer region (Seq ID No. 14). The resulting amplimer was fragmented by restriction enzymes and DNA fragments that were produced were cloned, maintained in Stable2 cells at 30 degrees C and sequenced. As a result of this sequencing, two nonoverlapping contigs were assembled (Seq ID No. 15, 5' portion of amplimer; and Seq ID No. 16, 3' portion of amplimer). Sequence from the downstream contig (Seq ID No. 16) was used to design a set of primers (Seq ID No. 17 and Seq ID No. 18) that were used to amplify a contiguous fragment near the enhancer (Seq ID No. 19). A subclone of each Seq ID No. 12 and Seq ID No. 19 were used to build a targeting vector (Seq ID No. 20). This vector was transfected into porcine fetal fibroblasts that were subsequently subjected to selection with G418. Resulting colonies were screened by PCR to detect potential targeting events (Seq ID No. 21 and Seq ID No. 22, 5' screen primers; and Seq ID No. 23 and Seq Id No 43, 3' screen primers, and Seq ID No. 24 and Seq Id No 24, endogenous screen primers). Targeting was confirmed by southern blotting. Southern blot strategy design was facilitated by cloning additional kappa sequence, it corresponds to the template for germline kappa transcript (Seq ID No. 25). Fetal pigs were generated by nuclear transfer.

### Nuclear Transfer.

The targeted fetal fibroblasts were used as nuclear donor cells. Nuclear transfer was performed by methods that are well known in the art (see, e.g., Dai et al., Nature Biotechnology 20: 251-255,2002; and Polejaeva et al., Nature 407:86-90,2000).

Oocytres were collected 46-54 h after the hCG injection by reverse flush of the oviducts using pre-warmed Dulbecco's phosphate buffered saline (PBS) containing bovine serum albumin (BSA; 4 gl⁻¹) (as described in Polejaeva, LA., et al. (Nature 407, 86-90 (2000)). Enucleation of in *vitro*-matured oocytes (BioMed, Madison, WI) was begun between 40 and 42 hours post-maturation as described in Polejaeva, I.A., et al. (Nature 407, 86-90 (2000)). Recovered oocytes were washed in PBS containing 4 gl⁻¹ BSA at 38°C, and transferred to calcium-free phosphate-buffered NCSU-23 medium at 38°C for transport to the laboratory. For enucleation, we incubated the oocytes in calcium-free phosphate-buffered NCSU-23 medium containing 5 µg ml⁻¹ cytochalasin B (Sigma) and 7.5 µg ml⁻¹ Hoechst 33342 (Sigma) at 38°C for 20 min. A small amount of cytoplasm from directly beneath the first polar body was then aspirated using an 18 µM glass pipette (Humagen, Charlottesville, Virginia). We exposed the aspirated karyoplast to ultraviolet light to confirm the presence of a metaphase plate.

For nuclear transfer, a single fibroblast cell was placed under the zona pellucida in contact with each enucleated oocyte. Fusion and activation were induced by application of an AC pulse of 5 V for 5 s followed by two DC pulses of 1.5 kV/cm for 60 µs each using an ECM2001 Electrocell Manipulator(BTX Inc., San Diego, CA). Fused embryos were cultured in NCSU-23 medium for 1-4 h at 38.6°C in a humidified atmosphere of 5% CO₂, and then transferred to the oviduct of an estrus-synchronized recipient gilt. Crossbred gilts (large white/Duroc/landrace) (280-400 lbs) were synchronized as recipients by oral administration of 18-20 mg Regu-Mate (Altrenogest, Hoechst, Warren, NJ) mixed into their feed. Regu-Mate was fed for 14 consecutive days. Human chorionic gonadotropin (hCG, 1,000 units; Intervet America, Millsboro, DE) was administered intra-muscularly 105 h after the last Regu-Mate treatment. Embryo transfers were done 22-26 h after the hCG injection.

Nuclear transfer using kappa targeted cells produced 33 healthy pigs from 5 litters. These pigs have one functional wild-type allele of porcine Ig light-chain kappa and one disrupted Ig light-chain kappa allele.

| | |
|---|---|
| Seq ID 10: kappa J to C 5' primer (kjc5'1) | caaggaqaccaagctggaactc |
| | |
| Seq ID 11: kappa J to C 3' primer (kjc3'2) | tgatcaagcacaccacagagacag |
| | |
| Seq ID 13: 5' primer for Kappa C to E (porKCS1) | gatgccaagccatccgtcttcatc |
| Seq ID 14: 3' primer for Kappa C to E (porKCA1) | tgaccaaagcagtgtgacggttgc |
| | |
| Seq ID 17: kappa 5' primer for amplification of enhancer region (K3'arm1S) | ggatcaaacacgcatcctcatggac |
| | |
| Seq ID 18: kappa 3' primer for amplification of enhancer region (K3'arm1A) | ggtgattggggcatggttgagg |
| | |
| Seq ID 21: kappa screen, 5' primer, 5' (kappa5armS) | cgaacccctgtgtatatagtt |
| | |
| Seq ID 22: kappa screen, 3' primer, 5' (kappaNeoA) | gagatgaggaagaggagaaca |
| | |
| Seq ID 23: kappa screen, 5' primer, 3' (kappaNeoS) | gcattgtctgagtaggtgtcatt |
| | |
| Seq ID 24: kappa screen, 3' primer, 5' (kappa5armProbe3') | cgcttcttgcagggaacacgat |
| | |
| Seq ID No 43, Kappa screen, 3' primer (kappa3armA2) | GTCTTTGGTTTTTGCTGAGGGTT |

*Southern blot analysis of cell and pig tissue samples.* Cells or tissue samples were lysed overnight at 60°C in lysis buffer (10mM Tris, pH 7.5, 10mM EDTA, 10mM NaCl, 0.5% (w/v) Sarcosyl, 1 mg/ml proteinase K) and the DNA precipitated with ethanol. The DNA was then digested with SacI and separated on a 1% agarose gel. After electrophoresis, the DNA was transferred to a nylon membrane and probed with digoxigenin-labeled probe (SEQ ID No 42). Bands were detected using a chemiluminescent substrate system (Roche Molecular Biochemicals).

### Probe for Kappa Southern:

Kappa5ArmProbe 5'/3'

### EXAMPLE 3

### Characterization of the Porcine Lambda Gene Locus

To disrupt or disable porcine lambda, a targeting strategy has been devised that allows for the removal or disruption of the region of the lambda locus that includes a concatamer of J to C expression cassettes. BAC clones that contain portions of the porcine genome can be generated. A portion of the porcine Ig lambda-chain locus was isolated from a 3X redundant porcine BAC library. In general, BAC libraries can be generated by fragmenting pig total genomic DNA, which can then be used to derive a BAC library representing at least three times the genome of the whole animal. BACs that contain porcine lambda chain immunoglobulin can then be selected through hybridization of probes selective for porcine lambdachain immunoglobulin as described herein.

BAC clones containing a lambda J-C flanking region (see Figure 3), can be independently fragmented and subcloned into a plasmid vector. Individual subclones have been screened by PCR for the presence of a portion of the J to C intron. We have cloned several of these cassettes by amplifying from one C region to the next C region. This amplification was accomplished by using primers that are oriented to allow divergent extension within any one C region (Seq ID 26 and Seq ID 27). To obtain successful amplification, the extended products converge with extended products originated from adjacent C regions (as opposed to the same C region). This strategy produces primarily amplimers that extend from one C to the adjacent C. However, some amplimers are the result of amplification across the adjacent C and into the next C which lies beyond the adjacent C. These multi-gene amplimers contain a portion of a C, both the J and C region of the next J-C unit, the J region of the third J-C unit, and a portion of the C region of the third J-C unit. Seq ID 28 is one such amplimer and represents sequence that must be removed or disrupted.

Other porcine lambda sequences that have been cloned include: Seq ID No. 32, which includes 5' flanking sequence to the first lambda J/C region of the porcine lambda light chain genomic sequence; Seq ID No. 33, which includes 3' flanking sequence to the J/C cluster region of the porcine lambda light chain genomic sequence, from approximately 200 base pairs downstream of lambda J/C; Seq ID No. 34, which includes 3' flanking sequence to the J/C cluster region of the porcine lambda light chain genomic sequence, approximately 11.8 Kb downstream of the J/C cluster, near the enhancer; Seq ID No. 35, which includes approximately 12 Kb downstream of lambda, including the enhancer region; Seq ID No. 36, which includes approximately 17.6 Kb downstream of lambda; Seq ID No. 37, which includes approximately 19.1 Kb downstream of lambda; Seq ID No. 38, which includes approximately 21.3 Kb downstream of lambda; and Seq ID No. 39, which includes approximately 27 Kb downstream of lambda.

| | |
|---|---|
| SeqID26: 5'primer for lambda C to C amplimer (lamC5') | ccttcctcctgcacctgtcaac |
| | |
| Seq ID 27: 3' primer for lambda C to C amplimer (lamC3') | tagacacaccagggtggccttg |

### Example 4

### Production of Targeting Vectors for the Lambda Gene

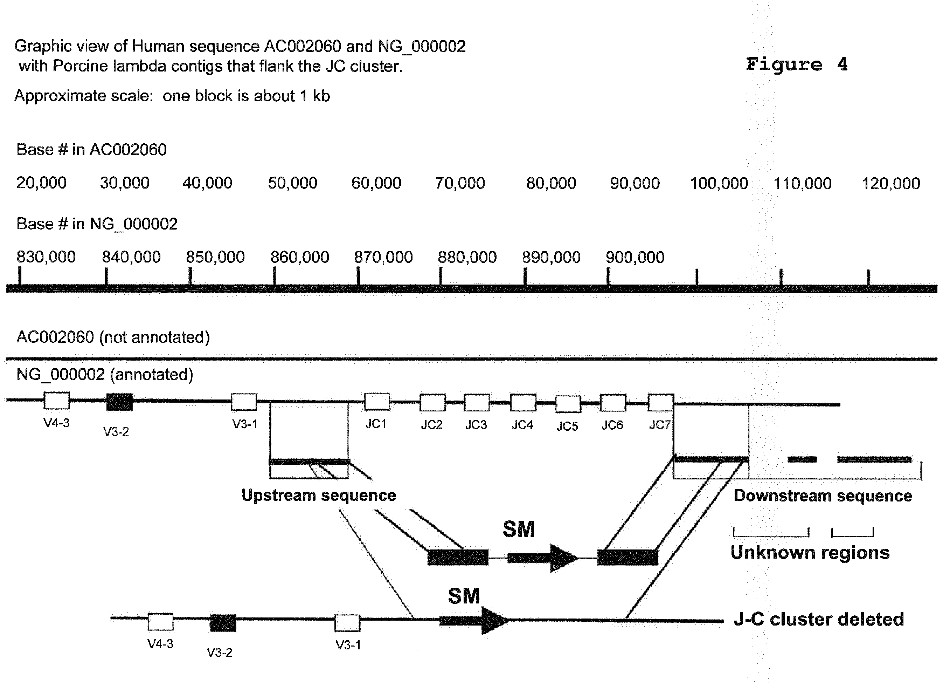

In one example, a vector has been designed and built with one targeting arm that is homologous to a region upstream of J1 and the other arm homologous to a region that is downstream of the last C (see Figure 4). One targeting vector is designed to target upstream of J1. This targeting vector utilizes a selectable marker that can be selected for or against. Any combination of positive and negative selectable markers described herein or known in the art can be used. A fusion gene composed of the coding region of Herpes simplex thymidine kinase (TK) and the Tn5 aminoglycoside phosphotransferase (Neo resistance) genes is used. This fusion gene is flanked by recognition sites for any site specific recombinase (SSRRS) described herein or known in the art, such as lox sites. Upon isolation of targeted cells through the use of G418 selection, Cre is supplied in trans to delete the marker gene (See Figure 5). Cells that have deleted the marker gene are selected by addition of any drug known in the art that can be metabolized by TK into a toxic product, such as ganciclovir. The resulting genotype is then targeted with a second vector. The second targeting vector (Figure 6) is designed to target downstream of last C and uses a positive/negative selection system that is flanked on only one side by a specific recombination site (lox). The recombination site is placed distally in relation to the first targeting event. Upon isolation of the targeted genotype, Cre is again supplied in trans to mediate deletion from recombination site provided in the first targeting event to the recombination site delivered in the second targeting event. The entire J to C cluster will be removed. The appropriate genotype is again selected by administration of ganciclovir.

In another example, insertional targeting vectors are used to disrupt each C regions independently. An insertional targeting vector will be designed and assembled to disrupt individual C region genes. There are at least 3 J to C regions in the J-C cluster. We will begin the process by designing vectors to target the first and last C regions and will include in the targeting vector site-specific recombination sites. Once both insertions have been made, the intervening region will be deleted with the site-specific recombinase.

### Example 5: Crossbreeding of Heavy chain single knockout with Kappa single knockout pigs.

To produce pigs that have both one disrupted Ig heavy chain locus and one disrupted Ig light-chain kappa allele, single knockout animals were crossbred. The first pregnancy yielded four fetuses, two of which screened positive by both PCR and Southern for both heavy-chain and kappa targeting events (see examples 1 and 2 for primers). Fetal fibroblasts were isolated, expanded and frozen. A second pregnancy resulting from the mating of a kappa single knockout with a heavy chain single knockout produced four healthy piglets.

Fetal fibroblast cells that contain a heavy chain single knockout and a kappa chain single knockout will be used for further targeting. Such cells will be used to target the lambda locus via the methods and compositins described herein. The resulting offspring will be hereozygous knockouts for heavy chain, kappa chain and lambda chain. These animals will be further crossed with animals containing the human Ig genes as decribed herein and then crossbred with other single Ig knockout animals to produce porcine Ig double knockout animals with human Ig replacement genes.

Aspects of the disclosure:
1. A transgenic ungulate that lacks any expression of functional endogenous immunoglobulins.
2. The transgenic ungulate of embodiment 1, wherein the ungulate lacks any expression of endogenous heavy chain immunoglobulins.
3. The transgenic ungulate of embodiment 1, wherein the ungulate lacks any expression of endogenous light chain immunoglobulins.
4. The transgenic ungulate of embodiment 3, wherein the ungulate lacks any expression of endogenous kappa chain immunoglobulin.
5. The transgenic ungulate of embodiment 3, wherein the ungulate lacks any expression of endogenous lambda chain immunoglobulin.
6. The transgenic ungulate of embodiment 1, wherein the ungulate is selected from the group consisting of a porcine, bovine, ovine and caprine.
7. The transgenic ungulate of embodiment 6, wherein the ungulate is a porcine.
8. The transgenic ungulate of embodiment 1, wherein the ungulate is produced via nuclear transfer.
9. The transgenic ungulate of embodiment 1, wherein the ungulate expresses an exogenous immunoglobulin loci.
10. The transgenic ungulate of embodiment 9, wherein the exogeous immunoglobulin loci is a heavy chain immunoglobulin or fragment thereof.
11. The transgenic ungulate of embodiment 9, wherein the exogeous immunoglobulin loci is a light chain immunoglobulin or fragment thereof.
12. The transgenic ungulate of embodiment 11, wherein the light chain locus is a kappa chain locus or fragment thereof.
13. The transgenic ungulate of embodiment 11, wherein the light chain locus is a lambda chain locus or fragment thereof.
14. The transgenic ungulate of embodiment 9, wherein the xenogenous locus is a human immunoglobulin locus or fragment thereof.
15. The transgenic ungulate of embodiment 9, wherein an artificial chromosome contains the xenogenous immunoglobulin.
15. The transgenic ungulate of embodiment 15, wherein the artificial chromosomes comprise a mammalian artificial chromosome.
16. The transgenic ungulate of embodiment 15, wherein the mammalian artificial chromosome comprises one or more of human chromosome 14, human chromosome 2, and human chromosome 22 or fragments thereof.
17. A transgenic mammal that lacks any expression of an endogenous lambda chain immunoglobulin.
18. A transgenic ungulate that expresses a xenogenous immunoglobulin loci or fragment thereof, wherein the immunoglobulin is expressed from an immunoglobulin locus that is integrated within an endogenous ungulate chromosome.
19. The transgenic ungulate of embodiment 18, wherein the xenogenous immunoglobulin is a human immunoglobulin or fragment thereof.
20. The transgenic ungulate of embodiment 18, wherein the xenogenous immunoglobulin locus is inherited by offspring.
21. The transgenic ungulate of embodiment 18, wherein the xenogenous immunoglobulin locus is inherited through the male germ line by offspring.
22. The transgenic ungulate of embodiment 18, wherein the ungulate is a porcine, sheep, goat or cow.
23. The transgenic ungulate of embodiment 22, wherein the ungulate is a porcine.
24. The transgenic ungulate of embodiment 18, wherein the ungulate is produced through nuclear transfer.
25. The transgenic ungulate of embodiment 18, wherein the immunoglobulin loci are expressed in B cells to produce xenogenous immunoglobulin in response to exposure to one or more antigens.
26. The transgenic ungulateof embodiment 18, wherein an artificial chromosome comprises the xenogenous immunoglobulin.
27. The transgenic ungulate of embodiment 18, wherein the artificial chromosome comprises a mammalian artificial chromosome.
28. The transgenic ungulate of embodiment 27, wherein the artificial chromosomes comprises a yeast artificial chromosome.
29. The transgenic ungulate of embodiment 26, wherein the artificial chromosome comprises one or more of human chromosome 14, human chromosome 2, and human chromosome 22 or fragment thereof.
30. A transgenic ungulate cell, tissue or organ derived from the transgenic ungulate of embodiment 1.
31. A transgenic ungulate cell, tissue or organ derived from the transgenic ungulate of embodiment 18.
32. The cell of embodiment 30 or 31, wherein the cell is a somatic, reproductive or germ cell.
33. The cell of embodiment 32, wherein the cell is a B cell.
34. The cell of embodiment 33, wherein the cell is a fibroblast cell.
35. A porcine animal comprising a xenogenous immunoglobulin locus.
36. The porcine of embodiment 35, wherein an artificial chromosome contains the xenogenous locus.
37. The porcine of embodiment 36, wherein the artificial chromosome comprises one or more xenogenous immunoglobulin loci that undergo rearrangement and can produce a xenogenous immunoglobulin in response to exposure to one or more antigens.
38. The procine cell derived from the animal of embodiment 35.
39. The procine cell of embodiment 36, wherein the cell is a somatic cell, a B cell or a fibroblast.
40. The porcine of embodiment 35, wherein the xenogenous immunoglobulin is a human immunoglobulin.
41. The porcine of embodiment 36, wherein the one or more artificial chromosomes comprise a mammalian artificial chromosome.
42. The porcine of embodiment 41, wherein the mammalian artificial chromosome comprises one or more of human chromosome 14, human chromosome 2, and human chromosome 22 or fragments thereof.
43 A method of producing xenogenous antibodies, the method comprising the steps of: (a) administering one or more antigens of interest to an ungulate whose cells comprise one or more artificial chromosomes and lack any expression of functional endogenous immunoglobulin, each artificial chromosome comprising one or more xenogenous immunoglobulin loci that undergo rearrangement, resulting in production of xenogenous antibodies against the one or more antigens; and (b) recovering the xenogenous antibodies from the ungulate.
44. The method of embodiment 43, wherein the immunoglobulin loci undergo rearrangement in a B cell.
45. The method of embodiment 43, wherein the exogeous immunoglobulin loci is a heavy chain immunoglobulin or fragment thereof.
46. The method of embodiment 43, wherein the exogeous immunoglobulin loci is a light chain immunoglobulin or fragment thereof.
47. The method of embodiment 43, wherein the xenogenous locus is a human immunoglobulin locus or fragment thereof.
48. The method of embodiment 43, wherein an artificial chromosome contains the xenogenous immunoglobulin.
49. The method of embodiment 48, wherein the artificial chromosomes comprise a mammalian artificial chromosome.
50. The method of embodiment 49, wherein the mammalian artificial chromosome comprises one or more of human chromosome 14, human chromosome 2, and human chromosome 22 or fragments thereof.
51. An isolated nucleotide sequence comprising porcine heavy chain immunoglobulin or fragment thereof, wherein the heavy chain immunoglobulin includes at least one joining region and at least one constant immunoglobulin region.
52. The nucleotide sequence of embodiment 51, wherein the heavy chain immunoglobulin comprises at least one variable region, at least two diversity regions, at least four joining regions and at least one constant region.
53. The nucleotide sequence of embodiment 52, wherein the heavy chain immunoglobulin comprises Seq ID No. 29.
54. The nucleotide sequence of embodiment 51, wherein the heavy chain immunoglobulin comprises Seq ID No. 4.
55. The nucleotide sequence of embodiment 53 or 54, wherein the sequence is at least 80, 85, 90, 95, 98 or 99% homologous to Seq ID Nos 4 or 29.
56. The nucleotide sequence of embodiment 53 or 54, wherein the sequence contains at least 17, 20, 25 or 30 contiguous nucleotides of Seq ID No 4 or residues 1- 9,070 of Seq ID No 29.
57. The nucleotide sequence of embodiment 53 or 54, wherein the sequence comprises residues 9,070-11039 of Seq ID No 29.
58.An isolated nucleotide sequences that hybridizes to Seq ID No 4 or 29.
59. A targeting vector comprising:
   (a) a first nucleotide sequence comprising at least 17 contiguous nucleic acids homologous to SEQ ID No 29;
   (b) a selectable marker gene; and
   (c) a second nucleotide sequence comprising at least 17 contiguous nucleic acids homologous to SEQ ID No 29, which does not overlap with the first nucleotide sequence.
60. The targeting vector of embodiment 59 wherein the selectable marker comprises an antibiotic resistence gene.
61. The targeting vector of embodiment 59 wherein the first nucleotide sequence represents the 5' recombination arm.
62.The targeting vector of embodiment 59 wherein the second nucleotide sequence represents the 3' recombination arm.
63.A cell transfected with the targeting vector of embodiment 59.
64. The cell of embodiment 63 wherein at least one allele of a porcine heavy chain immunoglobulin locus has been rendered inactive.
65.A porcine animal comprising the cell of embodiment 64.
66. An isolated nucleotide sequence comprising an ungulate kappa light chain immunoglobulin locus or fragment thereof.
67.The nucleotide sequence of embodiment 66, wherein the ungulate is a porcine.
68.The nucleotide sequence of embodiment 66, wherein the ungulate kappa light chain immunoglobulin locus comprises at least one joining region, one constant region and/or one enhancer region.
69.The nucleotide sequence of embodiment 66, wherein the nucleotide sequence comprises at least five joining regions, one constant region and one enhancer region.
70.The nucleotide sequence of embodiment 69 comprising Seq ID No. 30.
71.The nucleotide sequence of embodiment 69 comprising Seq ID No. 12.
72. The nucleotide sequence of embodiment 70 or 71, wherein the sequence contains at least 17, 20, 25 or 30 contiguous nucleotides of Seq ID No 12 or 30.
73. An isolated nucleotide sequences that hybridizes to Seq ID No 12 or 30.
74. A targeting vector comprising:
   (a) a first nucleotide sequence comprising at least 17 contiguous nucleic acids homologous to SEQ ID No 30;
   (b) a selectable marker gene; and
   (c) a second nucleotide sequence comprising at least 17 contiguous nucleic acids homologous to SEQ ID No 30, which does not overlap with the first nucleotide sequence.
75. The targeting vector of embodiment 74 wherein the selectable marker comprises an antibiotic resistence gene.
76. The targeting vector of embodiment 74 wherein the first nucleotide sequence represents the 5' recombination arm.
77. The targeting vector of embodiment 74 wherein the second nucleotide sequence represents the 3' recombination arm.
78. A cell transfected with the targeting vector of embodiment 74.
79. The cell of embodiment 78 wherein at least one allele of a kappa chain inununoglobulin locus has been rendered inactive.
80. A porcine animal comprising the cell of embodiment 79.
81. An isolated nucleotide sequence comprising an ungulate lambda light chain immunoglobulin locus.
82. The nucleotide sequence of embodiment 81, wherein the ungulate is a porcine.
83. The nucleotide sequence of embodiment 81, wherein the ungulate is a bovine.
84. The nucleotide sequence of embodiment 81, wherein the ungulate lambda light chain immunoglobulin locus comprises a concatamer of J to C units.
85. The nucleotide sequence of embodiment 81, wherein the ungulate lambda light chain immunoglobulin locus comprises at least one joining region-constant region pair and/or at least one variable region, for example, as represented by Seq ID No. 31.
86. The nucleotide sequence of embodiment 82 comprising Seq ID No. 28.
87. The nucleotide sequence of embodiment 83 comprising Seq ID No. 31.
88. The nucleotide sequence of embodiment 86 or 87, wherein the sequence contains at least 17, 20, 25 or 30 contiguous nucleotides of Seq ID No 28 or 31.
89. An isolated nucleotide sequences that hybridizes to Seq ID No 28 or 31.
90. A targeting vector comprising:
   (a) a first nucleotide sequence comprising at least 17 contiguous nucleic acids homologous to SEQ ID No 28 or 31;
   (b) a selectable marker gene; and
   (c) a second nucleotide sequence comprising at least 17 contiguous nucleic acids homologous to SEQ ID No 28 or 31, which does not overlap with the first nucleotide sequence.
91. The targeting vector of embodiment 90 wherein the selectable marker comprises an antibiotic resistence gene.
92. The targeting vector of embodiment 90 wherein the first nucleotide sequence represents the 5' recombination arm.
93. The targeting vector of embodiment 90 wherein the second nucleotide sequence represents the 3' recombination arm.
94. A cell transfected with the targeting vector of embodiment 90.
95. The cell of embodiment 94 wherein at least one allele of a lambda chain immunoglobulin locus has been rendered inactive.
96. A porcine animal comprising the cell of embodiment 95.
97. A method to circularize at least 100 kb of DNA, wherein the DNA can then be integrated into a host genome via a site specific recombinase.
98. The method of embodiment 97, wherein at least 100, 200, 300, 400, 500, 1000, 2000, 5000, 10,000 kb of DNA can be circularized.
99. The method of embodiment 97, wherein the circularization of the DNA can be accomplished by attaching site specific recombinase target sites at each end of the DNA sequence and then applying a site specific recombinase to the DNA sequence.
100. The method of embodiment 97, wherein the site specific recombinase target site is Lox.
101. The method of embodiment 97, wherein an artificial chromosome contains the DNA sequence.
102. The method of embodiment 101, wherein the artificial chromosome is a yeast artificial chromosome or a mammalian artificial chromosome.
103. The method of embodiment 101, wherein the artificial chromosome comprises a DNA sequence that encodes a human immunoglobulin locus or fragment thereof.
104. The method of embodiment 103, the human immunoglobulin locus or fragment thereof comprises human chromosome 14, human chromosome 2, and/ or human chromosome 22.
105. A transgenic ungulate that lacks expression of at least one allele of an endogenous immunoglobulin wherein the immunoglobulin is selected from the group consisting of heavy chain, kappa light chain and lambda light chain or any combination thereof.
106. The transgenic ungulate of embodiment 105, wherein xenogenous immunoglobulin is expressed.
107. A method to produce the transgenic ungulate of embodiment 106, wherein a transgenic ungulate that lacks expression of at least one allele of an endogenous immunoglobulin wherein the immunoglobulin is selected from the group consisting of heavy chain, kappa light chain and lambda light chain or any combination thereof is bred with an ungulate that expresses an xenogenous immunoglobulin.
108. The transgenic ungulate of any of embodiments 105-107, wherein the ungulate is a porcine.
109. The transgenic ungulate of embodiment 106 or 107, wherein the xenogenous immunoglobulin is a human immunoglobulin locus or fragment thereof.
110. The transgenic ungulate of embodiment 109, wherein an artificial chromosome contains the human Mununoglobulin locus or fragment thereof.
111. A cell derived from the ungulate of embodiment 105.
112. The transgenic ungulate of embodiment 1, 18, 105 or 106, further comprising an additional genetic modifications to eliminate the expression of a xenoantigen.
113. The transgenic ungulate of embodiment 112, wherein the ungulate lacks expression of at least one allele of the alpha-1,3-galactosyltransferase gene.
114. The transgenic ungulate of embodiment 112, wherein the ungulate is a porcine.

## Claims

1. A transgenic porcine animal lacking expression of at least one allele of porcine endogenous kappa light chain immunoglobulin gene, in which the at least one allele of porcine kappa light chain gene is inactivated via a genetic targeting event, wherein the genetic targeting event comprises disruption of the kappa constant region.

2. A porcine animal according to claim 1, wherein the porcine further lacks expression of at least one allele of porcine heavy chain immunoglobulin gene, in which the at least one allele of porcine heavy chain gene is inactivated via a genetic targeting event.

3. A porcine animal according to claim 1 or claim 2, wherein the porcine further lacks expression of at least one allele of porcine lambda light chain gene, in which the at least one allele of porcine lambda chain gene is inactivated via a genetic targeting event, and wherein the porcine comprises a xenogenous immunoglobulin locus.

4. A method for producing the transgenic porcine of any one of claims 1-3, comprising (i) a step of gene disruption through (a) substitution, deletion or insertion techniques and/or (b) homologous recombination; and/or (ii) a step of cloning using a donor nucleus from a cell in which at least one allele of the immunoglobulin gene has been inactivated.

5. A porcine animal according to any one of claims 1 to 3, wherein (i) the genes are disrupted through (a) substitution, deletion or insertion techniques and/or (b) homologous recombination; or (ii) the animal is produced by cloning using a donor nucleus from a cell in which at least one allele of the immunoglobulin gene has been inactivated.

6. A porcine animal according to claim 1 or claim 2 comprising a xenogenous immunoglobulin locus.

7. The porcine animal of claim 6, wherein the xenogenous immunoglobulin locus is a heavy chain immunoglobulin locus or a fragment thereof that can undergo rearrangement or a light chain immunoglobulin locus or a fragment thereof that can undergo rearrangement.

8. A porcine animal of any one of claims 1 to 3 or 5 to 7 that expresses a xenogenous immunoglobulin, wherein the immunoglobulin is expressed from a xenogenous immunoglobulin locus or a fragment thereof that can undergo rearrangement that is integrated within an endogenous porcine chromosome.

9. The porcine animal of any one of claims 6-8, wherein the xenogenous immunoglobulin locus is a kappa or lambda chain locus or a fragment thereof that can undergo rearrangement; and/or wherein the xenogenous locus is a human immunoglobulin locus or fragment thereof that can undergo rearrangement.

10. The porcine animal of claim 6, claim 7 or claim 9, wherein an artificial chromosome contains the xenogenous immunoglobulin locus.

11. The porcine animal of claim 10, wherein a) the artificial chromosome comprises a mammalian artificial chromosome, wherein optionally the mammalian artificial chromosome comprises one or more of human chromosome 14, human chromosome 2, and human chromosome 22 or fragments thereof; or b) the artificial chromosome comprises a yeast artificial chromosome.

12. The porcine animal of claim 8, wherein a) the xenogenous immunoglobulin locus is inherited by offspring; or b) the xenogenous immunoglobulin locus is inherited through the male germ line by offspring; or c) the animal is produced through nuclear transfer; and/or d) the xenogenous immunoglobulin is expressed in B cells in response to exposure to one or more antigens.

13. A cell, tissue or organ derived from the porcine animal of any one of the preceding claims, wherein the cell tissue or organ lacks expression of at least one allele of porcine endogenous kappa light chain immunoglobulin gene, in which the at least one allele of porcine kappa light chain gene is inactivated via a genetic targeting event, wherein the genetic targeting event comprises disruption of the kappa constant region.

14. The cell of claim 13, wherein a) the cell is a somatic, reproductive or germ cell; or b) the cell is a B cell or a fibroblast cell.

15. The porcine animal of claim 10, wherein the artificial chromosome comprises one or more xenogenous immunoglobulin loci that undergo rearrangement and can produce a xenogenous immunoglobulin in response to exposure to one or more antigens.

16. The porcine animal of any one of claims 1 to 3, 5 to 12 or 15, further comprising an additional genetic modification to eliminate the expression of a xenoantigen, wherein optionally the animal lacks expression of at least one allele of the alpha-1,3-galactosyltransferase gene.

## Patentansprüche

1. Transgenes Schwein, dem die Expression mindestens eines Allels des endogenen Schweine-kappa-leichte-Kette-Immunglobulingens fehlt, wobei das mindestens eine Allel des Schweine-kappa-leichte-Kette-Gens über ein genetisches Targeting-Ereignis inaktiviert worden ist, wobei das genetische Targeting-Ereignis die Disruption der kappa-konstanten-Region umfasst.

2. Schwein nach Anspruch 1, wobei dem Schwein außerdem die Expression mindestens eines Allels des Schweine-schwere-Kette-Immunglobulingens fehlt, wobei das mindestens eine Allel des Schweine-schwere-Kette-Gens über ein genetisches Targeting-Ereignis inaktiviert worden ist.

3. Schwein nach Anspruch 1 oder Anspruch 2, wobei dem Schwein außerdem die Expression mindestens eines Allels des Schweine-lambda-leichte-Kette-Gens fehlt, wobei das mindestens eine Allel des Schweine-lambda-Kette-Gens über ein genetisches Targeting-Ereignis inaktiviert worden ist und wobei das Schwein einen xenogenen Immunglobulinlocus umfasst.

4. Verfahren zur Herstellung des transgenen Schweins nach einem der Ansprüche 1-3, umfassend (i) einen Schritt der Gendisruption durch (a) Substitutions-, Deletions- oder Insertionstechniken und/oder (b) der homologen Rekombination; und/oder (ii) einen Schritt der Klonierung unter Verwendung eines Donorkerns aus einer Zelle, in der mindestens ein Allel des Immunglobulingens inaktiviert worden ist.

5. Schwein nach einem der Ansprüche 1 bis 3, wobei (i) die Gene durch (a) Substitutions-, Deletions- oder Insertionstechniken oder (b) homologe Rekombination disruptiert wurden; oder (ii) das Tier durch Klonierung unter Verwendung eines Donorkerns aus einer Zelle, in der mindestens ein Allel des Immunglobulingens inaktiviert ist, hergestellt wurde.

6. Schwein nach Anspruch 1 oder Anspruch 2, das einen xenogenen Immunglobulinlocus umfasst.

7. Schwein nach Anspruch 6, wobei der xenogene Immunglobulinlocus ein schwere-Kette-Immunglobulinlocus oder ein Fragment davon ist, der/das einer Umlagerung unterliegen kann, oder ein leichte-Kette-Immunglobulinlocus oder ein Fragment davon ist, der/das einer Umlagerung unterliegen kann.

8. Schwein nach einem der Ansprüche 1 bis 3 oder 5 bis 7, das ein xenogenes Immunglobulin exprimiert, wobei das Immunglobulin von einem xenogenen Immunglobulinlocus oder einem Fragment davon exprimiert wird, der/das einer Umlagerung unterliegen kann und in ein endogenes Schweinechromosom integriert ist.

9. Schwein nach einem der Ansprüche 6-8, wobei der xenogene Immunglobulinlocus ein kappa- oder lambda-Kette-Locus oder ein Fragment davon ist, der/das einer Umlagerung unterliegen kann; und/oder wobei der xenogene Locus ein menschlicher Immunglobulinlocus oder ein Fragment davon ist, der/das einer Umlagerung unterliegen kann.

10. Schwein nach Anspruch 6, Anspruch 7 oder Anspruch 9, wobei ein künstliches Chromosom den xenogenen Immunglobulinlocus enthält.

11. Schwein nach Anspruch 10, wobei a) das künstliche Chromosom ein künstliches Säugetierchromosom umfasst, wobei gegebenenfalls das künstliche Säugetierchromosom eines oder mehrere aus dem menschlichen Chromosom 14, dem menschlichen Chromosom 2 und dem menschlichen Chromosom 22 oder Fragmente davon umfasst; oder b) das künstliche Chromosomen ein künstliches Hefechromosom umfasst.

12. Schwein nach Anspruch 8, wobei a) der xenogene Immunglobulinlocus von Nachkommen geerbt wird; oder b) der xenogene Immunglobulinlocus über die männliche Keimbahn von Nachkommen geerbt wird; oder c) das Tier durch Kerntransfer erzeugt wird; und/oder d) das xenogene Immunglobulin in B-Zellen als Reaktion auf die Exposition gegenüber einem oder mehreren Antigenen exprimiert wird.

13. Zelle, Gewebe oder Organ, die/das von dem Schwein nach einem der vorhergehenden Ansprüche stammt, wobei der Zelle, dem Gewebe oder dem Organ die Expression mindestens eines Allels des endogenen Schweine-kappa-leichte-Kette-Immunglobulingens fehlt, wobei das mindestens eine Allel des Schweine-kappa-leichte-Kette-Gens über ein genetisches Targeting-Ereignis inaktiviert worden ist, wobei das genetische Targeting-Ereignis die Disruption der kappa-konstanten-Region umfasst.

14. Zelle nach Anspruch 13, wobei a) die Zelle eine somatische Zelle, Fortpflanzungs- oder Keimzelle ist; oder b) die Zelle eine B-Zelle oder eine Fibroblastenzelle ist.

15. Schwein nach Anspruch 10, wobei das künstliche Chromosom einen oder mehrere xenogene Immunglobulinloci umfasst, die einer Umlagerung unterliegen und als Reaktion auf die Exposition gegenüber einem oder mehreren Antigenen ein xenogenes Immunglobulin produzieren können.

16. Schwein nach einem der Ansprüche 1 bis 3, 5 bis 12 oder 15, das weiterhin eine zusätzliche genetische Modifikation umfasst, um die Expression eines Xenoantigens zu beseitigen, wobei dem Tier gegebenenfalls die Expression mindestens eines Allels des alpha-1,3-Galactosyltransferase-Gens fehlt.

## Revendications

1. Animal porcin transgénique ne présentant pas l'expression d'au moins un allèle de gène d'immunoglobuline de chaîne légère kappa endogène porcin, dans lequel l'au moins un allèle de gène de chaîne légère kappa porcin est inactivé via un évènement de ciblage génétique, dans lequel l'évènement de ciblage génétique comprend une rupture de la région constante kappa.

2. Animal porcin selon la revendication 1, le porcin ne présentant également pas l'expression d'au moins un allèle de gène d'immunoglobuline de chaîne lourde porcin, dans lequel l'au moins un allèle de gène de chaîne lourde porcin est inactivé via un évènement de ciblage génétique.

3. Animal porcin selon la revendication 1 ou la revendication 2, le porcin ne présentant également pas l'expression d'au moins un allèle de gène de chaîne légère lambda porcin, dans lequel l'au moins un allèle de gène de chaîne lambda porcin est inactivé via un évènement de ciblage génétique, et le porcin comprenant un locus d'immunoglobuline xénogène.

4. Procédé de production du porcin transgénique de l'une quelconque des revendications 1 à 3, comprenant (i) une étape de rupture de gène par (a) des techniques de substitution, délétion ou insertion et/ou (b) recombinaison homologue ; et/ou (ii) une étape de clonage au moyen d'un noyau donneur d'une cellule dans lequel au moins un allèle du gène d'immunoglobuline a été inactivé.

5. Animal porcin selon l'une quelconque des revendications 1 à 3, dans lequel (i) les gènes sont rompus par (a) des techniques de substitution, délétion ou insertion et/ou (b) recombinaison homologue ; ou (ii) l'animal est produit par clonage au moyen d'un noyau donneur d'une cellule dans lequel au moins un allèle du gène d'immunoglobuline a été inactivé.

6. Animal porcin selon la revendication 1 ou la revendication 2 comprenant un locus d'immunoglobuline xénogène.

7. Animal porcin de la revendication 6, dans lequel le locus d'immunoglobuline xénogène est un locus d'immunoglobuline de chaîne lourde ou un fragment de celui-ci qui peut subir un réarrangement ou un locus d'immunoglobuline de chaîne légère ou un fragment de celui-ci qui peut subir un réarrangement.

8. Animal porcin de l'une quelconque des revendications 1 à 3 ou 5 à 7 qui exprime une immunoglobuline xénogène, dans lequel l'immunoglobuline est exprimée à partir d'un locus d'immunoglobuline xénogène ou un fragment de celui-ci qui peut subir un réarrangement qui est intégré dans un chromosome porcin endogène.

9. Animal porcin de l'une quelconque des revendications 6 à 8, dans lequel le locus d'immunoglobuline xénogène est un locus de chaîne kappa ou lambda ou un fragment de celui-ci qui peut subir un réarrangement ; et/ou dans lequel le locus xénogène est un locus d'immunoglobuline humain ou un fragment de celui-ci qui peut subir un réarrangement.

10. Animal porcin de la revendication 6, la revendication 7 ou la revendication 9, dans lequel un chromosome artificiel contient le locus d'immunoglobuline xénogène.

11. Animal porcin de la revendication 10, dans lequel a) le chromosome artificiel comprend un chromosome artificiel de mammifère, dans lequel, facultativement, le chromosome artificiel de mammifère comprend l'un ou plusieurs parmi le chromosome humain 14, le chromosome humain 2 et le chromosome humain 22 ou des fragments de ceux-ci ; ou b) le chromosome artificiel comprend un chromosome artificiel de levure.

12. Animal porcin de la revendication 8, dans lequel a) le locus d'immunoglobuline xénogène est hérité par descendance ; ou b) le locus d'immunoglobuline xénogène est hérité par l'intermédiaire de la lignée germinale masculine par descendance ; ou c) l'animal est produit par transfert nucléaire ; et/ou d) l'immunoglobuline xénogène est exprimée dans des lymphocytes B en réponse à l'exposition à un ou plusieurs antigènes.

13. Cellule, tissu ou organe dérivé de l'animal porcin de l'une quelconque des revendications précédentes, la cellule, le tissu ou l'organe ne présentant pas l'expression d'au moins un allèle de gène d'immunoglobuline de chaîne légère kappa endogène porcin, dans lequel l'au moins un allèle de gène de chaîne légère kappa porcin est inactivé via un évènement de ciblage génétique, dans lequel l'évènement de ciblage génétique comprend une rupture de la région constante kappa.

14. Cellule de la revendication 13, où a) la cellule est une cellule somatique, reproductrice ou germinale ; ou b) la cellule est un lymphocyte B ou une cellule de fibroblaste.

15. Animal porcin de la revendication 10, dans lequel le chromosome artificiel comprend un ou plusieurs locus d'immunoglobuline xénogènes qui subissent un réarrangement et peuvent produire une immunoglobuline xénogène en réponse à l'exposition à un ou plusieurs antigènes.

16. Animal porcin de l'une quelconque des revendications 1 à 3, 5 à 12 ou 15, comprenant en outre une modification génétique additionnelle pour éliminer l'expression d'un xénoantigène, dans lequel, facultativement, l'animal ne présente pas l'expression d'au moins un allèle du gène d'alpha-1,3-galactosyltransférase.


### SEQUENCE LISTING

<110> Revivicor, Inc.
<120> Ungulates with Genetically Modified Immune Systems
<130> P046919EP
<140> EP 05818708.9
   <141> 2005-10-24
<150> US 60/621,433
   <151> 2004-10-22
<160> 43
<170> PatentIn version 3.3
<210> 1
   <211> 666
   <212> DNA
   <213> Sus scrofa
<400> 1
   tctagaagac gctggagaga ggccagactt cctcggaaca gctcaaagag ctctgtcaaa      60
   gccagatccc atcacacgtg ggcaccaata ggccatgcca gcctccaagg gccgaactgg     120
   gttctccacg gcgcacatga agcctgcagc ctggcttatc ctcttccgtg gtgaagaggc     180
   aggcccggga ctggacgagg ggctagcagg gtgtggtagg caccttgcgc cccccacccc     240
   ggcaggaacc agagaccctg gggctgagag tgagcctcca aacaggatgc cccacccttc     300
   aggccacctt tcaatccagc tacactccac ctgccattct cctctgggca cagggcccag     360
   cccctggatc ttggccttgg ctcgacttgc acccacgcgc acacacacac ttcctaacgt     420
   gctgtccgct cacccctccc cagcgtggtc catgggcagc acggcagtgc gcgtccggcg     480
   gtagtgagtg cagaggtccc ttcccctccc ccaggagccc caggggtgtg tgcagatctg     540
   ggggctcctg tcccttacac cttcatgccc ctcccctcat acccaccctc caggcgggag     600
   gcagcgagac ctttgcccag ggactcagcc aacgggcaca cgggaggcca gccctcagca     660
   gctggg                                                                666
<210> 2
   <211> 25
   <212> DNA
   <213> Sus scrofa
<400> 2
   ggccagactt cctcggaaca gctca 25
<210> 3
   <211> 23
   <212> DNA
   <213> Sus scrofa
<400> 3
   ttccaggaga aggtgacgga gct 23
<210> 4
   <211> 9175
   <212> DNA
   <213> Sus scrofa
<400> 4
   ggccagactt cctcggaaca gctcaaagag ctctgtcaaa gccagatccc atcacacgtg 60
   ggcaccaata ggccatgcca gcctccaagg gccgaactgg gttctccacg gcgcacatga 120
   agcctgcagc ctggcttatc ctcttccgtg gtgaagaggc aggcccggga ctggacgagg 180
   ggctagcagg gtgtggtagg caccttgcgc cccccacccc ggcaggaacc agagaccctg 240
   gggctgagag tgagcctcca aacaggatgc cccacccttc aggccacctt tcaatccagc 300
   tacactccac ctgccattct cctctgggca cagggcccag cccctggatc ttggccttgg 360
   ctcgacttgc acccacgcgc acacacacac ttcctaacgt gctgtccgct cacccctccc 420
   cagcgtggtc catgggcagc acggcagtgc gcgtccggcg gtagtgagtg cagaggtccc 480
   ttcccctccc ccaggagccc caggggtgtg tgcagatctg ggggctcctg tcccttacac 540
   cttcatgccc ctcccctcat acccaccctc caggcgggag gcagcgagac ctttgcccag 600
   ggactcagcc aacgggcaca cgggaggcca gccctcagca gctggctccc aaagaggagg 660
   tgggaggtag gtccacagct gccacagaga gaaaccctga cggaccccac aggggccacg 720
   ccagccggaa ccagctccct cgtgggtgag caatggccag ggccccgccg gccaccacgg 780
   ctggccttgc gccagctgag aactcacgtc cagtgcaggg agactcaaga cagcctgtgc 840
   acacagcctc ggatctgctc ccatttcaag cagaaaaagg aaaccgtgca ggcagccctc 900
   agcatttcaa ggattgtagc agcggccaac tattcgtcgg cagtggccga ttagaatgac 960
   cgtggagaag ggcggaaggg tctctcgtgg gctctgcggc caacaggccc tggctccacc 1020
   tgcccgctgc cagcccgagg ggcttgggcc gagccaggaa ccacagtgct caccgggacc 1080
   acagtgactg accaaactcc cggccagagc agccccaggc cagccgggct ctcgccctgg 1140
   aggactcacc atcagatgca caagggggcg agtgtggaag agacgtgtcg cccgggccat 1200
   ttgggaaggc gaagggacct tccaggtgga caggaggtgg gacgcactcc aggcaaggga 1260
   ctgggtcccc aaggcctggg gaaggggtac tggcttgggg gttagcctgg ccagggaacg 1320
   gggagcgggg cggggggctg agcagggagg acctgacctc gtgggagcga ggcaagtcag 1380
   gcttcaggca gcagccgcac atcccagacc aggaggctga ggcaggaggg gcttgcagcg 1440
   gggcgggggc ctgcctggct ccgggggctc ctgggggacg ctggctcttg tttccgtgtc 1500
   ccgcagcaca gggccagctc gctgggccta tgcttacctt gatgtctggg gccggggcgt 1560
   cagggtcgtc gtctcctcag gggagagtcc cctgaggcta cgctgggggg ggactatggc 1620
   agctccacca ggggcctggg gaccaggggc ctggaccagg ctgcagcccg gaggacgggc 1680
   agggctctgg ctctccagca tctggccctc ggaaatggca gaacccctgg cgggtgagcg 1740
   agctgagagc gggtcagaca gacaggggcc ggccggaaag gagaagttgg gggcagagcc 1800
   cgccaggggc caggcccaag gttctgtgtg ccagggcctg ggtgggcaca ttggtgtggc 1860
   catggctact tagattcgtg gggccagggc atcctggtca ccgtctcctc aggtgagcct 1920
   ggtgtctgat gtccagctag gcgctggtgg gccgcgggtg ggcctgtctc aggctagggc 1980
   aggggctggg atgtgtattt gtcaaggagg ggcaacaggg tgcagactgt gcccctggaa 2040
   acttgaccac tggggcaggg gcgtcctggt cacgtctcct caggtaagac ggccctgtgc 2100
   ccctctctcg cgggactgga aaaggaattt tccaagattc cttggtctgt gtggggccct 2160
   ctggggcccc cgggggtggc tcccctcctg cccagatggg gcctcggcct gtggagcacg 2220
   ggctgggcac acagctcgag tctagggcca cagaggcccg ggctcagggc tctgtgtggc 2280
   ccggcgactg gcagggggct cgggtttttg gacaccccct aatgggggcc acagcactgt 2340
   gaccatcttc acagctgggg ccgaggagtc gaggtcaccg tctcctcagg tgagtcctcg 2400
   tcagccctct ctcactctct ggggggtttt gctgcatttt gtgggggaaa gaggatgcct 2460
   gggtctcagg tctaaaggtc tagggccagc gccggggccc aggaaggggc cgaggggcca 2520
   ggctcggctc ggccaggagc agagcttcca gacatctcgc ctcctggcgg ctgcagtcag 2580
   gcctttggcc gggggggtct cagcaccacc aggcctcttg gctcccgagg tccccggccc 2640
   cggctgcctc accaggcacc gtgcgcggtg ggcccgggct cttggtcggc caccctttct 2700
   taactgggat ccgggcttag ttgtcgcaat gtgacaacgg gctcgaaagc tggggccagg 2760
   ggaccctagt tacgacgcct cgggtgggtg tcccgcaccc ctccccactt tcacggcact 2820
   cggcgagacc tggggagtca ggtgttgggg acactttgga ggtcaggaac gggagctggg 2880
   gagagggctc tgtcagcggg gtccagagat gggccgccct ccaaggacgc cctgcgcggg 2940
   gacaagggct tcttggcctg gcctggccgc ttcacttggg cgtcaggggg ggcttcccgg 3000
   ggcaggcggt cagtcgaggc gggttggaat tctgagtctg ggttcggggt tcggggttcg 3060
   gccttcatga acagacagcc caggcgggcc gttgtttggc ccctgggggc ctggttggaa 3120
   tgcgaggtct cgggaagtca ggagggagcc tggccagcag agggttccca gccctgcggc 3180
   cgagggacct ggagacgggc agggcattgg ccgtcgcagg gccaggccac accccccagg 3240
   tttttgtggg gcgagcctgg agattgcacc actgtgatta ctatgctatg gatctctggg 3300
   gcccaggcgt tgaagtcgtc gtgtcctcag gtaagaacgg ccctccaggg cctttaattt 3360
   ctgctctcgt ctgtgggctt ttctgactct gatcctcggg aggcgtctgt gccccccccg 3420
   gggatgaggc cggcttgcca ggaggggtca gggaccagga gcctgtggga agttctgacg 3480
   ggggctgcag gcgggaaggg ccccaccggg gggcgagccc caggccgctg ggcggcagga 3540
   gacccgtgag agtgcgcctt gaggagggtg tctgcggaac cacgaacgcc cgccgggaag 3600
   ggcttgctgc aatgcggtct tcagacggga ggcgtcttct gccctcaccg tctttcaagc 3660
   ccttgtgggt ctgaaagagc catgtcggag agagaaggga caggcctgtc ccgacctggc 3720
   cgagagcggg cagccccggg ggagagcggg gcgatcggcc tgggctctgt gaggccaggt 3780
   ccaagggagg acgtgtggtc ctcgtgacag gtgcacttgc gaaaccttag aagacggggt 3840
   atgttggaag cggctcctga tgtttaagaa aagggagact gtaaagtgag cagagtcctc 3900
   aagtgtgtta aggttttaaa ggtcaaagtg ttttaaacct ttgtgactgc agttagcaag 3960
   cgtgcgggga gtgaatgggg tgccagggtg gccgagaggc agtacgaggg ccgtgccgtc 4020
   ctctaattca gggcttagtt ttgcagaata aagtcggcct gttttctaaa agcattggtg 4080
   gtgctgagct ggtggaggag gccgcgggca gccctggcca cctgcagcag gtggcaggaa 4140
   gcaggtcggc caagaggcta ttttaggaag ccagaaaaca cggtcgatga atttatagct 4200
   tctggtttcc aggaggtggt tgggcatggc tttgcgcagc gccacagaac cgaaagtgcc 4260
   cactgagaaa aaacaactcc tgcttaattt gcatttttct aaaagaagaa acagaggctg 4320
   acggaaactg gaaagttcct gttttaacta ctcgaattga gttttcggtc ttagcttatc 4380
   aactgctcac ttagattcat tttcaaagta aacgtttaag agccgaggca ttcctatcct 4440
   cttctaaggc gttattcctg gaggctcatt caccgccagc acctccgctg cctgcaggca 4500
   ttgctgtcac cgtcaccgtg acggcgcgca cgattttcag ttggcccgct tcccctcgtg 4560
   attaggacag acgcgggcac tctggcccag ccgtcttggc tcagtatctg caggcgtccg 4620
   tctcgggacg gagctcaggg gaagagcgtg actccagttg aacgtgatag tcggtgcgtt 4680
   gagaggagac ccagtcgggt gtcgagtcag aaggggcccg gggcccgagg ccctgggcag 4740
   gacggcccgt gccctgcatc acgggcccag cgtcctagag gcaggactct ggtggagagt 4800
   gtgagggtgc ctggggcccc tccggagctg gggccgtgcg gtgcaggttg ggctctcggc 4860
   gcggtgttgg ctgtttctgc gggatttgga ggaattcttc cagtgatggg agtcgccagt 4920
   gaccgggcac caggctggta agagggaggc cgccgtcgtg gccagagcag ctgggagggt 4980
   tcggtaaaag gctcgcccgt ttcctttaat gaggactttt cctggagggc atttagtcta 5040
   gtcgggaccg ttttcgactc gggaagaggg atgcggagga gggcatgtgc ccaggagccg 5100
   aaggcgccgc ggggagaagc ccagggctct cctgtcccca cagaggcgac gccactgccg 5160
   cagacagaca gggcctttcc ctctgatgac ggcaaaggcg cctcggctct tgcggggtgc 5220
   tgggggggag tcgccccgaa gccgctcacc cagaggcctg aggggtgaga ctgaccgatg 5280
   cctcttggcc gggcctgggg ccggaccgag ggggactccg tggaggcagg gcgatggtgg 5340
   ctgcgggagg gaaccgaccc tgggccgagc ccggcttggc gattcccggg cgagggccct 5400
   cagccgaggc gagtgggtcc ggcggaacca ccctttctgg ccagcgccac agggctctcg 5460
   ggactgtccg gggcgacgct gggctgcccg tggcaggcct gggctgacct ggacttcacc 5520
   agacagaaca gggctttcag ggctgagctg agccaggttt agcgaggcca agtggggctg 5580
   aaccaggctc aactggcctg agctgggttg agctgggctg acctgggctg agctgagctg 5640
   ggctgggctg ggctgggctg ggctgggctg ggctggactg gctgagctga gctgggttga 5700
   gctgagctga gctggcctgg gttgagctgg gctgggttga gctgagctgg gttgagctgg 5760
   gttgagctgg gttgatctga gctgagctgg gctgagctga gctaggctgg ggtgagctgg 5820
   gctgagctgg tttgagttgg gttgagctga gctgagctgg gctgtgctgg ctgagctagg 5880
   ctgagctagg ctaggttgag ctgggctggg ctgagctgag ctaggctggg ctgatttggg 5940
   ctgagctgag ctgagctagg ctgcgttgag ctggctgggc tggattgagc tggctgagct 6000
   ggctgagctg ggctgagctg gcctgggttg agctgagctg gactggtttg agctgggtcg 6060
   atctgggttg agctgtcctg ggttgagctg ggctgggttg agctgagctg ggttgagctg 6120
   ggctcagcag agctgggttg ggctgagctg ggttgagctg agctgggctg agctggcctg 6180
   ggttgagctg ggctgagctg agctgggctg agctggcctg tgttgagctg ggctgggttg 6240
   agctgggctg agctggattg agctgggttg agctgagctg ggctgggctg tgctgactga 6300
   gctgggctga gctaggctgg ggtgagctgg gctgagctga tccgagctag gctgggctgg 6360
   tttgggctga gctgagctga gctaggctgg attgatctgg ctgagctggg ttgagctgag 6420
   ctgggctgag ctggtctgag ctggcctggg tcgagctgag ctggactggt ttgagctggg 6480
   tcgatctggg ctgagctggc ctgggttgag ctgggctggg ttgagctgag ctgggttgag 6540
   ctgggctgag ctgagggctg gggtgagctg ggctgaacta gcctagctag gttgggctga 6600
   gctgggctgg tttgggctga gctgagctga gctaggctgc attgagcagg ctgagctggg 6660
   ctgagcaggc ctggggtgag ctgggctagg tggagctgag ctgggtcgag ctgagttggg 6720
   ctgagctggc ctgggttgag gtaggctgag ctgagctgag ctaggctggg ttgagctggc 6780
   tgggctggtt tgcgctgggt caagctgggc cgagctggcc tgggttgagc tgggctcggt 6840
   tgagctgggc tgagctgagc cgacctaggc tgggatgagc tgggctgatt tgggctgagc 6900
   tgagctgagc taggctgcat tgagcaggct gagctgggcc tggagcctgg cctggggtga 6960
   gctgggctga gctgcgctga gctaggctgg gttgagctgg ctgggctggt ttgcgctggg 7020
   tcaagctggg ccgagctggc ctgggatgag ctgggccggt ttgggctgag ctgagctgag 7080
   ctaggctgca ttgagcaggc tgagctgggc tgagctggcc tggggtgagc tgggctgagc 7140
   taagctgagc tgggctggtt tgggctgagc tggctgagct gggtcctgct gagctgggct 7200
   gagctgacca ggggtgagct gggctgagtt aggctgggct cagctaggct gggttgatct 7260
   ggcagggctg gtttgcgctg ggtcaagctc ccgggagatg gcctgggatg agctgggctg 7320
   gtttgggctg agctgagctg agctgagcta ggctgcattg agcaggctga gctgggctga 7380
   gctggcctgg ggtgagctgg gctgggtgga gctgagctgg gctgaactgg gctaagctgg 7440
   ctgagctgga tcgagctgag ctgggctgag ctggcctggg gttagctggg ctgagctgag 7500
   ctgagctagg ctgggttgag ctggctgggc tggtttgcgc tgggtcaagc tgggccgagc 7560
   tggcctgggt tgagctgggc tgggctgagc tgagctaggc tgggttgagc tgggctgggc 7620
   tgagctgagc taggctgcat tgagctggct gggatggatt gagctggctg agctggctga 7680
   gctggctgag ctgggctgag ctggcctggg ttgagctggg ctgggttgag ctgagctggg 7740
   ctgagctggg ctcagcagag ctgggttgag ctgagctggg ttgagctggg gtgagctggg 7800
   ctgagcagag ctgggttgag ctgagctggg ttgagctggg ctcgagcaga gctgggttga 7860
   gctgagctgg gttgagctgg gctcagcaga gctgggttga gctgagctgg gttgagctgg 7920
   gctgagctag ctgggctcag ctaggctggg ttgagctgag ctgggctgaa ctgggctgag 7980
   ctgggctgaa ctgggctgag ctgggctgag ctgggctgag cagagctggg ctgagcagag 8040
   ctgggttggt ctgagctggg ttgagctggg ctgagctggg ctgagcagag ttgggttgag 8100
   ctgagctggg ttcagctggg ctgagctagg ctgggttgag ctgggttgag ttgggctgag 8160
   ctgggctggg ttgagcggag ctgggctgaa ctgggctgag ctgggctgag cggaactggg 8220
   ttgatctgaa ttgagctggg ctgagccggg ctgagccggg ctgagctggg ctaggttgag 8280
   cttgggtgag cttgcctcag ctggtctgag ctaggttggg tggagctagg ctggattgag 8340
   ctgggctgag ctgagctgat ctggcctcag ctgggctgag gtaggctgaa ctgggctgtg 8400
   ctgggctgag ctgagctgag ccagtttgag ctgggttgag ctgggctgag ctgggctgtg 8460
   ttgatctttc ctgaactggg ctgagctggg ctgagctggc ctagctggat tgaacggggg 8520
   taagctgggc caggctggac tgggctgagc tgagctaggc tgagctgagt tgaattgggt 8580
   taagctgggc tgagatgggc tgagctgggc tgagctgggt tgagccaggt cggactgggt 8640
   taccctgggc cacactgggc tgagctgggc ggagctcgat taacctggtc aggctgagtc 8700
   gggtccagca gacatgcgct ggccaggctg gcttgacctg gacacgttcg atgagctgcc 8760
   ttgggatggt tcacctcagc tgagccaggt ggctccagct gggctgagct ggtgaccctg 8820
   ggtgacctcg gtgaccaggt tgtcctgagt ccgggccaag ccgaggctgc atcagactcg 8880
   ccagacccaa ggcctgggcc ccggctggca agccaggggc ggtgaaggct gggctggcag 8940
   gactgtcccg gaaggaggtg cacgtggagc cgcccggacc ccgaccggca ggacctggaa 9000
   agacgcctct cactcccctt tctcttctgt cccctctcgg gtcctcagag agccagtctg 9060
   ccccgaatct ctaccccctc gtctcctgcg tcagcccccc gtccgatgag agcctggtgg 9120
   ccctgggctg cctggcccgg gacttcctgc ccagctccgt caccttctcc tggaa 9175
<210> 5
   <211> 9200
   <212> DNA
   <213> Sus scrofa
<400> 5
<210> 6
   <211> 26
   <212> DNA
   <213> Sus scrofa
<400> 6
   tctagaagac gctggagaga ggccag 26
<210> 7
   <211> 25
   <212> DNA
   <213> Sus scrofa
<400> 7
   taaagcgcat gctccagact gcctt 25
<210> 8
   <211> 22
   <212> DNA
   <213> Sus scrofa
<400> 8
   catcgccttc tatcgccttc tt 22
<210> 9
   <211> 22
   <212> DNA
   <213> Sus scrofa
<400> 9
   aagtacttgc cgcctctcag ga 22
<210> 10
   <211> 22
   <212> DNA
   <213> Sus scrofa
<400> 10
   caaggagacc aagctggaac tc 22
<210> 11
   <211> 24
   <212> DNA
   <213> Sus scrofa
<400> 11
   tgatcaagca caccacagag acag 24
<210> 12
   <211> 4277
   <212> DNA
   <213> Sus scrofa
<400> 12
   caaggaacca agctggaact caaacgtaag tcaatccaaa cgttccttcc ttggctgtct 60
   gtgtcttacg gtctctgtgg ctctgaaatg attcatgtgc tgactctctg aaaccagact 120
   gacattctcc agggcaaaac taaagcctgt catcaaactg gaaaactgag ggcacatttt 180
   ctgggcagaa ctaagagtca ggcactgggt gaggaaaaac ttgttagaat gatagtttca 240
   gaaacttact gggaagcaaa gcccatgttc tgaacagagc tctgctcaag ggtcaggagg 300
   ggaaccagtt tttgtacagg agggaagttg agacgaaccc ctgtgtatat ggtttcggcg 360
   cggggaccaa gctggagctc aaacgtaagt ggctttttcc gactgattct ttgctgtttc 420
   taattgttgg ttggcttttt gtccattttt cagtgttttc atcgaattag ttgtcaggga 480
   ccaaacaaat tgccttccca gattaggtac cagggagggg acattgctgc atgggagacc 540
   agagggtggc taatttttaa cgtttccaag ccaaaataac tggggaaggg ggcttgctgt 600
   cctgtgaggg taggttttta tagaagtgga agttaagggg aaatcgctat ggttcacttt 660
   tggctcgggg accaaagtgg agcccaaaat tgagtacatt ttccatcaat tatttgtgag 720
   atttttgtcc tgttgtgtca tttgtgcaag tttttgacat tttggttgaa tgagccattc 780
   ccagggaccc aaaaggatga gaccgaaaag tagaaaagag ccaactttta agctgagcag 840
   acagaccgaa ttgttgagtt tgtgaggaga gtagggtttg tagggagaaa ggggaacaga 900
   tcgctggctt tttctctgaa ttagcctttc tcatgggact ggcttcagag ggggtttttg 960
   atgagggaag tgttctagag ccttaactgt gggttgtgtt cggtagcggg accaagctgg 1020
   aaatcaaacg taagtgcact tttctactcc tttttctttc ttatacgggt gtgaaattgg 1080
   ggacttttca tgtttggagt atgagttgag gtcagttctg aagagagtgg gactcatcca 1140
   aaaatctgag gagtaagggt cagaacagag ttgtctcatg gaagaacaaa gacctagtta 1200
   gttgatgagg cagctaaatg agtcagttga cttgggatcc aaatggccag acttcgtctg 1260
   taaccaacaa tctaatgaga tgtagcagca aaaagagatt tccattgagg ggaaagtaaa 1320
   attgttaata ttgtggatca cctttggtga agggacatcc gtggagattg aacgtaagta 1380
   ttttttctct actaccttct gaaatttgtc taaatgccag tgttgacttt tagaggctta 1440
   agtgtcagtt ttgtgaaaaa tgggtaaaca agagcatttc atatttatta tcagtttcaa 1500
   aagttaaact cagctccaaa aatgaatttg tagacaaaaa gattaattta agccaaattg 1560
   aatgattcaa aggaaaaaaa aattagtgta gatgaaaaag gaattcttac agctccaaag 1620
   agcaaaagcg aattaatttt ctttgaactt tgccaaatct tgtaaatgat ttttgttctt 1680
   tacaatttaa aaaggttaga gaaatgtatt tcttagtctg ttttctctct tctgtctgat 1740
   aaattattat atgagataaa aatgaaaatt aataggatgt gctaaaaaat cagtaagaag 1800
   ttagaaaaat atatgtttat gttaaagttg ccacttaatt gagaatcaga agcaatgtta 1860
   tttttaaagt ctaaaatgag agataaactg tcaatactta aattctgcag agattctata 1920
   tcttgacaga tatctccttt ttcaaaaatc caatttctat ggtagactaa atttgaaatg 1980
   atcttcctca taatggaggg aaaagatgga ctgaccccaa aagctcagat ttaagaaaac 2040
   ctgtttaagg aaagaaaata aaagaactgc attttttaaa ggcccatgaa tttgtagaaa 2100
   aataggaaat attttaataa gtgtattctt ttattttcct gttattactt gatggtgttt 2160
   ttataccgcc aaggaggccg tggcaccgtc agtgtgatct gtagacccca tggcggcctt 2220
   ttttcgcgat tgaatgacct tggcggtggg tccccagggc tctggtggca gcgcaccagc 2280
   cgctaaaagc cgctaaaaac tgccgctaaa ggccacagca accccgcgac cgcccgttca 2340
   actgtgctga cacagtgata cagataatgt cgctaacaga ggagaataga aatatgacgg 2400
   gcacacgcta atgtggggaa aagagggaga agcctgattt ttatttttta gagattctag 2460
   agataaaatt cccagtatta tatcctttta ataaaaaatt tctattagga gattataaag 2520
   aatttaaagc tattttttta agtggggtgt aattctttca gtagtctctt gtcaaatgga 2580
   tttaagtaat agaggcttaa tccaaatgag agaaatagac gcataaccct ttcaaggcaa 2640
   aagctacaag agcaaaaatt gaacacagca gccagccatc tagccactca gattttgatc 2700
   agttttactg agtttgaagt aaatatcatg aaggtataat tgctgataaa aaaataagat 2760
   acaggtgtga cacatcttta agtttcagaa atttaatggc ttcagtagga ttatatttca 2820
   cgtatacaaa gtatctaagc agataaaaat gccattaatg gaaacttaat agaaatatat 2880
   ttttaaattc cttcattctg tgacagaaat tttctaatct gggtctttta atcacctacc 2940
   ctttgaaaga gtttagtaat ttgctatttg ccatcgctgt ttactccagc taatttcaaa 3000
   agtgatactt gagaaagatt atttttggtt tgcaaccacc tggcaggact attttagggc 3060
   cattttaaaa ctcttttcaa actaagtatt ttaaactgtt ctaaaccatt tagggccttt 3120
   taaaaatctt ttcatgaatt tcaaacttcg ttaaaagtta ttaaggtgtc tggcaagaac 3180
   ttccttatca aatatgctaa tagtttaatc tgttaatgca ggatataaaa ttaaagtgat 3240
   caaggcttga cccaaacagg agtatcttca tagcatattt cccctccttt ttttctagaa 3300
   ttcatatgat tttgctgcca aggctatttt atataatctc tggaaaaaaa atagtaatga 3360
   aggttaaaag agaagaaaat atcagaacat taagaattcg gtattttact aactgcttgg 3420
   ttaacatgaa ggtttttatt ttattaaggt ttctatcttt ataaaaatct gttccctttt 3480
   ctgctgattt ctccaagcaa aagattcttg atttgttttt taactcttac tctcccaccc 3540
   aagggcctga atgcccacaa aggggacttc caggaggcca tctggcagct gctcaccgtc 3600
   agaagtgaag ccagccagtt cctcctgggc aggtggccaa aattacagtt gacccctcct 3660
   ggtctggctg aaccttgccc catatggtga cagccatctg gccagggccc aggtctccct 3720
   ctgaagcctt tgggaggaga gggagagtgg ctggcccgat cacagatgcg gaaggggctg 3780
   actcctcaac cggggtgcag actctgcagg gtgggtctgg gcccaacaca cccaaagcac 3840
   gcccaggaag gaaaggcagc ttggtatcac tgcccagagc taggagaggc accgggaaaa 3900
   tgatctgtcc aagacccgtt cttgcttcta aactccgagg gggtcagatg aagtggtttt 3960
   gtttcttggc ctgaagcatc gtgttccctg caagaagcgg ggaacacaga ggaaggagag 4020
   aaaagatgaa ctgaacaaag catgcaaggc aaaaaaggcc ttaggatggc tgcaggaagt 4080
   tagttcttct gcattggctc cttactggct cgtcgatcgc ccacaaacaa cgcacccagt 4140
   ggagaacttc cctgttactt aaacaccatt ctctgtgctt gcttcctcag gggctgatgc 4200
   caagccatcc gtcttcatct tcccgccatc gaaggagcag ttagcgaccc caactgtctc 4260
   tgtggtgtgc ttgatca 4277
<210> 13
   <211> 24
   <212> DNA
   <213> Sus scrofa
<400> 13
   gatgccaagc catccgtctt catc 24
<210> 14
   <211> 24
   <212> DNA
   <213> Sus scrofa
<400> 14
   tgaccaaagc agtgtgacgg ttgc 24
<210> 15
   <211> 2027
   <212> DNA
   <213> Sus scrofa
<400> 15
   gatgccaagc catccgtctt catcttcccg ccatcgaagg agcagttagc gaccccaact 60
   gtctctgtgg tgtgcttgat caataacttc ttccccagag aaatcagtgt caagtggaaa 120
   gtggatgggg tggtccaaag cagtggtcat ccggatagtg tcacagagca ggacagcaag 180
   gacagcacct acagcctcag cagcaccctc tcgctgccca cgtcacagta cctaagtcat 240
   aatttatatt cctgtgaggt cacccacaag accctggcct cccctctggt cacaagcttc 300
   aacaggaacg agtgtgaggc ttagaggccc acaggcccct ggcctgcccc cagccccagc 360
   ccccctcccc acctcaagcc tcaggccctt gccccagagg atccttggca atcccccagc 420
   ccctcttccc tcctcatccc ctccccctct ttggctttaa ccgtgttaat actggggggt 480
   gggggaatga ataaataaag tgaacctttg cacctgtgat ttctctctcc tgtctgattt 540
   taaggttgtt aaatgttgtt ttccccatta tagttaatct tttaaggaac tacatactga 600
   gttgctaaaa actacaccat cacttataaa attcacgcct tctcagttct cccctcccct 660
   cctgtcctcc gtaagacagg cctccgtgaa acccataagc acttctcttt acaccctctc 720
   ctgggccggg gtaggagact ttttgatgtc ccctcttcag caagcctcag aaccattttg 780
   agggggacag ttcttacagt cacattcctg tgatctaatg actttagtta ccgaaaagcc 840
   agtctctcaa aaagaaggga acggctagaa accaagtcat agaaatatat atgtataaaa 900
   tatatatata tccatatatg taaaataaca aaataatgat aacagcatag gtcaacaggc 960
   aacagggaat gttgaagtcc attctggcac ttcaatttaa gggaatagga tgccttcatt 1020
   acattttaaa tacaatacac atggagagct tcctatctgc caaagaccat cctgaatgcc 1080
   ttccacactc actacaaggt taaaagcatt cattacaatg ttgatcgagg agttcccgtt 1140
   gtggctcagc aggttaagaa cgtgactggt atccaggagg atgcgggttt ggtccccagc 1200
   ctcgctcagt ggattaagga tccagtgttg ctgcaagatc acgggctcag atcccgtgtt 1260
   ctatggctat ggtgtaggct ggtagctgca tgcagcccta atttgacccc tagcctggga 1320
   actgccatat gccacatgtg aggcccttaa aacctaaaag aaaaaaaaag aaaagaaata 1380
   tcttacaccc aatttataga taagagagaa gctaaggtgg caggcccagg atcaaagccc 1440
   tacctgccta tcttgacacc tgatacaaat tctgtcttct agggtttcca acactgcata 1500
   gaacagaggg tcaaacatgc taccctccca gggactcctc ccttcaaatg acataaattt 1560
   tgttgcccat ctctgggggc aaaactcaac aatcaatggc atctctagta ccaagcaagg 1620
   ctcttctcat gaagcaaaac tctgaagcca gatccatcat gacccaagga agtaaagaca 1680
   ggtgttactg gttgaactgt atccttcaat tcaatatgct caatttccaa ctcccagtcc 1740
   ccgtaaatac aacccccttt gggaagagag tccttgcaga tgtagccacg ttaaaaagag 1800
   attatacaga aaggctagtg aggatgcagt gaaacgggat ctttcataca ttgctggtgg 1860
   aaatgtaaaa tgctgcaggc actctagaaa ataatttgcc agttttttga aaagctaaac 1920
   aaaatagttt agttgcattc tgggttattt atcccccaga aattaaaaat tatgtccgca 1980
   caaaaacgtg tacataatca ttcataacag ccttgtacga aaagctt 2027
<210> 16
   <211> 3918
   <212> DNA
   <213> Sus scrofa
<400> 16
   ggatccttaa cccactaatc gaggatcaaa cacgcatcct catggacaat atgttgggtt 60
   cttagcctgc tgagacacaa caggaactcc cctggcacca ctttagaggc cagagaaaca 120
   gcacagataa aattccctgc cctcatgaag cttatagtct agctggggag atatcatagg 180
   caagataaac acatacaaat acatcatctt aggtaataat atatactaag gagaaaatta 240
   caggggagaa agaggacagg aattgctagg gtaggattat aagttcagat agttcatcag 300
   gaacactgtt gctgagaaga taacatttag gtaaagaccg aagtagtaag gaaatggacc 360
   gtgtgcctaa gtgggtaaga ccattctagg cagcaggaac agcgatgaaa gcactgaggt 420
   gggtgttcac tgcacagagt tgttcactgc acagagttgt gtggggaggg gtaggtcttg 480
   caggctctta tggtcacagg aagaattgtt ttactcccac cgagatgaag gttggtggat 540
   tttgagcaga agaataattc tgcctggttt atatataaca ggatttccct gggtgctctg 600
   atgagaataa tctgtcaggg gtgggatagg gagagatatg gcaataggag ccttggctag 660
   gagcccacga caataattcc aagtgagagg tggtgctgca ttgaaagcag gactaacaag 720
   acctgctgac agtgtggatg tagaaaaaga tagaggagac gaaggtgcat ctagggtttt 780
   ctgcctgagg aattagaaag ataaagctaa agcttataga agatgcagcg ctctggggag 840
   aaagaccagc agctcagttt tgatccatct ggaattaatt ttggcataaa gtatgaggta 900
   tgtgggttaa cattatttgt tttttttttt tccatgtagc tatccaactg tcccagcatc 960
   atttatttta aaagactttc ctttccccta ttggattgtt ttggcacctt cactgaagat 1020
   caactgagca taaaattggg tctatttcta agctcttgat tccattccat gacctatttg 1080
   ttcatcttta ccccagtaga cactgccttg atgattaaag cccctgttac catgtctgtt 1140
   ttggacatgg taaatctgag atgcctatta gccaaccaag caagcacggc ccttagagag 1200
   ctagatatga gagcctggaa ttcagacgag aaaggtcagt cctagagaca tacatgtagt 1260
   gccatcacca tgcggatggt gttaaaagcc atcagactgc aacagactgt gagagggtac 1320
   caagctagag agcatggata gagaaaccca agcactgagc tgggaggtgc tcctacatta 1380
   agagattagt gagatgaagg actgagaaga ttgatcagag aagaaggaaa atcaggaaaa 1440
   tggtgctgtc ctgaaaatcc aagggaagag atgttccaaa gaggagaaaa ctgatcagtt 1500
   gtcagctagc gtcaattggg atgaaaatgg accattggac agagggatgt agtgggtcat 1560
   gggtgaatag ataagagcag cttctataga atggcagggg caaaattctc atctgatcgg 1620
   catgggttct aaagaaaacg ggaagaaaaa attgagtgca tgaccagtcc cttcaagtag 1680
   agaggtggaa aagggaagga ggaaaatgag gccacgacaa catgagagaa atgacagcat 1740
   ttttaaaaat tttttatttt attttattta tttatttttg ctttttaggg ctgcccctgc 1800
   aacatatgga ggttcccagg ttaggggtct aatcagagct atagctgcca gcctacacca 1860
   cagccatagc aatgccagat ctacatgacc tacaccacag ctcacagcaa cgccggatcc 1920
   ttaacccact gagtgaggcc agagatcaaa cccatatcct tatggatact agtcaggttc 1980
   attaccactg agccaaaatg ggaaatcctg agtaatgaca gcatttttta atgtgccagg 2040
   aagcaaaact tgccaccccg aaatgtctct caggcatgtg gattattttg agctgaaaac 2100
   gattaaggcc caaaaaacac aagaagaaat gtggaccttc ccccaacagc ctaaaaaatt 2160
   tagattgagg gcctgttccc agaatagagc tattgccaga cttgtctaca gaggctaagg 2220
   gctaggtgtg gtggggaaac cctcagagat cagagggacg tttatgtacc aagcattgac 2280
   atttccatct ccatgcgaat ggccttcttc ccctctgtag ccccaaacca ccacccccaa 2340
   aatcttcttc tgtctttagc tgaagatggt gttgaaggtg atagtttcag ccactttggc 2400
   gagttcctca gttgttctgg gtctttcctc cggatccaca ttattcgact gtgtttgatt 2460
   ttctcctgtt tatctgtctc attggcaccc atttcattct tagaccagcc caaagaacct 2520
   agaagagtga aggaaaattt cttccaccct gacaaatgct aaatgagaat caccgcagta 2580
   gaggaaaatg atctggtgct gcgggagata gaagagaaaa tcgctggaga gatgtcactg 2640
   agtaggtgag atgggaaagg gggggcacag gtggaggtgt tgccctcagc taggaagaca 2700
   gacagttcac agaagagaag cgggtgtccg tggacatctt gcctcatgga tgaggaaacc 2760
   gaggctaaga aagactgcaa aagaaaggta aggattgcag agaggtcgat ccatgactaa 2820
   aatcacagta accaacccca aaccaccatg ttttctccta gtctggcacg tggcaggtac 2880
   tgtgtaggtt ttcaatatta ttggtttgta acagtaccta ttaggcctcc atcccctcct 2940
   ctaatactaa caaaagtgtg agactggtca gtgaaaaatg gtcttctttc tctatgcaat 3000
   ctttctcaag aagatacata actttttatt ttatcatagg cttgaagagc aaatgagaaa 3060
   cagcctccaa cctatgacac cgtaacaaag tgtttatgat cagtgaaggg caagaaacaa 3120
   aacatacaca gtaaagaccc tccataatat tgtgggctgg cccaacacag gccaggttgt 3180
   aaaagctttt tattctttga tagaggaatg gatagtaatg tttcaacctg gacagagatc   3240
   atgttcactg aatccttcca aaaattcatg ggtagtttga attataagga aaataagact   3300
   taggataaat actttgtcca gatcccagag ttaatgccaa aatcagtttt cagactccag   3360
   gcagcctgat caagagccta aactttaaag acacagtccc ttaataacta ctattcacag   3420
   ttgcactttc agggcgcaaa gactcattga atcctacaat agaatgagtt tagatatcaa   3480
   atctctcagt aatagatgag gagactaaat agcgggcatg acctggtcac ttaaagacag   3540
   aattgagatt caaggctagt gttctttcta cctgttttgt ttctacaaga tgtagcaatg   3600
   cgctaattac agacctctca gggaaggaat tcacaaccct cagcaaaaac caaagacaaa   3660
   tctaagacaa ctaagagtgt tggtttaatt tggaaaaata actcactaac caaacgcccc   3720
   tcttagcacc ccaatgtctt ccaccatcac agtgctcagg cctcaaccat gccccaatca   3780
   ccccagcccc agactggtta ttaccaagtt tcatgatgac tggcctgaga agatcaaaaa   3840
   agcaatgaca tcttacaggg gactaccccg aggaccaaga tagcaactgt catagcaacc   3900
   gtcacactgc tttggtca                                                 3918
<210> 17
   <211> 25
   <212> DNA
   <213> Sus scrofa
<400> 17
   ggatcaaaca cgcatcctca tggac 25
<210> 18
   <211> 22
   <212> DNA
   <213> Sus scrofa
<400> 18
   ggtgattggg gcatggttga gg 22
<210> 19
   <211> 3759
   <212> DNA
   <213> Sus scrofa
<400> 19
   ggatcaaaca cgcatcctca tggacaatat gttgggttct tagcctgctg agacacaaca     60
   ggaactcccc tggcaccact ttagaggcca gagaaacagc acagataaaa ttccctgccc    120
   tcatgaagct tatagtctag ctggggagat atcataggca agataaacac atacaaatac    180
   atcatcttag gtaataatat atactaagga gaaaattaca ggggagaaag aggacaggaa    240
   ttgctagggt aggattataa gttcagatag ttcatcagga acactgttgc tgagaagata    300
   acatttaggt aaagaccgaa gtagtaagga aatggaccgt gtgcctaagt gggtaagacc    360
   attctaggca gcaggaacag cgatgaaagc actgaggtgg gtgttcactg cacagagttg    420
   ttcactgcac agagttgtgt ggggaggggt aggtcttgca ggctcttatg gtcacaggaa 480
   gaattgtttt actcccaccg agatgaaggt tggtggattt tgagcagaag aataattctg 540
   cctggtttat atataacagg atttccctgg gtgctctgat gagaataatc tgtcaggggt 600
   gggataggga gagatatggc aataggagcc ttggctagga gcccacgaca ataattccaa 660
   gtgagaggtg gtgctgcatt gaaagcagga ctaacaagac ctgctgacag tgtggatgta 720
   gaaaaagata gaggagacga aggtgcatct agggttttct gcctgaggaa ttagaaagat 780
   aaagctaaag cttatagaag atgcagcgct ctggggagaa agaccagcag ctcagttttg 840
   atccatctgg aattaatttt ggcataaagt atgaggtatg tgggttaaca ttatttgttt 900
   tttttttttc catgtagcta tccaactgtc ccagcatcat ttattttaaa agactttcct 960
   ttcccctatt ggattgtttt ggcaccttca ctgaagatca actgagcata aaattgggtc 1020
   tatttctaag ctcttgattc cattccatga cctatttgtt catctttacc ccagtagaca 1080
   ctgccttgat gattaaagcc cctgttacca tgtctgtttt ggacatggta aatctgagat 1140
   gcctattagc caaccaagca agcacggccc ttagagagct agatatgaga gcctggaatt 1200
   cagacgagaa aggtcagtcc tagagacata catgtagtgc catcaccatg cggatggtgt 1260
   taaaagccat cagactgcaa cagactgtga gagggtacca agctagagag catggataga 1320
   gaaacccaag cactgagctg ggaggtgctc ctacattaag agattagtga gatgaaggac 1380
   tgagaagatt gatcagagaa gaaggaaaat caggaaaatg gtgctgtcct gaaaatccaa 1440
   gggaagagat gttccaaaga ggagaaaact gatcagttgt cagctagcgt caattgggat 1500
   gaaaatggac cattggacag agggatgtag tgggtcatgg gtgaatagat aagagcagct 1560
   tctatagaat ggcaggggca aaattctcat ctgatcggca tgggttctaa agaaaacggg 1620
   aagaaaaaat tgagtgcatg accagtccct tcaagtagag aggtggaaaa gggaaggagg 1680
   aaaatgaggc cacgacaaca tgagagaaat gacagcattt ttaaaaattt tttattttat 1740
   tttatttatt tatttttgct ttttagggct gcccctgcaa catatggagg ttcccaggtt 1800
   aggggtctaa tcagagctat agctgccagc ctacaccaca gccatagcaa tgccagatct 1860
   acatgaccta caccacagct cacagcaacg ccggatcctt aacccactga gtgaggccag 1920
   agatcaaacc catatcctta tggatactag tcaggttcat taccactgag ccaaaatggg 1980
   aaatcctgag taatgacagc attttttaat gtgccaggaa gcaaaacttg ccaccccgaa 2040
   atgtctctca ggcatgtgga ttattttgag ctgaaaacga ttaaggccca aaaaacacaa 2100
   gaagaaatgt ggaccttccc ccaacagcct aaaaaattta gattgagggc ctgttcccag 2160
   aatagagcta ttgccagact tgtctacaga ggctaagggc taggtgtggt ggggaaaccc 2220
   tcagagatca gagggacgtt tatgtaccaa gcattgacat ttccatctcc atgcgaatgg 2280
   ccttcttccc ctctgtagcc ccaaaccacc acccccaaaa tcttcttctg tctttagctg 2340
   aagatggtgt tgaaggtgat agtttcagcc actttggcga gttcctcagt tgttctgggt 2400
   ctttcctcct gatccacatt attcgactgt gtttgatttt ctcctgttta tctgtctcat 2460
   tggcacccat ttcattctta gaccagccca aagaacctag aagagtgaag gaaaatttct 2520
   tccaccctga caaatgctaa atgagaatca ccgcagtaga ggaaaatgat ctggtgctgc 2580
   gggagataga agagaaaatc gctggagaga tgtcactgag taggtgagat gggaaagggg 2640
   tgacacaggt ggaggtgttg ccctcagcta ggaagacaga cagttcacag aagagaagcg 2700
   ggtgtccgtg gacatcttgc ctcatggatg aggaaaccga ggctaagaaa gactgcaaaa 2760
   gaaaggtaag gattgcagag aggtcgatcc atgactaaaa tcacagtaac caaccccaaa 2820
   ccaccatgtt ttctcctagt ctggcacgtg gcaggtactg tgtaggtttt caatattatt 2880
   ggtttgtaac agtacctatt aggcctccat cccctcctct aatactaaca aaagtgtgag 2940
   actggtcagt gaaaaatggt cttctttctc tatgaatctt tctcaagaag atacataact 3000
   ttttatttta tcataggctt gaagagcaaa tgagaaacag cctccaacct atgacaccgt 3060
   aacaaaatgt ttatgatcag tgaagggcaa gaaacaaaac atacacagta aagaccctcc 3120
   ataatattgt gggtggccca acacaggcca ggttgtaaaa gctttttatt ctttgataga 3180
   ggaatggata gtaatgtttc aacctggaca gagatcatgt tcactgaatc cttccaaaaa 3240
   ttcatgggta gtttgaatta taaggaaaat aagacttagg ataaatactt tgtccaagat 3300
   cccagagtta atgccaaaat cagttttcag actccaggca gcctgatcaa gagcctaaac 3360
   tttaaagaca cagtccctta ataactacta ttcacagttg cactttcagg gcgcaaagac 3420
   tcattgaatc ctacaataga atgagtttag atatcaaatc tctcagtaat agatgaggag 3480
   actaaatagc gggcatgacc tggtcactta aagacagaat tgagattcaa ggctagtgtt 3540
   ctttctacct gttttgtttc tacaagatgt agcaatgcgc taattacaga cctctcaggg 3600
   aaggaattca caaccctcag caaaaaccaa agacaaatct aagacaacta agagtgttgg 3660
   tttaatttgg aaaaataact cactaaccaa acgcccctct tagcacccca atgtcttcca 3720
   ccatcacagt gctcaggcct caaccatgcc ccaatcacc 3759
<210> 20
   <211> 9010
   <212> DNA
   <213> Sus scrofa
<400> 20
<210> 21
   <211> 21
   <212> DNA
   <213> Sus scrofa
<400> 21
   cgaacccctg tgtatatagt t 21
<210> 22
   <211> 21
   <212> DNA
   <213> Sus scrofa
<400> 22
   gagatgagga agaggagaac a 21
<210> 23
   <211> 23
   <212> DNA
   <213> Sus scrofa
<400> 23
   gcattgtctg agtaggtgtc att 23
<210> 24
   <211> 22
   <212> DNA
   <213> Sus scrofa
<400> 24
   cgcttcttgc agggaacacg at 22
<210> 25
   <211> 6772
   <212> DNA
   <213> Sus scrofa
<400> 25
   gcacatggta ggcaaaggac tttgcttctc ccagcacatc tttctgcaga gatccatgga 60
   aacaagactc aactccaaag cagcaaagaa gcagcaagtt ctcaagtgat ctcctctgac 120
   tccctcctcc caggctaatg aagccatgtt gcccctgggg gattaagggc aggtgtccat 180
   tgtggcaccc agcccgaaga caagcaattt gatcaggttc tgagcactcc tgaatgtgga 240
   ctctggaatt ttctcctcac cttgtggcat atcagcttaa gtcaagtaca agtgacaaac 300
   aacataatcc taagaagaga ggaatcaagc tgaagtcaaa ggatcactgc cttggattct 360
   actgtgaatg atgacctgga aaatatcctg aacaacagct tcagggtgat catcagagac 420
   aaaagttcca gagccaggta gggaaaccct caagccttgc aaagagcaaa atcatgccat 480
   tgggttctta acctgctgag tgatttacta tatgttactg tgggaggcaa agcgctcaaa 540
   tagcctgggt aagtatgtca aataaaaagc aaaagtggtg tttcttgaaa tgttagacct 600
   gaggaaggaa tattgataac ttaccaataa ttttcagaat gatttataga tgtgcactta 660
   gtcagtgtct ctccaccccg cacctgacaa gcagtttaga atttattcta agaatctagg 720
   tttgctgggg gctacatggg aatcagcttc agtgaagagt ttgttggaat gattcactaa 780
   attttctatt tccagcataa atccaagaac ctctcagact agtttattga cactgctttt 840
   cctccataat ccatctcatc tccgtccatc atggacactt tgtagaatga caggtcctgg 900
   cagagactca cagatgcttc tgaaacatcc tttgccttca aagaatgaac agcacacata 960
   ctaaggatct cagtgatcca caaattagtt tttgccacaa tggttcttat gataaaagtc 1020
   tttcattaac agcaaattgt tttataatag ttgttctgct ttataataat tgcatgcttc 1080
   actttctttt cttttctttt tttttctttt tttgcttttt agtgccgcag gtgcagcata 1140
   tgaaatttcc caggctaggg gtcaaatcag aactacacct actggcctac gccacagcca 1200
   cagcaactca ggatctaagc catgtcggtg acctacacta cagctcatgg caatgccaga 1260
   tccttaaccc aatgagcgag gccagggatc gaacccatgt cctcatggat actagtcagg 1320
   ctcattatcc gctgagccat aacaggaact cccgagtttg ctttttatca aaattggtac 1380
   agccttattg tttctgaaaa ccacaaaatg aatgtattca cataatttta aaaggttaaa 1440
   taatttatga tatacaagac aatagaaaga gaaaacgtca ttgcctcttt cttccacgac 1500
   aacacgcctc cttaattgat ttgaagaaat aactactgag catggtttag tgtacttctt 1560
   tcagcaatta gcctgtattc atagccatac atattcaatt aaaatgagat catgatatca 1620
   cacaatacat accatacagc ctatagggat ttttacaatc atcttccaca tgactacata 1680
   aaaacctacc taaaaaaaaa aaaaacccta cttcatcctc ctattggctg ctttgtgctc 1740
   cattaaaaag ctctatcata attaggttat gatgaggatt tccattttct acctttcaag 1800
   caacatttca atgcacagtc ttatatacac atttgagcct acttttcttt ttctttcttt 1860
   ttttggtttt tttttttttt ttttttttgg tctttttgtc ttttctaagg ctgcatatgg 1920
   aggttcccag gctagctgtc taatcagaac tatagctgct ggcctacgcc acatccacag 1980
   caatacaaga tctgagccat gtctgcaact tacaccacag ctcacagcaa cggtggatcc 2040
   ttaaaccact gagcaaggcc agggatcaaa cccataactt catggctcct agttggattt 2100
   gttaaccact gagccatgat ggcaactcct gagcctactt ttctaatcat ttccaaccct 2160
   aggacacttt tttaagtttc atttttctcc ccccaccccc tgttttctga agtgtgtttg 2220
   cttccactgg gtgacttcac tcccaggatc tcatctgcag gatactgcag ctaagtgtat 2280
   gagctctgaa tttgaatccc aactctgcca ctcaaaggga taggagtttc cgatgtggcc 2340
   caatgggatc agtggcatct ctgcagtgcc aggacgcagg ttccatccct ggcccagcac 2400
   agtgggttaa gaatctggca ttgctgcagc tgaggcatag atttcaattg tgcctcagat 2460
   ctgatccttg gcccaaggac tgcatatgcc tcagggcaac caaaaaagag aaaagggggg 2520
   tgatagcatt agtttctaga tttgggggat aattaaataa agtgatccat gtacaatgta 2580
   tggcattttg taaatgctca acaaatttca actattatgg agttcccatc atggctcagt 2640
   ggaagggaat ctgattagca tccatgagga cacaggtcca accccgacct tgctcagtgg 2700
   gcattgctgt gagctgtggc atgggttaca gacgaagctc ggatctggca ttgctgtggc 2760
   tgtggtgtaa gccagcaact acagctctca ttcagcccct agcctgggaa cctccatatg 2820
   cctaaaagac aaaaaataaa atttaaatta aaaataaaga aatgttaact attatgattg 2880
   gtactgcttg cattactgca aagaaagtca ctttctatac tctttaatat cttagttgac 2940
   tgtgtgctca gtgaactatt ttggacactt aatttccact ctcttctatc tccaacttga 3000
   caactctctt tcctctcttc tggtgagatc cactgctgac tttgctcttt aaggcaacta 3060
   gaaaagtgct cagtgacaaa atcaaagaaa gttaccttaa tcttcagaat tacaatctta 3120
   agttctcttg taaagcttac tatttcagtg gttagtatta ttccttggtc ccttacaact 3180
   tatcagctct gatctattgc tgattttcaa ctatttattg ttggagtttt ttcctttttt 3240
   ccctgttcat tctgcaaatg tttgctgagc atttgtcaag tgaagatact ggactgggcc 3300
   ttccaaatat aagacaatga aacatcggga gttctcatta tggtgcagca gaaacgaatc 3360
   caactaggaa atgtgaggtt gcaggttcga tccctgccct tgctcagtgg gttaaggatc 3420
   cagcattacc gtgagctgtg gtgtaggttg cagacgtggc tcagatcctg cgttgctgtg 3480
   gctgtggcat aggctggcag ctctagctct gattcgaccg ctagcctggg aacctccatg 3540
   cgccccgagt gcagccctta aaaagcaaaa aaaaaagaaa gaaagaaaaa gacaatgaaa 3600
   catcaaacag ctaacaatcc agtagggtag aaagaatctg gcaacagata agagcgatta 3660
   aatgttctag gtccagtgac cttgcctctg tgctctacac agtcgtgcca cttgctgagg 3720
   gagaaggtct ctcttgagtt gagtcctgaa agacattagt tgttcacaaa ctaatgccag 3780
   tgagtgaagg tgtttccaag cagagggaga gtttggtaaa aagctggaag tcacagaaag 3840
   actctaaaga gtttaggatg gtgggagcaa catacgctga gatggggctg gaaggttaag 3900
   agggaaacaa ctatagtaag tgaagctgga ctcacagcaa agtgaggacc tcagcatcct 3960
   tgatggggtt accatggaaa caccaaggca caccttgatt tccaaaacag caggcacctg 4020
   attcagccca atgtgacatg gtgggtaccc ctctagctct acctgttctg tgacaactga 4080
   caaccaacga agttaagtct ggattttcta ctctgctgat ccttgttttt gtttcacacg 4140
   tcatctatag cttcatgcca aaatagagtt caaggtaaga cgcgggcctt ggtttgatat 4200
   acatgtagtc tatcttgttt gagacaatat ggtggcaagg aagaggttca aacaggaaaa 4260
   tactctctaa ttatgattaa ctgagaaaag ctaaagagtc ccataatgac actgaatgaa 4320
   gttcatcatt tgcaaaagcc ttcccccccc cccaggagac tataaaaaag tgcaattttt 4380
   taaatgaact tatttacaaa acagaaatag actcacagac ataggaaacg aacagatggt 4440
   taccaagggt gaaagggagt aggagggata aataaggagt ctggggttag cagatacacc 4500
   ccagtgtaca caaaataaac aacagggacc tactatatag cacagggaac tatatgcagt 4560
   agcttacaat aacctataat ggaaaagaat gtgaaaaaga atatatgtat gcgtgtgtgt 4620
   gtaactgaat cactttgctg taacctgaat ctaacataac attgtaaatc aactacagtt 4680
   tttttttttt ttaagtgcag ggttttggtg tttttttttt ttcatttttg tttttgtttt 4740
   tgttttttgc tttttagggc cacacccaga catatggggg ttcccaggct aggggtctaa 4800
   ttagagctac agttgccggc ttgcaccaca gccacagcaa catcagatcc gagccgcact 4860
   tgcgacttac accacagctc atggcaatac cagatcctta acccactgag caaggcccag 4920
   ggatcgtacc cgcaacctca tggttcctag tcagattcat ttctgctgcg ctacaatggg 4980
   aactccaagt gcagtttttt gtaatgtgct tgtctttctt tgtaattcat attcatccta 5040
   cttcccaata aataaataaa tacataaata ataaacatac cattgtaaat caactacaat 5100
   tttttttaaa tgcagggttt ttgttttttg ttttttgttt tgtctttttg ccttttctag 5160
   ggccgctccc atggcatatg gaggttccca ggctaggggt cgaatcggag ctgtagccac 5220
   cggcctacgc cagagccaca gcaacgcggg atccgagccg cgtctgcaac ctacaccaca 5280
   gctcacggca acgccggatc gttaacccac tgagcaaggg cagggatcga acctgcaacc 5340
   tcatggttcc tagtcagatt cgttaactac tgagccacaa cggaaactcc taaagtgcag 5400
   tttttaaatg tgcttgtctt tctttgtaat ttacactcaa cctacttccc aataaataaa 5460
   taaataaaca aataaatcat agacatggtt gaattctaaa ggaagggacc atcaggcctt 5520
   agacagaaat acgtcatctt ctagtatttt aaaacacact aaagaagaca aacatgctct 5580
   gccagagaag cccagggcct ccacagctgc ttgcaaaggg agttaggctt cagtagctga 5640
   cccaaggctc tgttcctctt cagggaaaag ggtttttgtt cagtgagaca gcagacagct 5700
   gtcactgtgg tggacgttcg gccaaggaac caagctggaa ctcaaacgta agtcaatcca 5760
   aacgttcctt ccttggctgt ctgtgtctta cggtctctgt ggctctgaaa tgattcatgt 5820
   gctgactctc tgaaaccaga ctgacattct ccagggcaaa actaaagcct gtcatcaaac 5880
   cggaaaactg agggcacatt ttctgggcag aactaagagt caggcactgg gtgaggaaaa 5940
   acttgttaga atgatagttt cagaaactta ctgggaagca aagcccatgt tctgaacaga 6000
   gctctgctca agggtcagga ggggaaccag tttttgtaca ggagggaagt tgagacgaac 6060
   ccctgtgtat atggtttcgg cgcggggacc aagctggagc tcaaacgtaa gtggcttttt 6120
   ccgactgatt ctttgctgtt tctaattgtt ggttggcttt ttgtccattt ttcagtgttt 6180
   tcatcgaatt agttgtcagg gaccaaacaa attgccttcc cagattaggt accagggagg 6240
   ggacattgct gcatgggaga ccagagggtg gctaattttt aacgtttcca agccaaaata 6300
   actggggaag ggggcttgct gtcctgtgag ggtaggtttt tatagaagtg gaagttaagg 6360
   ggaaatcgct atggttcact tttggctcgg ggaccaaagt ggagcccaaa attgagtaca 6420
   ttttccatca attatttgtg agatttttgt cctgttgtgt catttgtgca agtttttgac 6480
   attttggttg aatgagccat tcccagggac ccaaaaggat gagaccgaaa agtagaaaag 6540
   agccaacttt taagctgagc agacagaccg aattgttgag tttgtgagga gagtagggtt 6600
   tgtagggaga aaggggaaca gatcgctggc tttttctctg aattagcctt tctcatggga 6660
   ctggcttcag agggggtttt tgatgaggga agtgttctag agccttaact gtgggttgtg 6720
   ttcggtagcg ggaccaagct ggaaatcaaa cgtaagtgca cttttctact cc 6772
<210> 26
   <211> 22
   <212> DNA
   <213> Sus scrofa
<400> 26
   ccttcctcct gcacctgtca ac 22
<210> 27
   <211> 22
   <212> DNA
   <213> Sus scrofa
<400> 27
   tagacacacc agggtggcct tg 22
<210> 28
   <211> 5918
   <212> DNA
   <213> Sus scrofa
<400> 28
   ccttcctcct gcacctgtca actcccaata aaccgtcctc cttgtcattc agaaatcatg 60
   ctctccgctc acttgtgtct acccattttc gggcttgcat ggggtcatcc tcgaaggtgg 120
   agagagtccc ccttggcctt ggggaagtcg aggggggcgg ggggaggcct gaggcatgtg 180
   ccagcgaggg gggtcacctc cacgcccctg aggaccttct agaaccaggg gcgtggggcc 240
   accgcctgag tggaaggctg tccacttttc ccccgggccc ccaggctccc tcctccgtgt 300
   ggaccttgtc cacctctgac tggcccagcc actcatgcat tgtttccccg aaaccccagg 360
   acgatagctc agcacgcgac agtgtccccc tctgagggcc tctgtccatt tcaggacgac 420
   ccgcatgtac agcgtgacca ctctgctcac gcccactcac cacgtcctag agccccaccc 480
   ccagccccat ccttaggggc acagccagct ccgaccgccc cggggacacc accctctgcc 540
   ccttccccag gccctccctg tcacacgcac cacagggccc tccgtcccga gaccctgctc 600
   cctcatccct cggtcccctc aggtagcctt ccacccgcgt gtgtcccgag gtcccagatg 660
   cagcaaggcc cctgggacaa cgccagatct ctgctctccc cgacccctca gaagccagcc 720
   cacgcctggc cccaccacca ctgcctaacg tccaagtgtc cataggcctc gggacctcca 780
   agtccaggtt ctgcctctgg gattccgcca tgggtctgcc tgggaaatga tgcacttgga 840
   ggagctcagc atgggatgcg ggaccttgtc tctaggcgct ccctcaggat cccacagctg 900
   ccctgtgaga cacacacaca cacacacaca cacacacaca cacacacaca cacacaaaca 960
   cgcatgcacg cacgccggca cacacgctat tgcagagatg gccacggtag ctgtgcctcg 1020
   aggccgagtg gagtgtctag aactctcggg ggtcccctct gcagacgaca ctgctccatc 1080
   ccccccgtgc cctgaagggc tcctcactct cccatcagga tctctccaag ctgctgacct 1140
   ggagaggaag gggcctggga caggcgggga cactcagacc tccctgctgc ccctcctctg 1200
   cctgggcttg gacggctccc cccttcccac gggtgaaggt gcaggtgggg agagggcacc 1260
   cccctcagcc tcccagaccc agaccagccc ccgtggcagg ggcagcctgt gagcctccag 1320
   ccagatgcag gtggcctggg gtggggggtg gagggggcgg gaggtttatg tttgaggctg 1380
   tatcactgtg taatattttc ggcggtggga cccatctgac cgtcctcggt gagtctcccc 1440
   ttttctctcc tccttgggga tccgagtgaa atctgggtcg atcttctctc cgttctcctc 1500
   cgactggggc tgaggtctga acctcggtgg ggtccgaaga ggaggcccct aggccaggct 1560
   cctcagcccc tccagcccga ccggccctct tgacacaggg tccagctaag ggcagacatg 1620
   gaggctgcta gtccagggcc aggctctgag acccaagggc gctgcccaag gaacccttgc 1680
   cccagggacc ctgggagcaa agctcctcac tcagagcctg cagccctggg gtctgaggac 1740
   aaggagggac tgaggactgg gcgtggggag ttcaggcggg gacaccaggt ccagggaggt 1800
   gacaaaggcg ctgggagggg gcggacggtg ccggggactc ctcctgggcc ctgtgggctc 1860
   ggggtccttg tgaggaccct gagggactga ggggcccctg ggcctaggga cttgcagtga 1920
   gggaggcagg gagtgtccct tgagaacgtg gcctccgcgg gctgggtccc cctcgtgctc 1980
   ccagccggga ggacacccca gagcaagcgc cccaggtggg cggggagggt ctcctcacag 2040
   gggcagctga cagatagagg cccccgccag gcagatgctt gatcctggca gttatactgg 2100
   gttcgcacaa ctttccctga acaaggggcc ctccgaacag acacagacgc aacccagtcg 2160
   acccaggctc agcacagaaa atgcactgac acccaaaacc ctcatctggg ggcctggccg 2220
   gcatcccgcc ccaggaccca aggcccctgc cccctggcag ccctggacac ggtcctctgt 2280
   gggcggtggg gtcggggctg tggtgacggt ggcatcgggg agcctgtgcc ccctccctga 2340
   aagggcggag aggctcaaga ggggacagaa atgtcctccc ctaggaagac ctcggacggg 2400
   ggcggggggg tggtctccga cagacagatg cccgggaccg acagacctgc cgagggaaga 2460
   gggcacctcg gtcgggttag gctccaggca gcacgaggga gcgaggctgg gagggtgagg 2520
   acatgggagc ctgaggagga gctggagact tcagcaggcc cccagctccg ggcttcgggc 2580
   tctgagatgc tcggacgcaa ggtgagtgac cccacctgtg gctgacctga cctcaggggg 2640
   acaaggctca gcctgggact ctgtgtcccc atcgcctgca caggggattc ccctgatgga 2700
   cactgagcca acgacctccc gtctctcccc gacccccagg tcagcccaag gccactccca 2760
   cggtcaacct cttcccgccc tcctctgagg agctcggcac caacaaggcc accctggtgt 2820
   gtctaataag tgacttctac ccgggcgccg tgacggtgac ctggaaggca ggcggcacca 2880
   ccgtcaccca gggcgtggag accaccaagc cctcgaaaca gagcaacaac aagtacgcgg 2940
   ccagcagcta cctggccctg tccgccagtg actggaaatc ttccagcggc ttcacctgcc 3000
   aggtcaccca cgaggggacc attgtggaga agacagtgac gccctccgag tgcgcctagg 3060
   tccctgggcc cccaccctca ggggcctgga gccacaggac ccccgcgagg gtctccccgc 3120
   gaccctggtc cagcccagcc cttcctcctg cacctgtcaa ctcccaataa accgtcctcc 3180
   ttgtcattca gaaatcatgc tctccgctca cttgtgtcta cccattttcg ggcttgcatg 3240
   gggtcatcct cgaaggtgga gagagtcccc cttggccttg gggaaatcga ggggggcggg 3300
   gggaggcctg aggcatgtgc cagcgagggg ggtcacctcc acgcccctga ggaccttcta 3360
   gaaccagggg cgtggggcca ccgccagagt ggaaggctgt ccacttttcc cccgggcccc 3420
   caggctccct cctccgtgtg gaccttgtcc acctctgact ggcccagcca ctcatgcatt 3480
   gtttccccga aaccccagga cgatagctca gcacgcgaca gtgtccccct ctgagggcct 3540
   ctgtccattt caggacgacc cgcatgtaca gcgtgaccac tctgctcacg cccactcacc 3600
   acgtcctaga gccccacccc cagccccatc cttaggggca cagccagctc cgaccgcccc 3660
   ggggacacca ccctctgccc cttccccagg ccctccctgt cacacgcacc acagggccct 3720
   ccgtcccgag accctgctcc ctcatccctc ggtcccctca ggtagccttc cacccgcgtg 3780
   tgtcccgagg tcccagatgc agcaaggccc ctgggacaac gccagatctc tgctctcccc 3840
   gaccctcaga agccagccca cgcctggccc accaccactg cctaacgtcc aagtgtccat 3900
   aggctcggga cctccaagtc caggttctgc ctctgggatt ccgccatggg tctgcctgga 3960
   atgatgcact tggaggagct cagcatggga tgcggaactt gtctagcgct cctcagatcc 4020
   acagctgcct gtgagacaca cacacacaca cacacacacc aaacacgcat gcacgcacgc 4080
   cggcacacac gctattacag agatggccac ggtagctgtg cctcgaggcc gagtggagtg 4140
   tctagaactc tcgggggtcc cctctgcaga cgacactgct ccatcccccc cgtgccctga 4200
   agggctcctc actctcccat caggatctct ccaagctgct gacctggaga ggaaggggcc 4260
   tgggacaggc ggggacactc agacctccct gctgcccctc ctctgcctgg gcttggacgg 4320
   ctcccccctt cccacgggtg aaggtgcagg tggggagagg gcacccccct caccctccca 4380
   gacccagacc agcccccgtg gcaggggcag cctgtgagcc tccagccaga tgcaggtggc 4440
   ctggggtggg gggtggaggg ggcgggaggt ttatgtttga ggctgtattc atctgtgtaa 4500
   tattttcggc ggtgggaccc atctgaccgt cctcggtgag tctccccttt tctttcctcc 4560
   ttggggatcc gagtgaaatc tgggtcgatc ttctctccgt tctcctccga ctggggctga 4620
   ggtctgaacc tcggtggggt ccgaagagga ggcccctagg ccggctcctc agcccctcca 4680
   gcccgacccg ccctcttgac acagggtcca gctaagggca gacatggctg ctagtccagg 4740
   gccaggctct gagacccaag ggcgctgccc aaggaaccct tgccccaggg accctgggag 4800
   caaagctcct cactcagagc ctgcagccct ggggtctgag gacaaggagg gactgaggac 4860
   tgggcgtggg gagttcaggc ggggacaccg ggtccaggga ggtgacaaag gcgctgggag 4920
   ggggcggacg gtgccggaga ctcctcctgg gccctgtggg ctcgtggtcc ttgtgaggac 4980
   cctgagggct gaggggcccc tgggcctagg gacttgcagt gagggaggca gggagtgtcc 5040
   cttgagaacg tggcctccgc gggctgggtc cccctcgtgc tcccagcagg gaggacaccc 5100
   cagagcaagc gccccaggtg ggcggggagg gtctcctcac aggggcagct gacagataga 5160
   cggcccccgc cagacagatg cttgatcctg gtcagtactg ggttcgccac ttccctgaac 5220
   aggggccctc cgaacagaca cagacgcaga ccaggctcag cacagaaaat gcactgacac 5280
   ccaaaaccct catctggggg cctggccggc atcccgcccc aggacccaag gcccctgccc 5340
   cctggcagcc ctggacacgg tcctctgtgg gcggtggggt cggggctgtg gtgacggtgg 5400
   catcggggag cctgtgcccc ctccctgaaa gggcggagag gctcaagagg ggacagaaat 5460
   gtcctcccct aggaagacct cggacggggg cgggggggtg gtctccgaca gacagatgcc 5520
   cgggaccgac agacctgccg agggaagagg gcacctcggt cgggttaggc tccaggcagc 5580
   acgagggagc gaggctggga gggtgaggac atgggagcct gaggaggagc tggagacttc 5640
   agcaggcccc cagctccggg cttcgggctc tgagatgctc ggacgcaagg tgagtgaccc 5700
   cacctgtggc tgacctgacc tgacctcagg gggacaaggc tcagcctggg actctgtgtc 5760
   cccatcgcct gcacagggga ttcccctgat ggacactgag ccaacgacct cccgtctctc 5820
   cccgaccccc aggtcagccc aaggccactc ccacggtcaa cctcttcccg ccctcctctg 5880
   aggagctcgg caccaacaag gccaccctgg tgtgtcta 5918
<210> 29
   <211> 11051
   <212> DNA
   <213> Sus scrofa
<400> 29
   tctagaagac gctggagaga ggccagactt cctcggaaca gctcaaagag ctctgtcaaa 60
   gccagatccc atcacacgtg ggcaccaata ggccatgcca gcctccaagg gccgaactgg 120
   gttctccacg gcgcacatga agcctgcagc ctggcttatc ctcttccgtg gtgaagaggc 180
   aggcccggga ctggacgagg ggctagcagg gtgtggtagg caccttgcgc cccccacccc 240
   ggcaggaacc agagaccctg gggctgagag tgagcctcca aacaggatgc cccacccttc 300
   aggccacctt tcaatccagc tacactccac ctgccattct cctctgggca cagggcccag 360
   cccctggatc ttggccttgg ctcgacttgc acccacgcgc acacacacac ttcctaacgt 420
   gctgtccgct cacccctccc cagcgtggtc catgggcagc acggcagtgc gcgtccggcg 480
   gtagtgagtg cagaggtccc ttcccctccc ccaggagccc caggggtgtg tgcagatctg 540
   ggggctcctg tcccttacac cttcatgccc ctcccctcat acccaccctc caggcgggag 600
   gcagcgagac ctttgcccag ggactcagcc aacgggcaca cgggaggcca gccctcagca 660
   gctggctccc aaagaggagg tgggaggtag gtccacagct gccacagaga gaaaccctga 720
   cggaccccac aggggccacg ccagccggaa ccagctccct cgtgggtgag caatggccag 780
   ggccccgccg gccaccacgg ctggccttgc gccagctgag aactcacgtc cagtgcaggg 840
   agactcaaga cagcctgtgc acacagcctc ggatctgctc ccatttcaag cagaaaaagg 900
   aaaccgtgca ggcagccctc agcatttcaa ggattgtagc agcggccaac tattcgtcgg 960
   cagtggccga ttagaatgac cgtggagaag ggcggaaggg tctctcgtgg gctctgcggc 1020
   caacaggccc tggctccacc tgcccgctgc cagcccgagg ggcttgggcc gagccaggaa 1080
   ccacagtgct caccgggacc acagtgactg accaaactcc cggccagagc agccccaggc 1140
   cagccgggct ctcgccctgg aggactcacc atcagatgca caagggggcg agtgtggaag 1200
   agacgtgtcg cccgggccat ttgggaaggc gaagggacct tccaggtgga caggaggtgg 1260
   gacgcactcc aggcaaggga ctgggtcccc aaggcctggg gaaggggtac tggcttgggg 1320
   gttagcctgg ccagggaacg gggagcgggg cggggggctg agcagggagg acctgacctc 1380
   gtgggagcga ggcaagtcag gcttcaggca gcagccgcac atcccagacc aggaggctga 1440
   ggcaggaggg gcttgcagcg gggcgggggc ctgcctggct ccgggggctc ctgggggacg 1500
   ctggctcttg tttccgtgtc ccgcagcaca gggccagctc gctgggccta tgcttacctt 1560
   gatgtctggg gccggggcgt cagggtcgtc gtctcctcag gggagagtcc cctgaggcta 1620
   cgctgggggg ggactatggc agctccacca ggggcctggg gaccaggggc ctggaccagg 1680
   ctgcagcccg gaggacgggc agggctctgg ctctccagca tctggccctc ggaaatggca 1740
   gaacccctgg cgggtgagcg agctgagagc gggtcagaca gacaggggcc ggccggaaag 1800
   gagaagttgg gggcagagcc cgccaggggc caggcccaag gttctgtgtg ccagggcctg 1860
   ggtgggcaca ttggtgtggc catggctact tagattcgtg gggccagggc atcctggtca 1920
   ccgtctcctc aggtgagcct ggtgtctgat gtccagctag gcgctggtgg gccgcgggtg 1980
   ggcctgtctc aggctagggc aggggctggg atgtgtattt gtcaaggagg ggcaacaggg 2040
   tgcagactgt gcccctggaa acttgaccac tggggcaggg gcgtcctggt cacgtctcct 2100
   caggtaagac ggccctgtgc ccctctctcg cgggactgga aaaggaattt tccaagattc 2160
   cttggtctgt gtggggccct ctggggcccc cgggggtggc tcccctcctg cccagatggg 2220
   gcctcggcct gtggagcacg ggctgggcac acagctcgag tctagggcca cagaggcccg 2280
   ggctcagggc tctgtgtggc ccggcgactg gcagggggct cgggtttttg gacaccccct 2340
   aatgggggcc acagcactgt gaccatcttc acagctgggg ccgaggagtc gaggtcaccg 2400
   tctcctcagg tgagtcctcg tcagccctct ctcactctct ggggggtttt gctgcatttt 2460
   gtgggggaaa gaggatgcct gggtctcagg tctaaaggtc tagggccagc gccggggccc 2520
   aggaaggggc cgaggggcca ggctcggctc ggccaggagc agagcttcca gacatctcgc 2580
   ctcctggcgg ctgcagtcag gcctttggcc gggggggtct cagcaccacc aggcctcttg 2640
   gctcccgagg tccccggccc cggctgcctc accaggcacc gtgcgcggtg ggcccgggct 2700
   cttggtcggc caccctttct taactgggat ccgggcttag ttgtcgcaat gtgacaacgg 2760
   gctcgaaagc tggggccagg ggaccctagt ctacgacgcc tcgggtgggt gtcccgcacc 2820
   cctccccact ttcacggcac tcggcgagac ctggggagtc aggtgttggg gacactttgg 2880
   aggtcaggaa cgggagctgg ggagagggct ctgtcagcgg ggtccagaga tgggccgccc 2940
   tccaaggacg ccctgcgcgg ggacaagggc ttcttggcct ggcctggccg cttcacttgg 3000
   gcgtcagggg gggcttcccg gggcaggcgg tcagtcgagg cgggttggaa ttctgagtct 3060
   gggttcgggg ttcggggttc ggccttcatg aacagacagc ccaggcgggc cgttgtttgg 3120
   cccctggggg cctggttgga atgcgaggtc tcgggaagtc aggagggagc ctggccagca 3180
   gagggttccc agccctgcgg ccgagggacc tggagacggg cagggcattg gccgtcgcag 3240
   ggccaggcca caccccccag gtttttgtgg ggcgagcctg gagattgcac cactgtgatt 3300
   actatgctat ggatctctgg ggcccaggcg ttgaagtcgt cgtgtcctca ggtaagaacg 3360
   gccctccagg gcctttaatt tctgctctcg tctgtgggct tttctgactc tgatcctcgg 3420
   gaggcgtctg tgcccccccc ggggatgagg ccggcttgcc aggaggggtc agggaccagg 3480
   agcctgtggg aagttctgac gggggctgca ggcgggaagg gccccaccgg ggggcgagcc 3540
   ccaggccgct gggcggcagg agacccgtga gagtgcgcct tgaggagggt gtctgcggaa 3600
   ccacgaacgc ccgccgggaa gggcttgctg caatgcggtc ttcagacggg aggcgtcttc 3660
   tgccctcacc gtctttcaag cccttgtggg tctgaaagag ccatgtcgga gagagaaggg 3720
   acaggcctgt cccgacctgg ccgagagcgg gcagccccgg gggagagcgg ggcgatcggc 3780
   ctgggctctg tgaggccagg tccaagggag gacgtgtggt cctcgtgaca ggtgcacttg 3840
   cgaaacctta gaagacgggg tatgttggaa gcggctcctg atgtttaaga aaagggagac 3900
   tgtaaagtga gcagagtcct caagtgtgtt aaggttttaa aggtcaaagt gttttaaacc 3960
   tttgtgactg cagttagcaa gcgtgcgggg agtgaatggg gtgccagggt ggccgagagg 4020
   cagtacgagg gccgtgccgt cctctaattc agggcttagt tttgcagaat aaagtcggcc 4080
   tgttttctaa aagcattggt ggtgctgagc tggtggagga ggccgcgggc agccctggcc 4140
   acctgcagca ggtggcagga agcaggtcgg ccaagaggct attttaggaa gccagaaaac 4200
   acggtcgatg aatttatagc ttctggtttc caggaggtgg ttgggcatgg ctttgcgcag 4260
   cgccacagaa ccgaaagtgc ccactgagaa aaaacaactc ctgcttaatt tgcatttttc 4320
   taaaagaaga aacagaggct gacggaaact ggaaagttcc tgttttaact actcgaattg 4380
   agttttcggt cttagcttat caactgctca cttagattca ttttcaaagt aaacgtttaa 4440
   gagccgaggc attcctatcc tcttctaagg cgttattcct ggaggctcat tcaccgccag 4500
   cacctccgct gcctgcaggc attgctgtca ccgtcaccgt gacggcgcgc acgattttca 4560
   gttggcccgc ttcccctcgt gattaggaca gacgcgggca ctctggccca gccgtcttgg 4620
   ctcagtatct gcaggcgtcc gtctcgggac ggagctcagg ggaagagcgt gactccagtt 4680
   gaacgtgata gtcggtgcgt tgagaggaga cccagtcggg tgtcgagtca gaaggggccc 4740
   ggggcccgag gccctgggca ggacggcccg tgccctgcat cacgggccca gcgtcctaga 4800
   ggcaggactc tggtggagag tgtgagggtg cctggggccc ctccggagct ggggccgtgc 4860
   ggtgcaggtt gggctctcgg cgcggtgttg gctgtttctg cgggatttgg aggaattctt 4920
   ccagtgatgg gagtcgccag tgaccgggca ccaggctggt aagagggagg ccgccgtcgt 4980
   ggccagagca gctgggaggg ttcggtaaaa ggctcgcccg tttcctttaa tgaggacttt 5040
   tcctggaggg catttagtct agtcgggacc gttttcgact cgggaagagg gatgcggagg 5100
   agggcatgtg cccaggagcc gaaggcgccg cggggagaag cccagggctc tcctgtcccc 5160
   acagaggcga cgccactgcc gcagacagac agggcctttc cctctgatga cggcaaaggc 5220
   gcctcggctc ttgcggggtg ctggggggga gtcgccccga agccgctcac ccagaggcct 5280
   gaggggtgag actgaccgat gcctcttggc cgggcctggg gccggaccga gggggactcc 5340
   gtggaggcag ggcgatggtg gctgcgggag ggaaccgacc ctgggccgag cccggcttgg 5400
   cgattcccgg gcgagggccc tcagccgagg cgagtgggtc cggcggaacc accctttctg 5460
   gccagcgcca cagggctctc gggactgtcc ggggcgacgc tgggctgccc gtggcaggcc 5520
   tgggctgacc tggacttcac cagacagaac agggctttca gggctgagct gagccaggtt 5580
   tagcgaggcc aagtggggct gaaccaggct caactggcct gagctgggtt gagctgggct 5640
   gacctgggct gagctgagct gggctgggct gggctgggct gggctgggct gggctggact 5700
   ggctgagctg agctgggttg agctgagctg agctggcctg ggttgagctg ggctgggttg 5760
   agctgagctg ggttgagctg ggttgagctg ggttgatctg agctgagctg ggctgagctg 5820
   agctaggctg gggtgagctg ggctgagctg gtttgagttg ggttgagctg agctgagctg 5880
   ggctgtgctg gctgagctag gctgagctag gctaggttga gctgggctgg gctgagctga 5940
   gctaggctgg gctgatttgg gctgagctga gctgagctag gctgcgttga gctggctggg 6000
   ctggattgag ctggctgagc tggctgagct gggctgagct ggcctgggtt gagctgagct 6060
   ggactggttt gagctgggtc gatctgggtt gagctgtcct gggttgagct gggctgggtt 6120
   gagctgagct gggttgagct gggctcagca gagctgggtt gggctgagct gggttgagct 6180
   gagctgggct gagctggcct gggttgagct gggctgagct gagctgggct gagctggcct 6240
   gtgttgagct gggctgggtt gagctgggct gagctggatt gagctgggtt gagctgagct 6300
   gggctgggct gtgctgactg agctgggctg agctaggctg gggtgagctg ggctgagctg 6360
   atccgagcta ggctgggctg gtttgggctg agctgagctg agctaggctg gattgatctg 6420
   gctgagctgg gttgagctga gctgggctga gctggtctga gctggcctgg gtcgagctga 6480
   gctggactgg tttgagctgg gtcgatctgg gctgagctgg cctgggttga gctgggctgg 6540
   gttgagctga gctgggttga gctgggctga gctgagggct ggggtgagct gggctgaact 6600
   agcctagcta ggttgggctg agctgggctg gtttgggctg agctgagctg agctaggctg 6660
   cattgagcag gctgagctgg gctgagcagg cctggggtga gctgggctag gtggagctga 6720
   gctgggtcga gctgagttgg gctgagctgg cctgggttga ggtaggctga gctgagctga 6780
   gctaggctgg gttgagctgg ctgggctggt ttgcgctggg tcaagctggg ccgagctggc 6840
   ctgggttgag ctgggctcgg ttgagctggg ctgagctgag ccgacctagg ctgggatgag 6900
   ctgggctgat ttgggctgag ctgagctgag ctaggctgca ttgagcaggc tgagctgggc 6960
   ctggagcctg gcctggggtg agctgggctg agctgcgctg agctaggctg ggttgagctg 7020
   gctgggctgg tttgcgctgg gtcaagctgg gccgagctgg cctgggatga gctgggccgg 7080
   tttgggctga gctgagctga gctaggctgc attgagcagg ctgagctggg ctgagctggc 7140
   ctggggtgag ctgggctgag ctaagctgag ctgggctggt ttgggctgag ctggctgagc 7200
   tgggtcctgc tgagctgggc tgagctgacc aggggtgagc tgggctgagt taggctgggc 7260
   tcagctaggc tgggttgatc tggcagggct ggtttgcgct gggtcaagct cccgggagat 7320
   ggcctgggat gagctgggct ggtttgggct gagctgagct gagctgagct aggctgcatt 7380
   gagcaggctg agctgggctg agctggcctg gggtgagctg ggctgggtgg agctgagctg 7440
   ggctgaactg ggctaagctg gctgagctgg atcgagctga gctgggctga gctggcctgg 7500
   ggttagctgg gctgagctga gctgagctag gctgggttga gctggctggg ctggtttgcg 7560
   ctgggtcaag ctgggccgag ctggcctggg ttgagctggg ctgggctgag ctgagctagg 7620
   ctgggttgag ctgggctggg ctgagctgag ctaggctgca ttgagctggc tgggatggat 7680
   tgagctggct gagctggctg agctggctga gctgggctga gctggcctgg gttgagctgg 7740
   gctgggttga gctgagctgg gctgagctgg gctcagcaga gctgggttga gctgagctgg 7800
   gttgagctgg ggtgagctgg gctgagcaga gctgggttga gctgagctgg gttgagctgg 7860
   gctcgagcag agctgggttg agctgagctg ggttgagctg ggctcagcag agctgggttg 7920
   agctgagctg ggttgagctg ggctgagcta gctgggctca gctaggctgg gttgagctga 7980
   gctgggctga actgggctga gctgggctga actgggctga gctgggctga gctgggctga 8040
   gcagagctgg gctgagcaga gctgggttgg tctgagctgg gttgagctgg gctgagctgg 8100
   gctgagcaga gttgggttga gctgagctgg gttcagctgg gctgagctag gctgggttga 8160
   gctgggttga gttgggctga gctgggctgg gttgagcgga gctgggctga actgggctga 8220
   gctgggctga gcggaactgg gttgatctga attgagctgg gctgagccgg gctgagccgg 8280
   gctgagctgg gctaggttga gcttgggtga gcttgcctca gctggtctga gctaggttgg 8340
   gtggagctag gctggattga gctgggctga gctgagctga tctggcctca gctgggctga 8400
   ggtaggctga actgggctgt gctgggctga gctgagctga gccagtttga gctgggttga 8460
   gctgggctga gctgggctgt gttgatcttt cctgaactgg gctgagctgg gctgagctgg 8520
   cctagctgga ttgaacgggg gtaagctggg ccaggctgga ctgggctgag ctgagctagg 8580
   ctgagctgag ttgaattggg ttaagctggg ctgagatggg ctgagctggg ctgagctggg 8640
   ttgagccagg tcggactggg ttaccctggg ccacactggg ctgagctggg cggagctcga 8700
   ttaacctggt caggctgagt cgggtccagc agacatgcgc tggccaggct ggcttgacct 8760
   ggacacgttc gatgagctgc cttgggatgg ttcacctcag ctgagccagg tggctccagc 8820
   tgggctgagc tggtgaccct gggtgacctc ggtgaccagg ttgtcctgag tccgggccaa 8880
   gccgaggctg catcagactc gccagaccca aggcctgggc cccggctggc aagccagggg 8940
   cggtgaaggc tgggctggca ggactgtccc ggaaggaggt gcacgtggag ccgcccggac 9000
   cccgaccggc aggacctgga aagacgcctc tcactcccct ttctcttctg tcccctctcg 9060
   ggtcctcaga gagccagtct gccccgaatc tctaccccct cgtctcctgc gtcagccccc 9120
   cgtccgatga gagcctggtg gccctgggct gcctggcccg ggacttcctg cccagctccg 9180
   tcaccttctc ctggaactac aagaacagca gcaaggtcag cagccagaac atccaggact 9240
   tcccgtccgt cctgagaggc ggcaagtact tggcctcctc ccgggtgctc ctaccctctg 9300
   tgagcatccc ccaggaccca gaggccttcc tggtgtgcga ggtccagcac cccagtggca 9360
   ccaagtccgt gtccatctct gggccaggtg agctgggctc cccctgtggc tgtggcgggg 9420
   gcggggccgg gtgccgccgg cacagtgacg ccccgttcct gcctgcagtc gtagaggagc 9480
   agccccccgt cttgaacatc ttcgtcccca cccgggagtc cttctccagt actccccagc 9540
   gcacgtccaa gctcatctgc caggcctcag acttcagccc caagcagatc tccatggcct 9600
   ggttccgtga tgggaaacgg gtggtgtctg gcgtcagcac aggccccgtg gagaccctac 9660
   agtccagtcc ggtgacctac aggctccaca gcatgctgac cgtcacggag tccgagtggc 9720
   tcagccagag cgtcttcacc tgccaggtgg agcacaaagg gctgaactac gagaagaacg 9780
   cgtcctctct gtgcacctcc agtgagtgca gcccctcggg ccgggcggcg gggcggcggg 9840
   agccacacac acaccagctg ctccctgagc cttggcttcc gggagtggcc aaggcgggga 9900
   ggggctgtgc agggcagctg gagggcactg tcagctgggg cccagcaccc cctcaccccg 9960
   gcagggcccg ggctccgagg ggccccgcag tcgcaggccc tgctcttggg ggaagcccta 10020
   cttggcccct tcagggcgct gacgctcccc ccacccaccc ccgcctagat cccaactctc 10080
   ccatcaccgt cttcgccatc gccccctcct tcgctggcat cttcctcacc aagtcggcca 10140
   agctttcctg cctggtcacg ggcctcgtca ccagggagag cctcaacatc tcctggaccc 10200
   gccaggacgg cgaggttctg aagaccagta tcgtcttctc tgagatctac gccaacggca 10260
   ccttcggcgc caggggcgaa gcctccgtct gcgtggagga ctgggagtcg ggcgacaggt 10320
   tcacgtgcac ggtgacccac acggacctgc cctcgccgct gaagcagagc gtctccaagc 10380
   ccagaggtag gccctgccct gcccctgcct ccgcccggcc tgtgccttgg ccgccggggc 10440
   gggagccgag cctggccgag gagcgccctc ggccccccgc ggtcccgacc cacacccctc 10500
   ctgctctcct ccccagggat cgccaggcac atgccgtccg tgtacgtgct gccgccggcc 10560
   ccggaggagc tgagcctgca ggagtgggcc tcggtcacct gcctggtgaa gggcttctcc 10620
   ccggcggacg tgttcgtgca gtggctgcag aagggggagc ccgtgtccgc cgacaagtac 10680
   gtgaccagcg cgccggtgcc cgagcccgag cccaaggccc ccgcctccta cttcgtgcag 10740
   agcgtcctga cggtgagcgc caaggactgg agcgacgggg agacctacac ctgcgtcgtg 10800
   ggccacgagg ccctgcccca cacggtgacc gagaggaccg tggacaagtc caccggtaaa 10860
   cccaccctgt acaacgtctc cctggtcctg tccgacacgg ccagcacctg ctactgaccc 10920
   cctggctgcc cgccgcggcc ggggccagag cccccgggcg accatcgctc tgtgtgggcc 10980
   tgtgtgcaac ccgaccctgt cggggtgagc ggtcgcattt ctgaaaatta gaaataaaag 11040
   atctcgtgcc g 11051
<210> 30
   <211> 12048
   <212> DNA
   <213> Sus scrofa
<400> 30
   gcgtccgaag tcaaaaatat ctgcagcctt catgtattca tagaaacaag gaatgtctac 60
   attttccaaa gtgggaccag aatcttgggt catgtctaag gcatgtgcat ttgcacatgg 120
   taggcaaagg actttgcttc tcccagcaca tctttctgca gagatccatg gaaacaagac 180
   tcaactccaa agcagcaaag aagcagcaag ttctcaagtg atctcctctg actccctcct 240
   cccaggctaa tgaagccatg ttgcccctgg gggattaagg gcaggtgtcc attgtggcac 300
   ccagcccgaa gacaagcaat ttgatcaggt tctgagcact cctgaatgtg gactctggaa 360
   ttttctcctc accttgtggc atatcagctt aagtcaagta caagtgacaa acaacataat 420
   cctaagaaga gaggaatcaa gctgaagtca aaggatcact gccttggatt ctactgtgaa 480
   tgatgacctg gaaaatatcc tgaacaacag cttcagggtg atcatcagag acaaaagttc 540
   cagagccagg tagggaaacc ctcaagcctt gcaaagagca aaatcatgcc attgggttct 600
   taacctgctg agtgatttac tatatgttac tgtgggaggc aaagcgctca aatagcctgg 660
   gtaagtatgt caaataaaaa gcaaaagtgg tgtttcttga aatgttagac ctgaggaagg 720
   aatattgata acttaccaat aattttcaga atgatttata gatgtgcact tagtcagtgt 780
   ctctccaccc cgcacctgac aagcagttta gaatttattc taagaatcta ggtttgctgg 840
   gggctacatg ggaatcagct tcagtgaaga gtttgttgga atgattcact aaattttcta 900
   tttccagcat aaatccaaga acctctcaga ctagtttatt gacactgctt ttcctccata 960
   atccatctca tctccgtcca tcatggacac tttgtagaat gacaggtcct ggcagagact 1020
   cacagatgct tctgaaacat cctttgcctt caaagaatga acagcacaca tactaaggat 1080
   ctcagtgatc cacaaattag tttttgccac aatggttctt atgataaaag tctttcatta 1140
   acagcaaatt gttttataat agttgttctg ctttataata attgcatgct tcactttctt 1200
   ttcttttctt tttttttctt tttttgcttt ttagtgccgc aggtgcagca tatgaaattt 1260
   cccaggctag gggtcaaatc agaactacac ctactggcct acgccacagc cacagcaact 1320
   caggatctaa gccatgtcgg tgacctacac tacagctcat ggcaatgcca gatccttaac 1380
   ccaatgagcg aggccaggga tcgaacccat gtcctcatgg atactagtca ggctcattat 1440
   ccgctgagcc ataacaggaa ctcccgagtt tgctttttat caaaattggt acagccttat 1500
   tgtttctgaa aaccacaaaa tgaatgtatt cacataattt taaaaggtta aataatttat 1560
   gatatacaag acaatagaaa gagaaaacgt cattgcctct ttcttccacg acaacacgcc 1620
   tccttaattg atttgaagaa ataactactg agcatggttt agtgtacttc tttcagcaat 1680
   tagcctgtat tcatagccat acatattcaa ttaaaatgag atcatgatat cacacaatac 1740
   ataccataca gcctataggg atttttacaa tcatcttcca catgactaca taaaaaccta 1800
   cctaaaaaaa aaaaaaaccc tacttcatcc tcctattggc tgctttgtgc tccattaaaa 1860
   agctctatca taattaggtt atgatgagga tttccatttt ctacctttca agcaacattt 1920
   caatgcacag tcttatatac acatttgagc ctacttttct ttttctttct ttttttggtt 1980
   tttttttttt tttttttttt ggtctttttg tcttttctaa ggctgcatat ggaggttccc 2040
   aggctagctg tctaatcaga actatagctg ctggcctacg ccacatccac agcaatacaa 2100
   gatctgagcc atgtctgcaa cttacaccac agctcacagc aacggtggat ccttaaacca 2160
   ctgagcaagg ccagggatca aacccataac ttcatggctc ctagttggat ttgttaacca 2220
   ctgagccatg atggcaactc ctgagcctac ttttctaatc atttccaacc ctaggacact 2280
   tttttaagtt tcatttttct ccccccaccc cctgttttct gaagtgtgtt tgcttccact 2340
   gggtgacttc actcccagga tctcatctgc aggatactgc agctaagtgt atgagctctg 2400
   aatttgaatc ccaactctgc cactcaaagg gataggagtt tccgatgtgg cccaatggga 2460
   tcagtggcat ctctgcagtg ccaggacgca ggttccatcc ctggcccagc acagtgggtt 2520
   aagaatctgg cattgctgca gctgaggcat agatttcaat tgtgcctcag atctgatcct 2580
   tggcccaagg actgcatatg cctcagggca accaaaaaag agaaaagggg ggtgatagca 2640
   ttagtttcta gatttggggg ataattaaat aaagtgatcc atgtacaatg tatggcattt 2700
   tgtaaatgct caacaaattt caactattat ggagttccca tcatggctca gtggaaggga 2760
   atctgattag catccatgag gacacaggtc caaccccgac cttgctcagt gggcattgct 2820
   gtgagctgtg gcatgggtta cagacgaagc tcggatctgg cattgctgtg gctgtggtgt 2880
   aagccagcaa ctacagctct cattcagccc ctagcctggg aacctccata tgcctaaaag 2940
   acaaaaaata aaatttaaat taaaaataaa gaaatgttaa ctattatgat tggtactgct 3000
   tgcattactg caaagaaagt cactttctat actctttaat atcttagttg actgtgtgct 3060
   cagtgaacta ttttggacac ttaatttcca ctctcttcta tctccaactt gacaactctc 3120
   tttcctctct tctggtgaga tccactgctg actttgctct ttaaggcaac tagaaaagtg 3180
   ctcagtgaca aaatcaaaga aagttacctt aatcttcaga attacaatct taagttctct 3240
   tgtaaagctt actatttcag tggttagtat tattccttgg tcccttacaa cttatcagct 3300
   ctgatctatt gctgattttc aactatttat tgttggagtt ttttcctttt ttccctgttc 3360
   attctgcaaa tgtttgctga gcatttgtca agtgaagata ctggactggg ccttccaaat 3420
   ataagacaat gaaacatcgg gagttctcat tatggtgcag cagaaacgaa tccaactagg 3480
   aaatgtgagg ttgcaggttc gatccctgcc cttgctcagt gggttaagga tccagcatta 3540
   ccgtgagctg tggtgtaggt tgcagacgtg gctcagatcc tgcgttgctg tggctgtggc 3600
   ataggctggc agctctagct ctgattcgac cgctagcctg ggaacctcca tgcgccccga 3660
   gtgcagccct taaaaagcaa aaaaaaaaga aagaaagaaa aagacaatga aacatcaaac 3720
   agctaacaat ccagtagggt agaaagaatc tggcaacaga taagagcgat taaatgttct 3780
   aggtccagtg accttgcctc tgtgctctac acagtcgtgc cacttgctga gggagaaggt 3840
   ctctcttgag ttgagtcctg aaagacatta gttgttcaca aactaatgcc agtgagtgaa 3900
   ggtgtttcca agcagaggga gagtttggta aaaagctgga agtcacagaa agactctaaa 3960
   gagtttagga tggtgggagc aacatacgct gagatggggc tggaaggtta agagggaaac 4020
   aactatagta agtgaagctg gactcacagc aaagtgagga cctcagcatc cttgatgggg 4080
   ttaccatgga aacaccaagg cacaccttga tttccaaaac agcaggcacc tgattcagcc 4140
   caatgtgaca tggtgggtac ccctctagct ctacctgttc tgtgacaact gacaaccaac 4200
   gaagttaagt ctggattttc tactctgctg atccttgttt ttgtttcaca cgtcatctat 4260
   agcttcatgc caaaatagag ttcaaggtaa gacgcgggcc ttggtttgat atacatgtag 4320
   tctatcttgt ttgagacaat atggtggcaa ggaagaggtt caaacaggaa aatactctct 4380
   aattatgatt aactgagaaa agctaaagag tcccataatg acactgaatg aagttcatca 4440
   tttgcaaaag ccttcccccc cccccaggag actataaaaa agtgcaattt tttaaatgaa 4500
   cttatttaca aaacagaaat agactcacag acataggaaa cgaacagatg gttaccaagg 4560
   gtgaaaggga gtaggaggga taaataagga gtctggggtt agcagataca ccccagtgta 4620
   cacaaaataa acaacaggga cctactatat agcacaggga actatatgca gtagcttaca 4680
   ataacctata atggaaaaga atgtgaaaaa gaatatatgt atgcgtgtgt gtgtaactga 4740
   atcactttgc tgtaacctga atctaacata acattgtaaa tcaactacag tttttttttt 4800
   ttttaagtgc agggttttgg tgtttttttt ttttcatttt tgtttttgtt tttgtttttt 4860
   gctttttagg gccacaccca gacatatggg ggttcccagg ctaggggtct aattagagct 4920
   acagttgccg gcttgcacca cagccacagc aacatcagat ccgagccgca cttgcgactt 4980
   acaccacagc tcatggcaat accagatcct taacccactg agcaaggccc agggatcgta 5040
   cccgcaacct catggttcct agtcagattc atttctgctg cgctacaatg ggaactccaa 5100
   gtgcagtttt ttgtaatgtg cttgtctttc tttgtaattc atattcatcc tacttcccaa 5160
   taaataaata aatacataaa taataaacat accattgtaa atcaactaca atttttttta 5220
   aatgcagggt ttttgttttt tgttttttgt tttgtctttt tgccttttct agggccgctc 5280
   ccatggcata tggaggttcc caggctaggg gtcgaatcgg agctgtagcc accggcctac 5340
   gccagagcca cagcaacgcg ggatccgagc cgcgtctgca acctacacca cagctcacgg 5400
   caacgccgga tcgttaaccc actgagcaag ggcagggatc gaacctgcaa cctcatggtt 5460
   cctagtcaga ttcgttaact actgagccac aacggaaact cctaaagtgc agtttttaaa 5520
   tgtgcttgtc tttctttgta atttacactc aacctacttc ccaataaata aataaataaa 5580
   caaataaatc atagacatgg ttgaattcta aaggaaggga ccatcaggcc ttagacagaa 5640
   atacgtcatc ttctagtatt ttaaaacaca ctaaagaaga caaacatgct ctgccagaga 5700
   agcccagggc ctccacagct gcttgcaaag ggagttaggc ttcagtagct gacccaaggc 5760
   tctgttcctc ttcagggaaa agggtttttg ttcagtgaga cagcagacag ctgtcactgt 5820
   ggtggacgtt cggccaagga accaagctgg aactcaaacg taagtcaatc caaacgttcc 5880
   ttccttggct gtctgtgtct tacggtctct gtggctctga aatgattcat gtgctgactc 5940
   tctgaaacca gactgacatt ctccagggca aaactaaagc ctgtcatcaa actggaaaac 6000
   tgagggcaca ttttctgggc agaactaaga gtcaggcact gggtgaggaa aaacttgtta 6060
   gaatgatagt ttcagaaact tactgggaag caaagcccat gttctgaaca gagctctgct 6120
   caagggtcag gaggggaacc agtttttgta caggagggaa gttgagacga acccctgtgt 6180
   atatggtttc ggcgcgggga ccaagctgga gctcaaacgt aagtggcttt ttccgactga 6240
   ttctttgctg tttctaattg ttggttggct ttttgtccat ttttcagtgt tttcatcgaa 6300
   ttagttgtca gggaccaaac aaattgcctt cccagattag gtaccaggga ggggacattg 6360
   ctgcatggga gaccagaggg tggctaattt ttaacgtttc caagccaaaa taactgggga 6420
   agggggcttg ctgtcctgtg agggtaggtt tttatagaag tggaagttaa ggggaaatcg 6480
   ctatggttca cttttggctc ggggaccaaa gtggagccca aaattgagta cattttccat 6540
   caattatttg tgagattttt gtcctgttgt gtcatttgtg caagtttttg acattttggt 6600
   tgaatgagcc attcccaggg acccaaaagg atgagaccga aaagtagaaa agagccaact 6660
   tttaagctga gcagacagac cgaattgttg agtttgtgag gagagtaggg tttgtaggga 6720
   gaaaggggaa cagatcgctg gctttttctc tgaattagcc tttctcatgg gactggcttc 6780
   agagggggtt tttgatgagg gaagtgttct agagccttaa ctgtgggttg tgttcggtag 6840
   cgggaccaag ctggaaatca aacgtaagtg cacttttcta ctcctttttc tttcttatac 6900
   gggtgtgaaa ttggggactt ttcatgtttg gagtatgagt tgaggtcagt tctgaagaga 6960
   gtgggactca tccaaaaatc tgaggagtaa gggtcagaac agagttgtct catggaagaa 7020
   caaagaccta gttagttgat gaggcagcta aatgagtcag ttgacttggg atccaaatgg 7080
   ccagacttcg tctgtaacca acaatctaat gagatgtagc agcaaaaaga gatttccatt 7140
   gaggggaaag taaaattgtt aatattgtgg atcacctttg gtgaagggac atccgtggag 7200
   attgaacgta agtatttttt ctctactacc ttctgaaatt tgtctaaatg ccagtgttga 7260
   cttttagagg cttaagtgtc agttttgtga aaaatgggta aacaagagca tttcatattt 7320
   attatcagtt tcaaaagtta aactcagctc caaaaatgaa tttgtagaca aaaagattaa 7380
   tttaagccaa attgaatgat tcaaaggaaa aaaaaattag tgtagatgaa aaaggaattc 7440
   ttacagctcc aaagagcaaa agcgaattaa ttttctttga actttgccaa atcttgtaaa 7500
   tgatttttgt tctttacaat ttaaaaaggt tagagaaatg tatttcttag tctgttttct 7560
   ctcttctgtc tgataaatta ttatatgaga taaaaatgaa aattaatagg atgtgctaaa 7620
   aaatcagtaa gaagttagaa aaatatatgt ttatgttaaa gttgccactt aattgagaat 7680
   cagaagcaat gttattttta aagtctaaaa tgagagataa actgtcaata cttaaattct 7740
   gcagagattc tatatcttga cagatatctc ctttttcaaa aatccaattt ctatggtaga 7800
   ctaaatttga aatgatcttc ctcataatgg agggaaaaga tggactgacc ccaaaagctc 7860
   agatttaaag aaatctgttt aagtgaaaga aaataaaaga actgcatttt ttaaaggccc 7920
   atgaatttgt agaaaaatag gaaatatttt aataagtgta ttcttttatt ttcctgttat 7980
   tacttgatgg tgtttttata ccgccaagga ggccgtggca ccgtcagtgt gatctgtaga 8040
   ccccatggcg gccttttttc gcgattgaat gaccttggcg gtgggtcccc agggctctgg 8100
   tggcagcgca ccagccgcta aaagccgcta aaaactgccg ctaaaggcca cagcaacccc 8160
   gcgaccgccc gttcaactgt gctgacacag tgatacagat aatgtcgcta acagaggaga 8220
   atagaaatat gacgggcaca cgctaatgtg gggaaaagag ggagaagcct gatttttatt 8280
   ttttagagat tctagagata aaattcccag tattatatcc ttttaataaa aaatttctat 8340
   taggagatta taaagaattt aaagctattt ttttaagtgg ggtgtaattc tttcagtagt 8400
   ctcttgtcaa atggatttaa gtaatagagg cttaatccaa atgagagaaa tagacgcata 8460
   accctttcaa ggcaaaagct acaagagcaa aaattgaaca cagcagccag ccatctagcc 8520
   actcagattt tgatcagttt tactgagttt gaagtaaata tcatgaaggt ataattgctg 8580
   ataaaaaaat aagatacagg tgtgacacat ctttaagttt cagaaattta atggcttcag 8640
   taggattata tttcacgtat acaaagtatc taagcagata aaaatgccat taatggaaac 8700
   ttaatagaaa tatattttta aattccttca ttctgtgaca gaaattttct aatctgggtc 8760
   ttttaatcac ctaccctttg aaagagttta gtaatttgct atttgccatc gctgtttact 8820
   ccagctaatt tcaaaagtga tacttgagaa agattatttt tggtttgcaa ccacctggca 8880
   ggactatttt agggccattt taaaactctt ttcaaactaa gtattttaaa ctgttctaaa 8940
   ccatttaggg ccttttaaaa atcttttcat gaatttcaaa cttcgttaaa agttattaag 9000
   gtgtctggca agaacttcct tatcaaatat gctaatagtt taatctgtta atgcaggata 9060
   taaaattaaa gtgatcaagg cttgacccaa acaggagtat cttcatagca tatttcccct 9120
   cctttttttc tagaattcat atgattttgc tgccaaggct attttatata atctctggaa 9180
   aaaaaatagt aatgaaggtt aaaagagaag aaaatatcag aacattaaga attcggtatt 9240
   ttactaactg cttggttaac atgaaggttt ttattttatt aaggtttcta tctttataaa 9300
   aatctgttcc cttttctgct gatttctcca agcaaaagat tcttgatttg ttttttaact 9360
   cttactctcc cacccaaggg cctgaatgcc cacaaagggg acttccagga ggccatctgg 9420
   cagctgctca ccgtcagaag tgaagccagc cagttcctcc tgggcaggtg gccaaaatta 9480
   cagttgaccc ctcctggtct ggctgaacct tgccccatat ggtgacagcc atctggccag 9540
   ggcccaggtc tccctctgaa gcctttggga ggagagggag agtggctggc ccgatcacag 9600
   atgcggaagg ggctgactcc tcaaccgggg tgcagactct gcagggtggg tctgggccca 9660
   acacacccaa agcacgccca ggaaggaaag gcagcttggt atcactgccc agagctagga 9720
   gaggcaccgg gaaaatgatc tgtccaagac ccgttcttgc ttctaaactc cgagggggtc 9780
   agatgaagtg gttttgtttc ttggcctgaa gcatcgtgtt ccctgcaaga agcggggaac 9840
   acagaggaag gagagaaaag atgaactgaa caaagcatgc aaggcaaaaa aggccttagg 9900
   atggctgcag gaagttagtt cttctgcatt ggctccttac tggctcgtcg atcgcccaca 9960
   aacaacgcac ccagtggaga acttccctgt tacttaaaca ccattctctg tgcttgcttc 10020
   ctcaggggct gatgccaagc catccgtctt catcttcccg ccatcgaagg agcagttagc 10080
   gaccccaact gtctctgtgg tgtgcttgat caataacttc ttccccagag aaatcagtgt 10140
   caagtggaaa gtggatgggg tggtccaaag cagtggtcat ccggatagtg tcacagagca 10200
   ggacagcaag gacagcacct acagcctcag cagcaccctc tcgctgccca cgtcacagta 10260
   cctaagtcat aatttatatt cctgtgaggt cacccacaag accctggcct cccctctggt 10320
   cacaagcttc aacaggaacg agtgtgaggc ttagaggccc acaggcccct ggcctgcccc 10380
   cagccccagc ccccctcccc acctcaagcc tcaggccctt gccccagagg atccttggca 10440
   atcccccagc ccctcttccc tcctcatccc ctccccctct ttggctttaa ccgtgttaat 10500
   actggggggt gggggaatga ataaataaag tgaacctttg cacctgtgat ttctctctcc 10560
   tgtctgattt taaggttgtt aaatgttgtt ttccccatta tagttaatct tttaaggaac 10620
   tacatactga gttgctaaaa actacaccat cacttataaa attcacgcct tctcagttct 10680
   cccctcccct cctgtcctcc gtaagacagg cctccgtgaa acccataagc acttctcttt 10740
   acaccctctc ctgggccggg gtaggagact ttttgatgtc ccctcttcag caagcctcag 10800
   aaccattttg agggggacag ttcttacagt cacattcctg tgatctaatg actttagtta 10860
   ccgaaaagcc agtctctcaa aaagaaggga acggctagaa accaagtcat agaaatatat 10920
   atgtataaaa tatatatata tccatatatg taaaataaca aaataatgat aacagcatag 10980
   gtcaacaggc aacagggaat gttgaagtcc attctggcac ttcaatttaa gggaatagga 11040
   tgccttcatt acattttaaa tacaatacac atggagagct tcctatctgc caaagaccat 11100
   cctgaatgcc ttccacactc actacaaggt taaaagcatt cattacaatg ttgatcgagg 11160
   agttcccgtt gtggctcagc aggttaagaa cgtgactggt atccaggagg atgcgggttt 11220
   ggtccccagc ctcgctcagt ggattaagga tccagtgttg ctgcaagatc acgggctcag 11280
   atcccgtgtt ctatggctat ggtgtaggct ggtagctgca tgcagcccta atttgacccc 11340
   tagcctggga actgccatat gccacatgtg aggcccttaa aacctaaaag aaaaaaaaag 11400
   aaaagaaata tcttacaccc aatttataga taagagagaa gctaaggtgg caggcccagg 11460
   atcaaagccc tacctgccta tcttgacacc tgatacaaat tctgtcttct agggtttcca 11520
   acactgcata gaacagaggg tcaaacatgc taccctccca gggactcctc ccttcaaatg 11580
   acataaattt tgttgcccat ctctgggggc aaaactcaac aatcaatggc atctctagta 11640
   ccaagcaagg ctcttctcat gaagcaaaac tctgaagcca gatccatcat gacccaagga 11700
   agtaaagaca ggtgttactg gttgaactgt atccttcaat tcaatatgct caatttccaa 11760
   ctcccagtcc ccgtaaatac aacccccttt  gggaagagag tccttgcaga tgtagccacg  11820
   ttaaaaagag attatacaga aaggctagtg aggatgcagt gaaacgggat ctttcataca  11880
   ttgctggtgg aaatgtaaaa tgctgcaggc actctagaaa ataatttgcc agttttttga   11940
   aaagctaaac aaaatagttt agttgcattc tgggttattt atcccccaga aattaaaaat  12000
   tatgtccgca caaaaacgtg tacataatca ttcataacag ccttgtac               12048
<210> 31
   <211> 126068
   <212> DNA
   <213> Bos sp.
<220>
   <221> misc_feature
   <222> (3591)..(3690)
   <223> n is a, c, g, or t
<220>
   <221> misc_feature
   <222> (6955)..(6955)
   <223> n is a, c, g, or t
<220>
   <221> misc_feature
   <222> (6967)..(6967)
   <223> n is a, c, g, or t
<220>
   <221> misc_feature
   <222> (6974)..(6974)
   <223> n is a, c, g, or t
<220>
   <221> misc_feature
   <222> (6976)..(6978)
   <223> n is a, c, g, or t
<220>
   <221> misc_feature
   <222> (6980)..(6980)
   <223> n is a, c, g, or t
<220>
   <221> misc_feature
   <222> (6985)..(6985)
   <223> n is a, c, g, or t
<220>
   <221> misc_feature
   <222> (6987)..(6988)
   <223> n is a, c, g, or t
<220>
   <221> misc_feature
   <222> (7000)..(7000)
   <223> n is a, c, g, or t
<220>
   <221> misc_feature
   <222> (7534)..(7633)
   <223> n is a, c, g, or t
<220>
   <221> misc_feature
   <222> (7842)..(7842)
   <223> n is a, c, g, or t
<220>
   <221> misc_feature
   <222> (11197)..(11197)
   <223> n is a, c, g, or t
<220>
   <221> misc_feature
   <222> (12793)..(12892)
   <223> n is a, c, g, or t
<220>
   <221> misc_feature
   <222> (18660)..(18759)
   <223> n is a, c, g, or t
<220>
   <221> misc_feature
   <222> (23231)..(23330)
   <223> n is a, c, g, or t
<220>
   <221> misc_feature
   <222> (27257)..(27356)
   <223> n is a, c, g, or t
<220>
   <221> misc_feature
   <222> (27727)..(27727)
   <223> n is a, c, g, or t
<220>
   <221> misc_feature
   <222> (32250)..(32349)
   <223> n is a, c, g, or t
<220>
   <221> misc_feature
   <222> (39216)..(39315)
   <223> n is a, c, g, or t
<220>
   <221> misc_feature
   <222> (41326)..(41425)
   <223> n is a, c, g, or t
<220>
   <221> misc_feature
   <222> (48650)..(48749)
   <223> n is a, c, g, or t
<220>
   <221> misc_feature
   <222> (55204)..(55204)
   <223> n is a, c, g, or t
<220>
   <221> misc_feature
   <222> (56436)..(56535)
   <223> n is a, c, g, or t
<220>
   <221> misc_feature
   <222> (61281)..(61281)
   <223> n is a, c, g, or t
<220>
   <221> misc_feature
   <222> (61394)..(61493)
   <223> n is a, c, g, or t
<220>
   <221> misc_feature
   <222> (68709)..(68808)
   <223> n is a, c, g, or t
<220>
   <221> misc_feature
   <222> (73149)..(73248)
   <223> n is a, c, g, or t
<220>
   <221> misc_feature
   <222> (80571)..(80670)
   <223> n is a, c, g, or t
<220>
   <221> misc_feature
   <222> (91799)..(91898)
   <223> n is a, c, g, or t
<220>
   <221> misc_feature
   <222> (106600)..(106699)
   <223> n is a, c, g, or t
<220>
   <221> misc_feature
   <222> (121987)..(121987)
   <223> n is a, c, g, or t
<220>
   <221> misc_feature
   <222> (121993)..(121993)
   <223> n is a, c, g, or t
<400> 31
<210> 32
   <211> 10012
   <212> DNA
   <213> Sus scrofa
<400> 32
   gccacgccca ctccatcatg cggggagggg atgggcagac cctccagaaa gaagctccct      60
   ggggtgcagg ttaacagctt tcccagacac agccagtact agagtgaggt gaataagaca     120
   tcctccttgc ttgtgaaatt taggaagtgc ccccaaacat cagtcattaa gataaataat      180
   attgaatgca cttttttttt tttatttttt ttttttgctt tttagggcct aatctgcagc     240
   atatggaagt tcccaggcta caagtcgaac cagagctgca gctgccagcc tacatcacag     300
   ccacagcaac accagatccg agccacatct gtgactaaca ctgcagttca cagcaacgcc     360
   agatccttaa cccattgagt gaggccaggg atcaaaccca catcctcatg gatactagtc     420
   tggttcgtaa accactgagc cacaagggga actcctgaat gcaatatttt tgaaaattga     480
   aattaaatct gtcactcttt cacttaagag tccccttaga ttggggaaaa tttaaatatc     540
   tgtcatctta gtgcatcttt gctcatatga tgtgaataaa atcccaaaat ccatatgaat     600
   gaagcatcaa aatgtacatg aagtcagcct gaccctgcac tgccctcact tgcctcatgt     660
   accccccacc tcaaaggaag atgcagaaag gagtccagcc cctacaccgc cacctgcccc     720
   caccactgga gcccctcagg tctcccacct ccttttctga gcttcagtct tcctgtggca     780
   ttgcctacct ctacagctgc cccctactag gccctccccc tggggctgag ctccaggcac     840
   tggactggga aagttagagg ttaaagcatg gaaaattccc aaagccacca gttccaggct     900
   gccccccacc ccaccgccac gtccaaaaag gggcatcttc ccagatctct ggctggtatt     960
   ggtaggaccc aggacatagt ctttatacca attctgctgt gtgtcttagg aaagaaactc 1020
   tccctctctg tgcttcagtt tcctcatcaa taaaaggagc aggccaggtt ggagggtctg 1080
   tgacgtctgc tgaagcagca ggattctctc tccttttgct ggaggagaac tgatccttca 1140
   cccccaggat caacagagaa gccaaggtct tcagccttcc tggggacccc tcagagggaa 1200
   ctcagggcca cagagccaga ccctgatgcc agaacctttg tcatatgccc agacggagac 1260
   ttcatccccc tcctcctcag accctccagg ccccaacagt gagatgctga agatattaag 1320
   agaagggcaa gtcagcttaa gtttgggggt agaggggaac agggagtgag gagatctggc 1380
   ctgagagata ggagccctgg tggccacagg aggactcttt gggtcctgtc ggatggacac 1440
   agggcggccc gggggcatgt tggagcccgg ctggttctta ccagaggcag gggcacccct 1500
   ctgacacggg agcagggcat gttccataca tgacacaccc ctctgctcca gggcaggtgg 1560
   gtggcggcac agaggagcca gggactctga gcaaggggtc caccagtggg gcagttggat 1620
   ccagacttct ctgggccagc gagagtctag ccctcagccg ttctctgtcc aggagggggg 1680
   tggggcaggc ctgggcggcc agagctcatc cctcaagggt tcccagggtc ctgccagacc 1740
   cagatttccg accgcagcca ccacaagagg atgtggtctg ctgtggcagc tgccaagacc 1800
   ttgcagcagg tgcagggtgg gggggtgggg gcacctgggg gcagctgggg tcactgagtt 1860
   cagggaaaac cccttttttc ccctaaacct ggggccatcc ctaggggaaa ccacaacttc 1920
   tgagccctgg gcagtggctg ctgggaggga agagcttcat cctggaccct gggggggaac 1980
   ccagctccaa aggtgcaagg ggcccaggtc caaggctaga gtgggccaag caccgcaatg 2040
   gccagggagt gggggaggtg gagctggact ggatcagggc ctccttggga ctccctacac 2100
   cctgtgtgac atgttagggt acccacaccc catcaccagt cagggcctgg cccatctcca 2160
   gggccaggga tgtgcatgta agtgtgtgtg agtgtgtgtg tgtggtgtag tacacccctt 2220
   ggcatccggt tccgaggcct tgggttcctc caaagttgct ctctgaatta ggtcaaactg 2280
   tgaggtcctg atcgccatca tcaacttcgt tctccccacc tcccatcatt atcaagagct 2340
   ggggagggtc tgggatttct tcccacccac aagccaaaag ataagcctgc tggtgatggc 2400
   agaagacaca ggatcctggg tcagagacaa aggccagtgt gtcacagcga gagaggcagc 2460
   cggactatca gctgtcacag agaggcctta gtccgctgaa ctcaggcccc agtgactcct 2520
   gttccactgg gcactggccc ccctccacag cgcccccagg ccccagggag aggcgtcaca 2580
   gcttagagat ggccctgctg aacagggaac aagaacaggt gtgccccatc cagcgcccca 2640
   ggggtgggac aggtgggctg gatttggtgt gaagcccttg agccctggaa cccaaccaca 2700
   gcagggcagt tggtagatgc catttgggga gaggccccag gagtaagggc catgggccct 2760
   tgagggggcc aggagctgag gacagggaca gagacggccc aggcagagga cagggccatg 2820
   aggggtgcac tgagatggcc actgccagca ggggcagctg ccaacccgtc cagggaactt 2880
   attcagcagt cagctggagg tgccattgac cctgagggca gatgaagccc aggccaggct 2940
   aggtgggctg tgaagacccc aggggacaga gctctgtccc tgggcagcac tggcctctca 3000
   ttctgcaggg cttgacggga tcccaaggcc tgctgcccct gatggtagtg gcagtaccgc 3060
   ccagagcagg accccagcat ggaaacccca acgggacgca gcctgcggag cccacaaaac 3120
   cagtaaggag ccgaagcagt catggcacgg ggagtgtgga cttccctttg atggggccca 3180
   ggcatgaagg acagaatggg acagcggcca tgagcagaaa atcagccgga ggggatgggc 3240
   ctaggcagac gctggcttta tttgaagtgt tggcattttg tctggtgtgt attgttggta 3300
   ttgattttat tttagtatgt cagtgacata ctgacatatt atgtaacgac atattattat 3360
   gtgttttaag aagcactcca agggaacagg ctgtctgtaa tgtgtccaga gaagagagca 3420
   agagcttggc tcagtctccc ccaaggaggt cagttcctca acaggggtcc taaatgtttc 3480
   ctggagccag gcctgaatca agggggtcat atctacacgt ggggcagacc catggaccat 3540
   tttcggagca ataagatggc agggaggata ccaagctggt cttacagatc cagggctttg 3600
   acctgtgacg cgggcgctcc tccaggcaaa gggagaagcc agcaggaagc tttcagaact 3660
   ggggagaaca gggtgcagac ctccagggtc ttgtacaacg caccctttat cctggggtcc 3720
   aggaggggtc actgagggat ttaagtgggg gaccatcaga accaggtttg tgttttggaa 3780
   aaatggctcc aaagcagaga ccagtgtgag gccagattag atgatgaaga agaggcagtg 3840
   gaaagtcgat gggtggccag gtagcaagag ggcctatgga gttggcaagt gaatttaaag 3900
   tggtggcacc agagggcaga tggggaggag caggcactgt catggactgt ctatagaaat 3960
   ctaaaatgta tacccttttt agcaatatgc agtgagtcat aaaagaacac atatatattt 4020
   aaattgtgta attccacttc taaggattca tcccaagggg ggaaaataat caaagatgta 4080
   accaaaggtt tacaaacaag aactcatcat taatcttcct tgttgttatt tcaacgatat 4140
   tattattatt actattatta ttattattat tttgtctttt tgcattttct agggccactc 4200
   ccacggcata gagaggttcc caggctaggg gtcaaatcgg agctacagct gccggcctac 4260
   gccagagcca cagcaacgca ggatctgagc cacagcaatg caggatctac accacagctc 4320
   atggtaacgc tggatcctta acccaatgag tgaggccagg gatcgaacct gtaacttcat 4380
   ggttcctagt cggattcatt aaccactgag ccacgacagg aactccaaca ttattaatga 4440
   tgggagaaaa ctggaagtaa cctaaatatc cagcagaaag ggtgtggcca aatacagcat 4500
   ggagtagcca tcataaggaa tcttacacaa gcctccaaaa ttgtgtttct gaaattgggt 4560
   ttaaagtacg tttgcatttt aaaaagcctg ccagaaaata cagaaaaatg tctgtgatat 4620
   gtctctggct gataggattt tgcttagttt taattttggc tttataattt tctatagtta 4680
   tgaaaatgtt cacaagaaga tatatttcat tttagcttct aaaataatta taacacagaa 4740
   gtaatttgtg ctttaaaaaa atattcaaca cagaagtata taaagtaaaa attgaggagt 4800
   tcccatcgtg gctcagtgat taacaaaccc aactagtatc catgaggata tggatttgat 4860
   ccctggcctt gctcagtggg ttgaggatcc agtgttgctg tgagctgtgg tgtaggttgc 4920
   agacacagca ctctggcgtt gctgtgactc tggcgtaggc cggcagctac agctccattt 4980
   ggacccttag cctgggaacc tccatatgcc tgagatacgg ccctaaaaag tcaaaagcca 5040
   aaaaaatagt aaaaattgag tgtttctact taccacccct gcccacatct tatgctaaaa 5100
   cccgttctcc agagacaaac atcgtcaggt gggtctatat atttccagcc ctcctcctgt 5160
   gtgtgtatgt ccgtaaaaca cacacacaca cacacacacg cacacacaca cacacgtatc 5220
   taattagcat tggtattagt ttttcaaaag ggaggtcatg ctctaccttt taggcggcaa 5280
   atagattatt taaacaaatc tgttgacatt ttctatatca acccataaga tctcccatgt 5340
   tcttggaaag gctttgtaag acatcaacat ctgggtaaac cagcatggtt tttagggggt 5400
   tgtgtggatt tttttcatat tttttagggc acacctgcag catatggagg ttcccaggct 5460
   aggggttgaa tcagagctgt agctgccggc ctacaccaca gccacagcaa cgccagatcc 5520
   ttaacccact gagaaaggcc agggattgaa cctgcatcct catggatgct ggtcagattt 5580
   atttctgctg agccacaaca ggaactccct gaaccagaat gcttttaacc attccacttt 5640
   gcatggacat ttagattgtt tccatttaaa aatacaaatt acaaggagtt cccgtcgtgg 5700
   ctcagtggta acgaattgga ctaggaacca tgaggtttcg ggttcgatcc ctggccttgc 5760
   tcggtgggtt aaggatccag cattgatgtg agatatggtg taggtcgcag acgtggctcg 5820
   gatcccacgt tgctgtggct ctggcgtagg ccggcaacaa cagctccgat tcgaccccta 5880
   gcctgggaac ctccatgtgc cacaggagca gccctagaaa aggcaaaaag acaaaaaaat 5940
   aaaaaattaa aatgaaaaaa taaaataaaa atacaaatta caagagacgg ctacaaggaa 6000
   atccccaagt gtgtgcaaat gccatatatg tataaaatgt actagtgtct cctcgcggga 6060
   aagttgccta aaagtgggtt ggctggacag agaggacagg ctttgacatt ctcataggta 6120
   gtagcaatgg gcttctcaaa atgctgttcc agtttacact caccatagca aatgacagtg 6180
   cctcttcctc tccacccttg ccaataatgt gacaggtgga tctttttcta ttttgtgtat 6240
   ctgacaagca aaaaatgaga acaggagttc ctgtcgtggt gcagtggaga caaatctgac 6300
   taggaaccat gaaatttcgg gttcaatccc tggcctcact cagtaggtaa aggatccagg 6360
   gttgcagtga gctgtggggt aggtcgcaga cacagtgcaa atttggccct gttgtggctg 6420
   tggtgtaggc cggcagctat agctccaatt ggacccctag cctgggaacc tccttatgcc 6480
   gtgggtgagg ccctaaaaaa aagagtgcaa aaaaaaaaaa taagaacaaa aatgatcatc 6540
   gtttaattct ttatttgatc attggtgaaa cttattttcc ttttatattt ttattgactg 6600
   attttatttc tcctatgaat ttaccggtca tagttttgcc tgggtgtttt tactccggtt 6660
   ttagttttgg ttggttgtat tttcttagag agctatagaa actcttcatc tatttggaat 6720
   agtaattcct cattaagtat ttgtgctgca aaaaattttc cctgatctgt tttatgcttt 6780
   tgtttgtggg gtctttcacg agaaagcctt tttagttttt acacctcagc ttggttgttt 6840
   ttcttgattg tgtctgtaat ctgcggccaa cataggaaac acatttttac tttagtgttt 6900
   ttttcctatt ttcttcaagt acgtccattg ttttggtgtc tgattttact ttgcctgggg 6960
   tttgtttttg tgtggcagga atataaactt atgtattttc caaatggaga gccaatggtt 7020
   gtatatttgt tgaattcaaa tgcaacttta tcaaacacca aatcatcgat ttatcacaac 7080
   tcttctctgg tttattgatc taatgatcaa ttcctgttcc acgctgtttt aattatttta 7140
   gctttgtgga ttttggtgcc tggtagagaa caaagcctcc attattttca ttcaaaatag 7200
   tcccgtctat tatctgccat tgttgtagta ttagacttta aaatcaattt actgattttc 7260
   aaaagttatt cctttggtga tgtggaatac tttatacttc ataaggtaca tggattcatt 7320
   tgtggggaat tgatgtcttt gctattgtgg ccatttgtca agttgtgtaa tattttaccc 7380
   atgccaactt tgcatattgt atgtgagttt attcccaggg tttttaatag gatgtttatt 7440
   gaagttgtca gtgtttccac aatttcatcg cctcagtgct tactgtttgc ataaaaggaa 7500
   acctactcac ttttgcctat tgctcttgta ttcaatcatt ttagttaact cttgtgttaa 7560
   ttttgagagt ttttcagctg actgtctggg gttttcttta atagactagc cctttgtctg 7620
   taaagaataa ttttatcgaa tttttcttaa cactcacact ctccccaccc ccacccccgc 7680
   tcatctcctt tcattgggtc aaatctgtag aatacaataa aagtaagagt gggaacctta 7740
   gcctttaagt cgattttgcc tttaaatgtg aatgttgcta tgtttcggga cattctcttt 7800
   atcaagttgc ggatgtttcc ttagataatt aacttaataa aagactggat gtttgctttc 7860
   ttcaaatcag aattgtgttg aatttatatt gctattctgt ttaattttgt ttcaaaaaat 7920
   ttacatgcac accttaaaga taaccatgac caaatagtcc tcctgctgag agaaaatgtt 7980
   ggccccaatg ccacaggtta cctcccgact cagataaact acaatgggag ataaaatcag 8040
   atttggcaaa gcctgtggat tcttgccata actctcagag catgacttgg gtgttttttc 8100
   cttttctaag tattttaatg gtatttttgt gttacaatag gaaatctagg acacagagag 8160
   tgattcaatg aggggaacgc attctgggat gactctaggc ctctggtttg gggagagctc 8220
   tattgaagta aagacaatga gaggaagcaa gtttgcaggg aactgtgagg aatttagatg 8280
   gggaatgttg ggtttgaggt ttctataggg cacgcaagca gagatgcact caggaggaag 8340
   aaggagcata aatctagagg caaaaagaga ggtcaggact ggaaatagag atgcgagaca 8400
   ccagggtggc agtcagagag cacagtgtgg gtcagaagac agtggaagaa cacaagggac 8460
   agagagggat ctccaacttc actgggatga gggccttgtt ggccttgacc tgagagattt 8520
   ccaggagttg agggtgggaa ggagagggct cctgcacatg tcctgacatg aaacggtgcc 8580
   cagcatatgg gtgcttggaa gacattgttg gacagatgga tggatgatgg atgatggatg 8640
   aatggatgga tggaagatga tggataaatg gatgatggat ggatggacag aaggacaaag 8700
   agatggacag aaagacagtg atctgagaga gcagagaagg cttcatgaaa ggacaggaac 8760
   tgaactgtct cagtgggtgg agacaatggt gtagggggtt tccacatgga ggcaccaggg   8820
   gtcaggaata atctagtgtc cacaggccca ggaaggaagc tgtctgcagg aaattgtggg   8880
   gaagaacctc agagtcctta aatgaggtca ggagtggtca ggagggtctg atcaggtaag   8940
   gactcatgtc catcatcaca tggtcaccta agggcatgta gctctcagca tctccatcag   9000
   gacagtctca gaatgggggc ggggtcacac actgggtgac tcaaggcgtg ggtcatgcct   9060
   gcctcggacg tgggcctggg catggggaca cctccagacc atgggcccgc ccagggctgc   9120
   actggcctct ggtgggctag ctacccgtcc aagcaacaca ggacacagcc ctacctgctg   9180
   caaccctgtg cccgaaacgc ccatctggtt cctgctccag cccggcccca gggaacagga   9240
   ctcaggtgct agcccaatgg ggttttgttc gagcctcagt cagcgtggta tttctccggc   9300
   agcgagactc agttcaccgc cttaggttaa gtggttctca tgaatttcct agcagtcctg   9360
   cactctgcta tgccgggaaa gtcacttttg tcgctggggg ctgtttcccc gtgcccttgg   9420
   agaatcaagg attgcccaac tttctctgtg ggggaggtgg ctggtcttgg ggtgaccagc   9480
   aggaagggcc ccaaaagcag gagcagctgc ctccagaata caactgtcgg ctacagctca   9540
   aacaggaggc ctggactggg gtttaaccac cagggcggca cgaaggagcg aggctgggag   9600
   ggtgaggaca tgggagcctg aggaggagct ggagacttca gcaggccccc agctccgggc   9660
   ttcgggctct gagatgctcg gacgcaaggt gagtgacccc acctgtggct gacctgacct   9720
   cagggggaca aggctcagcc tgagactctg tgtccccatc gcctgcacag gggattcccc   9780
   tgatggacac tgagccaacg acctcccgtc tctccccgac ccccaggtca gcccaaggcc   9840
   gcccccacgg tcaacctctt cccgccctcc tctgaggagc tcggcaccaa caaggccacc   9900
   ctggtgtgtc taataagtga cttctacccg aagggcgaat tccagcacac tggcggccgt   9960
   tactagtgga tccgagctcg gtaccaagct tgatgcatag cttgagtatc ta          10012
<210> 33
   <211> 4614
   <212> DNA
   <213> Sus scrofa
<400> 33
   agatctttaa accaccgagc aaggccaggg atcgaacccg catcctcatg aatcctagtt      60
   gggttcgtta accgctgaac cacaatggga actcctgtct ttcacattta attcacaacc     120
   tctccaggat tctgggggtg ggtggggaat cctaggtacc cactgggaaa gtaatccaag     180
   gggagaggct cacggactct agggatcggc ggaggaggga aggtatctcc caggaaactg     240
   gccaggacac attggtcctc cgccctcccc ttcctcccac tcctcctcca gacaggactg     300
   tgcccacccc ctgccacctt tctggccaga actgtccatg gcaggtgacc ttcacatgag     360
   cccttcctcc ctgcctgccc tagtgggacc ctccatacct ccccctggac cccgttgtcc     420
   tttctttcca gtgtggccct gagcataact gatgccatca tgggctgctg acccacccgg     480
   gactgtgttg tgcagtgagt cacttctctg tcatcagggc tttgtaattg atagatagtg 540
   tttcatcatc attaggaccg ggtggcctct atgctctgtt agtctccaaa cactgatgaa 600
   aaccttcgtt ggcatagtcc cagcttcctg ttgcccatcc ataaatcttg acttagggat 660
   gcacatcctg tctccaagca accacccctc ccctaggcta actataaaac tgtcccaatg 720
   gcccttgtgt ggtgcagagt tcatgcttcc agatcatttc tctgctagat ccatatctca 780
   ccttgtaagt catcctataa taaactgatc cattgattat ttgcttctgt tttttccatc 840
   tcaaaacagc ttctcagttc agttcgaatt ttttattccc tccatccacc catactttcc 900
   tcagcctggg gaacccttgc ccccagtccc atgcccttcc tccctctctg cccagctcag 960
   cacctgccca ccctcaccct tcctgtcact ccctaggact ggaccatcca ctggggccag 1020
   gacactccag cagccttggc ttcatgggct ctgaaatcca tggcccatct ctattcctca 1080
   ctggatggca ggttcagaga tgtgaaaggt ctaggaggaa gccaggaagg aaactgttgc 1140
   atgaaaggcc ggcctgatgg ttcagtactt aaataatatg agctctgagc tccccaggaa 1200
   ccaaagcatg gagggagtat gtgcctcaga atctctctga gattcagcaa agcctttgct 1260
   agagggaaaa tagtggctca accttgaggg ccagcatctt gcaccacagt taaaagtggg 1320
   tatttgtttt acctgaggcc tcagcattat gggaaccggg ctctgacaca aacacaggtg 1380
   cagcccggca gcctcagaac acagcaacga ccacaagctg ggacagctgc ccctgaacgg 1440
   ggagtccacc atgcttctgt ctcgggtacc accaggtcac catccctggg ggaggtagtt 1500
   ccatagcagt agtcccctga tttcgcccct cgggcgtgta gccaggcaag ctcctgcctc 1560
   tggacccagg gtggaccctt gctccccact accctgcaca tgccagacag tcaagaccac 1620
   tcccacctct gtctgaggcc cccttgggtg tcccagggcc cccgagctgt cctctactca 1680
   tggttcttcc acctgggtac aaaagaggcg agggacactt ttctcaggtt tgcggctcag 1740
   aaaggtacct tcctagggtt tgtccactgg gagtcacctc ccttgcatct caatgtcagt 1800
   ggggaaaact gggtcccatg gggggattag tgccactgtg aggcccctga agtctggggc 1860
   ctctagacac tatgatgatg agggatgtgg tgaaaaaccc caccccagcc cttcttgccg 1920
   ggaccctggg ctgtggctcc cccattgcac ttggggtcag aggggtggat ggtggctatg 1980
   gtcaggcatg tttcccatga gctgggggca ccctgggtga ctttctcctg tgaatcctga 2040
   attagcagct ataacaaatt gcccaaactc ttaggcttaa aacaacacac atttattcct 2100
   ctgggtccca gggtcagaag tccaaaatga gtcctatagg ctaaatttga ggtgtctctg 2160
   ggttgagctc ctcctggaag ccttttccag cctctagagt cccaagtcct tggctctggg 2220
   cccctccctc aagcttcaaa gccacagaag cttctaatct ctctcccttc ccctctgacc 2280
   tctgctccca tcctcatacc ctgtcccctc actctgaccc tcctgcctcc ctctttccct 2340
   tataaagacc ctgcatgggg ccacggagat aatccagggt aatcgcccct cttccagccc 2400
   ttaactccat cccatctgca aaatccctgt caccccataa tggacctact gatggtctgg 2460
   gggttaggac gtggacaact tggggcctta ttcatctgat cacaactcca gttcccagac 2520
   ccccagaccc ccgggcatta gggaaacttc tcccagttcc tctccctctg tgtcctgccc 2580
   agtctccagg atgggccact cccgagggcc cttcagctca ggctccccct cctttctccc 2640
   tggcctcttg tggccccatc tcctcctccg ctcacaggga gagaactttg atttcagctt 2700
   tggctctggg gctttgcttc cttctggcca ttggctgaag ggcgggtttc tccaggtctt 2760
   acctgtcagt catcaaaccg cccttggagg aagaccctaa tatgatcctt accctacaga 2820
   tggagactcg aggcccagag atcctgagtg acctgctcac attcacagca gggactgaac 2880
   cccagtcacc tacccaactc cagggctcag cgcttttttt tttttttttc tttttgcctt 2940
   ttcgagggcc gctcccgcaa catatggaga tttccaggct aggggtctaa ttggagcagt 3000
   cgacactggc ctaagccaaa gccacagcaa caagggcaag ccgcttctgc agcctatacc 3060
   acagctcacg gcaatgccgg atccttaacc cactgagcaa agccagggat tgaacctgca 3120
   acctcatgtt tcctagtcaa atttgttaac cactgaccca tgacgggaac tcccagggct 3180
   cagctcttga ctccaggttc gcagctgccc tcaaagcaat gcaaccctgg ctggccccgc 3240
   ctcatgcatc cggcctcctc cccaaagagc tctgagccca cctgggccta ggtcctcctc 3300
   cctgggactc atggcctaag ggtacagagt tactggggct gatgaaggga ccaatgggga 3360
   caggggcctc aaatcaaagt ggctgtctct ctcatgtccc ttcctctcct cagggtccaa 3420
   aatcagggtc agggccccag ggcaggggct gagagggcct ctttctgaag gccctgtctc 3480
   agtgcaggtt atgggggtct gggggagggt caatgcaggg ctcacccttc agtgccccaa 3540
   agcctagaga gtgagtgcct gccagtggct tcccaggccc aatcccttga ctgcctggga 3600
   atgctcaaat gcaggaactg tcacaacacc ttcagtcagg ggctgctctg ggaggaaaaa 3660
   cactcagaat tgggggttca gggaaggccc agtgccaagc atagcaggag ctcaggtggc 3720
   tgcagatggt gtgaacccca ggagcaggat ggccggcact ccccccagac cctccagagc 3780
   cccaggttgg ctgccctctt cactgccgac acccctgggt ccacttctgc cctttcccac 3840
   ctaaaacctt tagggctccc actttctccc aaatgtgaga catcaccacg gctcccaggg 3900
   agtgtccaga agggcatctg gctgagaggt cctgacatct gggagcctca ggccccacaa 3960
   tggacagacg ccctgccagg atgctgctgc agggctgtta gctaggcggg gtggagatgg 4020
   ggtactttgc ctctcagagg ccccggcccc accatgaaac ctcagtgaca ccccatttcc 4080
   ctgagttcac atacctgtat cctactccag tcaccttccc cacgaacccc tgggagccca 4140
   ggatgatgct ggggctggag ccacgaccag cccacgagtg atccagctct gccaatcagc 4200
   agtcatttcc caagtgttcc agccctgcca ggtcccacta cagcagtaat ggaggcccca 4260
   gacaccagtc cagcagttag agggctggac tagcaccagc tttcaagcct cagcatctca 4320
   aggtgaatgg ccagtgcccc tccccgtggc catcacagga tcgcagatat gaccctaggg 4380
   gaagaaatat cctgggagta aggaagtgcc catactcaag gatggcccct ctgtgaccta 4440
   acctgtccct gaggattgta cttccaggcg ttaaaacagt agaacgcctg cctgtgaacc 4500
   cccgccaagg gactgcttgg ggaggccccc taaaccagaa cacaggcact ccagcaggac 4560
   ctctgaactc tgaccaccct cagcaagtgg caccccccgc agcttccaag gcac 4614
<210> 34
   <211> 1151
   <212> DNA
   <213> Sus scrofa
<400> 34
   aacaagatgc taccccacca acaaaattca ccggagaaga caaggacagg gggttcctgg 60
   ggtcctgaca gggtcaccaa agagggttct ggggcagcag caactccagc cgcctcagaa 120
   cagagcctgg aagctgtacc ctcagagcag aggcggagag agaaagggcc tcttggtggg 180
   tcagcaggag cagaggctca gaggtggggg ttgcagcccc cccttcaaca ggccaacaca 240
   gtgaagcagc tgacccctcc accttggaga ccccagactc ctgtctccca cgccaccttg 300
   gtttttaagg taatttttat tttatatcag agtatggttg acttacaatg ttgtgttggt 360
   ttcaggtgta cagcagagtg attcacttct acatagactc atatctattc tttctcagat 420
   tcttttccca tataggttat tacagaatat tgagtagatc cctgctgatt acccattttt 480
   ataattgtat atgttaatcc caaactccta atttatccct ccccagacta tgattcttta 540
   tatctctatc tgtttcctaa tctgtctcct ctaagtcacc ctaggagagc agaggggtca 600
   cgtctgtcct gtcctggccc agccacctct ctccacccag gaatcccttg catttggtgc 660
   caagggcccg gccccgccct aaagagaaag gagaacggga tgtggacagg acaccgggca 720
   gagagggaca agcagaggat gccagggtag ggaggtctcc agggtggatg gtggtctgtc 780
   cgcaggcagg atgaggcagg aagggtgtgg atgtactcgg tgaggctggc gcatggcctg 840
   gagtgtcctg agccctggga ggcctcagcc ctggatcaga tctgtgattc caaagggcca 900
   ctgcatccag agaccgttga gtggcccatt gtcctgaacc atttatagaa cacaggacaa 960
   gcggtacctg actaagctgc tcacagattc catgaggctg atgccagggt tgtcacccca 1020
   tctcacaggc agggaaactg atgcatatac tgcagagcca ggcagaggcc ctcccagtgc 1080
   cccctcccag cctgtggccc ccctccagtg gctggacact gaggccacac tggggcaccc 1140
   tgtggagatc t 1151
<210> 35
   <211> 630
   <212> DNA
   <213> Sus scrofa
<400> 35
   agatctggcc aggccagaga agcccatgtg gtgacctccc tccatcactc cacgccctga     60
   cctgccaggg agcagaaagt aggcccaggg tggacccggt ggccacctgc caccccatgg    120
   ctgggagaag ggagggcctg ggcaaagggc ctgggaagcc tgtggtggga ccccagaccc    180
   cagggtggac agggagggtc ccacacccac agccatttgc ttccctctgt gggttcagtg    240
   tcctcatctc atctgtgggg agggggctga taatgaatct cccccattgg ggtgggcttg    300
   gggattaaag ggccagtgtc tgtgatatgc ctggaccata gtgaccctca ccctccccag    360
   ccattgctgt caccttccgg gctcttgccc aggcctgcct gacatgctgt gtgaccctgg    420
   gcaagatgat ccccctttct gggccccagc cttcctctct gctccggaag tgcttcctgg    480
   ggaaacctgt gggctggatc ctataggaaa cctgtccaat tcctggatgc acagaggggc    540
   agggaggccc tgggcctgga ggggcaggga ggctcgaggt gggagcaggg taggggccag    600
   tccagggcaa ggaggtgggt gggtagggtg                                      630
<210> 36
   <211> 947
   <212> DNA
   <213> Sus scrofa
<400> 36
   gatctgtgtt ccatctcaga gctatcttag cagagaggtg caggggcctc cagggccacc 60
   aaagtccagg ctcagccaga ggcaatgggg tatcgatgag ctacaggaca caggcgtcag 120
   cccagtgtca gggagaatca ccttgtttgt tttctgagtt cctcttaaaa tagagttaat 180
   tggtcttggc cttacggttt acaataacaa ctgcaccctg taaacaacgt gaagagtaca 240
   gaacaacaaa tgggggaaaa catatttcac ctgaaagagc caccgctcat attttgatgg 300
   atttccttct agtttaatcc tgttttaatt gtaaactgtt aaaacaaaca taaataaaga 360
   aaatgcatct gtaaagttta aaagtcatat ctatggtgat ggttgcaaaa cactgtgaat 420
   gttcactttg aaatcgtgaa ctctacgtga tatgcatgtc ccgttaatta acctcacagg 480
   ctcagaatgt ggttcattat ttctttaatt ttcctttaat tttatgtcct ctgtgtgtgc 540
   ccttaaacca actacttttc agctctgcct gtttttgacc ttcacataga tgacatttgt 600
   gagtgttttc tttctcaaca ctgggtctga tacccaccca cgctgtctgc tgtcactgcg 660
   gacgtggagg gccaccaccc agctatggcc ccagccaggc caacactgga tgaatctgcc 720
   cccagagcag ggccaccaac actggaggtg cagagagggt ttcttcaggg ccatcattat 780
   ccaaggcatt gtttctactg taagctttca aaatgcttcc cctgattatt aaaagaaata 840
   ataagatggg gggaaagtac aagaagggaa gtttccagcc cagcctgaag atcgtgctgg 900
   ttgtatctgg agcctgtctt cctgacaggc ctctattccc agagtta 947
<210> 37
   <211> 2067
   <212> DNA
   <213> Sus scrofa
<400> 37
   ggatcctagg gaagggaggg cgggggcctg gacaaagggg gcctaaagga cattctcacc 60
   tatcccactg gacccctgct gtgctctgag ggagggagca gagagggggt ctgaggcctt 120
   ttcccagctc ctctgagtcc ctcctccgag cacctggacg gaagcccctc ctcagggagt 180
   cctcagaccc ctcccctcca gccaggttgg cctgtgtgga gtccccagta agaatagaat 240
   gctcagggct tcgagctgag ccctggctac ttgggggggt gctggggatt gggggtgctg 300
   ggcggggagc tggggtgtca ctagatgcca gtaggctgtg ggctcgggtc tggggggtct 360
   gcacatgtgc agctgtggga aggccctatt ggtggtaccc tcagacacat atggcccctc 420
   aatttctgag accagagacc ccagtctggc cttcccagaa cagctgcccc tggtggggga 480
   gatgtagggg ggccttcagc ccaggacccc caacggcagg gcctgaggcc cccatcccct 540
   tgtcctgggc ccagagcctc agctatcagg cctatcagag atcctggctg cccagctcag 600
   gttccccagg agccagaggg aggccagggg ttactaggaa atccggaaag ggtctttgag 660
   gctgggcccc accctctcag ctttcacagg agaaacagag gcccacaggg ggcaaaggac 720
   ttgccagact cacaatgagc ccagcagctg gactcaaggc ccagtgttcg gccccacaac 780
   agcactcacg tgcccttgat cgtgaggggc cccctctcag ccaggcattc agacctgtga 840
   cctgcatcta agattcagca tcagccattc tgagctgaag agccctcagg gtctgcagtc 900
   aaggccacag ggccagacct ccaacggcca gacatcccag ccagattcct ttctggtcaa 960
   tgggccccag tctggcttgg ctcctgcagg cccagtgccg ccttcttccc ctgggcctgt 1020
   ggagtccagc ctttcagttt cccacccaca tcctcagcca caatccaggc tcagaggcaa 1080
   tgtccgtggg cagcccctgt gtgacccctc tgtgggtgat cctcagtcct acccttagca 1140
   gacagcgcat gaggggccct cttgaacctg agggatactc catgtcggag gggagaagct 1200
   ggccttcccc acccccactt ccaggccttg gggagcagag aaagacccca gacctgggtc 1260
   ccttctaaca ggccaggccc cagcccagct ctccaccagc cccaggggcc tcgggtccac 1320
   gcctggggac tggagggtgg gcctgtcagg cgctgaccca gaggcaggac agccaagttc 1380
   aggatcccag ccaggtggtc cccgtgcacc atgcaggggt gtcacccaca caggggtgtt 1440
   gccaccctca cctgactgtc ctcatgggcc acatggaggt atcctgggtt cattactggt 1500
   caacataccc gtgtccctgc agtgccccct ctggcgcacg cgtgcacgcg cacacgcaca 1560
   cactcataca gaggctccag ccaacagtgc cctctagtag gcactgctgt cacttctcta 1620
   aaaggtcgca atcatacttg taaagaccca agattgttca gaaatcccag atggagaagt 1680
   ctggaaagat ctttttctcc tttcacgggc tggggaaatg tgacctggcc aaggtcacac 1740
   agcaagtggt ggaaccctgg cccctgattc cagctcattc cagttcccaa ggccctgcca 1800
   gagcccagag gctgggccct ctggggcaga ggagctgggg tcctcccccc tacacagagc 1860
   acacagcccc gcaagagaga agagacacct tggggagagg aatctccaga ccagagatcc 1920
   cagtatgggt ctcctctatg ctgacgggat gggatgtcaa gaggggaggg ggctgggctt   1980
   tagggaaaca cacaaaaatc gctgagaaca ctgacaggtg cgacacaccc acccctaatg   2040
   ctaacctgtg gcccattact cagatct                                       2067
<210> 38
   <211> 4898
   <212> DNA
   <213> Sus scrofa
<400> 38
   gatcttctcc taagaccaag gaaaactggt cataccaggt ccacttgtcc cctgtggcca 60
   ttgtccctcc ttccccagaa gaaacaagca ctttccactc cacaagtagc tcctgatcag 120
   cttggaagcc cggtgctgct ctgggccctg gggacacggc aggggcatca gagaccaaat 180
   cctggaacaa agttccagtg ggtgaggcag gccggacaag caacacgtta taccataata 240
   tgaggcaaaa tataatgtga gttctttatg aaaggaaggg gttgcaggtg caactgttgg 300
   cttaggtgga tggtcacccc tgaatggagg agggggttcc cagggcatgt gcctggggag 360
   aagggctcct ggcaggaggg acagcaagtg caagggccct gtgatcaaat gtgcctggca 420
   agttgcagga acagctagaa ggccagcaag gttggaacca aggaaggggt caggggaggg 480
   gcagggccct cagggccttg cccagcagcc tgagcatctg gagatttgtc caaagtttca 540
   aatgtacctg ggcaacctca tgcccatata ccattcctaa cttctgcact taacatctct 600
   aggactggga cccagccagt caagcggggg gacccagaga gctccggtgt gaacaccgag 660
   gtgctggtgg gtctgcgtgt gtggacatag ggcagtcccg gtccttcctt cactaacacg 720
   gcccgggaag ccctgtgcct ccctggtgcg cgggtcggcg cttccggagg gtacaggccc 780
   acctggagcc cgggcacagt gcatgcaagt cgggttcacg gcaacctgag ctggctctgc 840
   agggcagtgg gactcacagc caggggtaca gggcagaccg gtcctgcctc tgcgcccctc 900
   cctggcctgt ggcccctgga cgtgatcccc aacagttagc atgccccgcc ggtgctgaga 960
   acctggacga ggtccgcagg cgtcactggg cggtcactga gcccgcccca ggccccctct 1020
   gccccttcct ggggtgaccg tggactcctg gatgaccctg gaccctagac ttcccagggt 1080
   gtctcgcgga ggttcctcag ccaggatctc tgcgtctcct ccttccatag aggggacggc 1140
   gcccccttgt ggccaaggag gggacggtgg gtcccggagc tggggcggag aacacaggga 1200
   gcccctccca gaccccgctc tgggcagaac ctgggaaggg atgtggccat cgggggatcc 1260
   ctccaggcca tctcctcaga tgggggctgg tcgactagct tctgagtcct ccaaggaacc 1320
   gggtccttct agtcatgact ctgcccagat gaagaaggag agcacttctc tccatcagga 1380
   ggatctgagc ttctcttaat tagaatcagc tccttggctt ctacccctta aaaaaaggta 1440
   cagaaacttt gcaccttgat ccagtatcag gggaatttat caatcaatgt gggagaaatt 1500
   ggcatcttta ccacactgaa tctttcaatc catgaatatc ctctctctct tccatgcata 1560
   ggttttaata attctcaatg gagtttaatg taagttttcc tcatagacaa ttgcctttgg 1620
   acatctcttt agactcatct ctagtaaact gatattctta atgcaattat aaaatgtatc 1680
   ctgcttaatg ttattttcta ttcatttgct gttatataga gatacaatga gtttccacat 1740
   ttgaaactgg atctggtaaa ttggctaccc tttttttata gattctatta atttttatac 1800
   attctgtggg acttgctaca tacttaatca tgtcacctgt gaagaatgac aatttggttg 1860
   ctaccctccc aattcttata tgtctcattt ctttccctct gctggtactc tggcagcagc 1920
   agggaagata atgggcctcc ttatcttgtc acaaaaggat gtttttaaag atttcgttat 1980
   aaaacataac gctttctggt tttctttaaa gattctctca ccagcttaag aaaattttct 2040
   tatactctgt atgataaatg ggtttttgac aatcatttgt tgcattttac ctagtgtttt 2100
   ctctgcatct ttatatgctt tttctccttt aatcctgaaa attgtttcga tttttctaac 2160
   attgaaccaa tcttacattc ctggaatgga tggaccagac tagtccacat gtttattctg 2220
   cccaatggct agattttgtg ttcaatattt tgttcagaat gtttgcatct atattcttga 2280
   gtgagacaga gctgcccttg ttaggtttca caaccgaggt tgtgttagct tcataaaatg 2340
   agacgtttat tctctaaaag aattgtttcg cttctctgga tgaatttgtg taaggttaga 2400
   attgcttacc agtgaagatc tcggggccag ttcttcttta ggggaagatt ttcaacaatt 2460
   aagctcaatg cctttagaag aactgagagt ttctattatt tcttgagtta aatatatgta 2520
   tttaattaga ctttctagga atagtctcat ttcatctcaa ataattgaca tatgctatta 2580
   aagcagattc tcatgaacca ttgtaggtat tccaggtcta gaaaaatgtt cccctttgca 2640
   tccctaatgt gtttaatttt caccttcttt cttttgttct tgagaaattc accaaatcat 2700
   tttcaatttc agtcatatcc caaagcaacc aactctctac cttcttgttt tatcatccct 2760
   gctggatttt tgttatctac ttcttcagta tttgttcttc cctttcttct attcctcatt 2820
   ccatttttcc cttgttttct aactttctga gatatatgct tagttccttc atttgaagcc 2880
   tttttatttt cttttttttt ttttggtctt tttgtctttt gttgttgttg ttgtgctatt 2940
   tcttgggccg ctcccgcggc atatggaggt tcccaggcta ggagtcgaat cggagctgta 3000
   gccaccggcc tacgccagag ccacagcaat gcgggatccg agccgcgtct gcaacctaca 3060
   ccacagctca tggcaacgcc ggatcgttaa cccactgagc aagggcagga accgaacccg 3120
   caacctcatg gttcctagtc ggattcgtaa ccactgtgcc acaacaggaa ctccgccttt 3180
   ttattttcta taaaaatttc tatgtacatt ttaaggttat aggtttcctt ctatgtaccc 3240
   cattggctgt atcctcaggg ttctgtggag tgatttcatt attgttcaag ttcaatatgt 3300
   cttctgattt tccaatttga atacctctct aaatcagtag gtgaatattt ctttttcttt 3360
   ttcttttctt ttcttctttt tttttttctt tcagccaggt ccatggcatg cagaaattcc 3420
   caggccagga atcaaactct caccatggca gtgacaatgt cggatccttt acccactagg 3480
   ccaccaggga actctgggag catatgtttt tatttcccga catctgagga tgcctagtat 3540
   gtcttcatta ttgatttcta gtttgccact gatttctagt attttgctca tagagtgtat   3600
   gctcaatggt tttggtcatt tgaaatgtat ttagtcctgc tttatgaccc agtatgtggt   3660
   cagttttgtc aatgttcctt ttctgcttga agagaaccta catgctgtaa ctctgggtgc   3720
   atgttctgta tataagtcta taggctgagc cgggggagcc ttctaatctg ccgttatctt   3780
   cttcgagtta ttctaggtac tatttcttag ccataaacct ttaaattctg atatcaatat   3840
   aatgacccca gcccgcttag ggtcggcact tcatgttatc tttttccatc catttaatcc   3900
   ctccccactg ttttggccac acccgtggga tatgggagtt cctgggccaa ggatcagatc   3960
   tgagccgcag ctgccaccta tgccacagca gcagcaatga tggatcttta acccactgca   4020
   ccacactggg gattgaaccc aagcctcagc agcaacccaa gctactgcag agacaacacc   4080
   agatccttaa cctgctgtgc catagcggga atttccatcc atttactttc aagccagctg   4140
   aataacctag cccaccatgc ctggacatgg gtgctctgct tcaaatgatt ttgttcagtc   4200
   agcatccatc tctgaaatgt gtgccaagca tttatatgca tgcaagagtc atgttggcac   4260
   ttctatcatt tccaacagtt cagtagcctt tgtatcatga catttcttgg ccttttctct   4320
   acaatatttg aggctgagca gactggccgt gcccctgtcc atgcttccag agcctgtgtg   4380
   cagacttctg ctctagacag agacagctaa ccatcctgca gtgcccagaa aacccaactc   4440
   aaagaccctc aagtaaggaa ggatttattg gctcacgtaa tctggaatcc aggcatgggg   4500
   tattcagggc cacctgaacc agaggccctg gccctgttct ctaagcttct tcctgccctg   4560
   ccctcgttct ggaagtgacc ctgaaggaca gcaatgaagg gcagctcccc cagggacaga   4620
   tgactgagag gtccatttca agtccaactt ggcctagatt gagaggcagc aagaaatatg   4680
   gacctacagt gagtcacagg atttaccagt ggtttggctg ggttgtcagt gttacaggct   4740
   aaacatttgg gtccctccaa aattaacatg ttgccactct aaccaccaaa atcatggtat   4800
   ttgggggtgg ggcccttgga ggtaattagg tttagaaaga atgaagaggg ggcccttgtg   4860
   atgggactag tgcctttata gagagagaag agagaggg                           4898
<210> 39
   <211> 1913
   <212> DNA
   <213> Sus scrofa
<400> 39
   cacctcatcc ccaaccacct ggatggtggc aagtggcagg ctgagaggct gcatatgagc 60
   tcatcaagag ggtccccacc ccacagaggc tgacccagct gccactgcca cctagtggct 120
   gatcggccaa gagcaggagc cccaggggca gctccattcc ctggggcggc cagggaacca 180
   cctggtggta ggacaattcc attgcacctc atccatcagg aaaaggtttg ccttccctgg 240
   cagtaatgca tcttcccata acatggtccc tggcctcttg gaatggcttg gccaccgtca 300
   tggcctcacc cacaaagcct tgtgtctcag caaggaactt attccacagc aaaggacttg 360
   cagcctggaa tgaactggtc tgactacata ccccattgcc cagaagtagg tggtctattg 420
   caaagtggag tggcttaccc aagactcagt tgtgcccaag ttgagagata gcatcctaaa 480
   atatgggctt atgtctcact ggctgaggtt tattctttga atcaaagaca attatatggt 540
   gtggtccccc cagagataga atacatgagt ctgggaatca agggatagaa gtaagaagag 600
   attttgtcac cattaatccc aataactcgc ccaaagaata tttgctttct gtcctggcag 660
   ctctgctgct ttggcaataa cttcctagaa tataatgtct ccaccagggg actccacaac 720
   ggttccattg atttgaagcc aatgggcaga ggaggggctg ccttactggt cggactggtc 780
   agccctgatt actaaggaga aatcaggcaa cttcaacaaa actaaggcag gggggacttt 840
   gtctagaacc caaagcacta agcatcttag tactttttag ttctcagagc ctccaagaac 900
   aaagatttag cccctcagca ccaccaggta aagaacaggt aaatccagct gaggacaaga 960
   gaaatattga atggatagag gaagaaagaa attatagata tcaactatgg cctcatgact 1020
   agagtctcca gattaagcgg aataaaaata cagatgatta gatctgaaca tcaggccaaa 1080
   caacgaacaa cagtttaagt gcgacctagg caatatttgg gacatactta tactaaaatt 1140
   ttttcgctat ttgagcatcc tgtattttat ctggcaactt tattcatccc tagcgaaaaa 1200
   ggaactgtgg taacttagtg tatttttact ttgctcatta ttgtgtatat acctacttgt 1260
   atttatcaat catatttact ctgttctcag tattacttta tatagcagtt ggtggtgatg 1320
   gttagcaaca tattcagtgg aactgtgact gaatttgagg agaaattaac agagttggct 1380
   gtggctacaa taacccttcg ggacatgtgt cccctcattt tggggagatg gttagatctc 1440
   tgggtaaatg ttagggcatc tgagccagaa accaagattt tgccagctgg tgcaatgtca 1500
   gattttacca gcagagggtg ccagaggaat gcggcaaaac ccgagtgcca gaaagcacct 1560
   ccctgttttc cagcttttct tcctttttat ttattttatt tacggcccag gagtccgtaa 1620
   tagcgctgag gatggcccag gctcttctca gcagccctga ctgactagtt cagcaatgcg 1680
   ctcaggcccc atctggccac cgggcagcct cttctgtggt agctccagcc tcagccagtg 1740
   caaaaggcta ccctacactg gcgccacttc tacaatcagc actggccaca ccctccacgc 1800
   catccggcac ggagccaggt gatctgccgg ccagattgca gttcgtgctg cctgagtcca 1860
   ggtgattaca ctggctgcat cttttctttc tggaccattc attccatttt ttt 1913
<210> 40
   <211> 394
   <212> DNA
   <213> Sus scrofa
<400> 40
   ctctgcactc actaccgccg gacgcgcact gccgtgctgc ccatggacca cgctggggag 60
   gggtgagcgg acagcacgtt aggaagtgtg tgtgtgcgcg tgggtgcaag tcgagccaag 120
   gccaagatcc aggggctggg ccctgtgccc agaggagaat ggcaggtgga gtgtagctgg 180
   attgaaaggt ggcctgaagg gtggggcatc ctgtttggag gctcactctc agccccaggg 240
   tctctggttc ctgccggggt ggggggcgca aggtgcctac cacaccctgc tagcccctcg 300
   tccagtcccg ggcctgcctc ttcaccacgg aagaggataa gccaggctgc aggcttcatg 360
   tgcgccgtgg agaacccagt tcggcccttg gagg 394
<210> 41
   <211> 333
   <212> DNA
   <213> Sus scrofa
<400> 41
   ggctgaagtc tgaggcctgg cagatgagct tggacgtgcg ctggggagta ctggagaagg 60
   actcccgggt ggggacgaag atgttcaaga cggggggctg ctcctctacg actgcaggca 120
   ggaacggggc gtcactgtgc cggcggcacc cggccccgcc cccgccacag ccacaggggg 180
   agcccagctc acctggccca gagatggaca cggacttggt gccactgggg tgctggacct 240
   cgcacaccag gaaggcctct gggtcctggg ggatgctcac agagggtagg agcacccggg 300
   aggaggccaa gtacttgccg cctctcagga cgg 333
<210> 42
   <211> 399
   <212> DNA
   <213> Sus scrofa
<400> 42
   gaagtgaagc cagccagttc ctcctgggca ggtggccaaa attacagttg acccctcctg 60
   gtctggctga accttgcccc atatggtgac agccatctgg ccagggccca ggtctccctc 120
   tgaagccttt gggaggagag ggagagtggc tggcccgatc acagatgcgg aaggggctga 180
   ctcctcaacc ggggtgcaga ctctgcaggg tgggtctggg cccaacacac ccaaagcacg 240
   cccaggaagg aaaggcagct tggtatcact gcccagagct aggagaggca ccgggaaaat 300
   gatctgtcca agacccgttc ttgcttctaa actccgaggg ggtcagatga agtggttttg 360
   tttcttggcc tgaagcatcg tgttccctgc aagaagcgg 399
<210> 43
   <211> 23
   <212> DNA
   <213> Sus scrofa
<400> 43
   gtctttggtt tttgctgagg gtt 23